# EUROPEAN PATENT APPLICATION

(11) **EP 1 354 877 A1**
(43) Date of publication of application: **22.10.2003**
(21) Application number: 02716308.8
(22) Date of filing: 17.01.2002
(51) Int. Cl.: C07D 213/74, C07D 239/42, C07D 401/14, C07D 213/76, C07D 213/75, C07D 401/10, C07D 401/12, C07D 405/12, A61K 31/4418, A61K 31/505, A61K 31/506, A61K 31/4439, A61K 31/498, A61K 31/444, A61K 31/443, A61P 37/08, A61P 37/06, A61P 1/04, A61P 21/04, A61P 29/00, A61P 13/12

(54) **HETERO-TRICYCLIC COMPOUNDS HAVING SUBSTITUTED AMINO GROUPS**

(30) Priority: 22.01.2001 JP 2001012888
(71) Applicant: SHIONOGI & CO., LTD., Osaka-shi, Osaka 541-0045 (JP)
(72) Inventor: TSURI, Tatsuo, c/o Shionogi & Co., Ltd., Osaka-shi, Osaka 553-0002 (JP); TAKECHI, Shozo, c/o Shionogi & Co., Ltd., Osaka-shi, Osaka 553-0002 (JP); HORIBE, Isao, Hirakata-shi, Osaka 573-0124 (JP)
(74) Representative: VOSSIUS & PARTNER
(86) International application number: JP0200260
(87) International publication number: WO02057237

(57) **Abstract**

The present invention provides a compound of the formula (I): 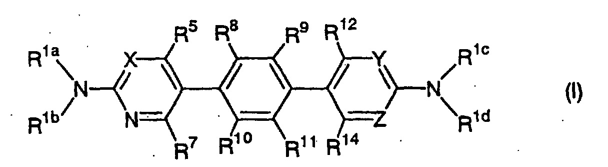 wherein X is N or CR⁴, Y is N or CR¹³, and Z is N or CR¹⁵, R^{1a}, R^{1b}, R^{1c} and R^{1d} are hydrogen, lower alkyl, lower alkenyl, lower alkynyl, lower alkylsulfonyl, cycloalkyl or the like, R⁴ to R¹⁵ are hydrogen, halogen, hydroxy, lower alkyl, lower alkoxy, acyl, carboxy, lower alkoxycarbonyl or the like,
which has superior immunosuppressive and/or anti-allergic activity and is useful for a pharmaceutical composition.

## Description

### Technical Field

The present invention relates to a novel aminoheterotricyclic compound and an IgE production suppressive composition, an anti-allergic composition and/or an immunosuppressive composition each comprising the same.

### Background Art

A serious problem of a transplantation of a tissue or an organ which is frequently performed in recent years is a rejection symptom for excluding a transplanted part after an operation. Prevention of the rejection symptom is very important for a success of the transplantation.

Various immunosuppressants such as azathioprine, corticoid, Cyclosporin A, Tacrolimus and the like are developed and come into practical use for prevention and a treatment of a rejection symptom against a transplantation of an organ or a tissue or a graft-versus-host reaction which is caused by a bone marrow transplantation. But they are not so satisfactory in view of their effects and side effects.

Allergic diseases such as atopic dermatitis, allergic rhinitis, bronchial asthma, allergic conjunctivitis and the, like tend to globally increase in recent years and become serious problems. The conventional antiinflammatory agents are suppressants of releasing chemical mediators from mast cells; receptor inhibitors of the released chemical mediators, suppressants of allergic inflammation response or the like. All of these are agents for symptomatic therapy and are not fundamental therapeutic agents for allergic diseases.

Although the compounds having a similar structure to a compound of the present invention and exhibiting an immunosuppressive or anti-allergic effect are described in WO98/04508, WO99/38829 and the like, more efficacious and safety medicaments are desired.

### Disclosure of Invention

The object of the present invention is to provide a novel aminoheterotricyclic compound, an IgE production suppressive composition, an anti-allergic composition and/or an immunosuppressive composition each containing the same.

The present invention provides
[1] A compound of the formula (I): 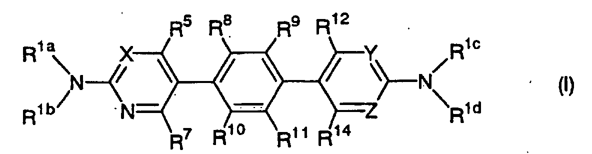 wherein X is N or CR⁴, Y is N or CR¹³, Z is N or CR¹⁵,
   R^{1a}, R^{1b}, R^{1c} and R^{1d} are each independently hydrogen;
   lower alkyl optionally substituted with at least one group selected from the group of (i)halogen, (ii)hydroxy, (iii)lower alkylthio, (iv)lower alkoxy, (v)amino, (vi)lower alkylamino, (vii)cycloalkyl, (viii)heterocyclyl, (ix)optionally substituted aryl wherein the substituents are lower alkoxycarbonyl or hydroxy, (x)lower alkoxycarbonyl, (xi)cyano, (xii)oxo and (xiii)carboxy;
   lower alkenyl;
   lower alkynyl;
   acyl optionally substituted with halogen or lower alkoxy;
   lower alkylsulfonyl optionally substituted with halogen;
   lower alkoxycarbonyl;
   cycloalkyl;
   arylsulfonyl;
   carbamoyl optionally substituted with alkyl;
   sulfamoyl optionally substituted with lower alkyl,
   R⁴, R⁵ R⁷ R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, optionally substituted lower alkenyl, optionally substituted lower alkenyloxy, optionally substituted cycloalkyoxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino; optionally substituted carbamoyl, optionally substituted sulfamoyl, guanidino, nitro; cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy,
   R⁴ and R^{1a} taken together may form optionally substituted C2 or C3 alkenylene,
   R¹³ and R^{1c} taken together may form optionally substituted C2 or C3 alkenylene,
   R⁸ and R⁹ taken together may form optionally substituted C2 or C3 alkenylenediamino or optionally substituted C2 or C3 alkylenediamino,
   R¹⁰ and R¹¹ taken together may form optionally substituted-C2 or C3 alkenylenediamino or optionally substituted C2 or C3 alkylenediamino,
   a prodrug, a pharmaceutically acceptable salt or solvate thereof (hereinafter referred to as Compound (I)),
[2] The compound as described in [1] wherein R^{1a} is lower alkylsulfonyl or 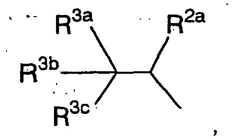 R^{1c} is lower alkylsulfonyl or 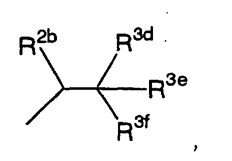 R^{1b} and R^{1d} are each independently hydrogen, lower alkyl or acyl,
   R^{2a} and R^{2b} are each independently hydrogen or lower alkyl,
   R^{3a} and R^{3d} are each independently hydrogen, lower alkyl or lower alkenyl,
   R^{3b}, R^{3e}, R^{3c} and R^{3f} are each independently hydrogen or lower alkyl,
   R^{3a} and R^{2a} or R^{3b} taken together may form optionally substituted lower alkylene,
   R^{3d} and R^{2b} or R^{3e} taken together may form optionally substituted lower alkylene, a prodrug, a pharmaceutically acceptable salt or solvate thereof,
[3] The compound as described in [1] wherein X is N or CR⁴, Y is CR¹³ and Z is CR¹⁵, a prodrug, a pharmaceutically acceptable salt or solvate thereof,
[4] The compound as described in [1] wherein X is N or CR⁴, Y is CR¹³ and Z is N, a prodrug, a pharmaceutically acceptable salt or solvate thereof,
[5] The compound as described in any one of [1] to [4] wherein at least one of R^{1b} and R^{1d} is hydrogen,
   a prodrug, a pharmaceutically acceptable salt or solvate thereof,
[6] The compound as described in any one of [1] to [4] wherein both of R^{1b} and R^{1d} are hydrogen,
   a prodrug, a pharmaceutically acceptable salt or solvate thereof,
[7] The compound as described in any one of [2] to [6] wherein R^{2a} and R^{2b} are each independently hydrogen or C1 to C3 alkyl,
   a prodrug, a pharmaceutically acceptable salt or solvate thereof,
[8] The compound as described in any one of [2] to [7] wherein both of R^{3a} and R^{3d} are hydrogen or C1 to C3 alkyl,
   a prodrug, a pharmaceutically acceptable salt or solvate thereof,
[9] The compound as described in any one of [2] to [6] wherein R^{3a} and R^{2a} or R^{3b} taken together may form unsubstituted alkylene and/or R^{3d} and R^{2b} or R^{3e} taken together may form unsubstituted alkylene,
   a prodrug, a pharmaceutically acceptable salt or solvate thereof,
[10] The compound as described in any one of [2] to [9] wherein R^{3b} and R^{3e} are each independently hydrogen or C1 to C3 alkyl,
   a prodrug, a pharmaceutically acceptable salt or solvate thereof,
[11] The compound as described in any one of [2] to [10] wherein R^{3c} and R^{3f} are each independently hydrogen or C1 to C3 alkyl,
   a prodrug, a pharmaceutically acceptable salt or solvate thereof,
[12] The compound as described in any one of [2] to [4] wherein both of R^{1b} and R^{1d} are hydrogen,
   R^{2a} p^{2b}, R^{3a}, R^{3d}, R^{3b}, R^{3e}, R^{3c} and R^{3f} are each independently hydrogen or all of R^{2a}, R^{2b}, R^{3a}, R^{3d}, R^{3b}, R^{3e}, R^{3c} and R^{3f} are C1 to C3 alkyl,
   R^{3a} and R^{2a} or R^{3b} taken together may form unsubstituted alkylene and/or R^{3a} and R^{2b} or R^{3e} taken together may form alkylene,
   a prodrug, a pharmaceutically acceptable salt or solvate thereof,
[13] The compound as described in any one of [2] to [4] wherein both of R^{1b} and R^{1d} are hydrogen, R^{2a}, R^{2b}, R^{3a}, R^{3d}, R^{3b}, R^{3e}, R^{3c} and R^{3f} are each independently hydrogen or methyl,
   a prodrug, a pharmaceutically acceptable salt or solvate thereof,
[14] The compound as described in any one of [1] to [4] wherein both of R^{1a} and R^{1c} are isopropyl and both of R^{1b} and R^{1d} are hydrogen,
   a prodrug, a pharmaceutically acceptable salt or solvate thereof,
[15] The compound as described in any one of [1] to [14] wherein X is N or CH and R⁶ and R⁷ are each independently hydrogen or lower alkyl,
   a prodrug, a pharmaceutically acceptable salt or solvate thereof,
[16] The compound as described in any one of [1] to [15] wherein R¹² is hydrogen, Y is CH, Z is N or CR¹⁵, R¹⁴ and R¹⁵ are each independently hydrogen, halogen, lower alkyl or lower alkoxy,
   a prodrug, a pharmaceutically acceptable salt or solvate thereof,
[17] The compound as described in any one of [1] to [16] wherein 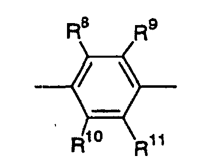 is 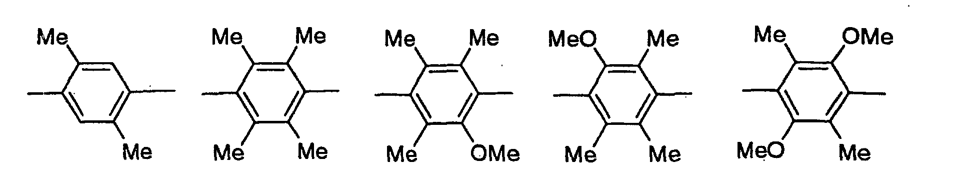 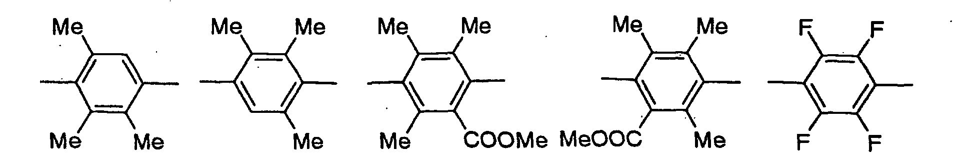 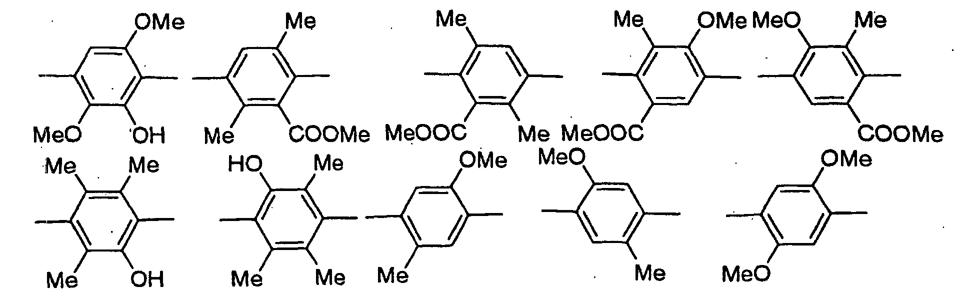 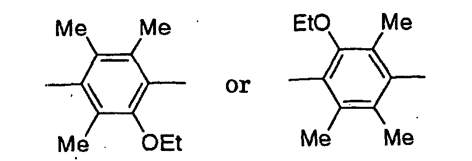 a prodrug, a pharmaceutically acceptable salt or solvate thereof,
[18] The compound as described in any one of [1] to [16] wherein 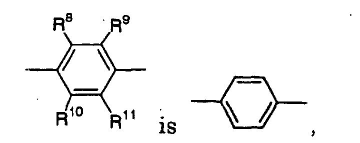 a prodrug, a pharmaceutically acceptable salt or solvate thereof,
[19] The compound as described in any one of [1] to [16] wherein 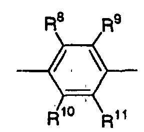 is 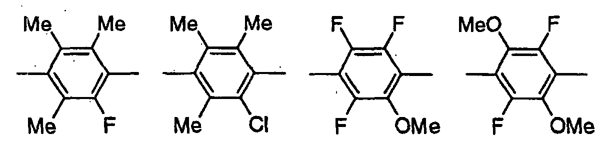 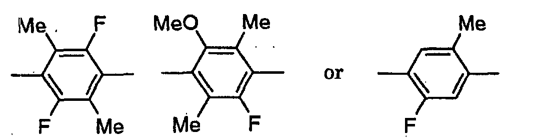 a prodrug, a pharmaceutically acceptable salt or solvate thereof,
[20] The compound as described in [1] wherein X and Y are CH, Z is N, R^{1a}, R^{1b}, R^{1c} and R^{1d} are each independently hydrogen; lower alkyl optionally substituted with cycloalkyl; lower alkenyl; acyl; lower alkylsulfonyl; or cycloalkyl,
   R⁵, R⁷, R¹² and R¹⁴ are each independently hydrogen or lower alkyl,
   R⁸, R⁹, R¹⁰ and R¹¹ are each independently hydrogen, halogen, lower alkyl or lower alkoxy,
   provided tat at least two of R⁸, R⁹, R¹⁰ and R¹¹ are each independently halogen, lower alkyl or lower alkoxy,
   a prodrug, a pharmaceutically acceptable salt or solvate thereof,
[21] The compound as described in [1] wherein X is N, Y and Z is CH, R^{1a}, R^{1b}, R^{1c} and R^{1d} are each independently hydrogen, lower alkyl or cycloalkyl,
   R⁵, R⁷, R¹² and R¹⁴ are hydrogen,
   R⁸, R⁹, R¹⁰ and R¹¹ are each independently lower alkyl or lower alkoxy,
   a prodrug, a pharmaceutically acceptable salt or solvate thereof,
[22] The compound as described in [1] wherein X is CH, Y is CR¹³, Z is CR¹⁵,
   R^{1a}, R^{1b}, R^{1c} and R^{1d} are each independently
   hydrogen;
   lower alkyl optionally substituted with at least one group selected from the group of cycloalkyl and heterocyclyl;
   lower alkenyl;
   acyl;
   lower alkylsulfonyl;
   or cycloalkyl,
   R⁵, R⁷, R¹² and R¹⁴ are hydrogen, R¹³ and R¹⁵ are each independently hydrogen or halogen,
   R⁸, R⁹, R¹⁰ and R¹¹ are each independently hydrogen, halogen, lower alkyl, lower alkoxy or lower alkoxycarbonyl,
   provided that at least three of R⁸, R⁹, R¹⁰ and R¹¹ are each independently halogen, lower alkyl, lower alkoxy or lower alkoxycarbonyl,
   R¹³ and R^{1c} taken together may form optionally substituted C2 or C3 alkenylene, a prodrug, a pharmaceutically acceptable salt or solvate thereof,
[23] A pharmaceutical composition comprising a compound, a prodrug, a pharmaceutically acceptable salt or solvate thereof of any one of [1] to [22],
[24] An IgE production suppressive composition comprising a compound, a prodrug, a pharmaceutically acceptable salt or solvate thereof of any one of [1] to [22],
[25] An anti-allergic composition comprising a compound, a prodrug, a pharmaceutically acceptable salt or solvate thereof of any one of [1] to [22],
[26] An immunosuppressive composition comprising a compound, a prodrug, a pharmaceutically acceptable salt or solvate thereof of any one of [1] to [22].
   As another embodiment, the present invention provides a method for suppressing an immune reaction, or a method for treating and/or preventing an anti-allergic disease each comprising administering a compound (I). As other embodiments, the present invention provides use of compound (I) for preparing a medicine for suppressing an immune reaction, and for treating and/or preventing an allergic disease.
[27] A method for suppressing IgE production comprising administering a compound, a prodrug, a pharmaceutically acceptable salt or solvate thereof of any one of [1] to [22].
[28] A method for treating and/or preventing an allergic disease comprising administering a compound, a prodrug, a pharmaceutically acceptable salt or solvate thereof of any one of [1] to [22].
[29] A method for suppressing an immune reaction comprising administering a compound, a prodrug, a pharmaceutically acceptable salt or solvate thereof of any one of [1]to [22].
[30] Use of a compound, a prodrug, a pharmaceutically acceptable salt or solvate thereof of any one of [1] to [22] for preparing a medicine for suppressing IgE production.
[31] Use of a compound, a prodrug, a pharmaceutically acceptable salt or solvate thereof of any one of [1] to [22] for preparing an anti-allergic agent.
[32] Use of a compound, a prodrug, a pharmaceutically acceptable salt or solvate thereof of any one of [1] to [22] for preparing an immunosuppressive agent.

In the present specification, the term "halogen" includes fluorine, chlorine, bromine and iodine. Fluorine or chlorine is preferable.

The term "lower alkyl" includes straight or branched chain alkyl having 1 to 10 carbon atoms. For example, included are methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl and the like.

The term "lower alkyl" as R^{1b} and R^{1d} includes alkyl having 2 to 6 carbon atoms, preferably 2 to 3 carbon atoms. The term "lower alkyl" as the other symbols includes alkyl having 1 to 6 carbon atoms, preferably 1 to 3 carbon atoms and most preferably methyl.

As substituents of "optionally substituted lower alkyl", exemplified are halogen; hydroxy; lower alkoxy optionally substituted with lower alkoxy; acyl; acyloxy; carboxy; lower alkoxycarbonyl; mercapt; lower alkylthio; amino optionally substituted with hydroxy, lower alkyl or optionally substituted acyl; imino optionally substituted with hydroxy, lower alkoxy, carboxy(lower)alkoxy, aryl(lower)alkoxy or heterocycle; hydrazono optionally substituted with carbamoyl or lower alkoxycarbonyl; carbamoyl optionally substituted with lower alkyl or amino; thiocarbamoyl optionally substituted with lower alkyl; cycloalkyl optionally substituted with lower alkyl or lower alkoxy; cycloalkenyl optionally substituted with lower alkyl; cyano; phenyl optionally substituted with at least one substituent selected from the group of hydroxy, lower alkyl, carboxy, lower alkoxycarbonyl and lower alkoxy; 5- or 6-membered heterocycle which may be substituted with lower alkyl and may fuse with benzene ring; and the like. The lower alkyl may be substituted with one or more of these substituents at any possible positions. Unsubstituted lower alkyl is preferable.

The lower alkyl part of "lower alkoxy" is the same as the above "lower alkyl".

As substituents for "optionally substituted lower alkoxy", exemplified are halogen; hydroxy; lower alkoxy optionally substituted with acyloxy; acyl; acyloxy optionally substituted with hydroxy or carboxy; carboxy; lower alkoxycarbonyl; lower alkylthio; amino optionally substituted with lower alkyl; phenyl optionally substituted with lower alkyl or lower alkoxy; heterocycle; heterocyclylcarbonyloxy and the like. Unsubstituted lower alkoxy is preferable.

The lower alkyl parts of "lower alkoxycarbonyl", "lower alkylsulfonyl", "lower alkylsulfonyloxy", "lower alkylsulfinyl", "lower alkylthio", "lower alkylamino",, "carboxy(lower)alkoxy", "aryl(lower)alkoxy", "acyloxy(lower)alkoxy", "cycloalkyl(lower)alkyl", "heterocyclyl(lower)alkyl" and "lower alkylenedioxy" are the same as the above "lower alkyl". Substituents for "optionally substituted lower alkoxycarbonyl", "optionally substituted lower alkylsulfonyl", "optionally substituted lower alkylsulfonyloxy", "optionally substituted lower alkylsulfinyl" and "optionally substituted lower alkylthio" are the same as those for the above "optionally substituted lower alkoxy".

The term "lower alkenyl" includes straight or branched chain alkenyl of 2 to 10 carbon atoms, preferably 2 to 8 carbon atoms, more preferably 3 to 6 carbon atoms having at least one double bond at any possible positions. For example, included are vinyl, propenyl such as 2-propenyl and the like, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like. Substituents for "optionally substituted lower alkenyl" are the same as those for the above "optionally substituted lower alkoxy". Unsubstituted alkenyl is preferable.

The lower alkenyl parts of "lower alkenyloxy", "lower alkenyloxythio" and "lower alkenyloxycarbonyl" are the same as the above "lower alkenyl". Substituents for "optionally substituted lower alkenyloxy", "optionally substituted lower alkenyloxycarbonyl" and "optionally substituted lower alkenylthio" are the same as those for the above "optionally substituted lower alkoxy".

The term "lower alkynyl" includes straight or branched chain alkynyl having 2 to 10 carbon atoms, preferably 2 to 8 carbon atoms, and more preferably 3 to 6 carbon atoms and is exemplified by ethynyl, propynyl such as 2-propynyl, butynyl such as 2-butynyl, pentynyl, hexynyl, heptynyl, octynyl, nonynyl, decynyl and the like. These have at least one triple bond and may have some double bonds at any possible positions.

The lower alkynyl parts of "lower alkynyloxy" and "lower alkynylthio" are the same as the above "lower alkynyl".

The term "acyl" includes straight or branched chain aliphatic acyl having 1 to 10 carbon atoms, preferably 1 to 6 carbon atoms, more preferably 1 to 4 carbon atoms, cyclic aliphatic acyl having 4 to 9 carbon atoms, preferably 4 to 7 carbon atoms and aroyl. For example, formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl, benzoyl and the like are included and acyl is preferable.

Substituents for "optionally substituted acyl" are the same as those for the above "optionally substituted lower alkoxy" and aroyl may further be substituted with lower alkyl. Unsubstituted acyl is preferable.

The acyl parts of "acyloxy", "acyloxy(lower)alkoxy", "hydroxy-substituted acyloxy" and "carboxy-substituted acyloxy" are the same as the above "acyl". Substituents for "optionally substituted acyloxy" are the same as those for the above "optionally substituted acyl".

The term "cycloalkyl" includes carbocycle having 3 to 6 carbon atoms and cyclopropyl, cyclobutyl, cyclopentyl, cyclohexyl and the like.

The cycloalkyl parts for "cycloalkyloxy" and "cycloalkyl(lower)alkyl" are the same as the above "cycloalkyl".

Examples of substituents for "optionally substituted cycloalkoxy" are lower alkyl, halogen, hydroxy, carboxy, lower alkoxycarbonyl, lower alkoxy, lower alkylenedioxy, imino optionally substituted with lower alkoxy, aryl, heterocyclyl and the like. Cycloalkoxy may be substituted with one or more of these substituents.

The term "cycloalkenyl" includes the group having at least one double bond at any possible positions in the above cycloalkyl and is exemplified by cyclopropenyl, cyclobutenyl, cyclopentenyl, cyclohexenyl, cyclohexadienyl and the like.

As substituents for "optionally substituted amino", exemplified are optionally substituted lower alkyl {wherein the substituents are lower alkoxy, cycloalkyl, optionally substituted amino (wherein the substituents are aroyl optionally substituted with acyloxy(lower)alkoxy), optionally substituted aryl (wherein the substituents are lower alkyl, lower alkoxy, carboxy or lower alkoxycarbonyl) or heterocycle}; lower alkenyl; lower alkynyl; cycloalkyl; aryl optionally substituted with lower alkyl, carboxy, 'acyl, lower alkoxycarbonyl; sulfamoyl optionally substituted with lower alkyl; optionally substituted lower alkoxycarbonyl (the substituents are halogen, acyloxy, acyloxy substituted with hydroxy, acyloxy substituted with carboxy or heterocyclylcarbonyloxy or the like); lower alkylsulfonyl and the like. Preferable substituents are lower alkyl, acyl or lower alkylsulfonyl.

The term "optionally substituted carbamoyl" includes carbamoyl optionally substituted with lower alkyl, lower alkenyl, lower alkynyl or the like.

The term "optionally substituted sulfamoyl" includes sulfamoyl optionally substituted with lower alkyl, lower alkenyl, lower alkynyl or the like.

The term "aryl" includes phenyl, naphthyl, anthryl, phenanthryl, indenyl and the like and phenyl is preferable.

The aryl parts for "arylsulfonyl", "arylsulfonyloxy" and "aryl(lower)alkoxy" are the same as the above "aryl", and phenyl is preferable.

Examples of the substituents for "optionally substituted arylsulfony" and "optionally substituted arylsulfonyloxy" are halogen; hydroxy; lower alkyl optionally substituted with halogen or carboxy; lower alkoxy optionally substituted with halogen, aryl, heterocyclyl or lower alkoxy; lower alkenyl; lower alkynyl; cycloalkyl; lower alkenyloxy; lower alkynyloxy; cycloalkoxy; acyl; acyloxy; carboxy; lower alkoxycarbonyl; lower alkenyloxycarbonyl; lower alkylthio; lower alkynylthio; amino optionally substituted with lower alkyl, cycloalkyl(lower)alkyl, heterocyclyl(lower)alkyl, lower alkenyl, cycloalkyl, acyl optionally substituted with halogen, lower alkoxycarbonyl, or lower alkylsulfonyl; guanidino; nitro; lower alkylsulfonyl; dihydroxyborane; lower alkylsulfonyloxy optionally substituted with halogen; arylsulfonyl; arylsulfonyloxy; aryl; heterocycle and the like. The aromatic carbocycle and aryl may be substituted with these substituents at one or more of any possible positions. Preferable examples are halogen; hydroxy; lower alkyl optionally substituted with halogen; lower alkoxy optionally substituted with aryl or lower alkoxy; lower alkenyloxy; acyloxy; lower alkylthio; amino optionally substituted with lower alkyl, lower alkenyl, acyl optionally substituted with halogen, or lower alkylsulfonyl; nitro; lower alkylsulfonyl; lower alkylsulfonyloxy optionally substituted with halogen; or arylsulfonyloxy.

The term "heterocyclyl" represents a 5- or 6-membered heterocyclic group which contains one or more of hetero atoms arbitrarily selected from the group of O, S and N. Examples are aromatic heterocyclyl such as pyrrole ring, imidazole ring, pyrazole ring, pyridine ring such as 4-pyridyl, pyridazine ring, pyrimidine ring, pyrazine ring, triazole ring, triazine ring, isoxazole ring, oxazole ring, isothiazole ring, thiazole ring, furan ring such as 2-furyl and 3-furyl, thiophene ring such as 3-thienyl or the like and aliphatic heterocycle such as tetrahydropyrane ring, dihydropyridine ring such as 1,2-dihydropyridyl, dihydropyridazine ring such as 2,3-dihydropyridazinyl, dihydropyrazine ring such as 1,2-dihydrlpyrazinyl, dioxane ring, oxathiorane ring, thiane ring, pyrolidine ring, pyrroline ring, imidazolidine ring, imidazoline ring, pyrazolidine ring, pyrazoline ring, piperidine ring, piperazine ring, morpholine ring or the like.

The heterocyclyl parts for "heterocyclylcarbonyloxy" and "heterocyclyl(lower)alkyl" are the same as the above "heterocyclyl".

The phrase "R^{3a} and R^{2a} or R^{3b} taken together may form optionally substituted alkylene" means that R^{3a}, R^{2a} and their adjacent carbon atoms, or R^{3a}, R^{3b} and their adjacent carbon atoms may be joined together to form optionally substituted cyclopropane, optionally substituted cyclobutane, optionally substituted cyclopentane, optionally substituted cyclohexane, optionally substituted cycloheptane and the like. The Examples of substituents are halogen, hydroxy, lower alkyl, lower alkoxy, acyl or the like.

The phrase "R^{3d} and R^{2b} or R^{3e} taken together may form optionally substituted alkylene" can be interpreted as the same manner.

The phrases "R⁴ and R^{1a} taken together may form optionally substituted C2 or C3 alkenylene" and "R¹³ and R^{1c} taken together may form optionally substituted C2 or C3 alkenylne" mean as follows, respectively: 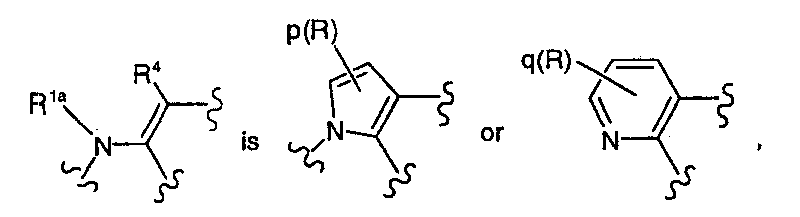 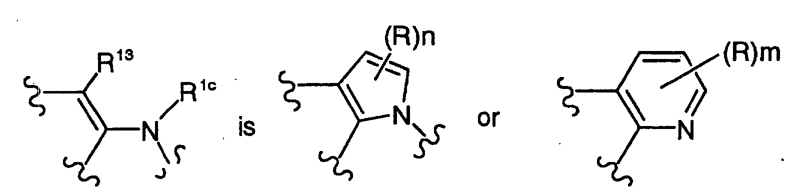 wherein R is halogen, hydroxy, lower alkyl, lower alkoxy, carboxy or lower alkoxycarbonyl, p and n are each independently an integer of 0 to 2, q and m are each independently an integer of 0 to 3.

The phrases "R⁸ and R⁹ taken together may form optionally substituted C2 or C3 alkenylendiamino or optionally substituted C2 or C3 alkylnediamino" and "R¹⁰ and R¹¹ taken together may form optionally substituted C2 or C3 alkenylendiamino or optionally substituted C2 or C3 alkylnediamino" means as follows, respectively: 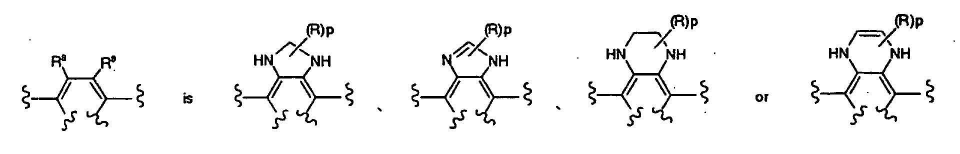 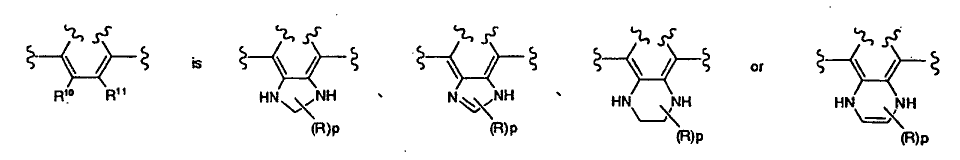 wherein R is oxo, lower alkyl, halogen, hydroxy, lower alkoxy, carboxy or lower alkoxycarbonyl, p and n are each independently an integer of 0 to 2, q and m are each independently an integer of 0 to 3, and R can be substituted on an N atom.

The term "compound (I)" includes formable and pharmaceutically acceptable salts of each compound. As "the pharmaceutically acceptable salt", exemplified are salts with mineral acids such as hydrochloric acid, sulfuric acid, nitric acid, phosphoric acid, hydrofluoric acid, hydrobromic acid and the like; salts with organic acids such as formic acid, acetic acid, tartaric acid, lactic acid, citric acid, fumaric acid, maleic acid, succinic acid and the like; salts with organic base such as ammonium, trimethylammonium, triethylammonium and the like; salts with alkaline metals such as sodium, potassium and the like and salts with alkaline earth metals such as calcium, magnesium and the like.

The compound of the present invention includes the solvate thereof and hydrate is preferable. An example of the solvate is a solvate with an organic solvent and/or water. The compound of the present invention may cooperate with arbitrary numbers of water molecules to give hydrate thereof.

The compound of the present invention includes all of stereoisomers, for example, atropisomers etc. thereof.

### Best Mode for Carrying Out the Invention

Compound (I) can be produced by the similar methods to WO98/04508, WO99/38829 and the like. A process for producing the compound (I) is as follows.

### Process for Producing Compound (I)

Compound (I) can be produced by reacting a compound of the formula (IIa) (hereinafter referred to as "a compound (IIa)") with a bicyclic compound of the formula (IIIa) (hereinafter referred to as "a compound (IIIa)") or by reacting a compound of the formula (IIb) (hereinafter referred to as "a compound (IIb)") with a bicyclic compound of the formula (IIIb) (hereinafter referred to as "a compound (IIIb)"). 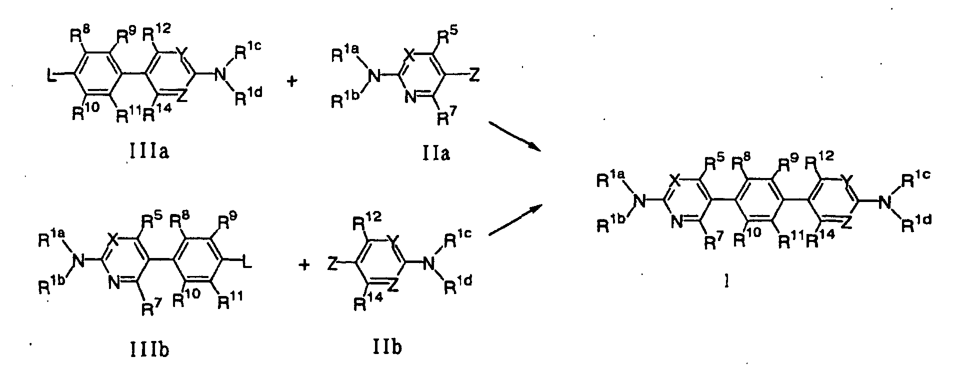 wherein either of L and Z is dihydroxyboryl, di(lower)alkyl boryl or di(lower) alkoxyboryl and the other is halogen or -OSO₂(C_{q}F_{2q+1}) (q is an integer of 0 to 4) and other symbols are the same as defined above.

The compound (I) can be produced by reacting the compound (IIa) with the compound (IIIa) or by reacting the compound (IIb) with the compound (IIIb) in a mixture of an appropriate solvent such as benzene, toluene, N, N-dimethylformamide, dimethoxyethane, tetrahydrofuran, dioxane, ethanol, methanol or the like and water or in an anhydrous solution in the presence of a palladium catalyst such as Pd(PPh₃)₄ PdCl₂(PPh₃)₂ PdCl₂(OAc)₂, PdCl₂(CH₃CN)₂ or the like, preferably Pd(PPh₃)₄, under a basic condition (for example, by K₃PO₄, NaHCO₃, NaOEt, Na₂CO₃, Ba(OH)₂, Cs₂CO₃, CsF, NaOH, Ag₂CO₃ or the like) at room temperature or heating for several tens minutes to several tens hours.

One of substituents L and Z of the compounds to be reacted may be any of the borane groups which are applicable in the Suzuki Reaction (Chemical Communication 1979, 866, Journal of Synthetic Organic Chemistry, Japan,1993, Vol.51, No.11, 91-100) and dihydroxyborane is preferable. The other may be any of the leaving groups which are applicable in the Suzuki Reaction, for example, halogen, -OSO₂(C_{q}F_{2q+1}) wherein q is an integer of 0 to 4, or the like. Specifically, halogen, trifluoromethanesulfonyloxy (hereinafter referred to as OTf) or the like is preferable and bromine, iodine or OTf is more preferable.

R^{1a}, R^{1b}, R^{1c} R^{1d}, R⁴ to R¹⁵ of the compounds (IIa), (IIIa), (IIb) and (IIIb) may be any of the groups which do not affect the Suzuki Reaction, for example, any groups other than halogen and -OSO₂(CqF_{2q+1}) wherein q is an integer of 0 to 4.

Even if either of R⁴ to R¹⁵ is halogen, these reactions can be carried out without difficulty when the reactivity of the substituent L with the substituent Z is higher than that of halogen with either of substituents L and Z.

Even if either of R⁴ to R¹⁵ is hydroxy, the above reactions can be preferably carried out. Preferably the above reactions may be carried out after the protection of hydroxy group with a usual hydroxy-protecting group such as methoxymethyl, benzyl, tert-butyldimethylsilyl, methanesulfonyl, p-toluenesulfonyl or the like, followed by deprotection by the usual methods.

As processes for producing the compound (I), the above mentioned Suzuki Reaction is most preferable in view of the efficiency and easiness but silicon, zinc, tin or the like can be used in place of the boryl group in the above scheme.

For example, in the case that one of L and Z is -SiR₃₋ᵣCHal)ᵣ wherein R are independently lower alkyl, Hal is halogen and r is an integer of 1 to 3 and the other is halogen or -OSO₂(C_{q}F_{2q+1}) wherein q is an integer of 0 to 4, the coupling reaction may be carried out using a usual palladium catalyst (Synlett (1991) 845-853, J. Org. Chem. 1996, 61, 7232-7233). Examples of preferable palladium catalysts are (i-Pr₃P)₂PdCl₂, [(dcpe)PdCl₂] (dcpe=Cy₂PCH₂CH₂PCy₂), (η³-C₃H₅PdCl)₂ and the like.

Even in the case that one of L and Z is -SnR'₃ wherein R' are each independently lower alkyl and the other is halogen, acetyloxy or -OSO₂(C_{q}F_{2q+1}) wherein q is an integer of 0 to 4, an objective compound can be obtained using a usual palladium catalyst (preferably Pd(PPh₃)₄ or the like) (Angew. Chem. Int. Ed. Engl. 25 (1986) 508-524).

In the case that one of L and Z is -Zn(Hal) wherein Hal is halogen and the other is halogen, an objective compound can be obtained (Acc. Chem. Res. 1982, 15, 340-348). Any usual palladium catalyst is applicable and Pd(PPh₃)_{4,} PdCl₂(dppf), PdCl₂(PPh₃)₂, PdCl₂(P(o-Tolyl)₃)₂, Pd(OAc)₂ and the like are exemplified as preferable examples.

All of these reactions may be carried out in a suitable solvent such as N,N-dimethylformamide, tetrahydrofuran or the like at room temperature or heating for several tens minutes to several tens hours.

As compound (IIIa) and (IIIb) in the above reactions, may be used known compounds or compounds which are derived from a compound of the following formula (Va) (hereinafter referred to as "a compound (Va)") or the following formula (Vb) (hereinafter referred to as "a compound (Vb)") which can be produced by the known method or the following method. 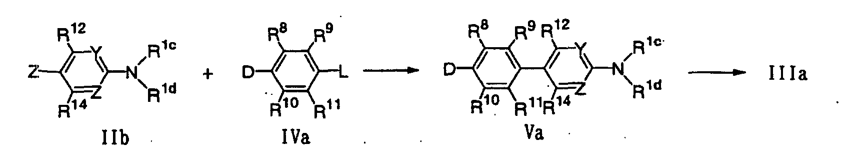 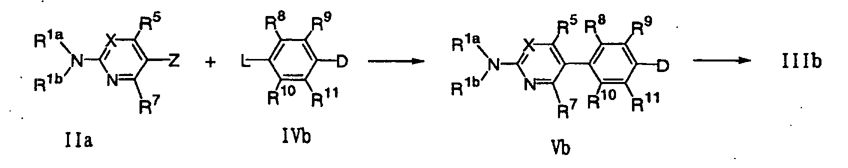 wherein D is any of the groups which do not affect the Suzuki Reaction of L with Z, and may be the same group as L when a compound of the formula (IVb) is a bisymmetric compound. The other symbols are the same as above.

The compound (IIb) is reacted with the compound (TVa) or the compound (IIa) is reacted with (IVb) to give the compound (Va) or (Vb). When the compound (IVa) or (IVb) is not a bisymmetric compound, D is preferably a group which does not affect the Suzuki Reaction of L with Z and can be easily converted to L. For example, hydroxy, hydrogen, formyl, nitro or the like is preferable. In the reaction of L with Z, silicon, zinc, tin or the like can be used in place of the borane group as mentioned above.

D is converted into a group L which is applicable to the Suzuki Reaction.

A compound wherein D is hydroxy may be reacted with a trifluoromethanesulfonating agent such as trifluoromethanesulfonic anhydride, trifluoromethanesulfonyl chloride, N-phenyltrifluoromethanesulfone imide or the like in a suitable solvent such as dichloromethane, chloroform, tetrahydrofuran or benzene in the presence of a base such as sodium hydride, pyridine, triethylamine, potassium carbonate or the like at - 20 °C or heating for several minutes to several tens hours to give an objective compound wherein L is OTf.

For example, a compound wherein D is hydrogen may be reacted with a halogenating agent such as bromine, chlorine, iodine, N-bromosuccinimide or the like in a suitable solvent such as acetic acid, dichloromethane, chloroform, carbon tetrachloride, benzene, water or the like at -20 °C or heating for several minutes to several tens hours to give an objective compound wherein L is halogen.

A compound wherein D is formyl may be oxidated by the Baeyer-Villiger reaction to give a compound wherein D is formyloxy, followed by hydrolysis to give a compound wherein D is hydroxy. The compound wherein L is OTf can be obtained by the similar process as mentioned above.

A compound wherein D is nitro may be reduced to give a compound wherein D is amino, followed by the Sandmeyer Reaction to give a compound L is halogen.

As mentioned above, Compound (I) of the present invention can be synthesized from (IIa), (IIb), (IIIa) or (IIIb) wherein the objective substituents R^{1a} to R^{1d} are previously introduced. Alternatively, Compound (I) can be synthesized by constructing the terphenyl structure, followed by introducing the target substituents R^{1a} to R^{1d} therein.

For example, if a target compound is a compound wherein any one of R^{1a} to R^{1d} is alkylsulfonyl optionally substituted with halogen or acyl optionally substituted with halogen, a tricyclic compound having an amino group may be reacted with a compound having a substituent corresponding to the target substituent (for example, methane sulfonic anhydride, trifluoroacetic anhydride) in a suitable solvent such as dichloromethane, tetrahydrofuran and the like in the presence of a base such as pyridine, triethylamine, dimethylaminopyridine or the like at ice-cooling or heating.

If a target compound is a compound wherein any one of R^{1a} to R^{1d} is lower alkyl, lower alkenyl, lower alkynyl or cycloalkyl, a tricyclic compound having an amino group may be reacted with a halogen compound, sulfonate compound or alcohol compound having a substituent corresponding to a target substituent (for example, lower alkyl halide, lower alkylsulfonate, lower alkylalcohol or the like in the presence of a base such as an alkaline metal hydride, an alkaline metal hydrogencarbonate, an alkaline metal carbonate, an organic base and the like at ice-cooling or heating.

A tricyclic compound having an amino group can be reacted with an aldehyde compound or a ketone compound having an target substituent (for example, a lower alkyl. aldehyde, a lower alkanone, a cycloalkanone and the like) in a suitable solvent such as dichloromethane, tetrahydrofuran or the like in the presence of an acidic catalyst such as acetic acid, pyridinium paratoluene sulfonate or the like at ice-cooling or heating to get Schiffs base. After isolation of the base, it may be reacted with a reductant to obtain an target compound. These reactions can be carried out in an one-pot system.

In the case that a compound has a substituent interfering of the above reaction, the substituent may be protected with a suitable protecting group in advance and the protecting group may be removed in a suitable step by the usual method. For example, if hydroxy interferes the reaction, it may be protected with methoxymethyl, methanesulfonyl, benzyl, trifluoromethanesulfonyl, tert-butyldimethylsilyl or the like, followed by deprotection in a suitable step.

For example, for a protection of hydroxy with methanesulfonyl, a compound which has hydroxy may be reacted with methanesulfonyl chloride in a solvent such as dichloromethane, chloroform, carbon tetrachloride or the like in the presence of a base such as triethylamine, pyridine or the like at ice-cooling or room temperature for several hours. The protected compound may be deprotected with 1-4 N sodium hydroxide, potassium hydroxide, aqueous solution thereof, sodium methoxide, ethyl magnesium bromide or the like in a solvent such as dimethylsulfoxide, dimethylformamide, tetrahydrofuran, dioxane, dimethoxyethane or the like at room temperature or heating for several tens minutes to several hours.

When methoxymethyl is used as a hydroxy-protecting group, a compound which has hydroxy may be reacted with chloromethylmethylether in a solvent such as tetrahydrofuran, dioxane, dimethoxyethane or the like in the presence of sodium hydride, diisopropylethylamine or the like to give a compound which has a protected hydroxy group. The compound may be subjected to a usual deprotection reaction with hydrochloric acid, sulfuric acid or the like in a solvent such as methanol, tetrahydrofuran, acetic acid or the like for a deprotection.

When tert-butyldimethylsilyl is used as a protecting group, a compound which has hydroxy may be reacted with tert-butyldimethylsilyl chloride, tert-butyldimethylsilyl triflate or the like in a solvent such as dimethylformamide, acetonitrile, tetrahydrofuran, dimethylformamide, dichloromethane or the like in the presence of imidazole, triethylamine, 2, 6-lutidine or the like. For a deprotection reaction the protected compound may be reacted with tetrabutylammonium fluoride or the like in a solvent such as tetrahydrofuran or the like.

Thus obtained compound of the present invention can be converted into a prodrug thereof. The term "prodrug" includes compounds which can easily be converted to the compound having the activity of the present invention in a living body. Any usual method for conversion into a prodrug may be used.

The methods for selecting and producing suitable prodrugs are described in Design of Prodrugs, Elsevier, Amsterdam 1985. According to this, a group generally used for prodrug may be introduced into carboxy, hydroxy, amino or the like at any position of the compound of the present invention.

For example, if a compound has a hydroxy group on any ring, -COCH₂CH₂COOH, -COCH=CHCOOH, -COCH₂SO₃H, -PO₃H₂, -COCH₂NMe₂, -CO-Py wherein Py means pyridyl can be introduced.

For example, if a compound has an amino group on any ring, -COOCR^{a}R^{b}OCOCH₂R^{c}
wherein R^{a} and R^{b} are each independently hydrogen or lower alkyl, R^{c} is H, -OH, -CONHR^{d}, -OCONHR^{d}, -(NHCOCR^{e}R^{f})ᵤNHCOCH₃, -(NHCOCR^{e}R^{f})ᵤNHCOC₂H₅, -CSNH₂, -(OCH₂CH₂)ₜOH, -OCH₃, -(OCH₂CH₂)ₜOCH₃, -COCH₃, -COC₂H₅, -OCOCH₃, -OCOC₂H₅, -NHOH, -NHCONH₂, -NHCSNH₂, -NHSO₂CH₃, -N(SO₂CH₃)₂, -SO₂NH₂, -SOMe, -SO₂CH₃, -OCH₂CONH₂, -OCH₂CON(CH₃)₂, -SO₂N(CH₃)₂, -PO(OCH₃)₂, -NHCSNHC₂H₅Et, -CH=NNHCONH₂, CH=NNHCSNH₂, -CH=NNHSO₂CH₃, triazolyl, tetrazolyl and the like, Rd, Re and R^{f} is hydrogen or lower alkyl, t is 1 or 2, u is an integer of 0 to 2,
-COOCH(Me)OCOCMe₃, -COOCH₂OCO(CH₂)₁₄Me, -COOCH₂OCO-Pyr, -CH₂NHCO-C₆H₄-o-OCH₂OAc and the like wherein Pyr means pyridyl and Ac means acetyl can be introduced.

When a prodrug is produced by introducing a substituted acyloxycarbonyl (-COOCR^{a}R^{b}OCOCH₂R^{c}) into an amino group at any position of the compound of the present invention, the amino group may be α-haloalkoxycarbonated, followed by being reacted with a suitable carboxylic acid under a suitable condition.

The above-mentioned acyloxyalkylcarbamate can be produced according to known methods described in WO96/18605 and the like.

A compound of the present invention having an amino group is reacted with chloroformate α-haloalkyl ester in a non-active solvent such as diethyl ether, tetrahydrofuran, 1,4-dioxane, ethyl acetate, toluene and the like in the presence of a base such as pyridine, triethylamine, N-methylmorpholine and the like at 0 °C to room temperature to obtain a haloalkoxycarbamate. Thus-obtained compound may be reacted with a salt (for example, an alkaline metal salt, an alkaline earth metal salt, a silver salt, a mercury salt) of a substituted carboxylic acid compound at room temperature or heating for several hours to several days to obtain a prodrug compound.

Regarding compounds having a substituent interfering with processes for obtaining a prodrug compound, the group may be protected with a suitable protecting group in advance, and the protected group may be deprotected by the conventional method at a suitable stage.

Compounds of the present invention has not only IgE production suppressive effect but also Th2 differentiation inhibitory effect.

The term "Th2 differentiation inhibitory effect" means an inhibitory effect on the differentiation of Th0 cells to Th2 cells. The compounds of the present invention can be used for a pharmaceutical composition for treating and/or preventing diseases induced by Th2 cells or cytokines produced from Th2 cells.

A Th2 differentiation inhibitory composition of the present invention shows suppressive effects on activating of B cells and antibody production by decreasing Th2 cells and cytokines derived from Th2 cells. Additionally, the composition has the following features.

The contact of B cells with Th2 cells and the stimulation of B cells by cytokines derived from Th2 cells are considered to be necessary for the activation of B cells in the resting stage. Thus, a Th2 differentiation inhibitory composition of the present invention can suppress the direct activation of B cells by Th2 cells themselves. Therefore, the composition of the present invention can treat and prevent allergic or autoimmune diseases more effectively than the conventional anti-allergic agents.

Some kinds of allergic diseases such as asthma, respiratory inflammation and the like are known to be caused by cytokines produced by and released from Th2 cells. Thus, a Th2 differentiation inhibitory composition of the present invention is expected to show a more effective treating effect than a pharmaceutical composition showing only IgE production suppressive effect.

For example, the compound of the present invention is expected to be useful for preventing or treating the following diseases.

Rejection symptom against a transplantation of an organ or a tissue, transplantation immunology (acute or chronic GVHD), autoimmune diseases (especially organ non-specific autoimmune diseases), mixed connective tissue disease(MCTD), injury caused by ischemia-reperfusion, ulcerative colitis, systemic lupus erythematodes, myasthenia gravis, systemic progressive scleroderma, rheumatoid arthritis, interstitial cystitis, Hashimoto's diseases, Basedow's diseases, autoimmune hemolytic anemia, idiopathic thrombocytopenic purpura, Goodpasture's syndrome, atrophic gastritis, pernicious anemia, Addison diseases, pemphigus, pemphigoid, lenticular uveitis, sympathetic ophthalmia, primary biliary cirrhosis, active chronic hepatitis, Sjogren's syndrome, multiple myositis, dermatomyositis, polyarteritis nodosa, rheumatic fever, glomerular nephritis (lupus nephritis, IgA nephtopathy, membranous nephropathy and the like), allergic encephalitis, atopic allergic diseases (for example, bronchial asthma, allergic rhinitis, allergic dermatitis, allergic conjunctivitis, pollinosis, urticaria, food allergy and the like), psoriasis, Omenn's syndrome, vernal conjunctivitis and hypereosinophilic syndrome and the like.

Especially, the composition of the present invention is expected to be useful for organ non-specific autoimmune diseases such as chronic GVHD, ulcerative colitis, systemic lupus erythematodes, myasthenia gravis, systemic progressive scleroderma, rheumatoid arthritis, glomerular nephritis, interstitial cystitis and the like.

Further, because an immunosuppressant is expected to be a treating agent for chronic renal insufficiency induced by non-immune mechanism (Kidney International vol.54 (1998), pp. 1510-1519 Kidney International vol.55 (1999), pp. 945-955), the compound of the present invention can be a drug for non-immune chronic renal insufficiency.

The compound of the present invention has merits such as low toxity, high bioavailability etc. Many of them are negative in a heterochromosome test and little effective on other enzymes. Thus, they can be a superior pharmaceutical' composition.

A compound of the present invention can be administered orally or parenterally as a suppressant on the IgE production, Th2 differentiation inhibitor, anti-allergic agent and/or immunosuppressant. In the case of oral administration, it may be in any usual form such as tablets, granules, powders, capsules, pills, solutions, syrups, buccal tablets, sublingual tablets and the like. When the compound is parenterally administered, any usual form is preferable, for example, injections (e.g., intravenous, intramuscular), suppositories, endermic agents, vapors and the like. Oral administration is particularly preferable.

A pharmaceutical composition may be manufactured by mixing an effective amount of a compound of the present invention with various pharmaceutical ingredients suitable for the administration form, such as excipients, binders, moistening agents, disintegrators, lubricants, diluents and the like. When the composition is of an injection, an active ingredient can be sterilized with a suitable carrier to give a pharmaceutical composition.

Specifically, examples of the excipients include lactose, saccharose, glucose, starch, calcium carbonate, crystalline cellulose and the like, examples of the binders include methylcellulose, carboxymethylcellulose, hydroxypropylcellulose, gelatin, polyvinylpyrrolidone and the like, examples of the disintegrators include carboxymethylcellulose, sodium carboxymethylcellulose, starch, sodium alginate, agar, sodium lauryl sulfate and the like, and examples of the lubricants include talc, magnesium stearate, macrogol and the like. Cacao oil, macrogol, methyl cellulose and the like may be used as base materials of suppositories. When the composition is manufactured as solutions, emulsified injections or suspended injections, dissolving accelerators, suspending agents, emulsifiers, stabilizers, preservatives, isotonic agents and the like may be added. For oral administration, sweetening agents, flavors and the like may be added.

Although the dosage of a compound of the present invention a suppressant on the IgE production, Th2 differentiation inhibitor, anti-allergic agent and/or immunosuppressant should be determined in consideration of the patient's age and body weight, the type and degree of diseases, the administration route or the like, a usual oral dosage for human adults is 0.05 - 100 mg/kg/day and preferable is 0.1 - 10 mg/kg/day. For parenteral administration, although the dosage highly varies with administration routes, a usual dosage is 0.005 - 10 mg/kg/day, preferably, 0.01 - 1 mg/kg/day. The dosage may be administered in one or several divisions per day.

The present invention is further explained by the following Examples and Experiments, which are not intended to limit the scope of the present invention.

### EXAMPLE

The present invention is further described in the following Examples, which are not intended to restrict the invention.

### Example 1 Synthesis of Compound 169

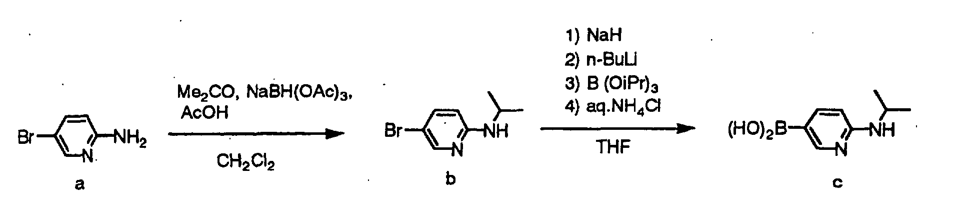

### (Step 1) Synthesis of Compound (b)

After 8.00 g of 2-amino-5-bromopyridine (a) (46.24 mmol) was dissolved in 64 ml of dichloromethane under nitrogen atmosphere, 10.19 ml of acetone (138.7 mmol) and 7.94 ml of acetic acid (138.7 mmol) were added and the mixture was stirred for 15 minutes at room temperature. To the reaction solution, 14.70 g of triacetoxy sodium borohydride (69.36 mmol) was added under ice-cooling and the mixture was stirred for 6 hours at room temperature. The reaction mixture was poured into water and extracted with ethyl acetate. The extract was washed with water and saturated brine successively, dried and concentrated. The residue was purified by a silica gel chromatography (hexane-ethyl acetate 1:7) to obtain Compound (b) (6.64 g; 67 % yield)

### (Step 2) Synthesis of Boric acid compound (c)

1.45 g of 60% sodium hydride (36.26 mmol) was washed with anhydrous hexane under nitrogen atmosphere to exclude a mineral oil. After 98 ml of THF was added thereto, 6.50 g of Compound (b) (30.22 mmol) was added and stirred for 1 hour at 50 °C. The reaction mixture was cooled to - 78 °C and 37.8 ml of n-butyl lithium (1.6 M) (60.44mmol) was added dropwise to the mixture. The mixture was reacted for 2 hours at - 78 °C and 25.10 ml of triisopropyl borate (108.78 mmol) was added to the mixture. After the mixture was warmed to room temperature, the reaction was stopped by adding 50 ml of saturated aqueous solution of ammonium chloride. The reaction mixture was extracted with ethyl acetate and the extract was washed with water and saturated brine, successively, dried and concentrated. The residue was recrystallized from isopropyl ether to obtain Boric acid compound (c) (5.37 g; 99% yield).

### (Step 3) Synthesis of Compound 169

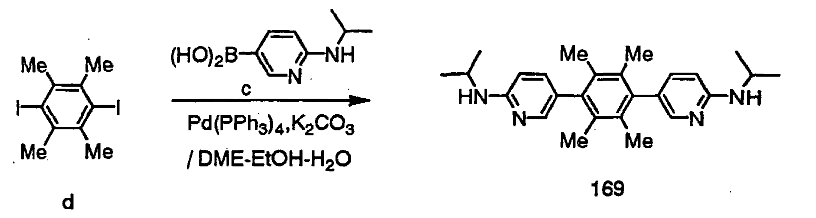

After 250 mg of 1,4-diiodo-2,3,5,6-tetramethylbenzene (d) (0.648 mmol) was dissolved in 3 ml of 1,2-dimethoxyethane, 0.5 ml of ethanol and 0.5 ml of water were added to the solution. To the solution, 537 mg of potassium carbonate (3.89 mmol) and 350 mg of Boric acid compound (c) (1.943 mmol) were added and 37.4 mg of tetrakis(triphenylphosphine)palladium (0) (0.324 mmol) was added under argon atmosphere. The suspension was refluxed overnight under argon atmosphere. After cooling, water was added to the reaction mixture and the mixture was extracted with ethyl acetate. The extract was washed with water and saturated brine, successively, dried and concentrated. The residue was recrystallized from methanol to obtain Compound 169 (213 mg; 82% yield).

### Example 2 Synthesis of Compound 13

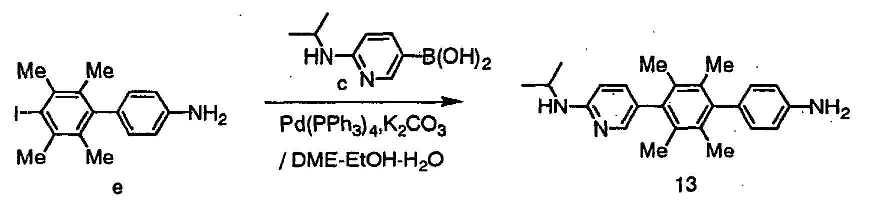

### (Step 1) Synthesis of Compound 13

500 mg of Compound (e) (1.424 mmol) was dissolved in 5 ml of 1,2-dimethoxyehtane, and 1 ml of ethanol and 1 ml of water were added to the mixture. After 590 mg of potassium carbonate (4.272 mmol) and 385 mg of Boric acid compound (c) (2.136 mmol) were added, 82.3 mg of tetrakis(triphenylphosphine)palladium(0) (0.0712 mmol) was added under argon atmosphere. The reaction suspension was refluxed overnight under argon atmosphere. After cooling, water was added to the solution and the mixture was extracted with ethyl acetate. The extract was washed with water and saturated brine, successively, dried and concentrated. The residue was recrystallized from hexane to obtain Compound 13 (337 mg; 66 % yield).

Other Compound (I) were synthesized by the similar methods. The structures are as follows.

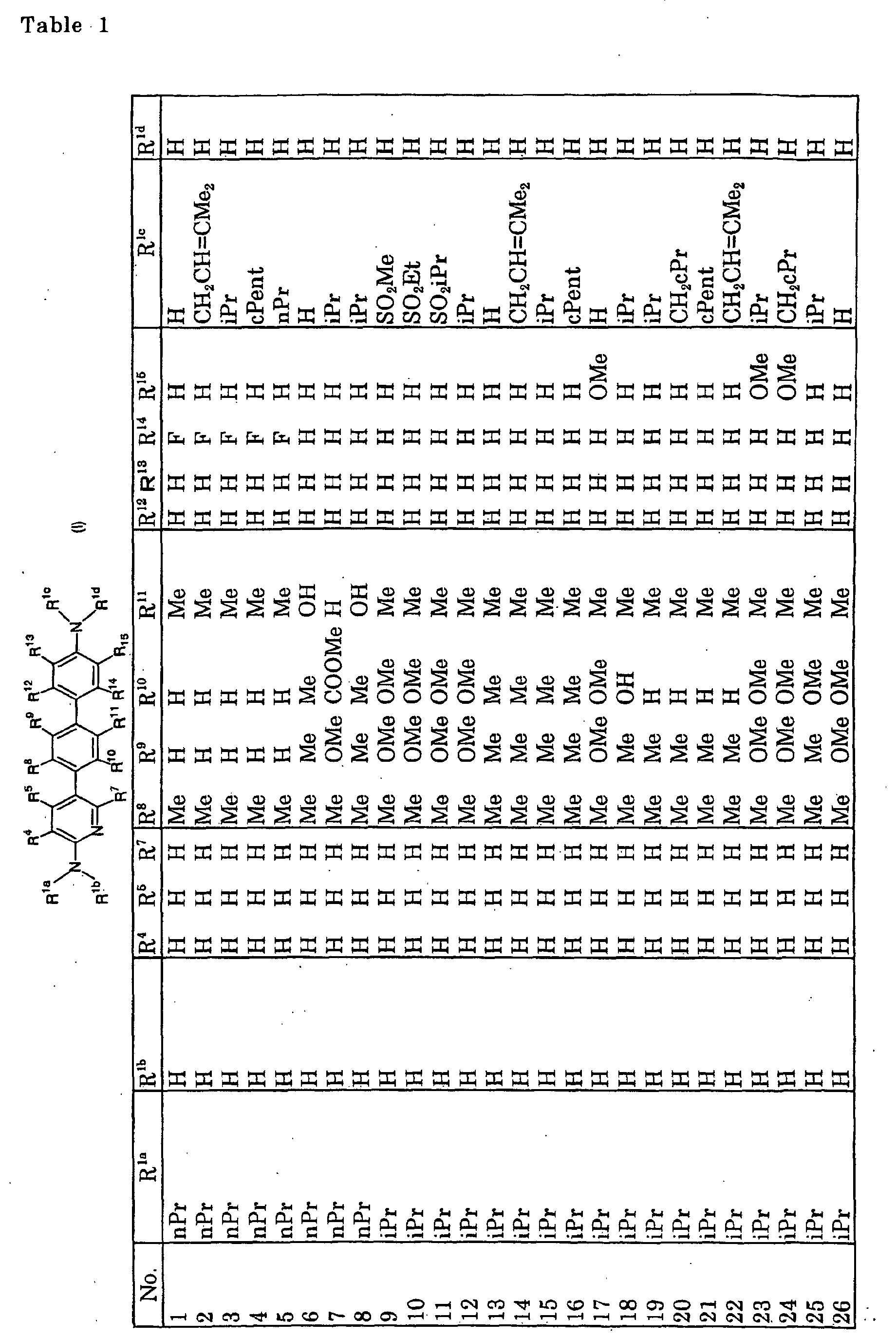

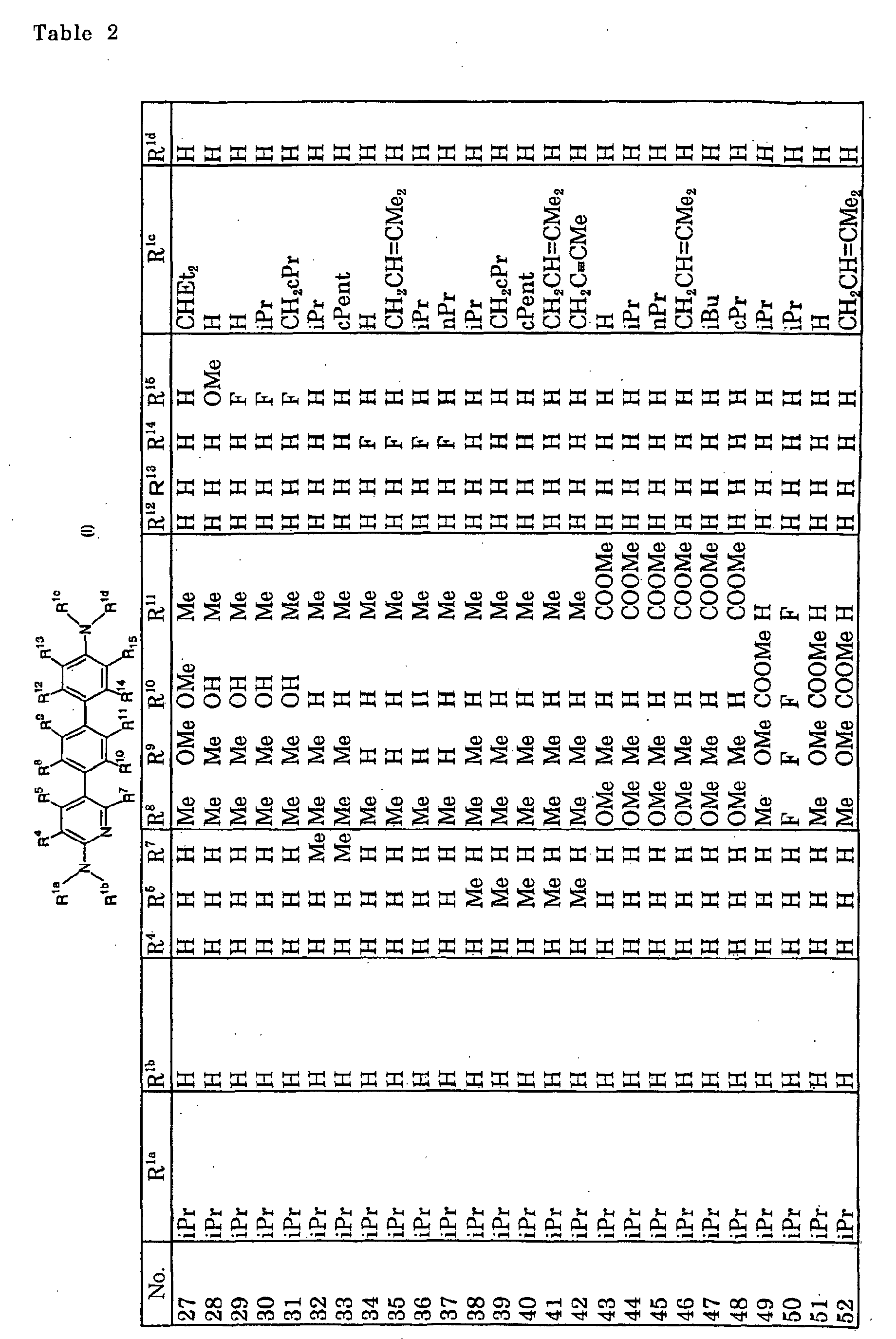

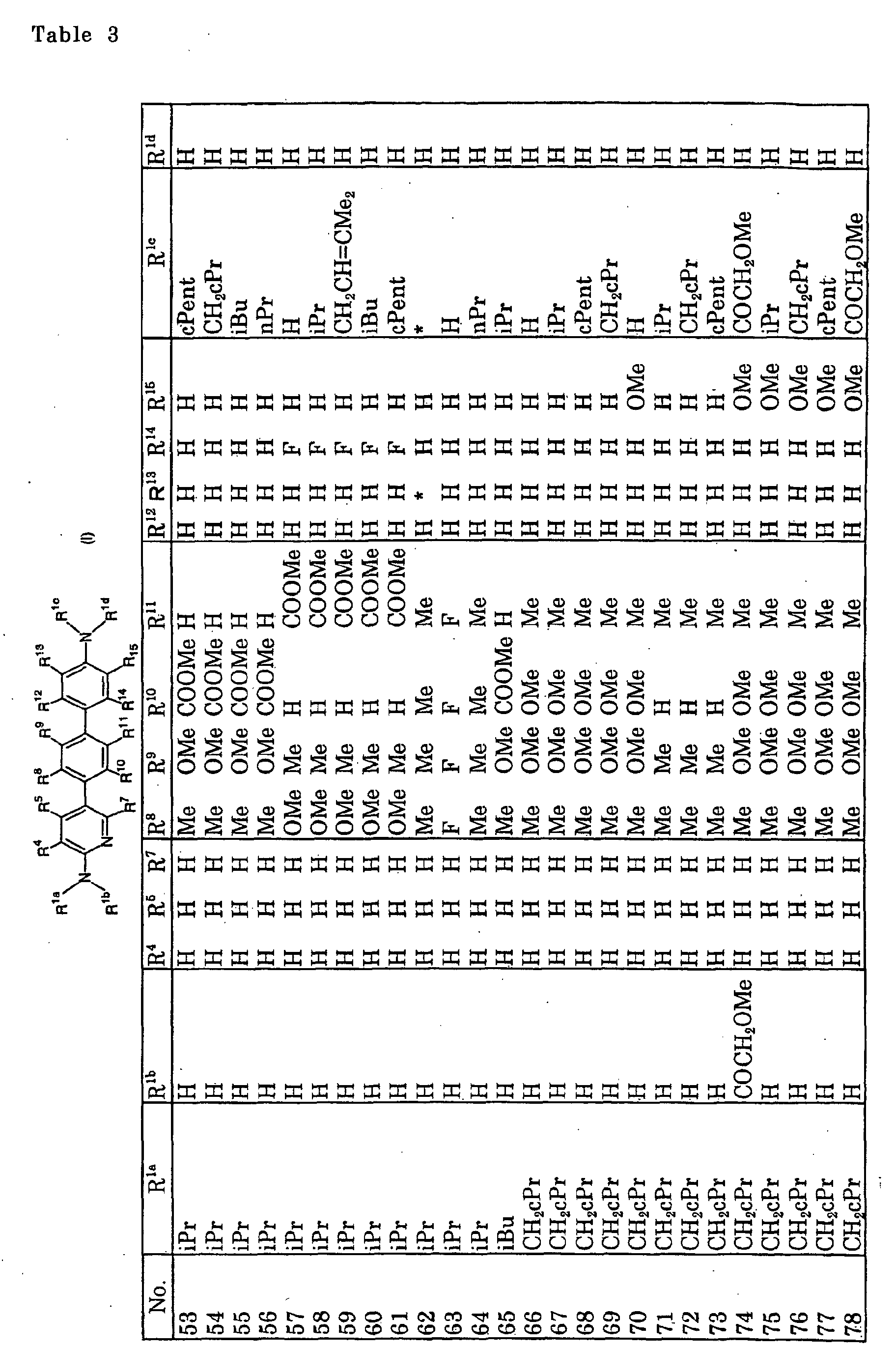

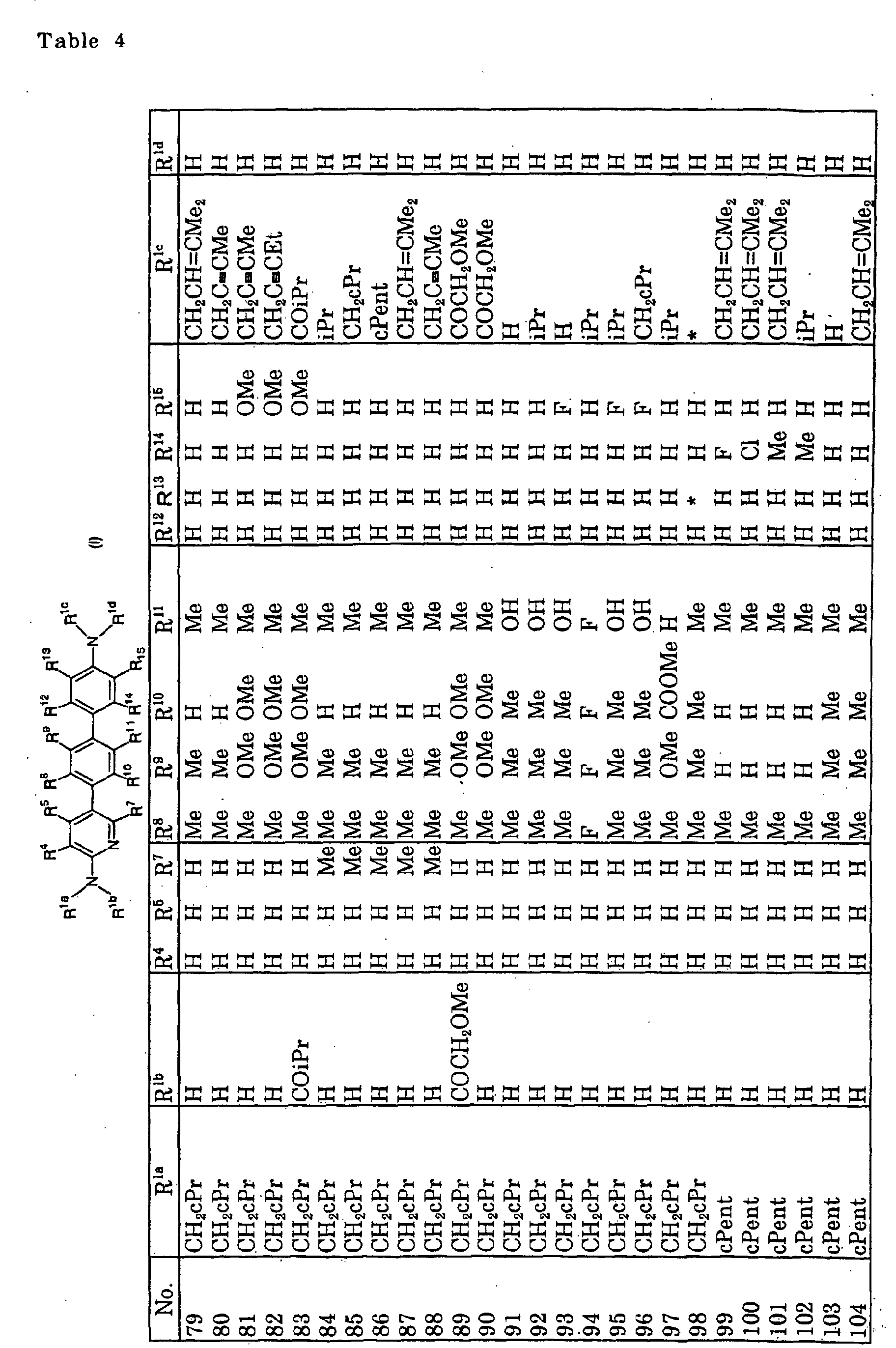

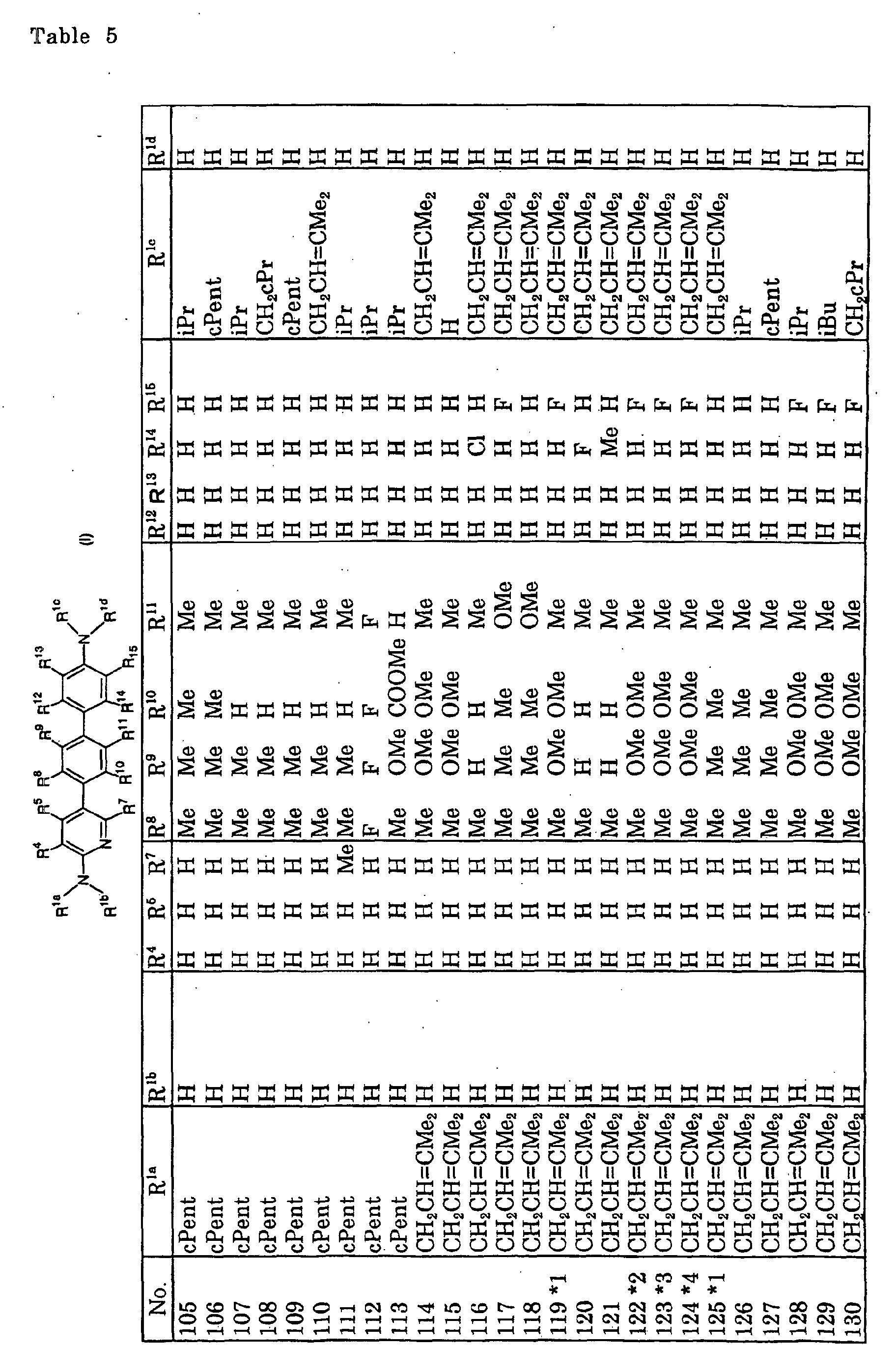

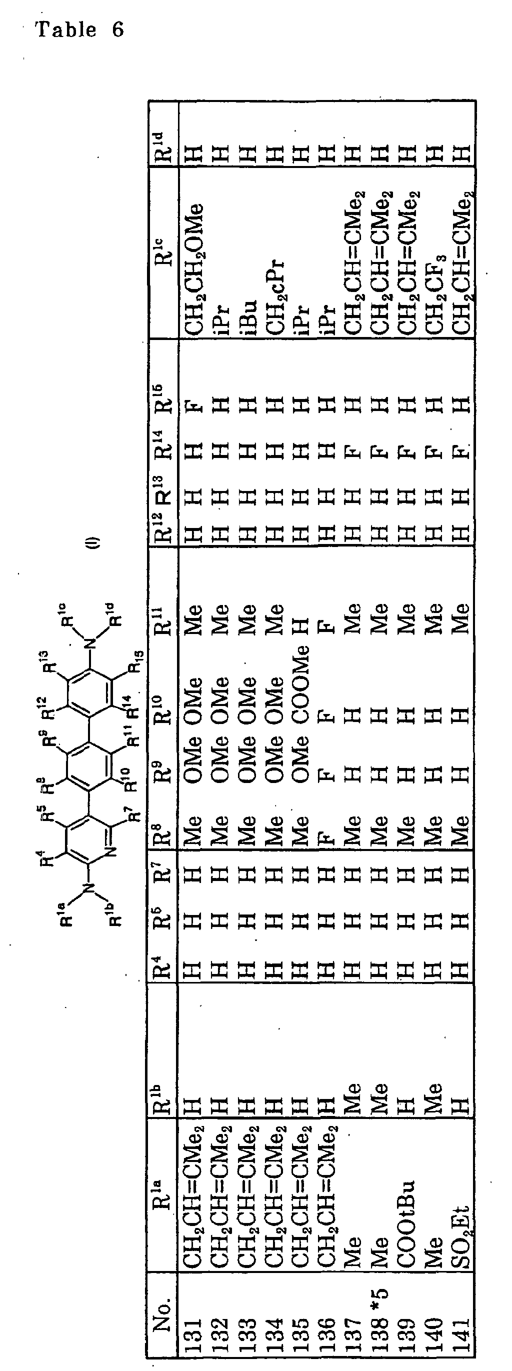

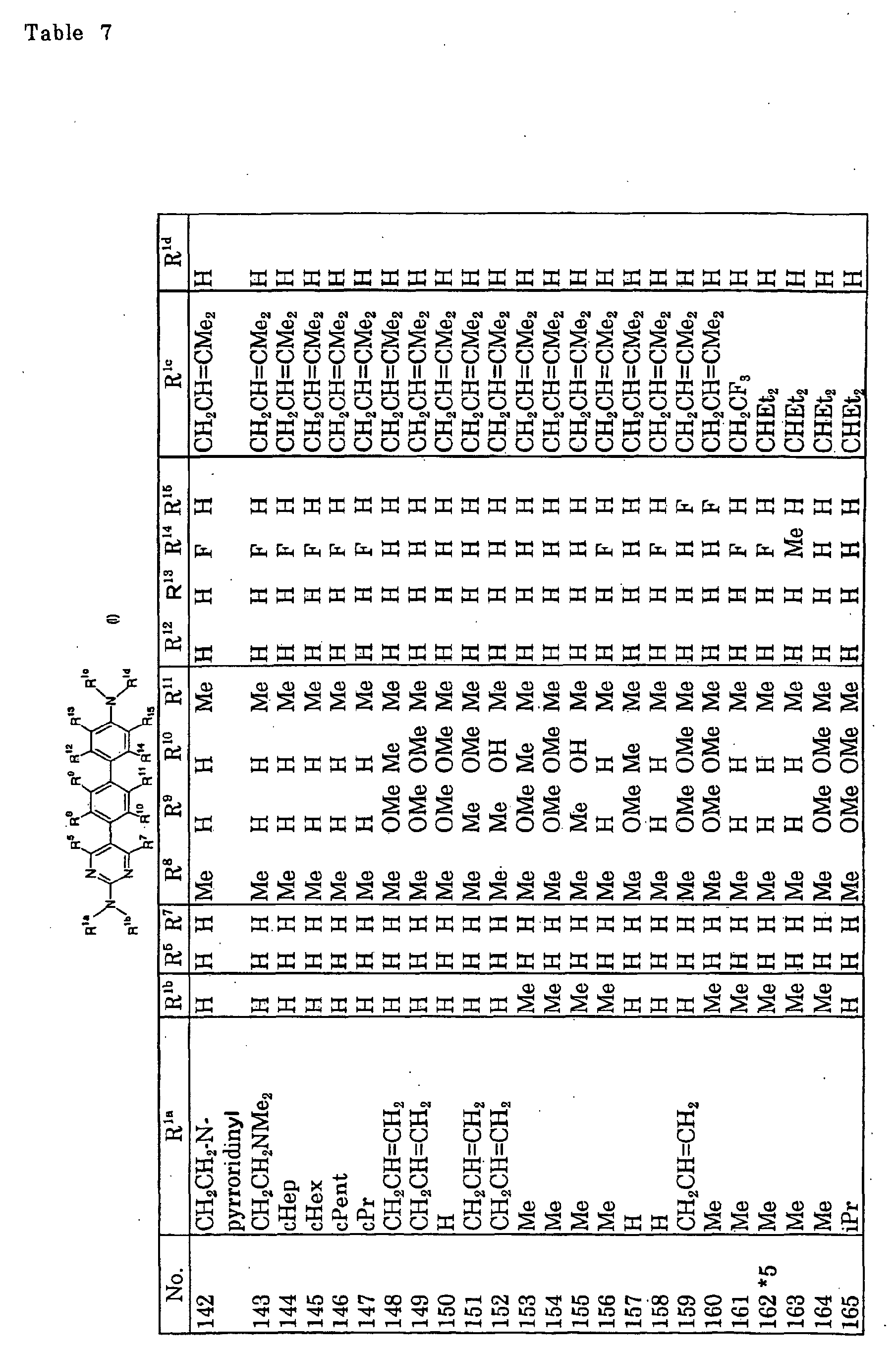

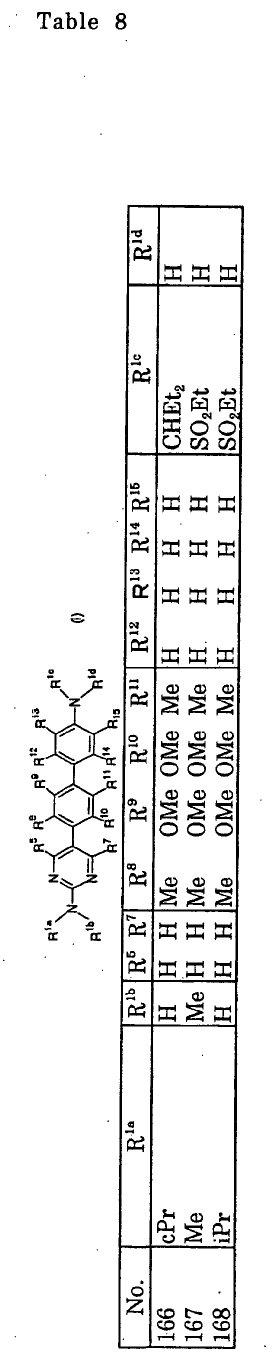

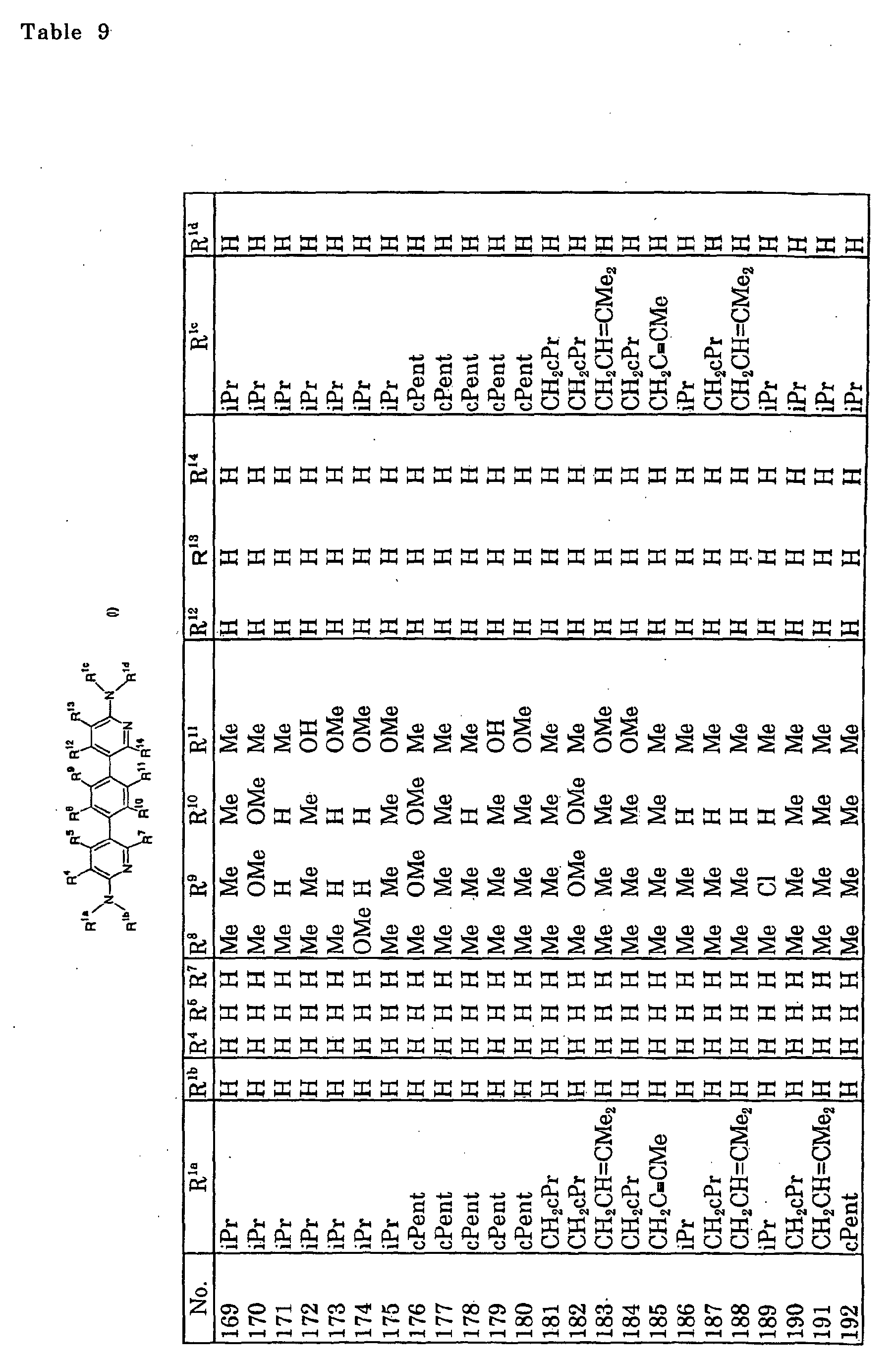

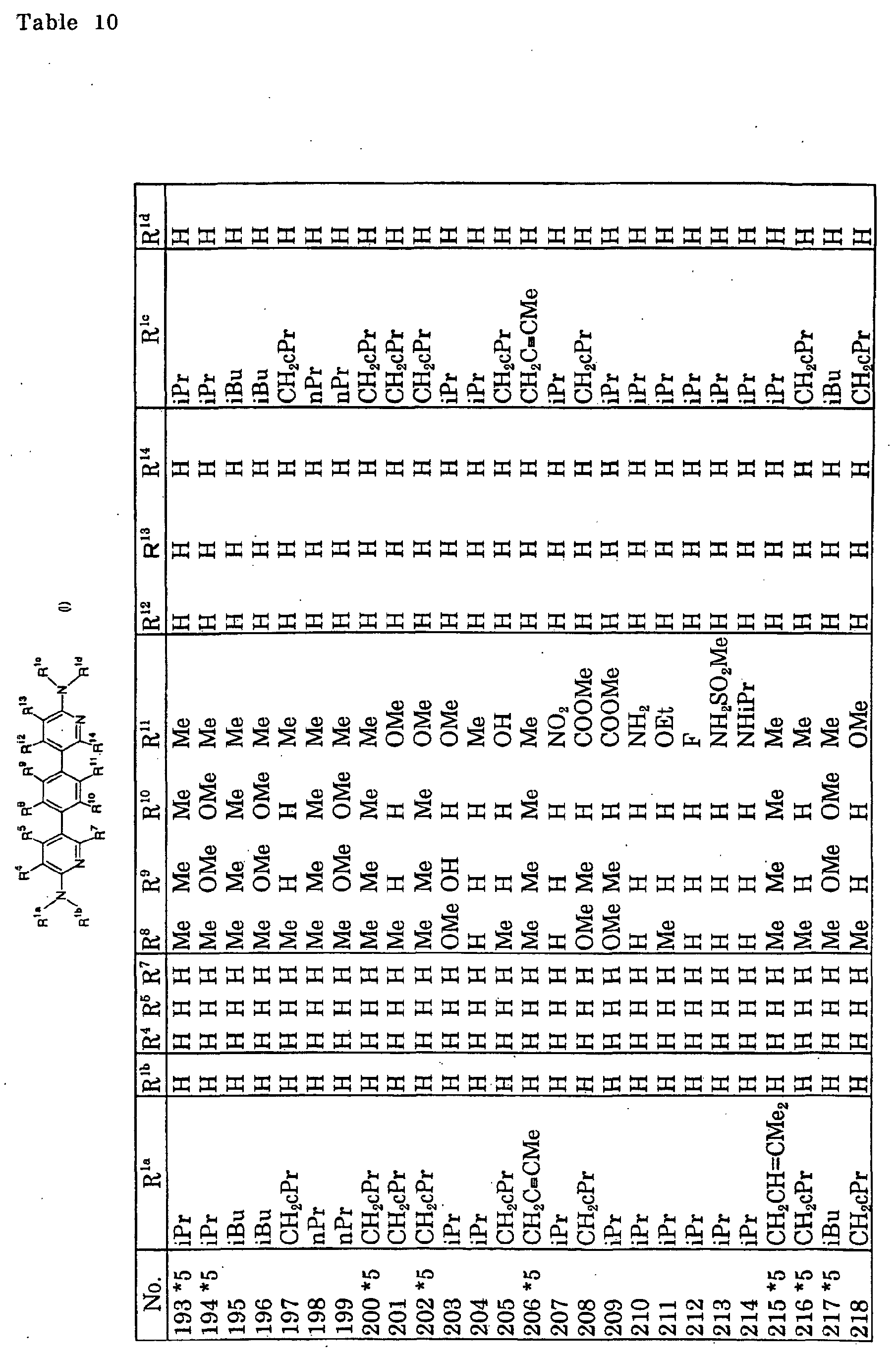

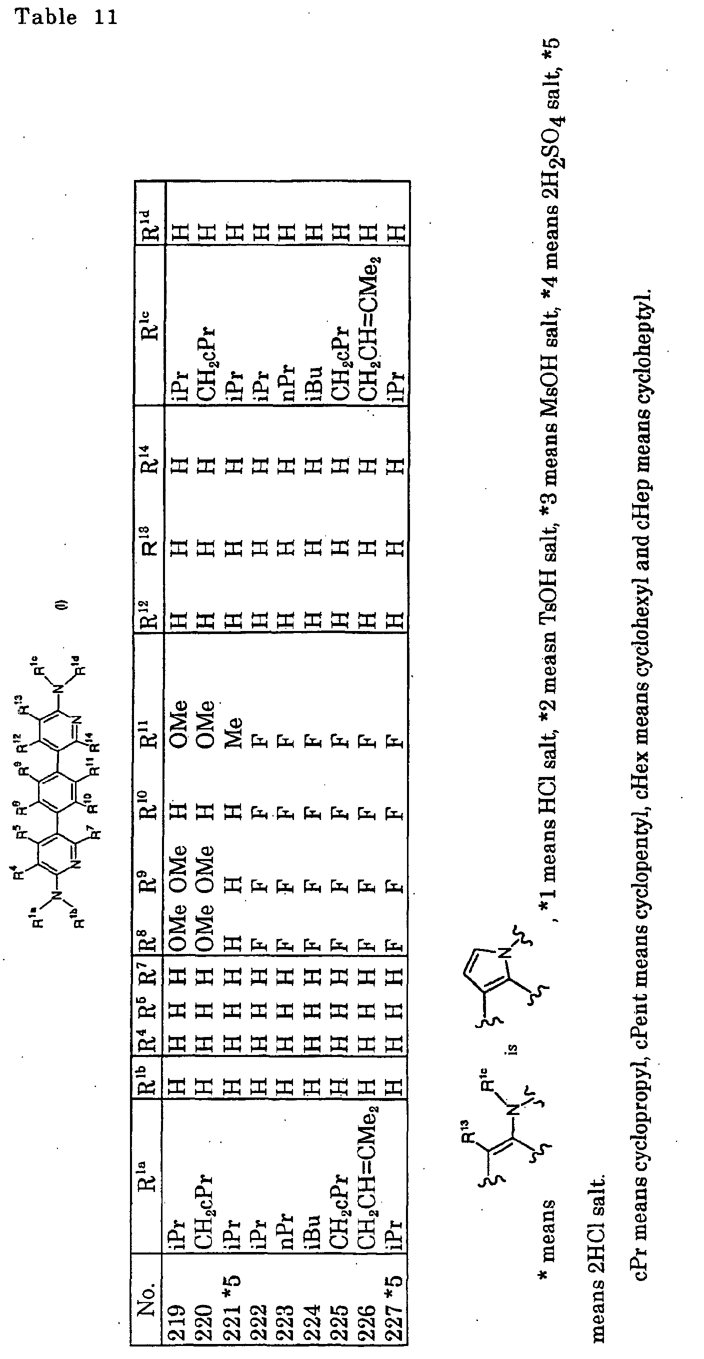

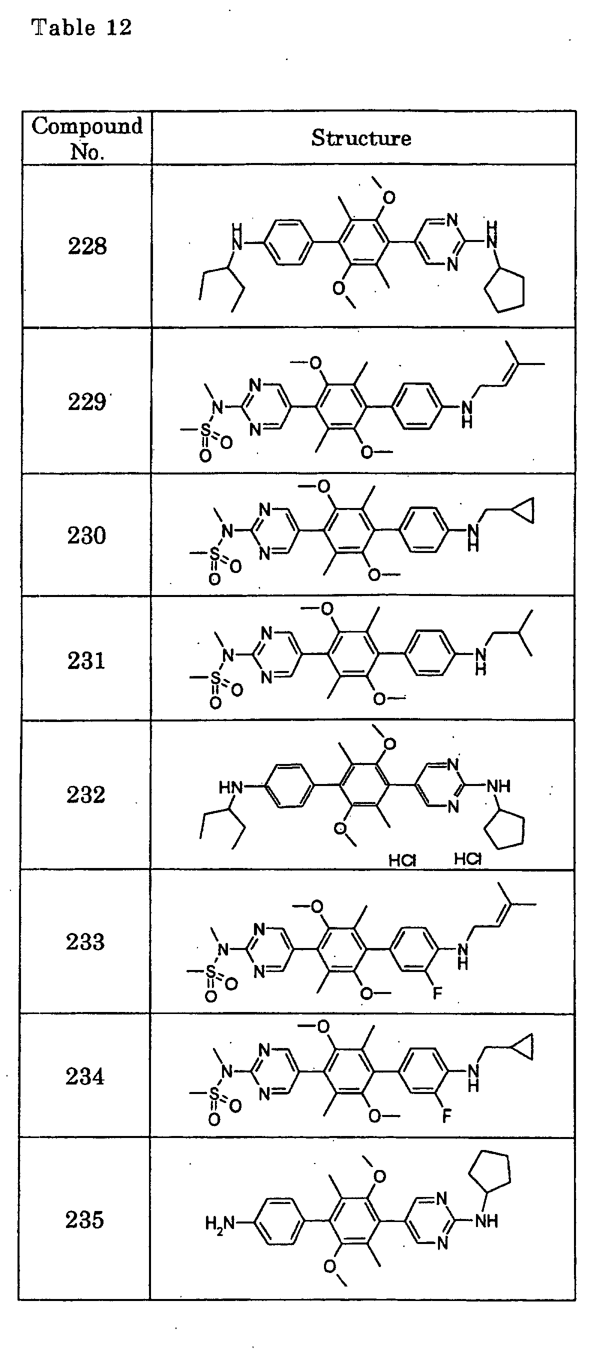

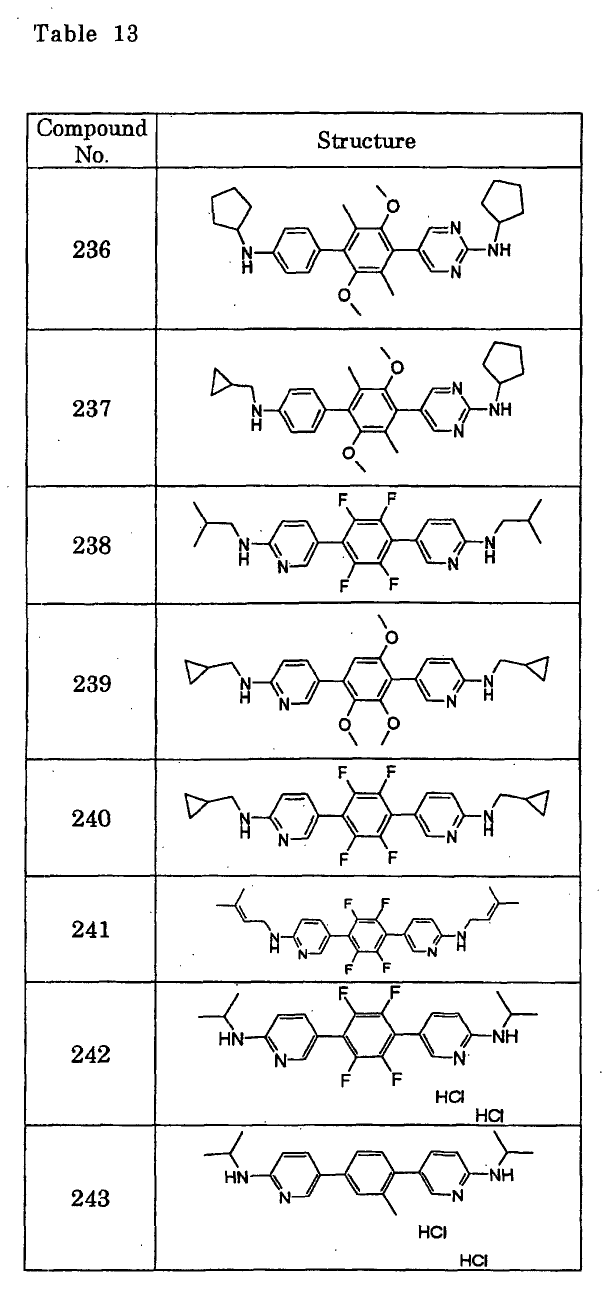

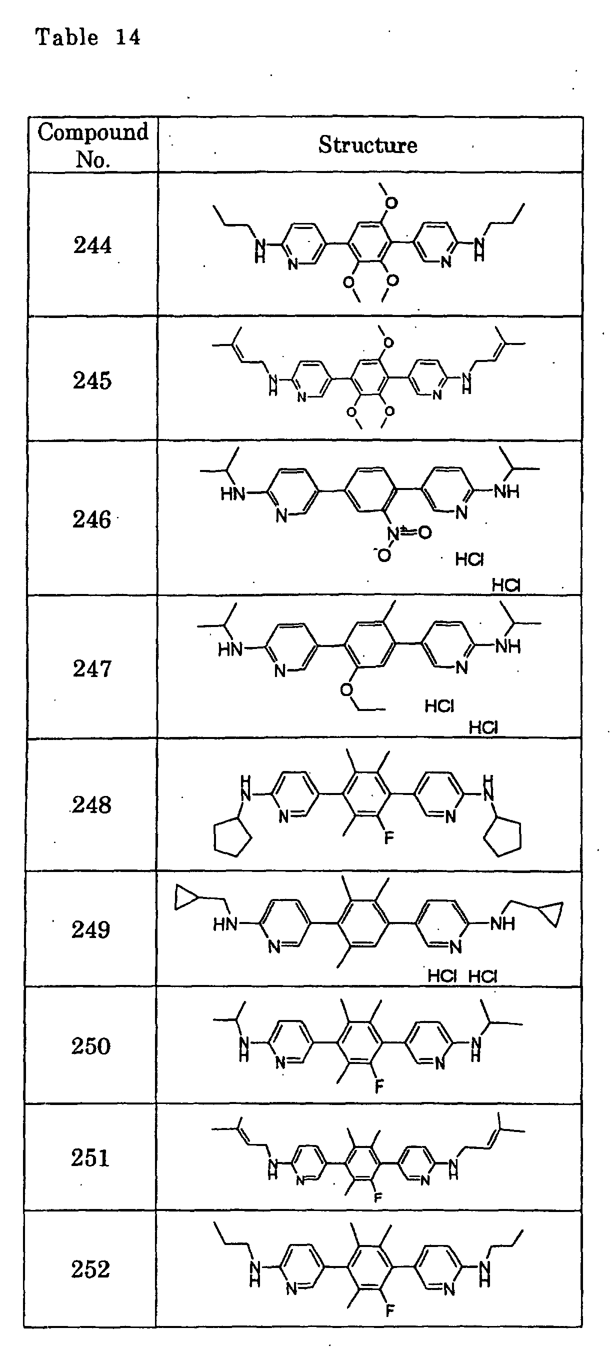

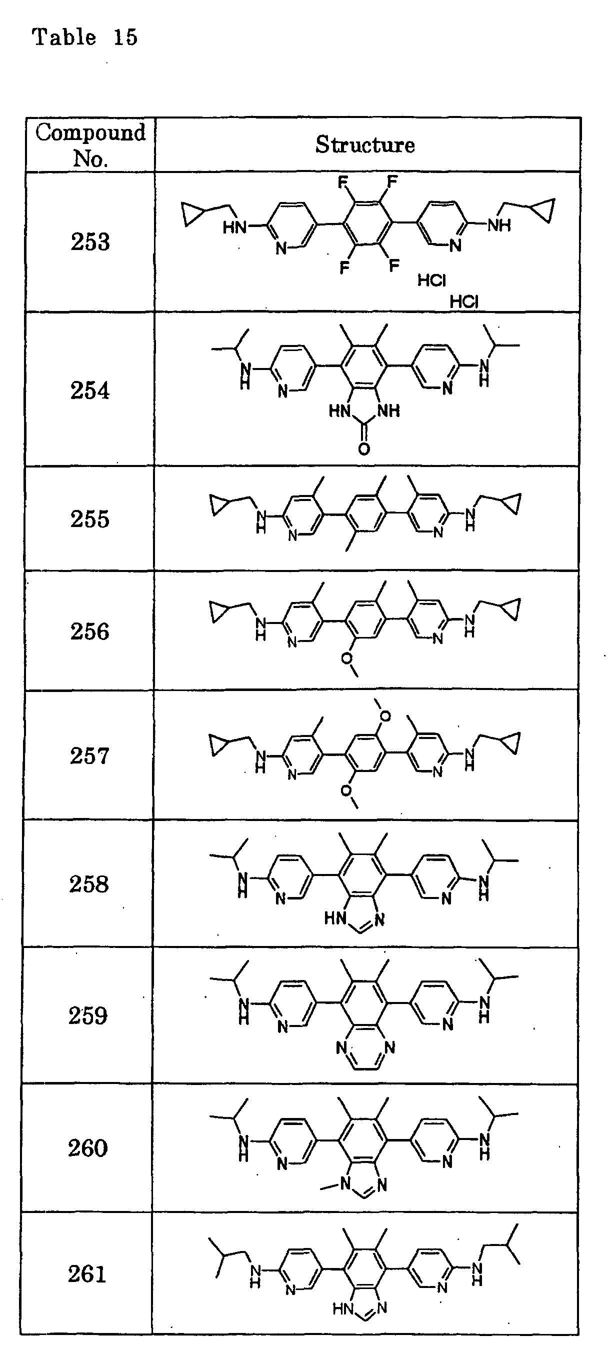

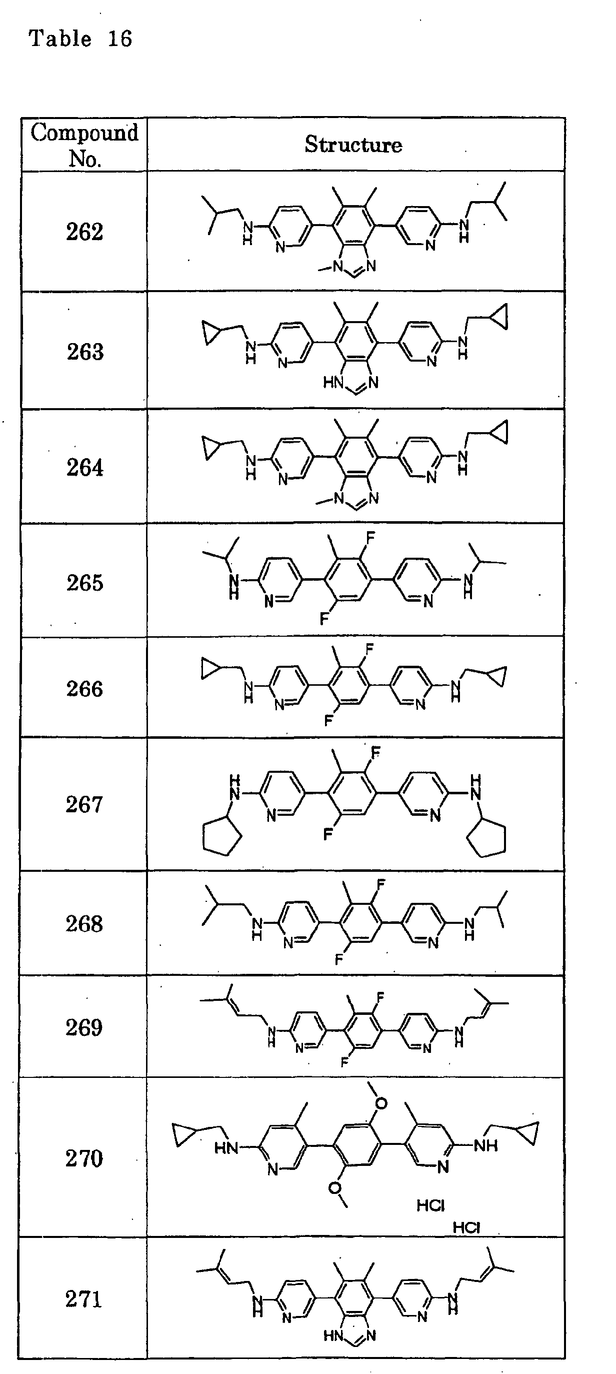

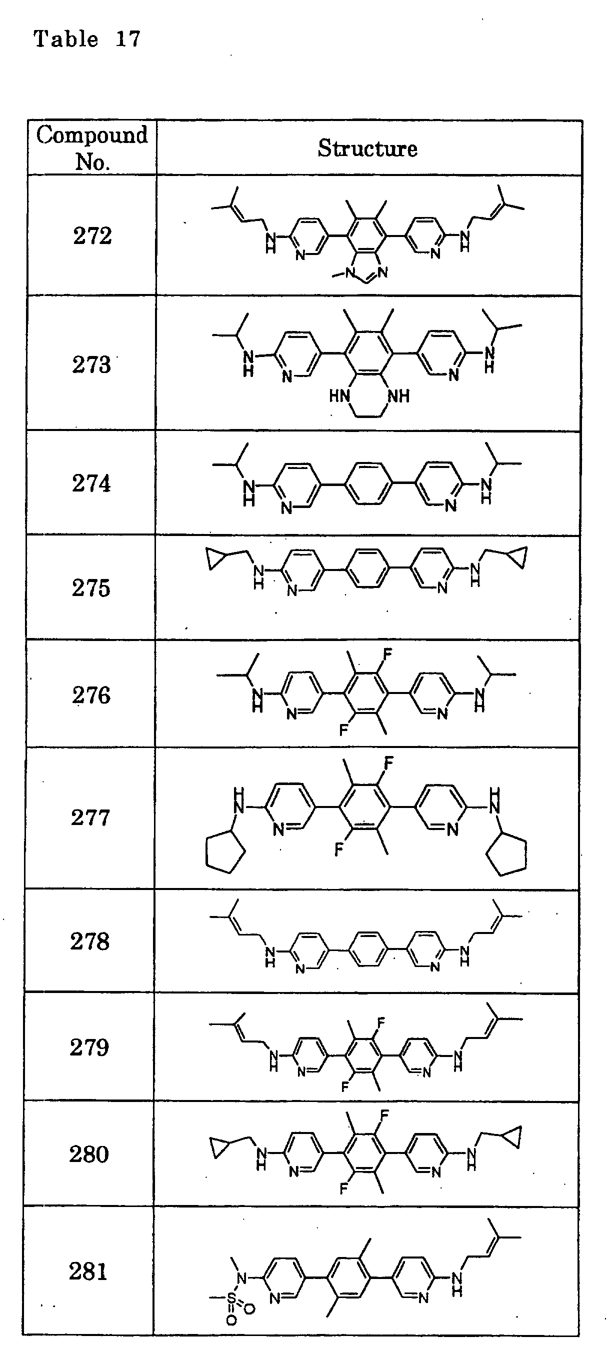

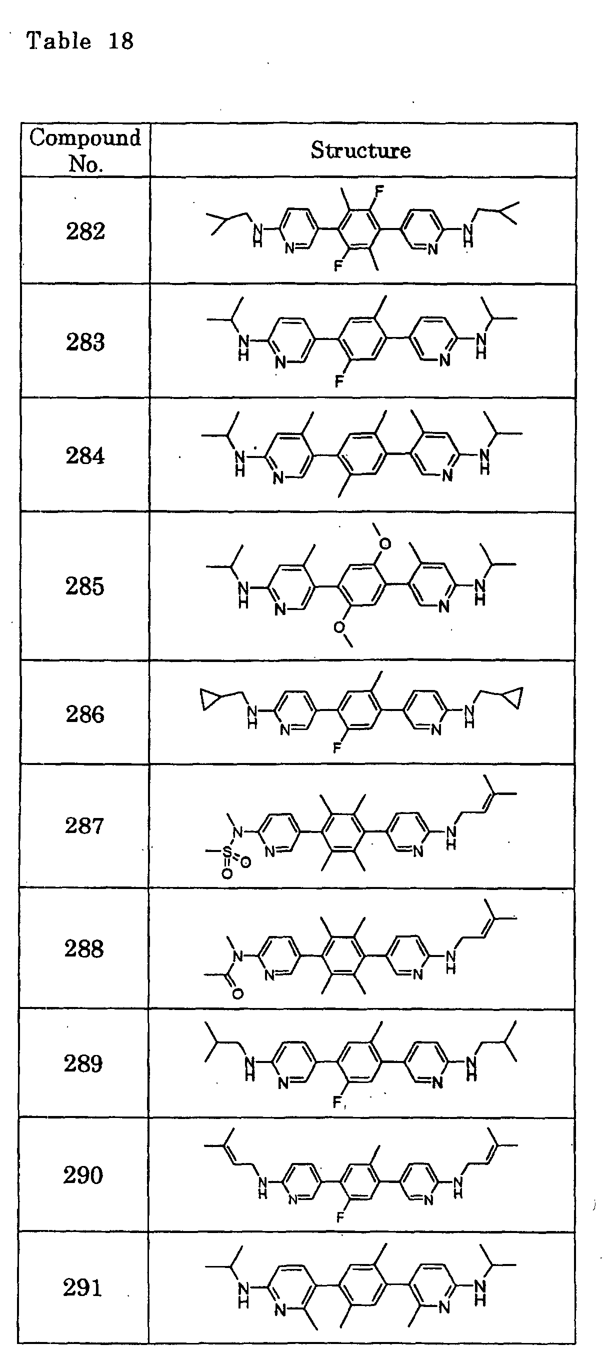

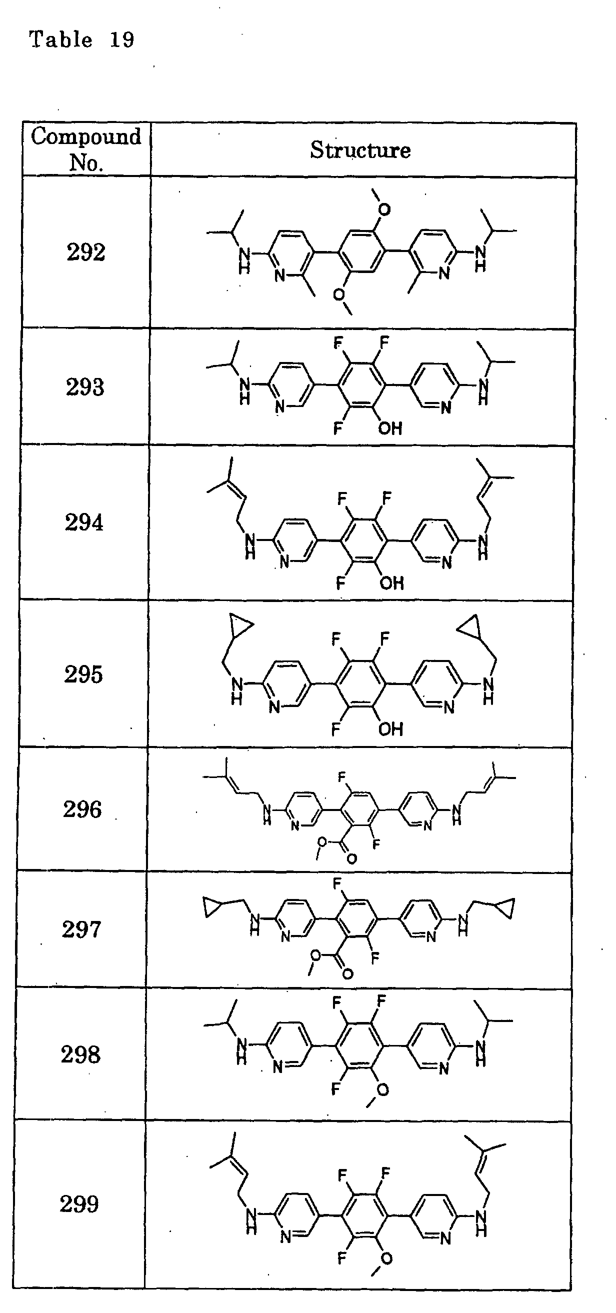

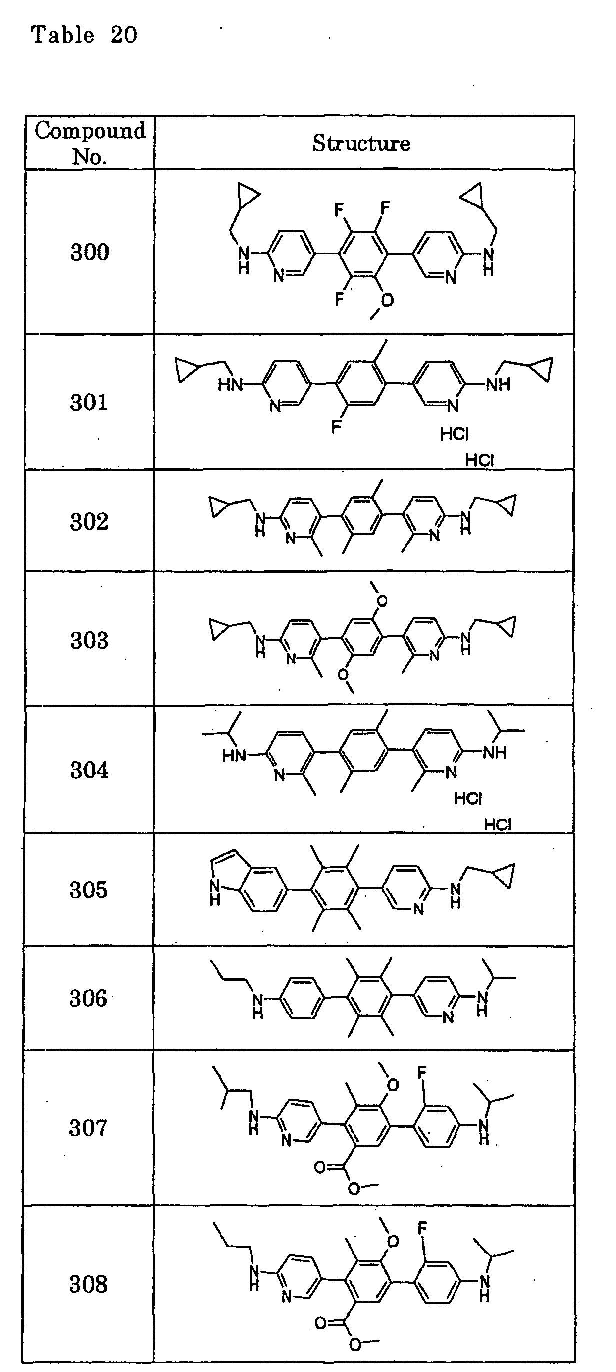

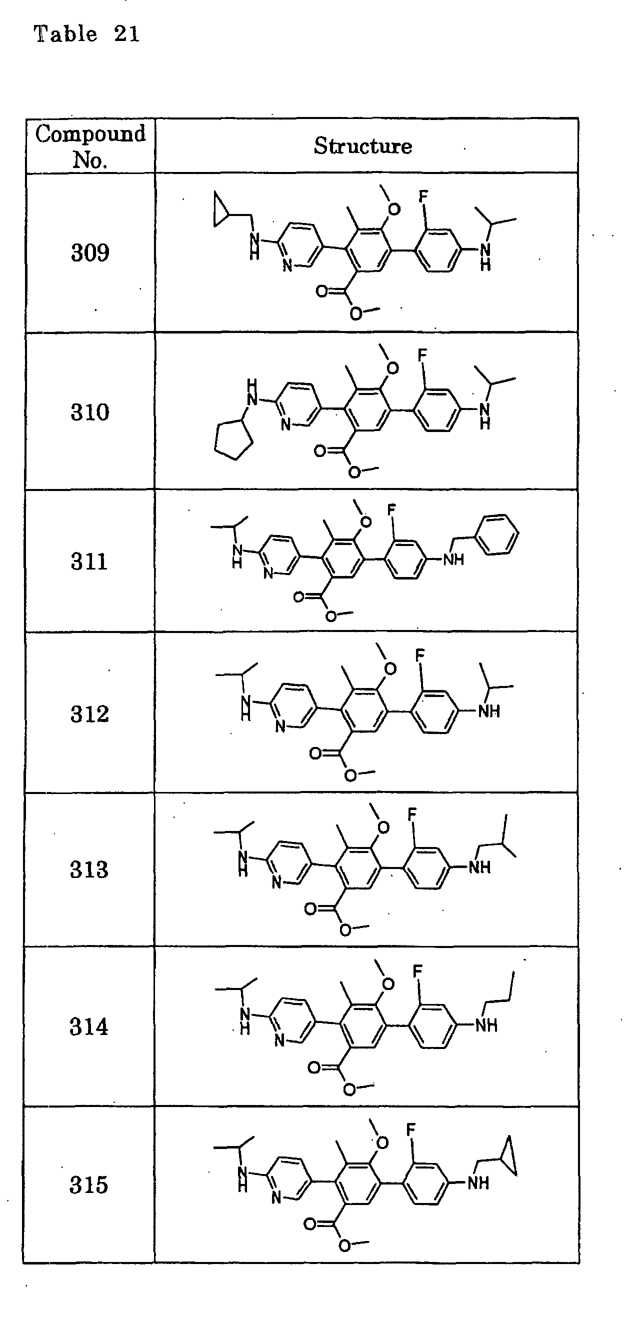

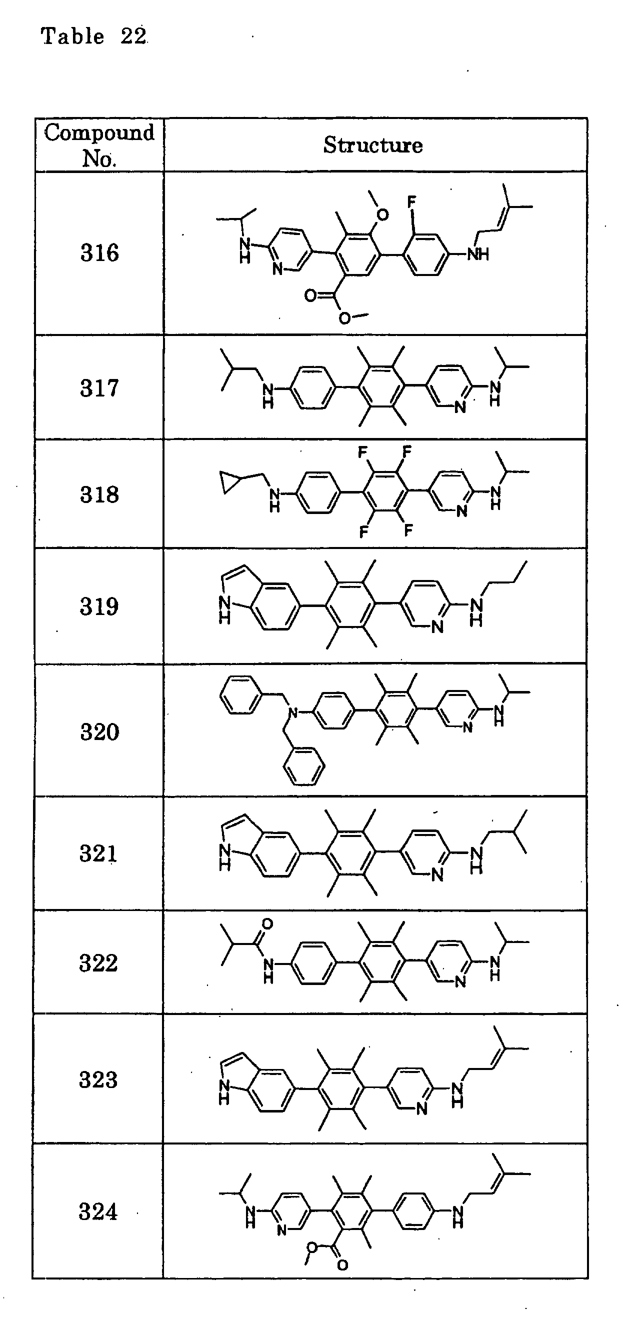

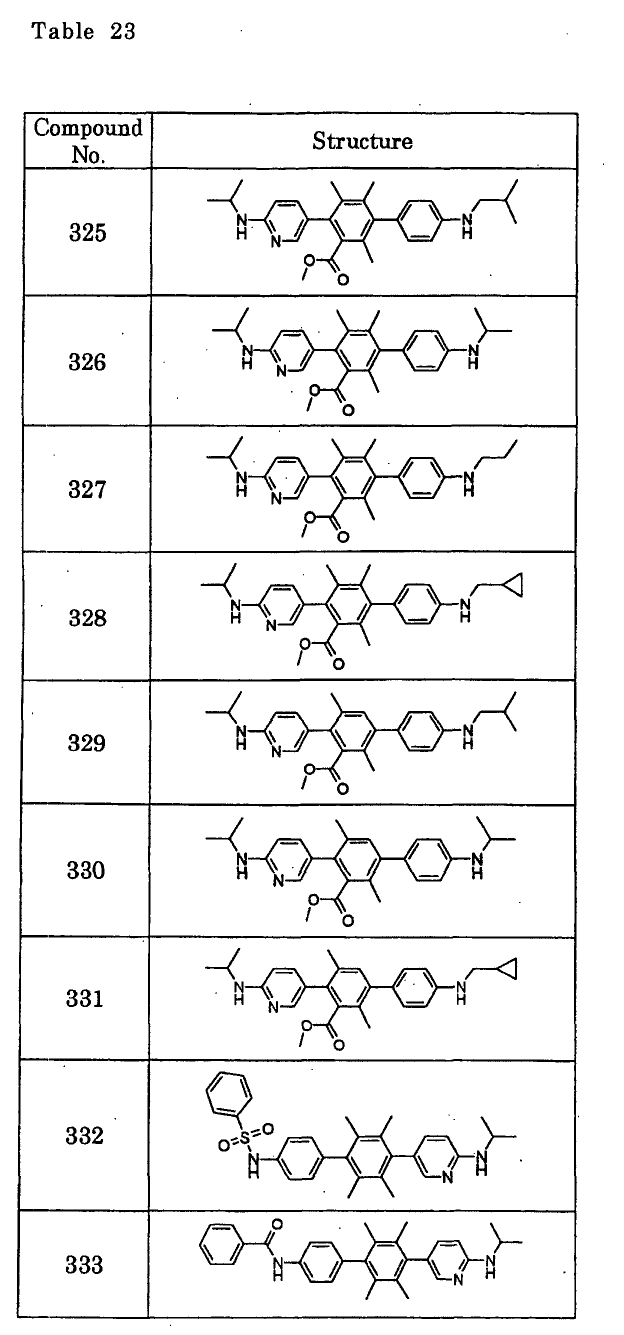

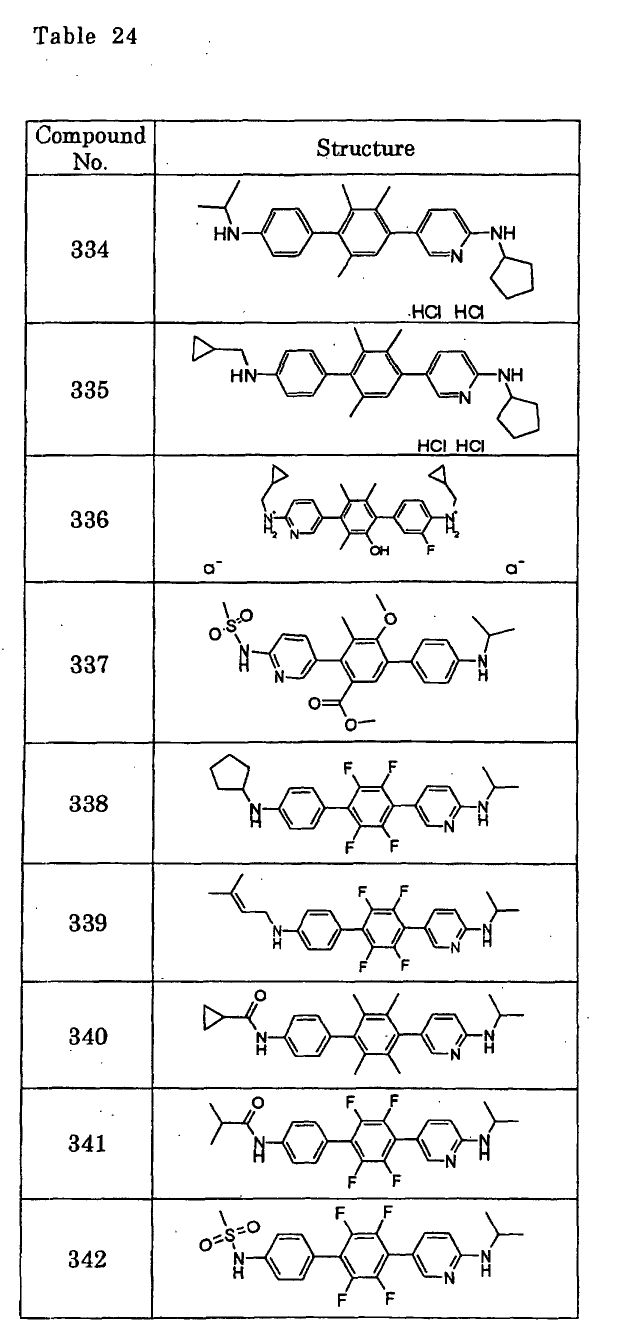

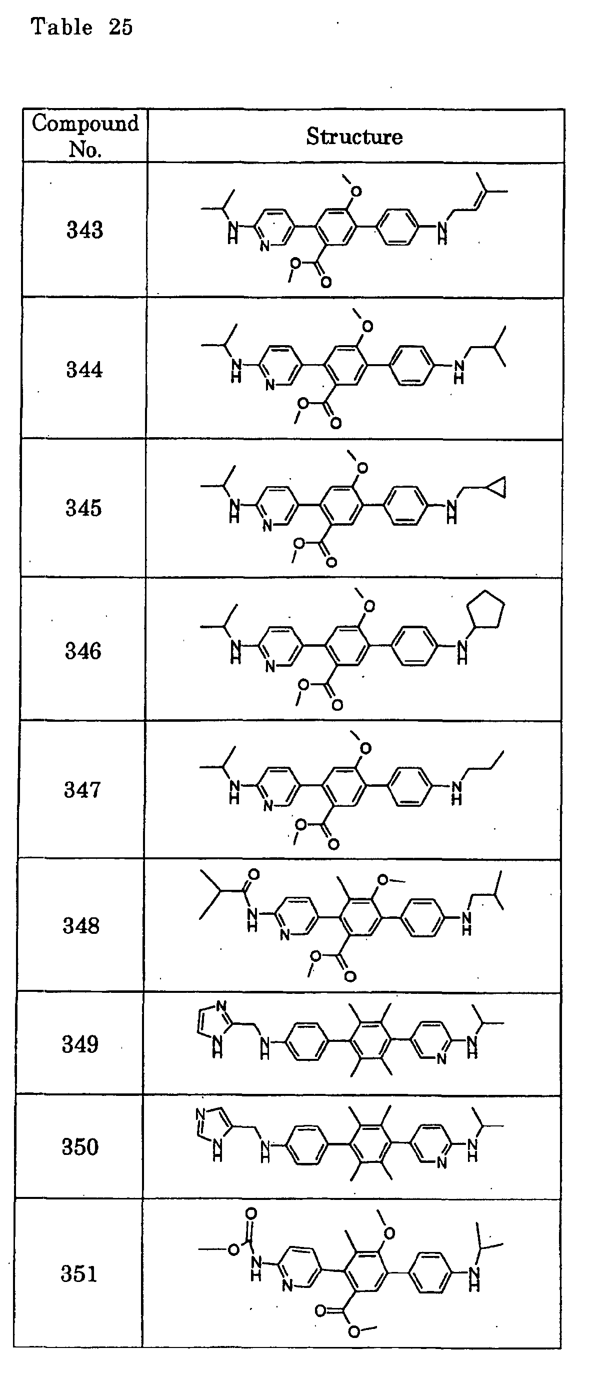

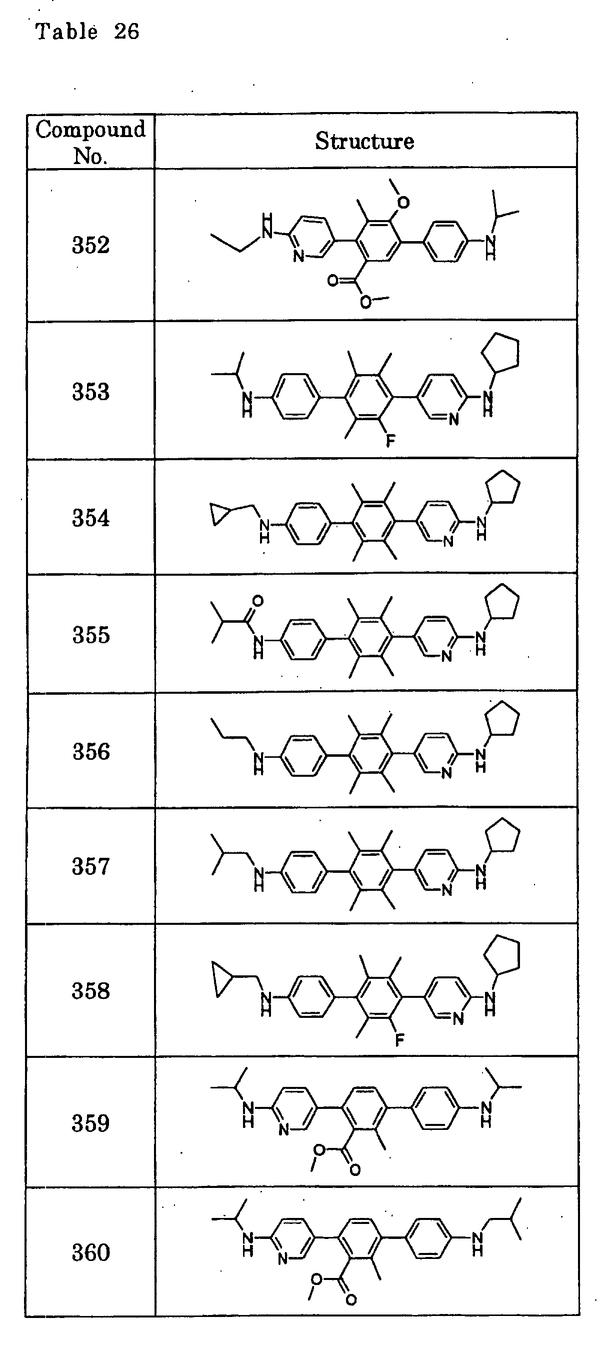

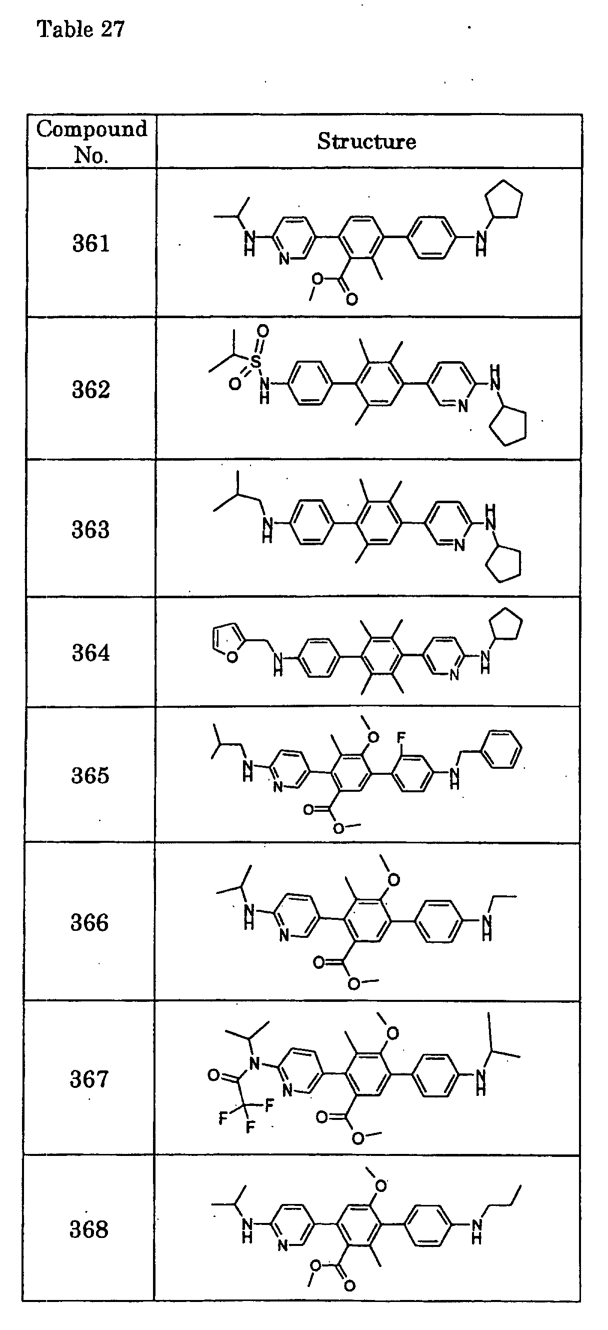

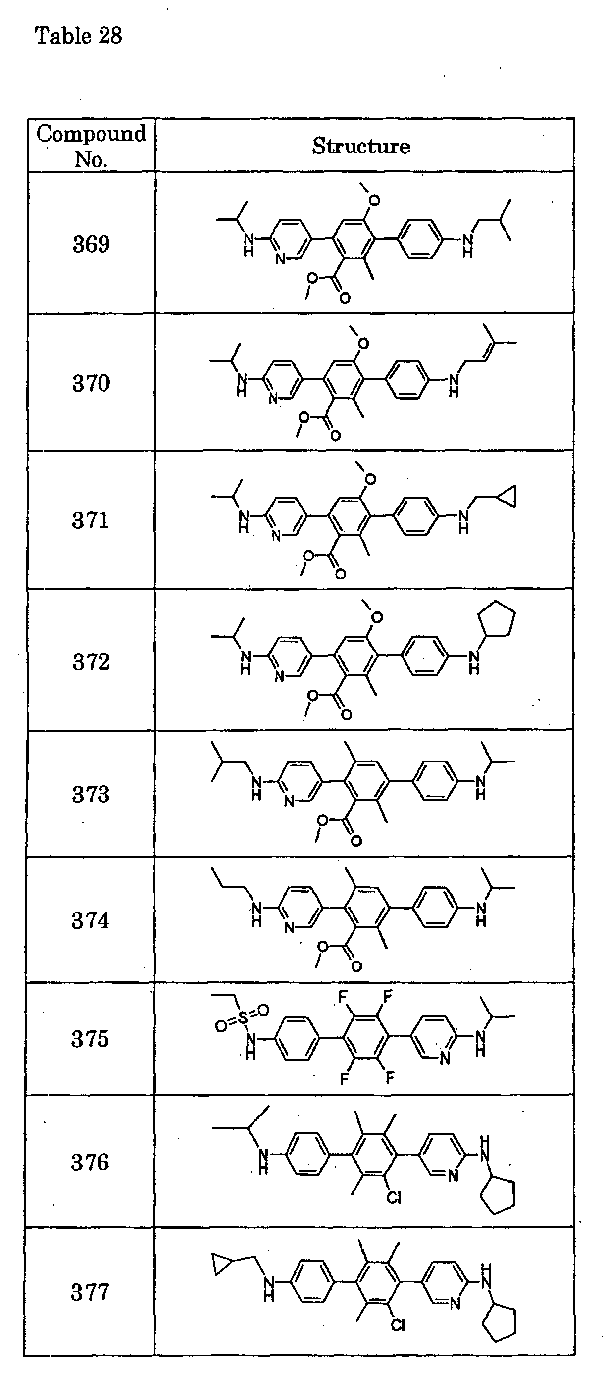

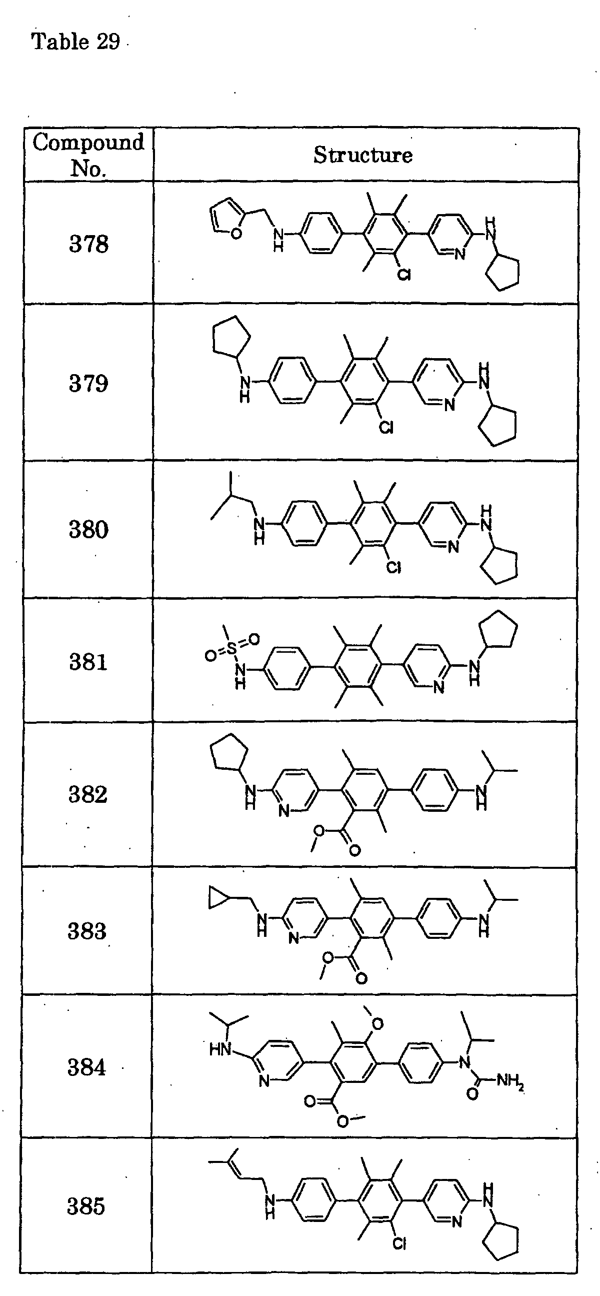

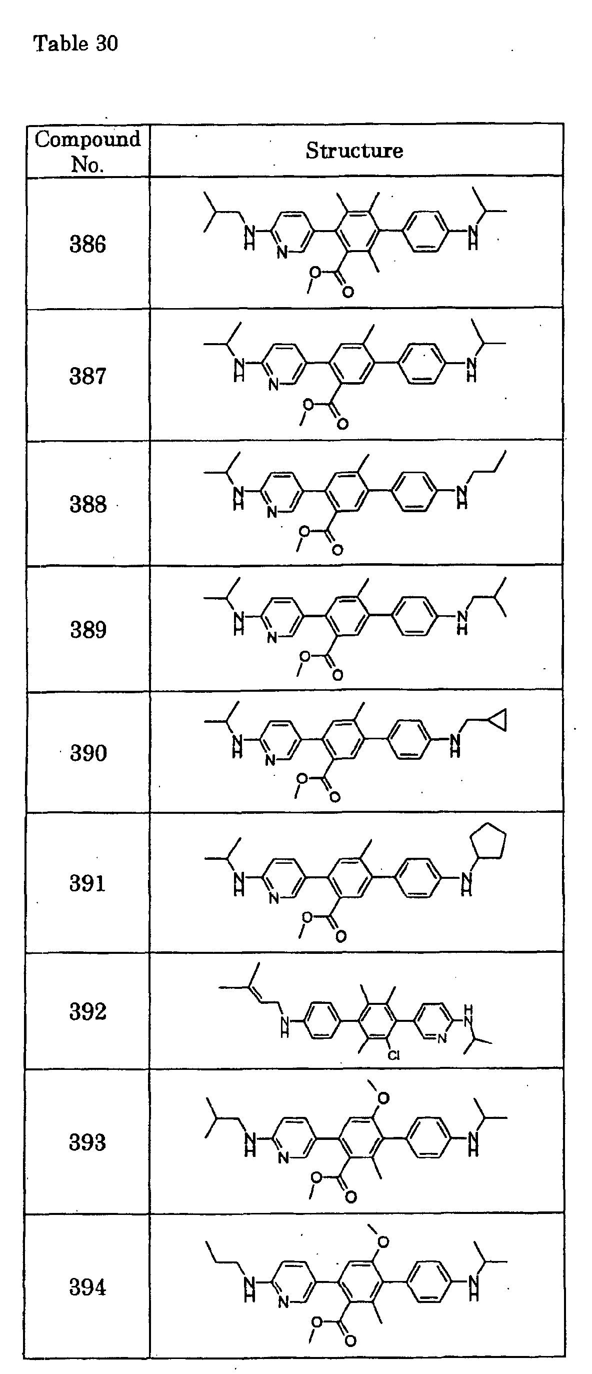

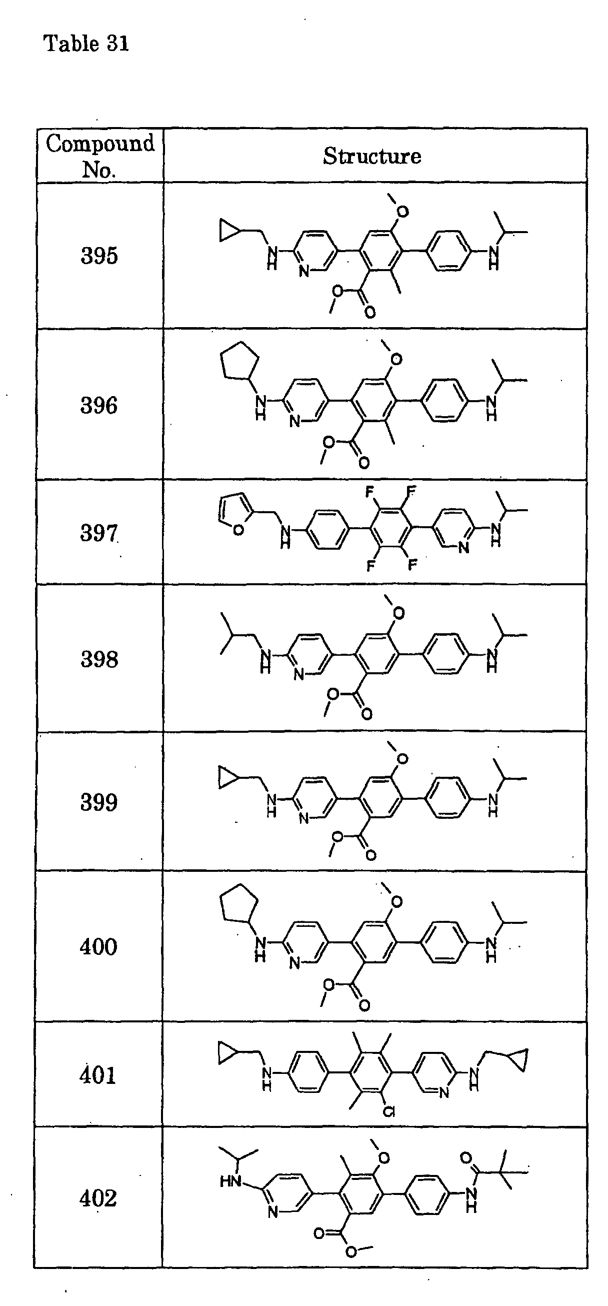

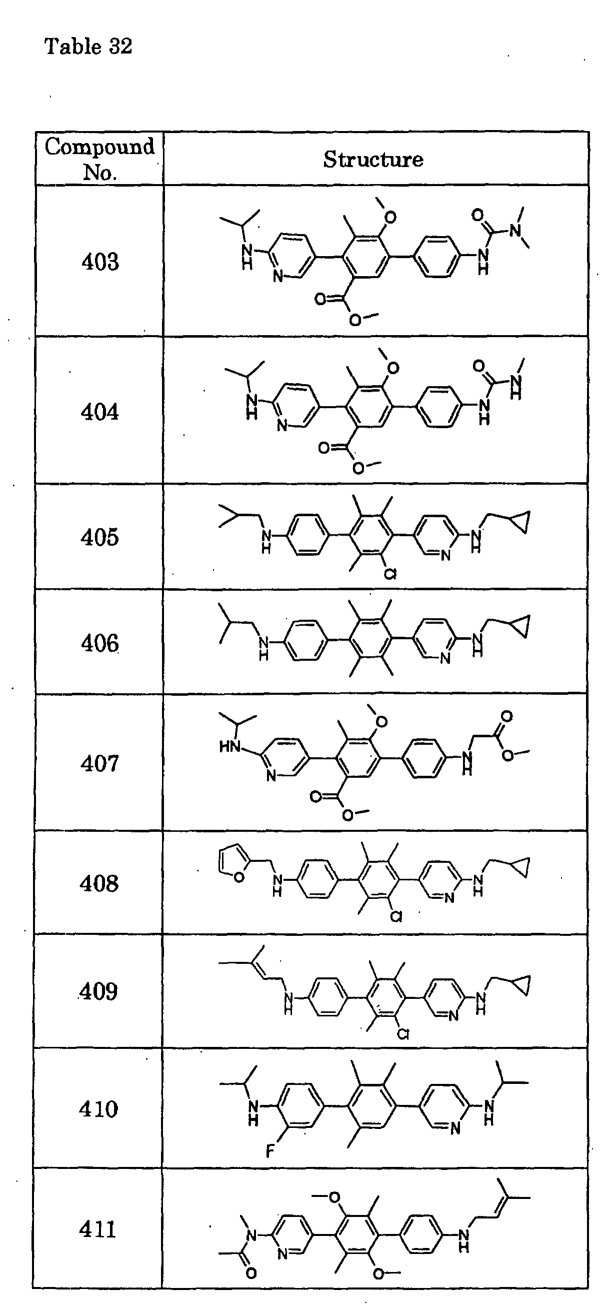

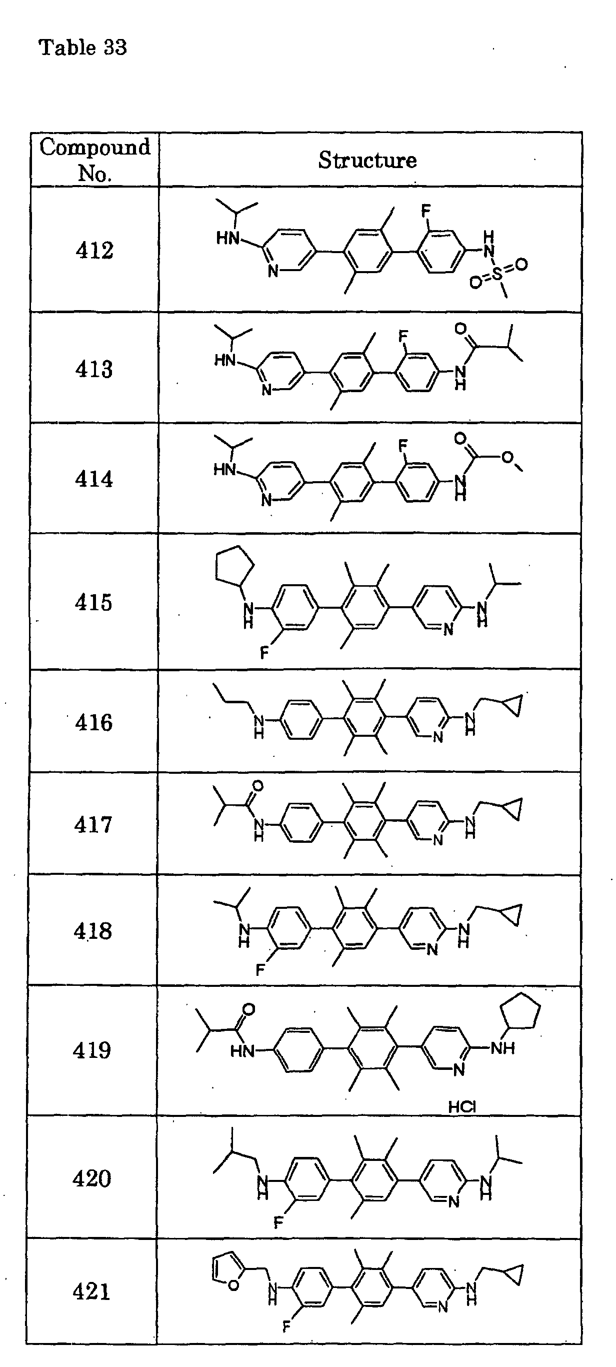

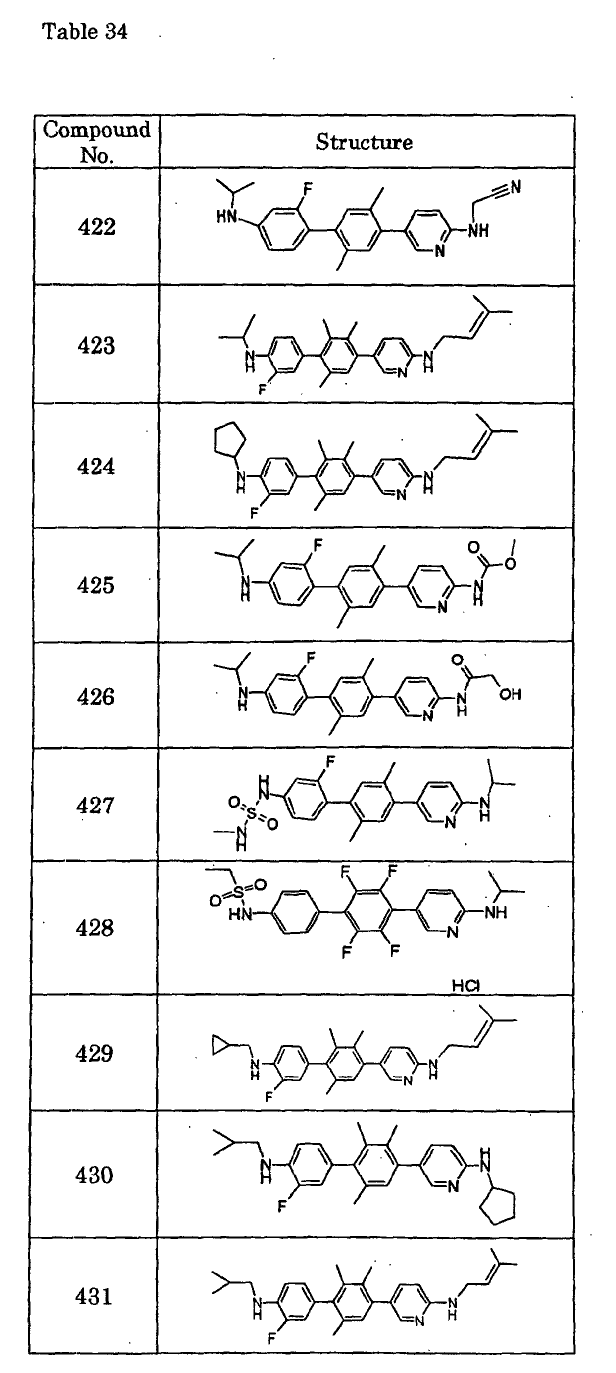

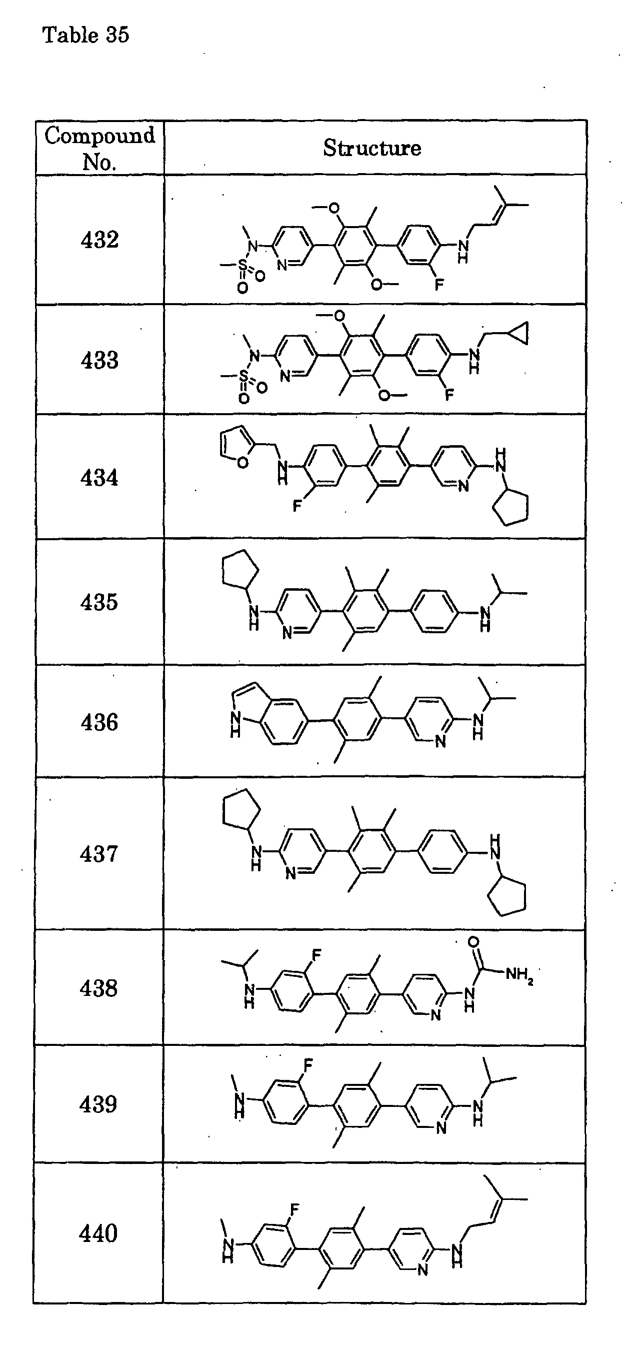

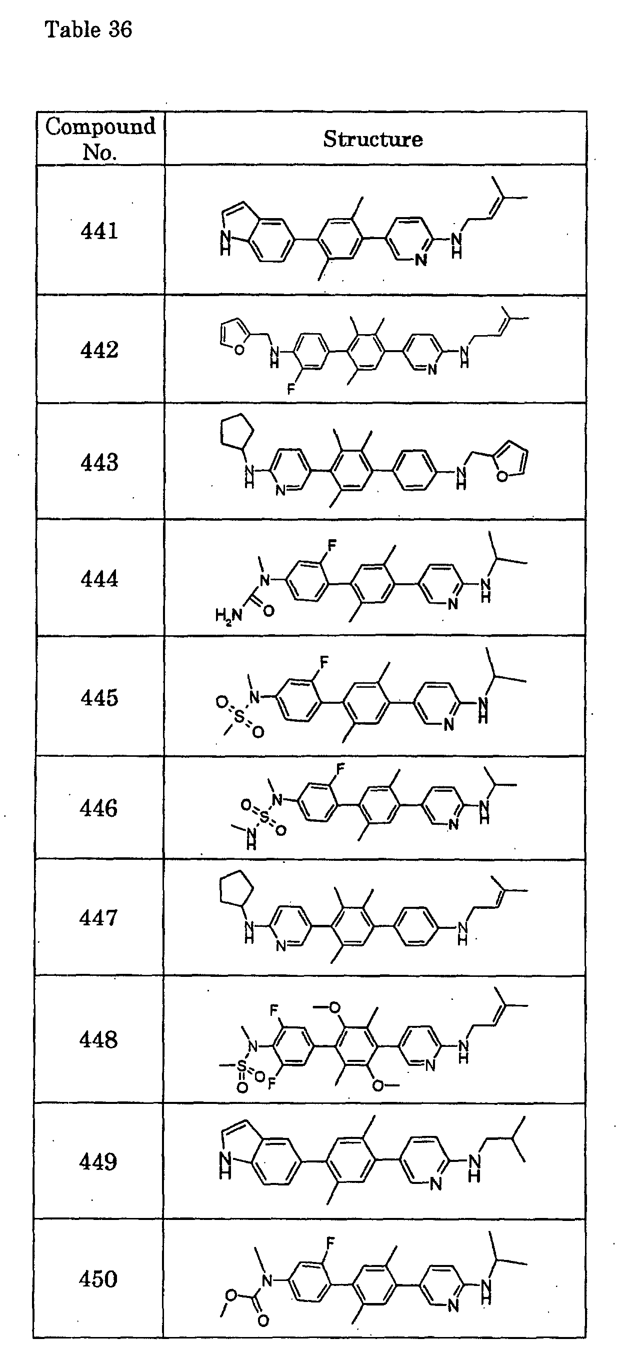

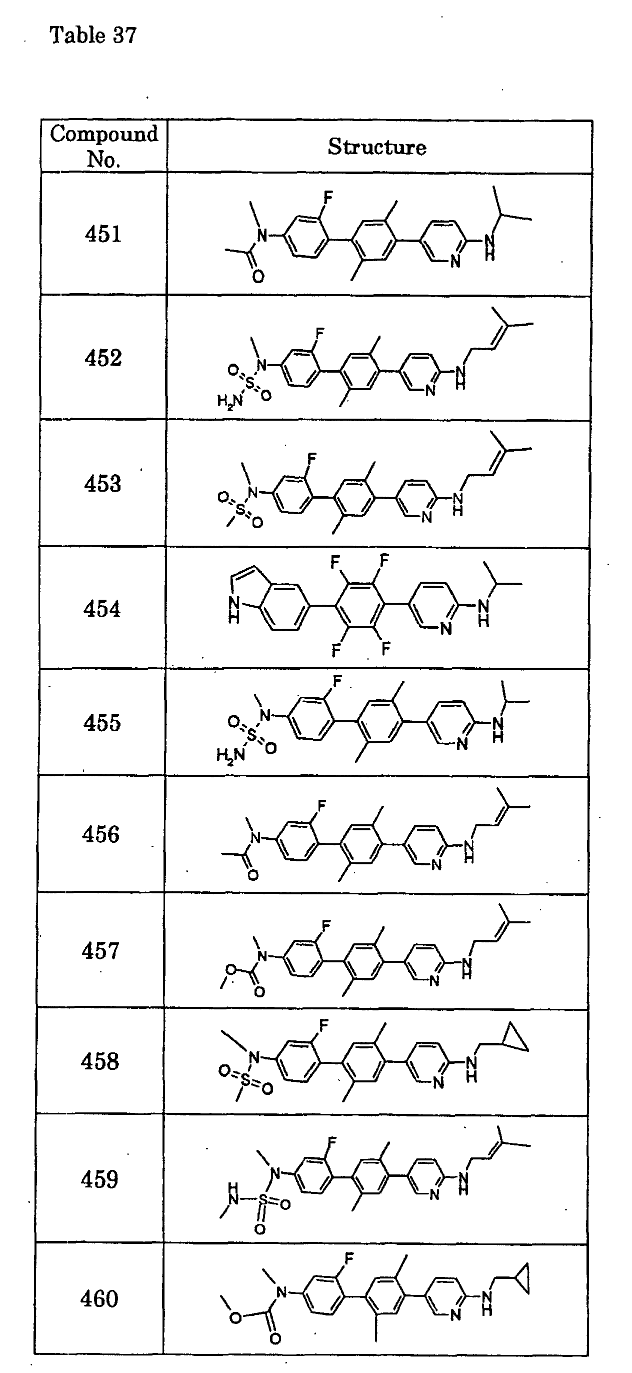

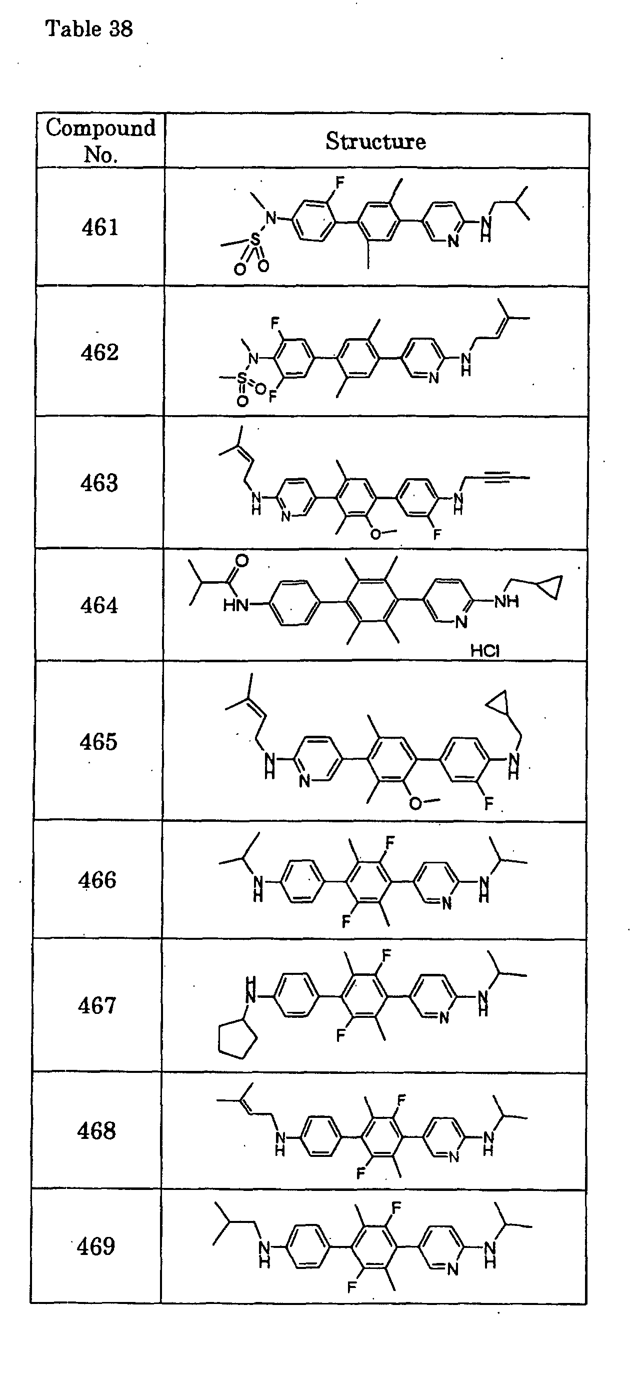

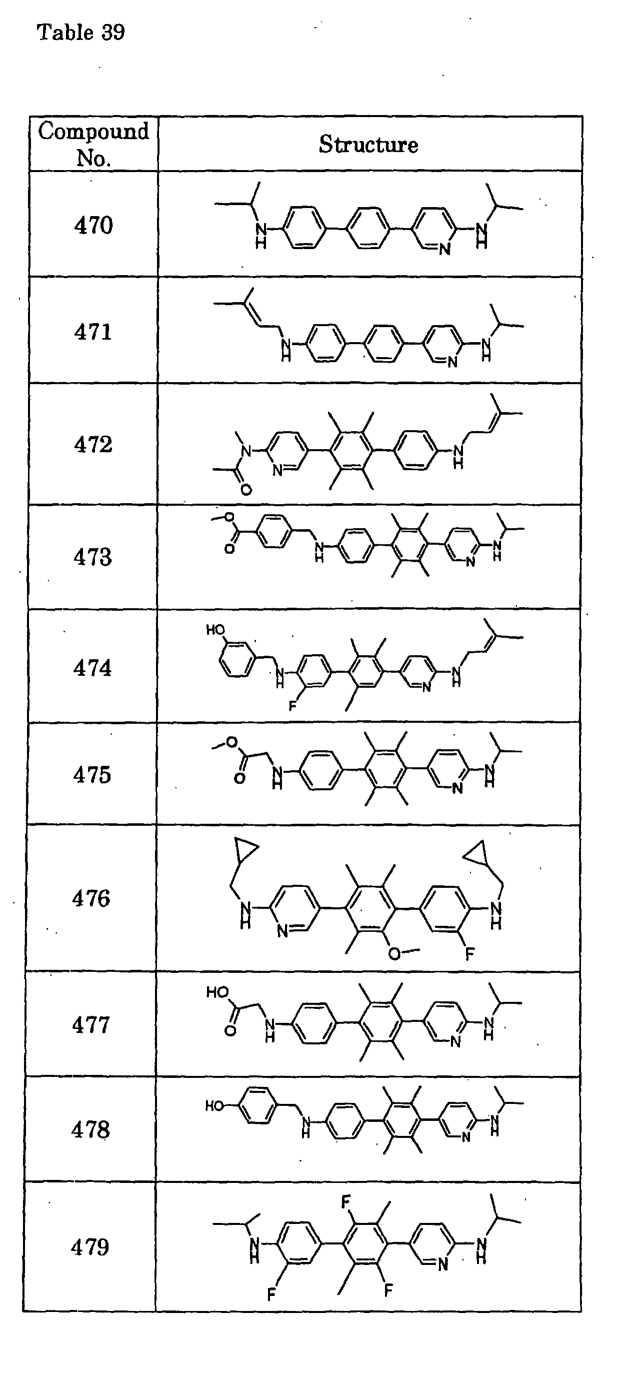

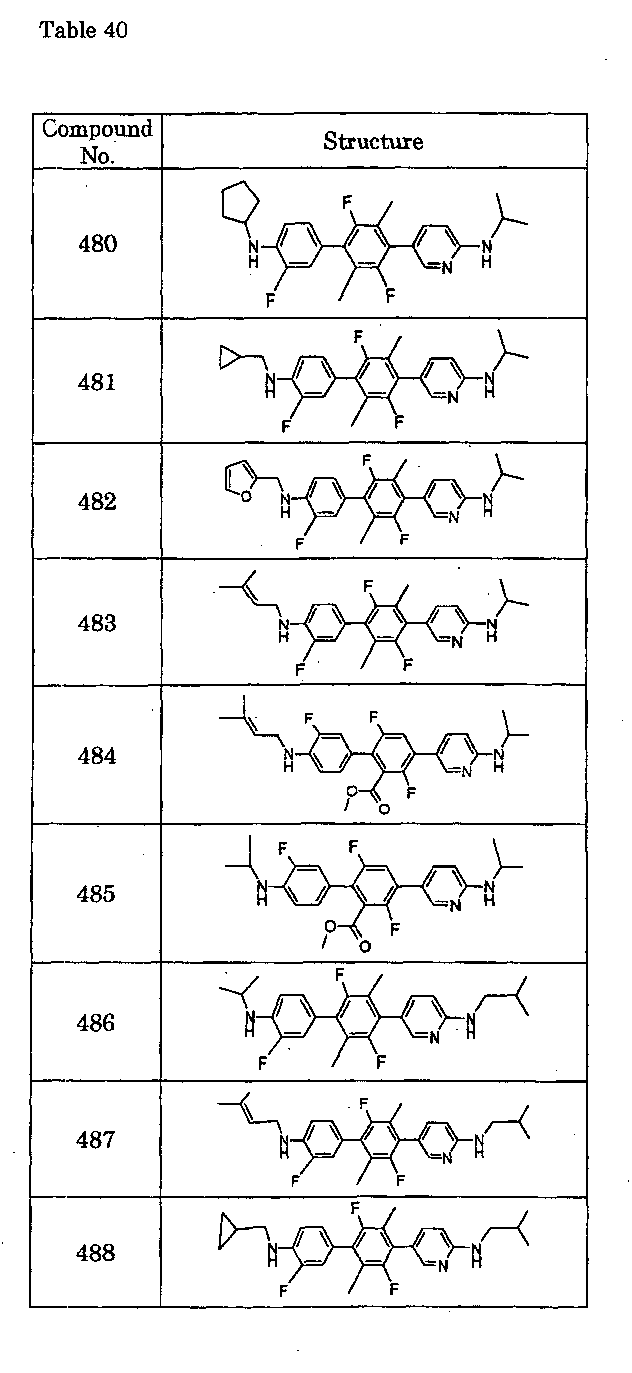

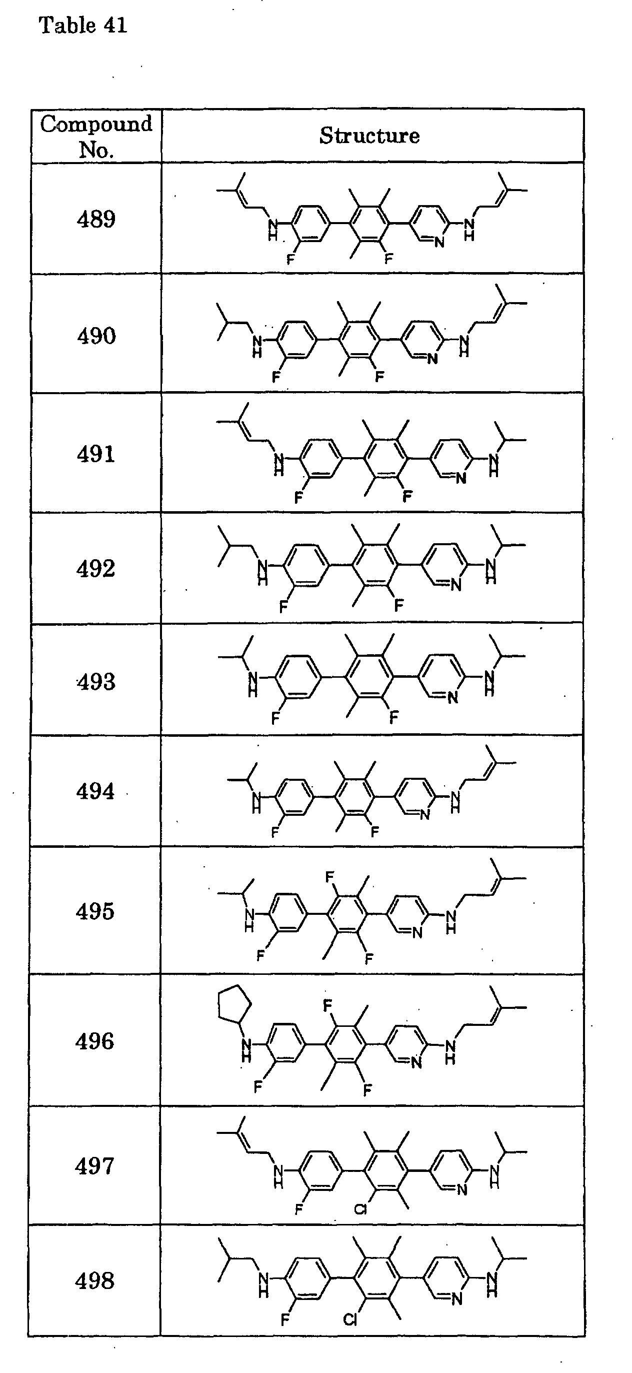

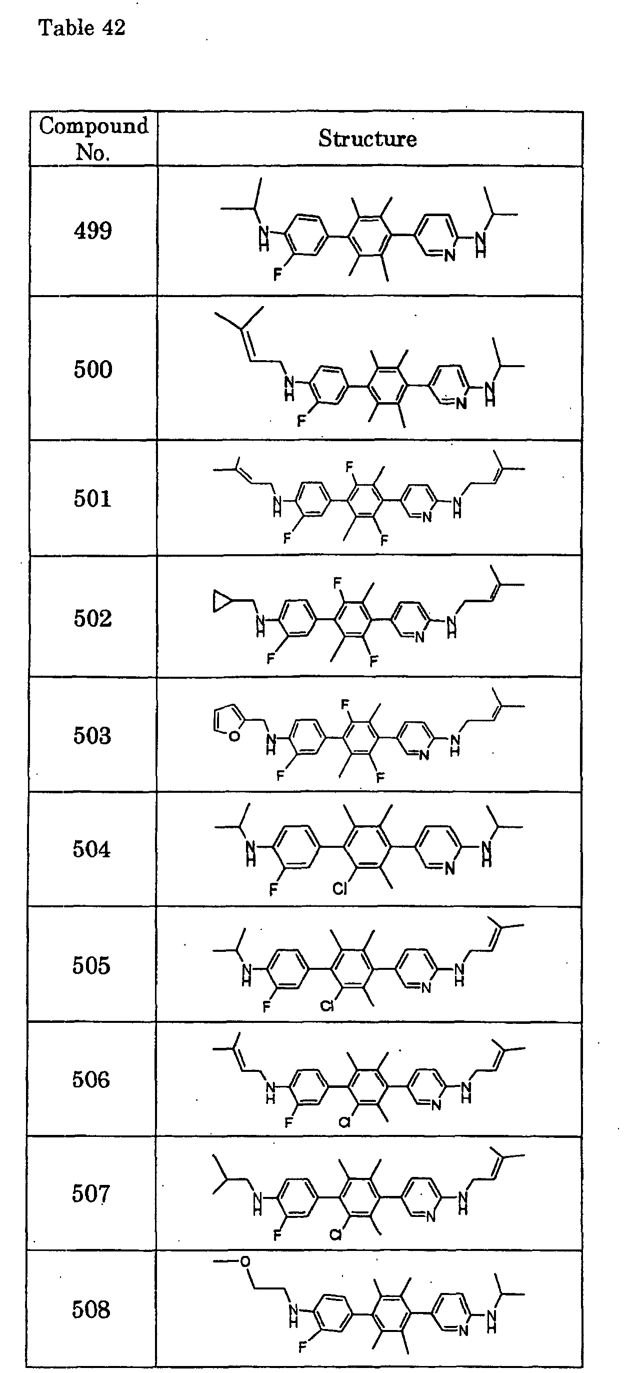

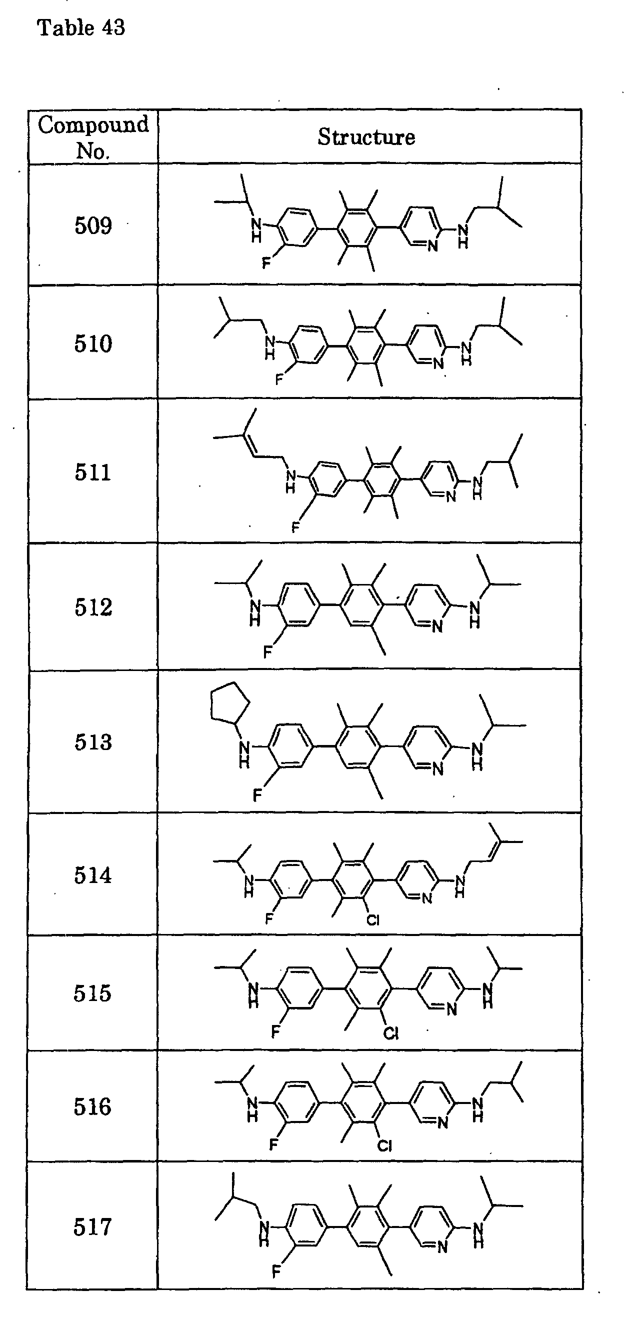

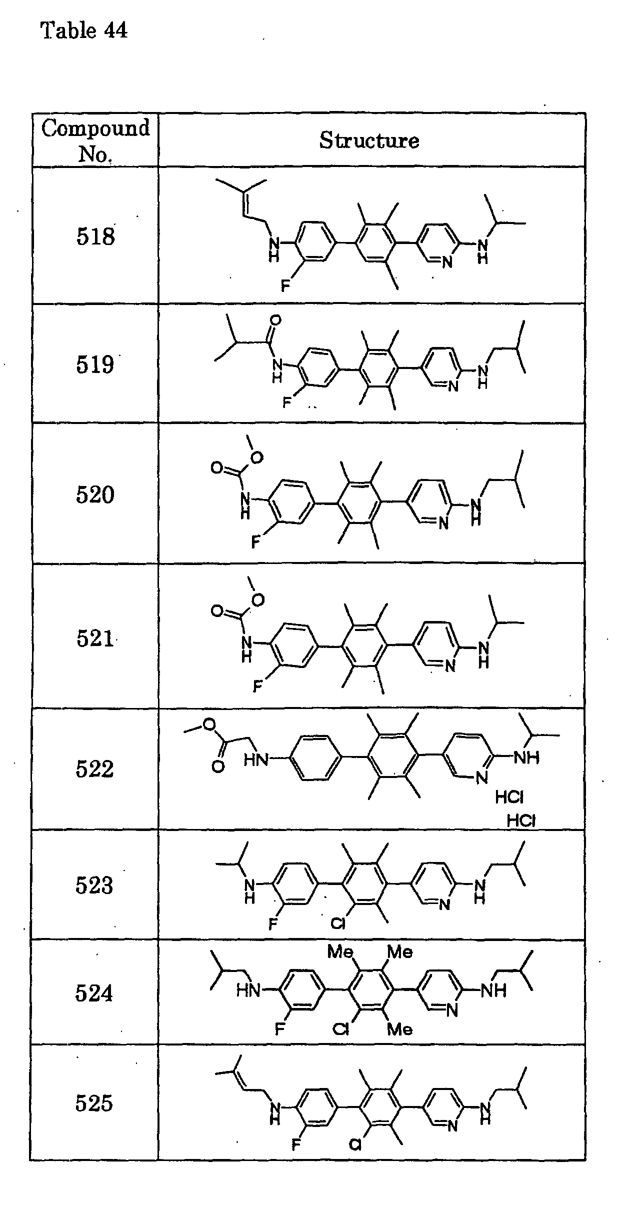

1: ¹H-NMR (CDCl₃)δ 1.033 (t,J=7.5Hz, 3H), 1.60-1.77 (m, 2H), 2.20 (s, 3H), 2.28 (s, 3H), 3.20-3.32 (m, 2H), 3.81 (s, 2H), 4.76 (s, 1H), 6.43-6.55 (m, 3H), 7.02-7.07 (m, 1H), 7.10 (s, 2H), 7.49 (dd, J=8.4, 2.4Hz, 1H), 8.09 (d, J=1.8Hz, 1H)
2: ¹H-NMR (CDCl₃)δ 1.03 (t, J=7.2Hz, 3H), 1.62-1.76 (m, 2H), 1:74 (s, 3H), 1.77 (s, 3H), 2.21 (s, 3H), 2.28 (s, 3H), 3.23-3.32 (m, 2H), 3.71 (d, J=6.3Hz, 2H), 4.62 (m, 1H), 5.35 (m, 1H), 6.34-6.48 (m, 3H), 7.06 (t, J=8.7Hz, 1H), 7.10 (s, 2H), 7.48 (dd, J=8.4, 2.1Hz, 1H), 8.11 (d, J=2.1H 1H)
3: ¹H-NMR (CDCl₃)δ 1.03 (t, J=7.5Hz, 3H), 1.25 (d, J=6.0Hz, 6H), 1.61-1.76 (m, 2H), 2.21 (s, 3H), 2.28 (s, 3H), 3.22-3.31 (m, 2H), 3.56-3.70 (m, 1H), 6.30-6.49 (m, 3H), 7.01-7.07 (m, 1H), 7.10 (s, 1H), 7.11 (s, 1H), 7.48 (dd, J=8.4, 2.1H 1H), 8.10 (d, J=1.5Hz, 1H)
4: ¹H-NMR (CDCl₃)δ 1.03 (t, J=7.5Hz, 3H), 1.44-1.80 (m, 8H), 1.97-2.12 (m, 2H), 2.21 (s, 3H), 2.28 (s, 3H), 3.21-3.32 (m, 2H), 3.71-3.90 (m, 2H), 4.66 (brs, 1H), 6.30-6.52 (m, 3H), 7.04 (t, J=8.1Hz, 1H), 7.10 (s, 1H), 7.11 (s, 1H), 7.48 (dd, J=8.4, 2.7Hz, 1H), 8.11 (s, 1H)
5: ¹H-NMR (CDCl₃)δ 0.99-1.07 (m, 6H), 1.60-1.76 (m, 4H), 2.21 (s, 3H), 2.78 (s, 3H), 3.06-3.15 (m, 2H), 3.23-3.32 (m, 2H), 3.82 (brs, 1H), 4.62 (brs, 1H), 6.34-6.48 (m, 3H), 7:05 (t, J=8.7Hz, 1H), 7.10 (s, 1H), 7.11 (s, 1H), 7.47 (dd, J=8.7, 2.4Hz, 1H), 8.11 (d, J=2.4Hz)
6: ¹H-NMR (CDCl₃)δ 1.04 (t, J=7.5Hz, 3H), 1.65-1.74 (m, 2H), 1.97 (s, 3H), 1.98 (s, 3H), 2.00 (s, 3H), 3.29 (dt, J=6.0, 6.8Hz, 2H), 3.80 (brs, 2H), 4.68 (brs, 1H), 4.87 (brs, 1H), 6.49 (d, J=8.4Hz, 1H), 6.82 (d, J=8.4Hz, 2H), 7.10 (d, J=6.9Hz, 2H), 7.26-7.31 (m, 1H), 7.90 (d, J=2.1Hz, 1H)
7: ¹H-NMR (CDCl₃)δ 1.03 (t, J=7.5Hz, 3H), 1.26 (d, J=6.3Hz, 6H), .1.64-1.74 (m, 2H), 2.10 (s, 3H), 3.22-3.31 (m, 2H), 3.44 (s, 3H), 3.62 (s, 3H), 3.69 (m, 1H), 4.62 (m, 1H), 6.45 (d, J=8.1Hz, 1H), 6.65 (d, J=8.4Hz, 2H), 7.30 (dd, J=8.4, 2.4Hz, 1H), 7.44 (d, J=8.4Hz, 2H), 7.71 (s, 1H), 7.86 (d, J=1.8Hz, 1H)
8: ¹H-NMR (CDCl₃)δ 1.04 (t, J=7.5Hz, 3H), 1.27 (t, J=5.7Hz, 6H), 1.64-1.74 (m, 2H), 1.97 (s, 3H), 1.98 (s, 3H), 2.02 (s, 3H), 3.28 (dt, J=6.0, 6.5Hz, 2H), 3.64-3.74 (m, 2H), 4.62 (brs, 1H), 4.95 (brs, 1H), 6.49 (d, J=8.7Hz, 1H), 6.71 (d, J=8.4Hz, 2H), 7.09 (d, J=7.2Hz, 2H), 7.25-7.30 (m, 1H), 7.91 (d, J=1.8Hz, 1H)
9: ¹H-NMR (DMSO-d₆)δ 1.16 (s, 3H), 1.19 (s, 3H), 1.93 (s, 3H), 1.98 (s, 3H), 3.04 (s, 3H), 3.23 (s, 3H), 3.27 (s, 3H), 4.01 (m, 1H), 6.37-6.52 (m, 2H), 7.22-7.30 (m, 4H), 7.86 (d, J=2.1Hz, 1H), 9.83 (brs, 1H).
10: ¹H-NMR (DMSO-d₆)δ 1.14-1.22 (m, 9H), 1.90 (s, 3H), 1.96 (s, 3H), 2.48 (s, 3H), 3.08-3.31 (m, 5H), 4.02 (m, 1H), 6.40 (m, 2H), 7.22 (m, 4H) , 7.83 (d, J=2.1Hz 1H), 9.84 (brs, 1H).
11: ¹H-NMR (DMSO-d₆)δ 1.14 (s, 3H), 1.16 (s, 3H), 1.25 (s, 3H), 1.27 (s, 3H), 1.90 (s, 3H), 1.96 (s, 3H), 3.20 (s, 3H), 3.25 (s, 3H), 4.02 (m, 1H), 6.40 (m, 2H), 7.22 (m, 4H) , 7.83 (d, J=1.8Hz, 1H), 9.82 (brs, 1H).
12: ¹H-NMR (CDCl₃)δ 1.25-1.30 (m, 12H), 2.07 (s, 3H), 2.09 (s, 3H), 3.32 (s, 3H), 3.36 (s, 3H), 3.69 (m, 1H), 3.90 (m, 1H), 4.51 (m, 1H), 6.46 (d, J=8.4Hz, 1H) , 6.66 (d, J=8.4Hz, 2H), 7.10 (d, J=8.1Hz 2H), 7.43 (d, J=7.5Hz, 1H), 8.04 (s, 1H)
13: ¹H-NMR (CDCl₃)δ 1.29 (d, J=6.3Hz, 6H), 1.98 (s, 6H), 2.01 (s, 6H), 3.69 (brs, 2H), 3.81-4.00 (m, 1H), 4.41 (d, J=8.7Hz, 1H), 6.47 (dd, J=8.7, 0.8Hz, 1H), 6.74-6.80 (m, 2H), 6.92-6.98 (m, 2H), 7.27 (dd, J=8.7, 2.0Hz, 1H), 7.91 (dd, J=2.0, 0.8Hz, 1H)
14: ¹H-NMR (CDCl₃)δ 1.29 (d, J=6.3Hz, 6H), 1.74 (s, 3H), 1.78 (s, 3H), 1.99 (s, 6H), 2.01 (s, 6H), 3.74 (d, J=6.6Hz, 2H), 3.85-3.98 (m, 1H), 4.42 (d, J=7.8Hz, 1H), 5.35-5.44 (m, 1H), 6.47 (dd, J=8.4, 0.9Hz, 1H), 6.67-6.72 (m, 2H), 6.93-7.00 (m, 2H), 7.28 (dd, J=8.4, 2.4Hz, 1H), 7.91 (dd, J=2.4, 0.9Hz, 1H)
15: ¹H-NMR (CDCl₃)δ 1.26 (d, J=6.3Hz, 6H), 1.30 (d, J=6.3Hz, 6H), 2.00 (s, 6H), 2.01 (s, 6H), 3.68 (sept, J=6.3Hz, 1H), 3.85-3.98 (m, 1H), 4.44-4.55 (m, 1H), 6.47 (d, J=8.7Hz, 1H), 6.62-6.70 (m, 2H), 6.91-6.98 (m, 2H), 7.28 (dd, J=8.7, 2.3Hz, 1H), 7.91 (d, J=2.3Hz, 1H)
16: ¹H-NMR (CDCl₃)δ 1.29 (d, J=6.3Hz, 6H), 1.47-1.84 (m, 6H), 2.00 (s, 6H), 2.01 (s, 6H), 2.00-2.12 (m, 2H), 3.78-3.90 (m, 1H), 3.84-3.99 (m, 1H), 4.44-4.54 (m, 1H), 6.47 (d, J=8.6Hz, 1H), 6.63-6.71 (m, 2H), 6.91-6.98 (m, 2H), 7.28 (dd, J=8.6, 2.1H 1H), 7.90 (d, J=2.1Hz, 1H)
17: ¹H-NMR (CDCl₃)δ 1.30 (d, J=6.3Hz, 6H), 2.06 (s, 3H), 2.10 (s, 3H), 3.35 (s, 3H), 3.36 (s, 3H), 3.86 (s, 3H), 3.84.3.93 (m, 1H), 4.72 (brs, 1H), 4.91 (brs, 2H), 6.50 (d, J=9.0Hz, 1H). 6.71-6.80 (m, 3H), 7.46 (dd, J=8.4, 2.1Hz, 1H), 8.01 (d, J=1.8Hz, 1H)
18: ¹H-NMR (CDCl₃)δ 1.26 (d, J=6.3Hz, 6H), 1.30 (d, J=6.6Hz, 6H), 1.96 (s, 3H), 1.97 (s, 3H), 2.01 (s, 3H), 3.20 (brs, 2H), 3.68 (sept, J=6.3Hz, 1H), 3.82-3.96 (m, 1H), 5.07 (brs, 1H), 6.53 (d, J=8.6Hz, 1H), 6.61-6.69 (m, 2H), 6.90-6.98 (m, 2H), 7.41 (dd, J=8.6, 2.0Hz, 1H), 7.99 (d, J=2.0Hz, 1H)
19: ¹H-NMR (CDCl₃)δ 1.25 (s, 3H), 1.27 (s, 6H), 1.29 (s, 3H), 2.05 (s, 6H), 2.20 (s,3H), 3.64-3.72 (m,1H), 3:86-3.98 (m, 1H), 4.48 (brs, 2H), 6.43 (d, J=8.4Hz, 1H), 6.66 (d, J=8.4Hz, 2H), 6.96 (d, J=8.4Hz, 2H), 6.98 (s, 1H), 7.45 (dd, J=8.4, 2.4Hz, 1H), 8.09 (d, J=2.4Hz, 1H)
20: ¹H-NMR (CDCl₃)δ 0.27-0.30 (m, 2H), 0.56-0.62 (m, 2H), 1.10-1.20 (m, 1H), 1.28 (s, 3H), 1.30 (s, 3H), 2.04 (s, 6H), 2.20 (s,3H), 3.02 (d, J=6.9Hz, 2H), 3.88-3.95 (m,1H), 4.65 (brs, 2H), 6.45 (d, J=9.0Hz, 1H), 6.69 (d, J=9.0Hz, 2H), 6.97 (d, J=9.0Hz, 2H), 6.98 (s, 1H), 7.46 (dd, J=9.0, 2.4Hz, 1H), 8.07 (d, J=2.4Hz, 1H)
21: ¹H-NMR (CDCl₃)δ 1.27 (s, 3H), 1.29 (s, 3H), 1.46-1.81 (m, 8H), 2.05 (s, 6H), 2.20 (s,3H), 3.79-3.89 (m,1H), 3.87-4.00 (m, 1H), 4.49 (brs, 1H), 6.44 (d, J=8.7Hz, 1H), 6.67 (d, J=8.7, 2H), 6.95 (d, J=8.7, 2H), 6.98 (s, 1H),.7.45 (dd, J=8.7, 2.4, 1H), 8.08 (d, J=2.4, 1H)
22: ¹H-NMR (CDCl₃)δ 1.27 (s, 3H), 1.29 (s, 3H), 1.74 (s, 3H), 1.78 (s, 3H), 2.05 (s, 6H), 2.20 (s,3H), 3.74 (d, J=6.6, 2H), 3.87-3.95 (m,1H), 4.50 (brs, 1H), 5.39 (t, J=6.6, 1H), 6.44 (d, J=8.7, 2H), 6.97 (d, J=8.7, 2H), 6.98 (s, 1H), 7.45 (dd, J=8.7, 2.4, 1H), 8.08 (d, J=2.4, 1H)
23: ¹H-NMR (CDCl₃)δ 1.29 (d, J=6.0Hz, 6H), 1.29 (d, J=6.3Hz, 6H), 2.08 (s, 3H), 2.10 (s, 3H), 3.35 (s, 3H), 3.37 (s, 3H), 3.63-3.72 (m, 1H), 3.84 (s, 3H), 3.85-3.98 (m, 1H), 4.08 (brs, 1H), 4.54 (brs, 1H), 6.47 (d, J=8.4Hz, 1H), 6.66-6.80 (m, 3H), 7.44 (dd, J=8.7, 2.1Hz, 1H), 8.04 (d, J=2.1Hz, 1H)
24: ¹H-NMR (CDCl₃)δ 0.28-0.31 (m, 2H), 0.58-0.61 (m, 2H), 1.16-1.21 (m, 1H), 1.29 (d, J=6.6Hz, 6H), 2.07 (s, 3H), 2.10 (s, 3H), 3.03 (d, J=6.9Hz, 2H), 3.35 (s, 3H), 3.36 (s, 3H), 3.85-3.95 (m, 1H), 3.87 (s, 3H), 4.37 (brs, 1H), 4.54 (brs, 1H), 6.47 (d, J=8.7Hz, 1H), 6.64 (d, J=7.8Hz, 1H), 6.74-6.81 (m, 2H), 7.43 (dd, J=8.7, 2.3Hz, 1H), 8.04 (dd, J=1.8, 0.3Hz, 1H)
25: ¹H-NMR (CDCl₃)δ 1.26 (d, J=6.3Hz, 6H), 1.29 (d, J=6.6Hz, 6H), 1.99 (s, 6H), 2.09 (s, 3H), 3.35 (s, 3H), 3.68 (sept, J=6.3Hz, 1H), 3.86-3.98 (m, 1H), 4.43.4.51 (m, 1H), 6.46 (d, J=8.7Hz, 1H), 6.66 (d, J=8.4Hz, 2H), 6.94 (d, J=8.4Hz, 2H), 7.43 (dd, J=8.7, 2.1Hz, 1H), 8.04 (d, J=2.1Hz, 1H)
26: ¹H-NMR (CDCl₃)δ 1.28 (s, 3H), 1.31 (s, 3H), 2.05 (s, 3H), 2.09 (s, 3H), 3.23 (s, 3H), 3.36 (s, 3H), 3.72 (brs, 2H), 3.90 (m, 1H), 4.58 (m, 1H) , 6.47 (d, J=8.4Hz, 1H), 6.77 (d, J=8.7Hz, 2H), 7.10 (d, J=8.4Hz, 2H), 7.43 (dd, J=2.4, 8.4Hz, 1H), 8.03 (d, J=1.8Hz, 1H)
27: ¹H-NMR (CDCl₃)δ 0.97 (t, J=7.5Hz, 6H), 1.28 (s, 3H), 1.30 (s, 3H), 1.59 (m, 4H), 2.08 (s, 3H), 2.09 (s, 3H), 3.28 (m, 1H), 3.33 (s, 3H), 3.36 (s, 3H), 3.90 (m, 2H), 4.48 (m, 2H), 6.46 (d, J=8.4Hz, 1H), 6.63 (d, J=8.7Hz, 2H), 7.08 (d, J=8.7Hz, 2H), 7.42 (dd, J=2.1, 8.4Hz, 1H), 8.04 (d, J=1.8Hz, 1H)
28: ¹H-NMR (CDCl₃)δ 1.30 (d, J=6.0Hz, 6H), 1.97 (s, 3H), 1.98 (s, 3H), 2.02 (s, 3H), 3.83 (s, 3H), 3.84 (brs, 2H), 3.89-3.97 (m, 1H), 3.92 (brs, 1H), 4.61 (brs, 1H), 6.52 (d, J=8.7Hz, 1H), 6.59 (d, J=7.8Hz, 1H), 6.60 (s, 1H), 7.39 (d, J=8.4Hz, 1H), 8.03 (brs, 1H)
29: ¹H-NMR (CDCl₃)δ 1.30 (d, J=6.3Hz, 3H), 1.303 (d, J=6.3Hz, 3H), 1.94 (s, 3H), 1.95 (s, 3H), 2.01 (s, 3H), 3.76 (s, 2H), 3.87-3.96 (m, 1H), 4.89 (brs, 2H), 6.45-6.88 (m, 4H), 7.41 (d, J=8.7Hz, 1H), 7.99 (d, J=2.1Hz, 1H)
30: ¹H-NMR (CDCl₃)δ 1.28-1.31 (m, 12H), 1.95 (s, 3H), 1.97 (s, 3H), 2:01 (s, 3H), 3.66-3.74 (m, 1H), 3.72 (brs, 1H), 3.88-3.99 (m, 1H), 4.69 (brs, 1H), 4.78 (brs, 1H), 6.52 (d, J=9.0Hz, 1H), 6.75-6.81 (m, 3H), 7.39 (d, J=9.0Hz, 1H), 8.01 (d, J=1.5Hz, 1H)
31: ¹H-NMR (CDCl₃)δ 0.28-0.31 (m, 2H), 0.58-0.62 (m, 2H), 1.13.1.21 (m, 1H), 1.29 (d, J=6.3Hz, 6H), 1.95 (s, 3H), 1.96 (s, 3H), 2.01 (s, 3H), 3.04 (d, J=5.4Hz, 1H), 3.06 (d, J=5.4Hz, 1H), 3.88-3.98 (m, 1H), 4.05 (brs, 1H), 4.68 (brs, 1H), 4.83 (brs, 1H), 6.51 (d, J=8.7Hz, 1H), 6.71-6.83 (m, 3H), 7.39 (d, J=8.7Hz, 1H), 8.01 (d, J=1.8Hz, 1H)
32: ¹H-NMR (CDCl₃) δ 1.25 (s, 3H), 1.26 (s, 3H), 1.27 (s, 3H), 1.28 (s, 3H), 2.01 (s, 3H), 2.03 (s, 6H), 2.18 (s,3H), 3.64-3.72 (m, 1H), 3.73-3.84 (m, 1H), 6.28 (d, J=8.4, 1H), 6.66 (d, J=8.4, 2H), 6.85 (s, 1H), 6.96 (d, J=8.4, 2H), 7.27 (d, J=8.4, 1H)
33: ¹H-NMR (CDCl₃)δ 1.26 (s, 3H), 1.28 (s, 3H), 1.50-1.76 (s, 8H), 2.02 (s, 3H), 2.02 (s, 6H), 2.18 (s,3H), 3.70-3.89 (m, 2H), 6.29 (d, J=8.4, 1H), 6.67 (d, J=8.4, 2H), 6.85 (s, 1H), 6.97 (d, J=8.4, 2H), 7.27 (d, J=8.4, 1H)
34: ¹H-NMR (CDCl₃)δ 1.28 (d, J=6.6Hz, 6H), 2.20 (s, 3H), 2.28 (s, 3H), 3.90 (m, 1H), 4.83 (brs, 1H), 6.40-6.54 (m, 3H), 7.04 (t, J=8.4Hz, 1H), 7.10 (s, 2H), 7.48 (dd, J=8.7, 2.1Hz, 1H), 8.07 (d, J=1.8Hz, 1H)
35: ¹H-NMR (CDCl₃)δ 1.28 (d, J=6.3Hz, 6H), 1.74 (s, 3H), 1.77 (s, 3H), 2.12 (s 3H), 2.28 (s, 3H), 3.72 (d, J=6.3Hz, 2H), 3.92 (m, 1H), 4.45 (d, J=8.4Hz, 1H), 5.35 (m, 1H), 6.35-6.47 (m, 3H), 7.05 (t, J=8.4Hz, 1H), 7.10 (s, 2H), 7.46 (dd, J=8.4, 2.1Hz, 1H), 8.11 (d, J=1.5Hz, 1H)
36: ¹H-NMR (CDCl₃)δ 1.28 (d, J=6.6Hz, 6H), 2.20 (s, 3H), 2.28 (s, 3H), 3.92 (m, 1H), 4.47 (d, J=7.8Hz, 1H), 6.40-6.54 (m, 3H), 7.04 (t, J=8.1Hz, 1H), 7.10 (s, 2H), 7.46 (dd, J=8.7, 2.4Hz, 1H), 8.10 (m, 1H)
37: ¹H-NMR (CDCl₃)δ 1.03 (t, J=7.5Hz, 3H), 1.28 (d, J=6.3Hz, 6H), 1.56-1.75 (m, 2H), 2.21 (s, 3H), 2.28 (s, 3H), 3.04-3.16 (m, 2H), 3.82 (brs, 1H), 3.92 (m, 1H), 4.46 (d, J=8.4Hz, 1H), 6.34-6.46 (m, 3H), 7.05 (t, J=8.4Hz, 1H), 7.10 (s, 2H), 7.46 (dd, J=8.7, 2.4Hz, 1H), 8.11 (d, J=2.1Hz, 1H)
38: ¹H-NMR (CDCl₃)δ 1.25 (s, 3H), 1.26 (s, 3H), 1.27 (s, 3H), 1.28 (s, 3H), 2.01 (s, 3H), 2.02 (s, 6H), 2.04 (s,3H), 3.64-3.72 (m, 1H), 3.82-3.97 (m, 1H), 6.29 (s, 1H), 6.66 (d, J=9.0, 2H), 6.85 (s, 1H), 6.93-6.98 (m, 2H), 7.85 (s,, 1H)
39: ¹H-NMR (CDCl₃)δ 0.24-0.31 (m, 2H), 0.54-0.62 (m, 2H), 1.08-1.21 (m, 1H), 1.26 (s, 3H), 1.28 (s, 3H), 2.01 (s, 3H), 2.02 (s, 6H), 2.03 (s,3H), 3.02 (d, J=6.9, 2H), 3.82-3.95 (m, 1H), 4.35 (brs, 1H), 6.29 (s, 1H), 6.69 (d, J=8.4, 2H), 6.85 (s, 1H), 6.95-7.00 (m, 2H), 7.85 (s,, 1H)
40: ¹H-NMR (CDCl₃)δ 1.27 (s, 3H), 1.29 (s, 3H), 1.44-1.90 (m, 8H), 2.01 (s, 3H), 2.02 (s, 6H), 2.04 (s,3H), 3.80-3.92 (m, 2H), 4.46 (brs, 1H), 6.30 (s, 1H), 6.67 (d, J=8.7, 2H), 6.85 (s, 1H), 6.93-6.98 (m, 2H), 7.85 (s,, 1H)
41: ¹H-NMR (CDCl₃)δ 1.27 (s, 3H), 1.29 (s, 3H), 1.74 (s, 3H), 1.78 (s, 3H), 2.01 (s, 3H), 2.02 (s, 6H), 2.04 (s,3H), 3.74 (d, J=6.6, 2H), 3.83-3.96 (m, 1H), 4.51 (brs, 1H), 5.39 (t, J=6.6, 1H), 6.30 (s, 1H), 6.69 (d, J=8.4, 2H), 6.85 (s, 1H), 6.95-7.01 (m, 2H), 7.84 (s,, 1H)
42: ¹H-NMR (CDCl₃)δ 1.26 (s, 3H), 1.29 (s, 3H), 1.85 (s, 3H), 2.02 (s, 9H), 2.04 (s,3H), 3.82-3.96 (m, 3H), 4.40 (brs, 1H), 6.30 (s, 1H), 6.74 (d, J=8.4, 2H), 6.86 (s, 1H), 6.99-7.03 (m, 2H), 7.85 (s,, 1H)
43: ¹H-NMR (CDCl₃)δ 1.29 (d, J=6.3 Hz, 6H), 2.09 (s, 3H ), 3.47 (s, 3H), 3.58 (s, 3H), 3.92 (m, 1H), 4.66 (m, 1H), 6.46 (d, J=8.7Hz, 1H), 6.74 (d, J=8.7Hz, 2H), 6.96 (d, J=8.7Hz, 2H), 7.63 (s, 1H), 7.75 (dd, J=8.7, 2.4Hz, 1H), 8.33 (d, J=2.4Hz,1H)
44: ¹H-NMR (CDCl₃)δ 1.25 (d, J=6.0 Hz, 6H), 1.28 (d, J=6.6Hz, 6H), 2.11 (s, 3H ), 3.47 (s, 3H), 3.57 (s, 3H), 3.70 (m, 1H), 3.93 (m, 1H), 4.57 (d, J=8.1Hz, 1H), 6.45 (d, J=8.4Hz, 1H), 6.62 (d, J=8.7Hz, 2H), 6.97 (d, J=8.7Hz, 2H), 7.60 (s, 1H), 7.74 (dd, J=8.7, 2.1Hz, 1H), 8.33 (d, J=2.1Hz, 1H)
45: ¹H-NMR (CDCl₃)δ 1.03 (t, J=7.5 Hz, 3H), 1.28 (d, J=6.6Hz, 6H), 1.54-1.76 (m, 2H), 2.11 (s, 3H), 3.09-3.15 (m, 2H), 3.47 (s, 3H), 3.58 (s, 3H), 3.92 (m, 1H), 4.57 (d, J=8.1Hz, 1H), 6.45 (d, J=8.7Hz, 1H), 6.65 (d, J=8.7Hz, 2H), 6.98 (d, J=8.7Hz, 2H), 7.61 (s, 1H), 7.74 (dd, J=8.7, 2.4Hz, 1H), 8.33 (d, J=2.4Hz,1H)
46: ¹H-NMR (CDCl₃)δ 1.28 (d, J=6.6 Hz, 6H), 1.74 (s, 3H), 1.77 (s, 3H), 2.11 (s, 3H), 3.47 (s, 3H), 3.59 (s, 3H),3.73 (d, J=6.6Hz, 2H), 3.92 (m, 1H), 4.58 (d, J=7.5Hz, 1H), 5.37 (m, 1H), 6.45 (d, J=8.1Hz, 1H), 6.65 (d, J=8.4Hz, 2H), 6.98 (d, J=8.4Hz, 2H), 7.61 (s, 1H), 7.74 (dd, J=8.7, 2.4Hz, 1H), 8.33 (d, J=2.4Hz, 1H)
47: ¹H-NMR (CDCl₃)δ 1.02 (d, J=6.6 Hz, 6H), 1.28 (d, J=6.6Hz, 6H), 1.90 (m, 1H), 2.11 (s, 3H), 2.97 (d, J=6.6Hz, 2H), 3.47 (s, 3H), 3.58 (s, 3H), 3.92 (m, 1H), 4.60 (d, J=7.2Hz, 1H), 6.45 (d, J=8.1H 1H), 6.64 (d, J=8.4Hz, 2H), 6.97 (d, J=8.9Hz, 2H), 7.60 (s, 1H), 7.75 (dd, J=8.4, 2.4Hz, 1H), 8.32 (d, J=2.4Hz, 1H)
48: ¹H-NMR (CDCl₃)δ 0.23-0.30 (m, 2H), 0.54-0.62 (m, 2H), 1.13 (m, 1H), 1.28 (d, J=6.6Hz, 6H), 2.10 (s, 3H), 3.00 (d, J=7.2Hz, 2H), 3.47 (s, 3H), 3.58 (s, 3H), 3.92 (m, 1H), 4.58 (m, 1H), 6.45 (d, J=8.7Hz, 1H), 6.65 (d, J=8.7Hz, 2H), 6.98 (d, J=8.7Hz, 2H), 7.61 (s, 1H), 7.74 (dd, J=8.7, 2.4Hz, 1H), 8.33 (d, J=2.4Hz, 1H)
49: ¹H-NMR (CDCl₃)δ 1.26 (d, J=6.6Hz, 6H), 1.28 (d, J=6.6Hz, 6H), 2.11 (s, 3H), 3.44 (s, 3H), 3.62 (s, 3H), 3.69 (m, 1H), 3.91 (m, 1H), 4.50 (m, 1H), 6.43 (d, J=8.1H 1H), 6.65 (d, J=8.7Hz, 2H), 7.30 (dd, J=8.4, 2.4Hz, 1H), 7.44 (d, J=8.7Hz, 2H), 7.70 (s, 1H), 7.86 (d, J=1.8Hz, 1H)
50: ¹H-NMR (CDCl₃)δ 1.25 (d, J=6.3Hz, 6H), 1.28 (d, J=6.6Hz, 6H), 3.69 (sept, J=6.3Hz, 1H), 3.87-4.02 (m, 1H), 4.66 (d, J=8.7Hz, 1H), 6.47 (d, J=9.0Hz, 1H), 6.63-6.69 (m, 2H), 7.28-7.36 (m, 2H), 7.54-7.61 (m, 1H), 8.22-8.27 (m, 1H)
51: ¹H-NMR (CDCl₃)δ 1.29(d, J=6.3Hz, 6H), 2.11(s, 3H), 3.43(s, 3H), 3.91(m, 1H), 4.68(brs, 1H), 6.45(d, J=8.4Hz, 1H), 6.77(d, J=8.7Hz, 1H), 7.32(dd, J=8.4, 2.1Hz, 1H), 7.43(d, J=8.4Hz, 2H), 7.70(s, 1H), 7.84(d, J=1.5Hz, 1H)
52: ¹H-NMR (CDCl₃)δ 1.28(d, J=6.3Hz, 6H), 1.74(s, 3H), 1:77(s, 3H), 2.11(s, 3H), 3.44(s, 3H), 3.63(s, 3H), 3.74(d, J=6.6Hz, 2H), 3.91(m, 1H), 4.63(m, 1H), 5.37(m, 1H), 6.45(d, J=8.1H 1H), 6.68(d, J=8.7Hz, 2H), 7.31(dd, J=8.7, 2.4Hz, 1H), 7.46(d, J=8.4Hz, 2H), 7.71(s, 1H), 7.85(d, J=2.1Hz, 1H)
53: ¹H-NMR (CDCl₃)δ 1.28(d, J=6.3Hz, 6H), 1.45-1.81(m, 6H), 2.00-2.13(m, 2H), 2.11(s, 3H), 3.44(s, 3H), 3.62(s, 3H), 3.79-3.98(m, 2H), 4.52(m, 1H), 6.44(d, J=8.4Hz, 1H), 6.67(d, J=8.7Hz, 1H), 7.30(dd, J=8.7, 2.4Hz, 1H), 7.44(d, J=8.4Hz, 2H), 7.71(s, 1H), 7.86(d, J=2.1Hz, 1H)
54: ¹H-NMR (CDCl₃)δ 0.24-0.31(m,2H), 0.55-0.62(m,2H), 1.14(m,1H), 1.28(d, J=6.3Hz, 6H), 2.11(s, 3H), 3.02(d, J=6.9Hz, 2H), 3.44(s,3H), 3.62(s, 3H), 3.91(m, 1H), 4.54(m, 1H), 6.44(d, J=8.4Hz, 1H), 6.69(d, J=8.7Hz, 1H), 7.30(dd, J=8.7, 2.7Hz, 1H), 7.47(d, J=8.4Hz, 2H), 7.70(s, 1H), 7.86(d, J=1.5Hz, 1H)
55: ¹H-NMR (CDCl₃)δ 1.02(d, J=6.6Hz, 6H), 1.28(d, J=6.6Hz, 6H), 1.93(m, 1H), 2.11(s, 3H), 2.99(d, J=6.9Hz, 2H), 3.44(s, 3H), 3.62(s, 3H), 3.91(m, 1H), 4.50(m, 1H), 6.44(d, J=8.7Hz, 1H), 6.67(d, J=8.4Hz, 2H), 7.30(dd, J=8.4, 5.1Hz, 1H), 7.45(d, J=8.7Hz, 2H), 7.70(s, 1H), 7.86(s, 1H)
56: ¹H-NMR (CDCl₃)δ 1.03(t, J=7.5Hz, 3H), 1.28(d, J=6.3Hz, 6H), 1.61-1.75(m, 2H), 2.11(s, 3H), 3.11-3.17(m, 2H), 3.44(s, 3H), 3.62(s, 3H), 3.91(m, 1H), 4.55(m, 1H), 6.44(d, J=8.4Hz, 1H), 6.68(d, J=8.7Hz, 2H), 7.30(dd, J=8.7, 2.7Hz, 1H), 7.45(dd, J=8.4, 1.6Hz, 2H), 7.71(s, 1H), 7.86(d, J=1.5Hz, 1H)
57: ¹H-NMR (CDCl₃)δ 1.29(d, J=6.3Hz, 6H), 2.09(s, 3H), 3.48(s, 3H), 3.64(s, 3H), 3.92(m, 1H), 4.83(m, 1H), 6.44-6.53(m, 3H), 6.86(t, J=8.1Hz, 1H), 7.76(d, J=2.1Hz, 1H), 7.79(d, J=2.4Hz, 1H), 8.32(d, J=1.5Hz, 1H)
58: ¹H-NMR (CDCl₃)δ 1.25(d, J=6.3Hz, 6H), 1.30(d, J=6.3Hz, 6H), 2.11(s, 3H), 3.48(s, 3H), 3.64(s, 3H), 3.92(m, 1H), 4.90(m, 1H), 6.31-6.40(m, 2H), 6.48(d, J=9.0Hz, 1H), 6.85(t, J=8.4Hz, 1H), 7.73(s, 1H), 7.78(dd, J=8.7, 2.1Hz, 1H), 8.32(d, J=2.1 1H)
59: ¹H-NMR (CDCl₃)δ 1.28(d, J=6.3Hz, 6H), 1.74(s, 3H), 1.78(s, 3H), 2.11(s, 3H), 3.48(s, 3H), 3.65(s, 3H), 3.71(d, J=6.3Hz, 2H), 3.93(m, 1H), 4.56(d, J=7.5Hz, 1H), 5.35(m, 1H), 6.32-6.49(m, 3H), 6.87(t, J=8.4Hz, 1H), 7.50-7.79(m, 2H), 8.34(d, J=2.1H 1H)
60: ¹H-NMR (CDCl₃)δ 1.02(d, J=6.6Hz, 6H), 1.29(d, J=6.3Hz, 6H), 1.92(m, 1H), 2.11(s, 3H), 2.96(brs, 2H), 3.48(s, 3H), 3.65(s, 3H), 3.80-4.00(m, 2H), 4.58(d, J=7.8Hz, 1H), 6.32-6.48(m, 3H), 6.86(t, J=8.7Hz, 1H), 7.74(s, 1H), 7.77(d, J=2.1H 1H), 8.34(d, J=1.5Hz, 1H)
61: ¹H-NMR (CDCl₃)δ 1.28(d, J=6Hz, 6H), 1.45-1.81(m, 6H), 2.00-2.13(m, 2H), 2.11(s, 3H), 3.48(s, 3H), 3.64(s, 3H), 3.71-4.00(m, 2H), 4.56(d, J=7.5Hz, 1H), 6.33-6.50(m, 3H), 6.78-6.93(m, 1H), 7.67-7.79(m, 1H), 7.73(s, 1H), 8.34(s, 1H)
62: ¹H-NMR (CDCl₃)δ 1.30 (d, J=6.3Hz, 6H), 1.97 (s, 6H), 2.03 (s, 6H), 3.85-3.99 (m, 1H), 4.40 (d, J=7.2Hz, 1H), 6.48 (d, J=8.6Hz, 1H), 6.55-6.61 (m, 1H), 6.97.7.04 (m, 1H), 7.26-7.28 (m, 1H), 7.30 (dd, J=8.6, 2.4Hz, 1H), 7.40-7.49 (m, 2H), 7.94 (d, J=2.4Hz, 1H), 8.24 (s, 1H)
63: ¹H-NMR (CDCl₃) δ 1.28 (d, J=6.3Hz, 6H), 3.88 (s, 2H), 3.88-4.02 (m, 1H), 4.62 (d, J=7.8Hz, 1H), 6.47 (dd, J=9.0, 0.6Hz, 1H), 6.75-6.82 (m, 2H), 7.28-7.35 (m, 2H), 7.54-7.61 (m, 1H), 8.26 (d, J=1.5Hz, 1H)
64: ¹H-NMR (CDCl₃)δ 1.04 (t, J=7.2Hz, 3H), 1.29 (d, J=6.6Hz, 6H), 1.69 (sextet, J=7.2Hz, 2H), 1.99 (s, 6H), 2.01 (s, 6H), 3.14 (t, J=7.2Hz, 2H), 3.64 (brs, 1H), 3.85-4.04 (m, 1H), 4.39 (d, J=8.4Hz, 1H), 6.46 (dd, J=8.7, 0.8Hz, 1H), 6.65-6.71 (m, 2H), 6.93-6.99 (m, 2H), 7.27 (dd, J=8.7, 2.1Hz, 1H), 7.91 (dd, J=2.1, 0.8Hz, 1H)
65: ¹H-NMR (CDCl₃)δ 1.02(d J=6.3Hz, 6H), 1.26(d, J=6.3Hz, 6H), 1.93(m, s), 2.10(s, 3H), 3.09-3.16(m, 2H), 3.44(s, 3H), 3.62(s, 3H), 3.69(m, 1H), 4.66(m, 1H), 6.45(d, J=8.1H 1H), 6.65(d, J=8.4Hz, 2H), 7.30(dd, J=8.4, 2.4Hz, 1H), 7.44(d, J=8.4Hz, 2H), 7.71(s, 1H), 7.86(d, J=1.8Hz, 1H)
66: ¹H-NMR (CDCl₃)δ 0.27-0.32 (m, 2H), 0.56-0.62 (m, 2H), 1.10-1.18 (m, 1H), 2.05 (s, 3H), 2.08 (s, 3H), 3.18 (d, J=5.7Hz, 1H), 3.20 (d, J=6.3Hz, 1H), 3.32 (s, 3H), 3.35 (s, 3H), 3.71 (brs, 2H), 4.81 (brs, 1H), 6.50 (d, J=8.7Hz, 1H), 6.77 (d, J=8.4Hz, 2H), 7.10 (d, J=8.1Hz, 2H), 7.44 (d, J=8.7Hz, 1H), 8.04 (d, J=2.1Hz, 1H)
67: ¹H-NMR (CDCl₃)δ 0.27-0.32 (m, 2H), 0.56-0.61 (m, 2H), 1.10-1.17 (m, 1H), 1.25-1.27 (m, 6H), 2.07 (s, 3H), 2.09 (s, 3H), 3.18 (d, J=5.1 1H), 3.20 (d, J=5.4Hz, 1H), 3.33 (s, 3H), 3.35 (s, 3H), 3.64-3.73 (m, 1H), 4.75 (brs, 1H), 6.49 (d, J=8.4Hz, 1H), 6.66 (d, J=8.4Hz, 2H), 7.10 (d, J=8.7Hz, 2H), 7.44 (d, J=8.7Hz, 1H), 8.05 (s, 1H)
68: ¹H-NMR (CDCl₃)δ 0.27-0.32 (m, 2H), 0.56-0.61 (m, 2H), 1.11-1.18 (m, 1H), 1.50-1.80 (m, 6H), 2.00-2.14 (m, 2H), 2.07 (s, 3H), 2.08 (s, 3H), 3.17 (d, J=5.1Hz, 1H), 3.20 (d, J=5.1Hz, 1H), 3.32 (s, 3H), 3.35 (s, 3H), 3.71 (brs, 1H), 3.79-3.89 (m, 1H), 4.80 (brs, 1H), 6.49 (d, J=8.7Hz, 1H), 6.67 (d, J=8.7Hz, 2H), 7.10 (d, J=8.4Hz, 2H), 7.44 (dd, J=8.7, 2.3Hz, 1H), 8.05 (d, J=2.1Hz, 1H)
69: ¹H-NMR (CDCl₃)δ 0.27-0.30 (m, 4H), 0.56-0.61 (m, 4H), 1.10-1.18 (m, 2H), 2.06 (s, 3H), 2.08 (s, 3H), 3.02 (d, J=7.2Hz, 2H), 3.18, (d, J=5.1H 1H), 3.20 (d, J=5.4Hz, 1H), 3.32 (s, 3H), 3.35 (s, 3H), 3.83 (brs, 1H), 4.78 (brs, 1H), 6.49 (d, J=8.4Hz, 1H), 6.69 (d, J=8.4Hz, 2H), 7.12 (d, J=8.7Hz, 2H), 7.44 (dd, J=8.4, 2.3Hz, 1H), 8.05 (d, J=1.5Hz, 1H)
70: ¹H-NMR (CDCl₃)δ 0.28-0.33 (m, 2H), 0.58-0.62 (m, 2H), 1.10-1.18 (m, 1H), 2.06 (s, 3H), 2.09 (s, 3H), 3.19 (d, J=4.5Hz, 2H), 3.35 (s, 3H), 3.36 (s, 3H), 3.86 (s, 3H), 4.68 (brs, 2H), 5.30 (brs, 1H), 6.54 (d, J=8.7Hz, 1H), 6.70-6.80 (m, 3H), 7.49 (dd, J=8.7, 1.8Hz, 1H), 8.01 (d, J=1.5Hz, 1H)
71: ¹H-NMR (CDCl₃)δ 0.25-0.31 (m, 2H), 0.55-0.61 (m, 2H), 1.09-1.20 (m, 1H), 1.25 (s, 3H), 1.27 (s, 3H), 2.05 (s, 6H), 2.20 (s,3H), 3.18 (dd, J=5.7, 6.9, 2H), 3.64-3.72 (m,1H), 4.75 (brs, 2H), 6.46 (d, J=8.7, 1H), 6.66 (d, J=8.7, 2H), 6.95 (d, J=8.7, 2H), 6.97 (s, 1H), 7.45 (dd, J=8.7, 2.1, 1H), 8.09 (d, J=2.1, 1H),
72: ¹H-NMR (CDCl₃)δ 0.25-0.32 (m, 4H), 0.55-0.62 (m, 4H), 1.12-1.20 (m, 2H), 2.04 (s, 6H), 2.19 (s,3H), 3.01 (d, J=7.2, 2H), 3.18 (dd, J=5.4, 6.9, 2H), 6.48 (d, J=8.4, 1H), 6.69 (d, J=8.4, 2H), 6.96 (d, J=8.4, 2H), 6.97 (s, 1H), 7.47 (dd, J=8.4, 2.4, 1H), 8.08 (d, J=2.4, 1H),
73: ¹H-NMR (CDCl₃)δ 0.26-0.34 (m, 2H), 0.55-0.63 (m, 2H), 1.10-1.19 (m, 1H), 1.44-1.95 (m, 8H), 2.05 (s, 6H), 2.19 (s,3H), 3.18 (t, J=5.7, 2H), 3.78-3.88 (m, 1H), 6.48-6.51(m, 1H), 6.67(d, J=8.7, 2H), 6.94 (d, J=8.7, 2H), 6.96 (s, 1H), 7.46-7.52(m, 1H), 8.03- 8.08 (m, 1H),
74: ¹H-NMR (CDCl₃)δ 0.13-0.16 (m, 2H), 0.43-0.46 (m, 2H), 1.04-1.10 (m, 1H), 2.07 (s, 3H), 2.08 (s, 3H), 3.34 (s, 3H), 3.37 (s, 3H), 3.374 (s, 3H), 3.55 (s, 3H), 3.83 (d, J=7.2Hz, 2H), 3.92 (s, 3H), 4.08 (s, 2H), 4.11 (s, 2H), 6.87-6.93 (m, 2H), 7.37 (d, J=8.1H 1H), 7.77 (dd, J=7.8, 2.4Hz, 1H), 8.47 (s, 1H), 8.49 (d, J=8.1Hz, 1H), 8.95 (s, 1H)
75: ¹H-NMR (CDCl₃)δ 0.28-0.33 (m, 2H), 0.56-0.62 (m, 2H), 1.10-1.18 (m, 1H), 1.29 (d, J=6.3Hz, 6H), 2.08 (s, 3H), 2.09 (s, 3H), 3.18 (d, J=5.1Hz, 1H), 3.20 (d, J=5.1Hz, 1H), 3.35 (s, 3H), 3.36 (s, 3H), 3.63-3.72 (m, 1H), 3.84 (s, 3H), 4.10 (brs, 1H), 4.82 (brs, 1H), 6.50 (d, J=8.7Hz, 1H), 6.66-6.80 (m, 3H), 7.45 (dd, J=8.4, 1.8Hz, 1H), 8.05 (d, J=2.1H 1H)
76: ¹H-NMR (CDCl₃)δ 0.28-0.32 (m, 4H), 0.56-0.61 (m, 4H), 1.13-1.21 (m, 2H), 2.07 (s, 3H), 2.09 (s, 3H), 3.03 (d, J=6.9Hz, 2H), 3.18 (d, J=5.1H 1H), 3.20 (d, J=5.1Hz, 1H), 3.35 (s, 3H), 3.36 (s, 3H), 3.87 (s, 3H), 4.38 (brs, 1H), 4.74 (brs, 1H), 6.49 (d, J=8.4Hz, 1H), 6.64 (d, J=7.8Hz, 1H), 6.74-6.81 (m, 2H), 7.44 (dd, J=8.7, 2.3Hz, 1H), 8.05 (d, J=1.8Hz, 1H)
77: ¹H-NMR (CDCl₃)δ 0.28-0.31 (m, 2H), 0.56-0.62 (m, 2H), 1.10-1.18 (m, 1H), 1.54-1.81 (m, 6H), 2.04-2.15 (m, 2H), 2.08 (s, 3H), 2.09 (s, 3H), 3.18 (d, J=5.4Hz, 1H), 3.20 (d, J=5.4Hz, 1H), 3.35 (s, 3H), 3.36 (s, 3H), 3.80-3.84 (m, 1H), 3.84 (s, 3H), 4.23 (brs, 1H), 4.75 (brs, 1H), 6.50 (d, J=8.7Hz, 1H), 6.68-6.80 (m, 3H), 7.44 (dd, J=8.4, 2.0Hz, 1H), 8.05 (d, J=1.8Hz, 1H)
78: ¹H-NMR (CDCl₃)δ 0.29-0.31 (m, 2H), 0.57-0.62 (m, 2H), 1.10-1.18 (m, 1H), 2.05 (s, 3H), 2.10 (s, 3H), 3.18 (d, J=5.1Hz, 1H), 3.20 (d, J=5.1Hz, 1H), 3.35 (s, 3H), 3.36 (s, 3H), 3.55 (s, 3H), 3.90 (s, 3H), 4.07 (s, 2H), 4.82 (s, 1H), 6.51 (d, J=8.7Hz, 1H), 6.87 (d, J=1.8Hz, 1H), 6.92 (dd, J=8.1, 1.7Hz, 1H), 7.44 (dd, J=8.7, 1.8Hz, 1H), 8.04 (s, 1H), 8.46 (d, J=8.1Hz, 1H), 8.93 (s, 1H)
79: ¹H-NMR (CDCl₃)δ 0.27-0.33 (m, 2H), 0.55-0.64 (m, 2H), 1.10-1.21 (m, 1H), 1.75 (s, 3H), 1.78 (s, 3H), 2.05 (s, 6H), 2.19 (s,3H), 3.17-3.21 (m, 2H),3.74 (d, J=6.9Hz, 2H), 5.36-5.43 (m, 1H), 6.49(d, J=8.7Hz, 1H), 6.69(d, J=8.7Hz, 2H), 6.97 (d, J=8.7Hz, 2H), 6.97 (s, 1H), 7.44-7.52(m, 1H), 8.03- 8.09 (m, 1H),
80: ¹H-NMR (CDCl₃)δ 0.26-0.31 (m, 2H), 0.55-0.61 (m, 2H), 1.10-1.20 (m, 1H), 1.84 (s, 3H), 2.04 (s, 6H), 2.20 (s,3H), 3.18 (dd, J=5.4, 7.2Hz, 2H),3.86.3.97 (m, 2H), 6.47(d, J=8.7Hz, 1H), 6.74(d, J=8.7Hz, 2H), 6.98 (s, 1H), 6.99 (d, J=8.7Hz, 2H), 7.46 (dd, J=2.1, 8.7Hz, 1H), 8.09 (d, J=2.1Hz 1H),
81: ¹H-NMR (CDCl₃)δ 0.28-0.32 (m, 2H), 0.56-0.62 (m, 2H), 1.11-1.19 (m, 1H), 1.84-1.85 (m, 3H), 2.07 (s, 3H), 2.09 (s, 3H), 3.18 (d, J=6.3Hz, 1H), 3.20 (d, J=6.0Hz, 1H), 3.35 (s, 3H), 3.36 (s, 3H), 3.85 (s, 3H), 3.95 (brs, 2H), 4.45 (brs, 1H), 4.95 (brs, 1H), 6.51 (d, J=8.7Hz, 1H), 6.72-6.83 (m, 3H), 7.46 (d, J=8.7Hz, 1H), 8.04 (d, J=1.8Hz, 1H)
82: ¹H-NMR (CDCl₃)δ 0.28-0.33 (m, 2H), 0.57-0.62 (m, 2H), 1.11-1.19 (m, 1H), 1.14 (t, J=7.5Hz, 3H), 2.07 (s, 3H), 2.09 (s, 3H), 2.22 (q, J=7.5Hz, 2H), 3.18 (d, J=5.7Hz, 1H), 3.20 (d, J=6.6Hz, 1H), 3.35 (s, 3H), 3.36 (s, 3H), 3.85 (s, 3H), 3.97 (brs, 2H), 4.44 (brs, 1H), 5.11 (brs, 1H), 6.53 (d, J=8.4Hz, 1H), 6.73-6.85 (m, 3H), 7.48 (d, J=8.1Hz 1H), 8.02 (s, 1H)
83: ¹H-NMR (CDCl₃)δ 0.06-0.09 (m, 2H), 0.39-0.44 (m, 2H), 1.00-1.08 (m, 1H), 1.14 (d, J=6.9Hz, 6H), 1.30 (d, J=6.9Hz, 6H), 2.07 (s, 3H), 2.09 (s, 3H), 2.59-2.70 (m, 2H), 3.34 (s, 3H), 3.37 (s, 3H), 3.80 (d, J=7.2Hz, 2H), 3.91 (s, 3H), 6.86 (d, J=1.5Hz, 1H), 6.92 (dd, J=8.4, 1.2Hz, 1H), 7.34 (d, J=8.1Hz, 1H), 7.75 (dd, J=8.1, 2.3Hz, 1H), 7.91 (brs, 1H), 8.48-8.52 (m, 2H)
84: ¹H-NMR (CDCl₃)δ 0.26-0.31 (m, 2H), 0.54-0.60 (m, 2H), 1.08-1.20 (m, 1H), 1.25 (s, 3H), 1.27 (s, 3H), 2.01 (s, 3H), 2.03 (s, 6H), 2.20 (s,3H), 3.10-3.14 (m, 2H), 3.64-3.74 (m, 1H), 6.31 (d, J=8.1Hz, 1H), 6.66 (d, J=8.7Hz, 2H), 6.84 (s, 1H), 6.96 (d, J=8.7Hz, 2H), 7.27 (d, J=8.1H 1H),
85: ¹H-NMR (CDCl₃)δ 0.25-0.38 (m, 4H), 0.55-0.65 (m, 4H), 1.08-1.20 (m, 2H), 2.00 (s, 3H), 2.02 (s, 6H), 2.25 (s,3H), 3.02 (d, J=6.6Hz, 2H), 3.12-3.18 (m, 2H), 6.39 (d, J=8.4Hz, 1H), 6.69 (d, J=7.2Hz, 2H), 6.83 (s, 1H), 6.96 (d, J=7.2Hz, 2H), 7.36 (d, J=8.4Hz, 1H)
86: ¹H-NMR (CDCl₃)δ 0.25-0.36 (m, 2H), 0.52-0.60 (m, 2H), 1.07-1.20 (m, 1H), 1.40-1.85 (m, 8H), 2.01 (s, 3H), 2.02 (s, 6H), 2.20 (s,3H), 3.06-3.16 (m, 2H), 3.78-3.88 (m, 1H), 6.31 (d, J=9.3Hz, 1H), 6.67 (d, J=8.1Hz, 2H), 6.84 (s, 1H), 6.96 (d, J=8.1Hz, 2H), 7.26 (d, J=9.3Hz, 1H)
87: ¹H-NMR (CDCl₃)δ 0.20-0.37 (m, 2H), 0.47-0.65 (m, 2H), 1.04-1.20 (m, 1H), 1.75 (s, 3H), 1.78 (s, 3H), 2.02 (s, 9H), 2.20 (s,3H), 3.04-3.20 (m, 2H), 3.64-3.80 (m, 2H), 5.30-5.42 (m, 1H), 6.29 (d, J=8.1Hz, 1H), 6.69 (d, J=7.2Hz, 2H), 6.85 (s, 1H), 6.98 (d, J=7.2Hz, 2H), 7.26 (d, J=8.1Hz, 1H)
88: ¹H-NMR (CDCl₃)δ 0.24-0.34 (m, 2H), 0.50-0.64 (m, 2H), 1.04-1.21 (m, 1H), 1.85 (s, 3H), 2.01 (s, 6H), 2.19 (s,3H), 3.06-3.18 (m, 2H), 3.78-3.95 (m, 2H), 6.29 (d, J=8.4Hz, 1H), 6.74 (d, J=8.1Hz, 2H), 6.85 (s, 1H), 7.01 (d, J=8.1Hz, 2H), 7.26 (d, J=8.4Hz, 1H)
89: ¹H-NMR-(CDCl₃)δ 0.12-0.17 (m, 2H), 0.42-0.48 (m, 2H), 1.04-1.12 (m, 1H). 2.06 (s, 3H), 2.08 (s, 3H), 3.33 (s, 3H), 3.34 (s, 3H), 3.37 (s, 3H), 3.55 (s, 3H), 3.83 (d, J=7.2Hz, 2H), 4.06 (s, 2H), 4.11 (s, 2H), 7.30 (d, J=8.1Hz, 2H), 7.37 (d, J=7.8Hz, 1H), 7.69 (d, J=8.7Hz, 2H), 7.76 (dd, J=8.4, 1.8Hz, 1H), 8.35 (s, 1H), 8.47 (d, J=2.4Hz, 1H)
90: ¹H-NMR (CDCl₃)δ 0.28-0.32 (m, 2H), 0.57-0.61 (m, 2H), 1.10-1.19 (m, 1H), 2.03 (s, 3H), 2.09 (s, 3H), 3.18 (d, J=5.1Hz 1H), 3.20 (d, J=5.1Hz, 1H), 3.31 (s, 3H), 3.36 (s, 3H), 3.54 (s, 3H), 4.06 (s, 2H), 4.83 (brs, 1H), 6.50 (d, J=8.4Hz, 1H), 7.30 (d, J=8.4Hz, 2H), 7.44 (dd, J=8.7, 2.3Hz, 1H), 7.66 (d, J=8.7Hz, 2H), 8.04 (d, J=1.8Hz, 1H), 8.33 (s, 1H)
91: ¹H-NMR (CDCl₃)δ 0.27-0.32 (m, 2H), 0.57-0.61 (m, 2H), 1.10-1.18 (m, 1H), 1.97 (s, 3H), 1.98 (s, 3H), 2.00 (s, 3H), 3.18 (d, J=5.7Hz, 1H), 3.20 (d, J=5.4Hz, 1H), 3.80 (brs, 2H), 4.87 (brs, 2H), 6.51 (d, J=8.4Hz, 1H), 6.82 (d, J=8.1Hz, 2H), 7.10 (d, J=7.2Hz, 2H), 7.26-7.29 (m, 1H), 7.90 (d, J=1.8Hz, 1H)
92: ¹H-NMR (CDCl₃)δ 0.27-0.32 (m, 2H), 0.57-0.61 (m, 2H), 1.10-1.19 (m, 1H), 1.27 (d, J=6.0Hz, 6H), 1.97 (s, 3H), 1.98 (s, 3H), 2.02 (s, 3H), 3.18 (d, J=5.1H 1H), 3.20 (d, J=6.6Hz, 1H), 3.62 (brs, 1H), 3.65-3.73 (m, 1H), 4.75 (brs, 1H), 4.95 (brs, 111), 6.50 (d, J=8.1Hz, 1H), 6.71 (d, J=8.1Hz, 2H), 7. 09 (d, J=7.5Hz, 2H), 7.26-7.29 (m, 1H), 7.92 (s, 1H)
93: ¹H-NMR (CDCl₃)δ 0.28-0.32 (m, 2H), 0.56-0.61 (m, 2H), 1,10-1. IS (m, 1H), 1.96 (s, 3H), 1.97 (s, 3H), 2.00 (s, 3H), 3.18 (d, J=5.4Hz, 1H), 3.20 (d, J=5.7Hz, 1H), 3.87 (brs, 2H), 4.72 (brs, 1H), 4.79 (brs, 1H), 6.50 (d, J=8.7Hz, 1H), 6.90-6.98 (m, 3H), 7.24-7.28 (m, 1H), 7.90 (d, J=1.8Hz, 1H)
94: ¹H-NMR (CDCl₃)δ 0.25-0.32 (m, 2H), 0.55-0.62 (m, 2H), 1.06-1.19 (m, 1H), 1.25 (d, J=6.3Hz, 6H), 3.20 (dd, J=6.9, 5.4Hz, 2H), 3.69 (sept, J=6.3Hz, 1H), 4.91-5.00 (m, 1H), 6.50 (d, J=8.7Hz, 1H), 6.62-6.69 (m, 2H), 7.28-7.36 (m, 2H), 7.59 (dd, J=8.7, 1.5Hz, 1H), 8.25 (d, J=1.5Hz, 1H)
95: ¹H-NMR (CDCl₃)δ 0.28-0.31 (m, 2H), 0.56-0.61 (m, 2H), 1.10-1.19 (m, 1H), 1.295 (d, J=6.0Hz, 3H), 1.30 (d, J=6.3Hz, 3H),1.97 (s, 3H), 1.98 (s, 3H), 2.01 (s, 3H), 3.18 (d, J=5.4Hz, 1H), 3.20 (d, J=5.7Hz, 1H), 3.65-3.73 (m, 1H), 3.86 (brs, 1H), 4.73 (brs, 1H), 4.87 (brs, 1H), 6.50 (d, J=8.7Hz, 1H), 6.79-6.95 (m, 3H), 7.25-7.29 (m, 1H), 7.90 (d, J=2.1H 1H)
96: ¹H-NMR (CDCl₃)δ 0.28-0.31 (m, 4H), 0.57-0.64 (m, 4H), 1.09-1.19 (m, 2H), 1.97 (s, 3H), 1.98 (s, 3H), 2.01 (s, 3H), 3.05 (d, J=5.4Hz, 1H), 3.07 (d, J=6.3Hz, 1H), 3.18 (d, J=5.4Hz, 1H), 3.20 (d, J=6.3Hz, 1H), 4.18 (brs, 1H), 4.75 (brs, 1H), 4.85 (brs, 1H), 6.50 (d, J=8.4Hz, 1H), 6.80 (t, J=8.0Hz, 1H), 6.94 (d, J=9.6Hz, 2H), 7.26-7.28 (m, 1H), 7.90 (d, J=1.8Hz, 1H)
97: ¹H-NMR (CDCl₃)δ 0.26-0.31(m, 2H), 0.53-0.61(m, 2H), 1.13(m, 1H), 1.25(d, J=6.0Hz, 6H), 2.10(s, 3H), .3.14-4.00(m, 2H), 3.44(s, 3H), 3.63(s, 3H), 3.69(m, 1H), 4.71(m, 1H), 6.46(dd, J=8.4, 0.6Hz, 1H), 6.65(d, J=8.4Hz, 2H), 7.11(d, J=0.6Hz, 114), 7.30(dd, J=8.1, 2.1Hz, 1H), 7.44(d, J=8.4Hz, 2H), 7.88(dd, J=2.1, 0.6Hz, 1H)
98: ¹H-NMR (CDCl₃)δ 0.27-0.34 (m, 2H), 0.55-0.63 (m, 2H), 1.08-1.22 (m, 1H), 1.97 (s, 6H), 2.03 (s, 6H), 3.20 (dd, J=6.9, 5.4Hz, 2H), 4.63-4.72 (m, 1H), 6.51 (d, J=8.4Hz, 1H), 6.55-6.59 (m, 1H), 6.96-7.03 (m, 1H), 7.25-7.27 (m, 1H), 7.31 (dd, J=8.4, 2.3Hz, 1H), 7.40-7.48 (m, 2H), 7.95 (d, J=2.3Hz, 1H), 8.25 (s, 1H)
99: ¹H-NMR (CDCl₃)δ 1.53-1.77 (m, 6H), 1.74 (s, 3H), 1.77 (s, 3H), 2.02-2.12 (m, 2H), 2.21 (s, 3H), 2.28 (s, 3H), 3.71 (d, J=6.0Hz, 2H), 4.01 (m, 1H), 4.69 (d, J=6.9Hz, 1H), 5.35 (m, 1H), 6.36-6.48(m, 3H), 7.03-7.10 (m, 3H), 7.47 (dd, J=2.4, 8.4Hz, 1H), 8.11 (d, J=1.5Hz, 1H)
100: ¹H-NMR (CDCl₃)δ 1.51-1.69 (m, 6H), 1.74 (s, 3H), 1.77 (s, 3H), 2.04-2.12 (in, 2H), 2.14 (s, 3H), 2.28 (s, 3H), 3.71 (d, J=4.5Hz, 2H), 4.01 (m, 1H), 4.66 (d, J=6.6Hz, 1H), 5.35 (m, 1H), 6.46 (d, J=8.4Hz, 1H), 6.54 (dd, J=2.4, 8.4Hz, 1H) , 6.70 (d, J=2.1Hz, 1H), 7.02-7.09 (m, 3H), 7.48 (dd, J=2.4, 8.4Hz, 1H) , 8.11 (d, J=2.1H 1H).
101: m.p=116-117°C
102: ¹H-NMR (CDCl₃)δ 1.23 (s, 3H), 1.25 (s, 3H), 1.55-1.95 (m, 1H), 2.04 (s, 3H), 2.07 (s, 3H), 2.27 (s, 3H), 3.66 (m, 1H), 4.00 (m, 1H), 4.67 (m, 1H), 6.45-6.50 (m, 3H), 6.93 (d, J=7.5Hz, 1H), 7.01 (s, 1H), 7.07 (s, 1H), 7.47 (dd, J=2.4, 8.4Hz, 1H), 8.10 (d, J=2.4Hz, 1H).
103: ¹H-NMR (CDCl₃)δ 1.50-1.85 (m, 6H), 1.98 (s, 6H), 2.01 (s, 6H), 2.03-2.11 (m, 2H), 3.68 (brs, 2H), 3.91-4.08 (m, 1H), 4.70 (d, J=6.0Hz, 1H), 6.51 (d, J=8.4Hz, 1H), 6.74-6.80 (m, 2H), 6.91-6.99 (m, 2H), 7.29 (dd, J=8.4, 2.1Hz, 1H), 7.89 (d, J=2.1Hz, 1H)
104: ¹H-NMR (CDCl₃)δ 1.50-1.83 (m, 6H), 1.72 (s, 3H), 1.76 (s, 3H), 1.91-2.15 (m, 2H), 2.01 (s, 12H), 3.90 (d, J=5.4Hz, 2H), 3.95-4.07 (m, 1H), 5.25-5.33 (m, 1H), 4.84 (brs, 1H), 6.52 (d, J=8.6Hz, 1H), 6.72-6.79 (m, 2H), 6.92-7.00 (m, 2H), 7.31 (dd, J=8.6, 1.8Hz, 1H), 7.89 (d, J=1.8Hz, 1H)
105: ¹H-NMR (CDCl₃)δ 1.26 (d, J=6.3Hz, 6H), 1.50-1.84 (m, 6H), 1.90-2.15 (m, 2H), 1.99 (s, 6H), 2.01 (s, 6H), 3.68 (sept, J=6.3Hz, 1H), 3.95-4.07 (m, 1H), 4.63-4.75 (m, 1H), 6.50 (d, J=8.4Hz, 1H), 6.62-6.69 (m, 2H), 6.90-6.98 (m, 2H), 7.29 (dd, J=8.4, 2.1Hz 1H), 7.90 (d, J=2.1Hz, 1H)
106: ¹H-NMR (CDCl₃)δ 1.48-1.83 (m, 12H), 1.92-2.12 (m, 4H), 2.00 (s, 6H), 2.01 (s, 6H), 3.78-3.88 (m, 1H), 3.96-4.06 (m, 1H), 4.64-4.74 (m, 1H), 6.51 (d, J=8.1Hz, 1H), 6.67 (d, J=8.3Hz, 2H), 6.957 (d, J=8.3Hz, 2H), 7.29 (dd, J=8.1, 2.1Hz, 1H), 7.90 (d, J=2.1H 1H)
107: ¹H-NMR (CDCl₃)δ 1.25 (s, 3H), 1.27 (s, 3H), 1.45-1.82 (m, 8H), 2.05 (s, 6H), 2.20 (s,3H), 3.64-3.72 (m, 1H), 3.99-4.10 (m, 1H), 6.47(d, J=9.0Hz, 1H), 6.66(d, J=8.4Hz, 2H), 6.95 (d, J=8.4Hz, 2H), 6.98 (s, 1H), 7.45 (dd, J=9.0, 2.4Hz, 1H), 8.08 (d, J=2.4Hz, 1H)
108: ¹H-NMR (CDCl₃)δ 0.25-0.30 (m, 2H), 0.55-0.62 (m, 2H), 1.10-1.21 (m, 1H), 1.47-1.85 (m, 8H), 2.04 (s, 6H), 2.20 (s,3H), 3.01 (d, J=6.9Hz, 2H), 3.95-4.07 (m, 1H), 6.47(d, J=8.7Hz, 1H), 6.69 (d, J=8.7Hz, 2H), 6.97 (d, J=8.7Hz, 2H), 6.98 (s, 1H), 7.46 (dd, J=8.7, 2.4Hz, 1H), 8.07 (d, J=2.4Hz, 1H)
109: ¹H-NMR (CDCl₃)δ 1.45-1.88 (m, 16H), 2.05 (s, 6H), 2.20 (s, 3H), 3.37-3.89 (m, 1H), 3.95-4.06 (m, 1H), 6.46(d, J=8.4Hz, 1H), 6.67 (d, J=8.7Hz, 2H), 6.95 (d, J=8.7Hz, 2H), 6.98 (s, 1H), 7.45 (dd, J=8.4Hz, 2.4, 1H), 8.08 (d, J=2.4Hz, 1H)
110: ¹H-NMR (CDCl₃)δ 1.41-1.80 (m, 8H), 1.74 (s, 3H), 1.78 (s, 3H), 2.05 (s, 6H), 2.20 (s,3H), 3.74 (d, J=6.6Hz, 2H), 3.97-4.06 (m, 1H), 5.36-5.43 (m, 1H), 6.47 (d, J=8.4Hz, 1H), 6.69 (d, J=8.7Hz, 2H), 6.97 (d, J=8.7Hz, 2H), 6.98 (s, 1H), 7.45 (dd, J=8.4, 2.4Hz, 1H), 8.08 (d, J=2.4Hz, 1H),
111: ¹H-NMR.(CDCl₃)δ 1.25 (s, 3H), 1.27 (s, 3H), 1.45.1.89 (m, 8H), 2.02 (s, 9H), 2.18 (s,3H), 3.59-3.80 (m, 1H), 3.81-4.00 (m, 1H), 6.32 (d, J=8:4Hz, 1H), 6.66 (d, J=7.8Hz, 2H), 6.85 (s, 1H), 6.95 (d, J=7.2Hz, 2H), 7.27 (d, J=8.4Hz, 1H)
112: ¹H-NMR (CDCl₃)δ 1.26 (d, J=6.2Hz, 6H), 1.48-1.83 (m, 6H), 2.02-2.14 (m, 2H), 3.69 (sept, J=6.2Hz, 1H), 3.7 (brs, 1H), 3.98-4.07 (m, 1H), 4:84 (d, J=6.6Hz, 1H), 6.50 (d, J=8.7Hz, 1H), 6.63-6.69 (m, 2H), 7.29-7.36 (m, 2H), 7.55-7.61 (m, 1H), 8.24 (d, J=1.5Hz, 1H)
113: ¹H-NMR (CDCl₃)δ 1.25(d, J=6.0Hz, 6H), 1.45-1.83(m, 6H), 2.00-2.14(m, 2H), 2.11(s, 3H), 3.44(s, 3H), 3.62(s, 3H), 3.69(m, 1H), 4.02(m, 1H), 4.63(d,J=6.9Hz, 1H), 6.46(d, J=8.1Hz, 1H), 6.65(d, J=8.4Hz, 2H), 7.30(dd, J=8.7, 2.1Hz, 1H), 7.44(d, J=8.4Hz, 2H), 7.70(6,1H), 7.86(d, J=1.8Hz, 1H)
114: mp 160-163 °C
115: mp 215-221 °C
116: ¹H-NMR (CDCl₃)δ 1.74 (s, 6H), 1.77 (s, 6H), 2.14 (s, 3H), 2.28 (s, 3H), 3.71 (d, J=6.6Hz, 2H), 3.89 (m, 2H), 5.36 (m, 2H), 6.44 (d, J=8.7Hz, 1H), 6.54 (dd, J=2.4, 8.4Hz, 1H), 6.70 (d, J=2.4Hz, 1H), 7.02-7.04 (m, 2H), 7.09 (s, 1H), 7.48 (dd, J=2.4, 8.4Hz, 1H), 8.41 (d, J=1.8Hz, 1H).
117: mp 134-138 °C
118: mp 166-168°C
120: ¹H-NMR (CDCl₃)δ 1.74 (s, 6H), 1.77 (s, 6H), 2.21 (s, 3H), 2.28 (s, 3H), 3.71 (d, J=5.4Hz, 2H), 3.90 (m, 2H), 4.47 (m, 1H), 5.36 (m, 2H), 6.36-6.45 (m, 3H), 7.03-7.10 (m, 3H), 7.46 (dd, J=2.4, 8.4Hz, 1H), 8.13 (dd, J=0.6, 2.4Hz, 1H).
121: m.p=79-81°C
125: ¹H-NMR (DMSO-d₆)δ 1.56 (s, 3H), 1.72 (s, 3H), 1.74 (s, 3H), 1.78 (s, 3H), 1.88 (s, 6H), 1.97 (s, 6H), 3.88 (d, J=6.9Hz, 2H), 3.97-4.11 (m, 2H), 5.33-5.42 (m, 2H), 7.08-7.24 (m, 3H), 7.30-7.45 (m, 1H), 7.70-7.82 (m, 2H), 8.90-9.05 (m, 1H)
126: ¹H-NMR (CDCl₃)δ 1.26 (d, J=6.3Hz, 6H), 1.75 (s, 3H), 1.78 (s, 3H), 2.00 (s, 6H), 2.01 (s, 6H), 3.68 (sept, J=6.3Hz, 1H), 3.91 (t, J=6.0Hz, 1H), 4.41-4.48 (m, 1H), 5.33-5.42 (m, 1H), 6.48 (d, J=8.6Hz, 1H), 6.62-6.69 (m, 2H), 6.91-6.98 (m, 2H), 7.28 (dd, J=8.6, 2.0Hz, 1H), 7.93 (d, J=2.0Hz, 1H)
127: ¹H-NMR (CDCl₃)δ 1.55-1.80 (m, 6H), 1.75 (s, 3H), 1.78 (s, 3H), 1.95-2.12 (m, 2H), 2.00 (s, 6H), 2.01 (s, 6H), 3.79-3.90 (m, 1H), 3.87-3.94 (m, 2H), 4.41-4.49 (m, 1H), 5.35-5.42 (m, 1H), 6.48 (d, J=8.6Hz, 1H), 6.67 (d, J=8.7Hz, 2H), 6.95 (d, J=8.7Hz, 2H), 7.28 (dd, J=8.6, 2.4Hz, 1H), 7.93 (d, J=2.4Hz, 1H)
128: mp 148-149 °C
129: mp 151-154 °C
130: mp 153-154 °C
131: mp 151-153 °C
132: mp 173-174°C
133: mp 157-158 °C
134: mp 160-162 °C
135: ¹H-NMR (CDCl₃)δ 1.26(d, J=6.3Hz, 6H), 1.74(s, 3H), 1.77(s, 3H), 2.10(s, 3H), 3.44(s, 3H), 3.63(s, 3H), 3.69(m, 1H), 3.84 -3.94(m, 2H), 4.53(brs, 1H), 5.36(m, 1H), 6.46(d, J=8.7Hz, 1H), 6.65(d, J=8.4Hz, 2H), 7.31(dd, J=8.7, 2.1Hz, 1H), 7.45(d, J=8.4Hz, 2H), 7.71(s, 1H), 7.88(d, J=2.1Hz, 1H)
136: ¹H-NMR (CDCl₃)δ 1.26 (d, J=6.6Hz, 6H), 1.74 (s, 3H), 1.77 (s, 3H), 3.69 (sept, J=6.6Hz, 1H), 3.91 (t, J=6.2Hz, 2H), 4.69-4.76 (m, 1H), 5.30-5.38 (m, 1H), 6.49 (dd, J=8.6, 0.6Hz, 1H), 6.63-6.69 (m, 2H), 7.29-7.37 (m, 2H), 7.58 (dd, J=8.6, 2.4Hz, 1H), 8.27 (dd, J=2.4, 0.6Hz, 1H)
141: ¹H-NMR (CDCl₃)δ 1.44 (t, J=7.2Hz, 3H), 1.74 (s, 3H), 1.78 (s, 3H), 2.22 (s, 3H), 2.27 (s, 3H), 3.27-3.35 (m, 2H), 3.72 (d, J=6.6Hz, 2H), 5.33 (m, 1H), 6.36-6.48 (m, 2H), 7.05 (t, J=8.4Hz, 1H), 7.09 (s, 1H), 7.14 (s, 1H), 7.38 (d, J=9.0Hz, 1H), 7.72 (dd, J=2.1, 8.7Hz, 1H), 8.28 (d, J=2.1Hz, 1H)
142: ¹H-NMR (CDCl₃)δ 1.74 (s, 3H), 1.78 (s, 3H), 1.85 (m, 4H), 2.20 (s, 3H), 2.29 (s, 3H), 2.67 (m, 4H), 2.84 (m, 1H), 3.59-3.78 (m, 3H), 5.35 (m, 1H), 5.81 (m, 4H), 6.36-6.46 (m, 2H), 7.02-7.08 (m, 2H),7.13 (s, 3H), 8.34 (s, 2H)
143: ¹H-NMR (CDCl₃)δ 1.73 (s, 3H), 1.77 (s, 3H), 2.22 (s, 3H), 2.29 (s, 9H), 2.57 (m, 2H), 2.53 (m, 2H), 3.70.3.80 (m, 3H), 5.35 (m, 1H), 5.71 (m, 1H), 6.35-6.45 (m, 2H), 7.00-7.12 (m, 3H), 7.25 (s, 1H), 8.33 (s, 2H)
144: ¹H-NMR (CDCl₃)δ 1.54-1.68 (m, 10H), 1.74 (s, 3H), 1.77 (s, 3H), 2.04-2.11 (m, 2H), 2.22 (s, 3H), 2.29 (s, 3H), 3.71 (d, J=6.3Hz, 2H), 4.08 (m, 1H), 5.14 (d, J=8.1Hz, 1H), 5.34 (m, 1H), 6.35-6.46 (m, 2H), 6.99 (m, 2H), 7.12 (s, 1H), 8.32 (s, 1H).
145: ¹H-NMR (CDCl₃)δ 1.22-1.65 (m, 8H), 1.74 (s, 3H), 1.77 (s, 3H), 2.06-2.12 (m, 2H), 2.21 (s, 3H), 2.29 (s, 3H), 3.71 (d, J=6.3Hz, 2H), 3.87 (m, 1H), 5.12 (d, J=8.1Hz 1H), 5.35 (m, 1H), 6.53-6.46 (m, 2H), 7.01-7.12 (m, 3H), 8.32 (s, 2H).
146: ¹H-NMR (CDCl₃)δ 1.49-1.69 (m, 6H), 1.74 (s, 3H), 1.77 (s, 3H), 2.05-2.13 (m, 2H), 2.22 (s, 3H), 2.29 (s, 3H), 3.71 (d, J=6.3Hz, 2H), 4.32 (m, 1H), 5.20 (d, J=10.2Hz, 1H), 5.35 (m, 1H), 6.36-6.46 (m, 2H), 7.01-7.12 (m, 3H), 8.33 (s, 2H).
147: ¹H-NMR (CDCl₃)δ 0.59-0.64 (m, 2H), 0.85-0.91 (m, 2H), 1.74 (s, 3H), 1.78 (s, 3H), 2.22 (s, 3H), 2.29 (s, 3H), 2.84 (m, 1H), 3.71 (d, J=6.9Hz, 2H), 5.33 (m, 1H), 5.51 (m, 1H), 6.36-6.46 (m, 2H), 7.02-7.14 (m, 3H), 8.39 (s, 2H)
162: ¹H-NMR (DMSO-d₆)δ 0.91 (t, J=7.5Hz, 6H), 1.53 (m, 4H), 2.14 (s, 3H), 2.24 (s, 3H), 3.21 (m, 1H), 6.51-6.61 (m, 2H), 7.02 (t, J=8.1Hz 1H), 7.09 (s, 1H), 7.14 (s, 1H), 8.49 (s, 2H)
163: ¹H-NMR (CDCl₃) δ 1.05 (t, J=7.2Hz, 6H), 2.01-2.12 (m, 4H), 2.13 (s, 3H), 2.30 (s, 3H), 3.27(s, 6H), 4.53 (m, 1H), 7.05-7.26 (m, 5H), 8.39 (s, 2H)
164: ¹H-NMR (CDCl₃)δ 0.97 (t, J=8.4Hz, 6H), 1.44-1.66 (m, 4H), 2.08 (s, 3H), 2.11 (s, 3H), 3.27 (s, 6H), 3.33 (s, 3H), 3.38 (s, 3H), 6.64 (d, J=8.7Hz, 2H), 7.07 (d, J=8.1Hz 2H), 8.34 (s, 2H)
165: ¹H-NMR (CDCl₃)δ 0.97 (t, J=6.3Hz, 6H), 1.31 (s, 3H), 1.34 (s, 3H), 1.57 (m, 4H), 2.08 (s, 3H), 2.12 (s, 3H), 3.23 (s, 3H), 3.39 (s, 3H), 4.20 (m, 1H), 5.02 (d, J=7.2Hz, 1H), 6.64 (d, J=8.7Hz, 2H), 7.06 (d, J=8.4Hz, 2H), 8.28 (s, 2H)
166: ¹H-NMR (CDCl₃)δ 0.63 (m, 2H), 0.88 (m, 2H), 0.97 (t, J=8.4Hz, 6H), 1.58 (m, 4H), 2.08 (s, 3H), 2.84 (m, 1H), 3.32-3.39 (m, 7H), 6.64 (d, J=8.1Hz, 2H), 7.07 (d, J=8.1Hz, 2H), 8.35 (s, 2H)
167: ¹H-NMR (CDCl₃)δ 1.44 (t, J=7.5Hz, 6H), 2.03 (s, 3H), 2.12 (s, 3H), 3.21-3.39 (m, 14H), 7.26-7.30 (m, 4H), 8.33 (s, 2H)
168: ¹H-NMR(CDCl₃)δ 1.30 (s, 3H), 1.32 (s, 3H), 1.44 (t, J=7.5Hz, 6H), 2.03 (s, 3H), 2.13 (s, 3H), 3.18-3.39 (m, 9H), 4.20 (m, 1H), 5.18 (d, J=8.1Hz, 1H),7.26-7.30 (m, 4H), 8.29 (s, 2H)
169: ¹H-NMR (CDCl₃)δ 1.29 (d, J=6.3Hz, 12H), 2.02 (s, 12H), 3.85-3.99 (m, 2H), 4.41 (d, J=7.5Hz, 2H), 6.44-6.50 (m, 2H), 7.22-7.29 (m, 2H), 7.87-7.93 (m, 2H)
170: ¹H-NMR (CDCl₃)δ 1.29 (d, J=6.3Hz, 12H), 2.10 (s, 6H), 3.36 (s, 6H), 3.86-3.99 (m, 2H), 4.46 (d, J=7.8Hz, 2H), 6.46 (d, J=8.4Hz, 2H), 7.42 (dd, J=8.4, 2.1Hz, 2H), 8.04 (d, J=2.1Hz, 2H)
171: ¹H-NMR (CDCl₃)δ 1.29 (d, J=6.3Hz, 12H), 2.29 (s, 6H), 3.85-4.00 (m, 2H), 4.43 (d, J=8.1Hz, 2H), 6.43 (d, J=8.4Hz, 2H), 7.11 (s, 2H), 7.45 (dd, J=8.4, 2.1Hz, 2H), 8.10 (d, J=2.1Hz,
172: ¹H-NMR (CDCl₃)δ 1.29 (d, J=6.3Hz, 12H), 1.98 (s, 3H), 2.00 (s, 3H), 2.02 (s, 3H), 3.84-4.01 (m, 2H), 4.46 (d, J=7.5Hz, 1H), 4.56 (d, J=7.5Hz, 1H), 4.9 (brs, 1H), 6.47 (d, J=8.7Hz, 1H), 6.51 (d, J=8.7Hz, 1H), 7.22-7.30 (m, 1H), 7.32-7.40 (m, 1H), 7.87-7.92 (m, 1H), 7.99-8.05 (m, 1H)
173: ¹H-NMR (CDCl₃)δ 1.26 (d, J=6.3Hz, 6H), 1.28 (d, J=6.3Hz, 6H), 2.27 (s, 3H), 3.80 (s, 3H), 3.86-4.00 (m, 2H), 4.45 (d, J=7.8Hz, 2H), 6.42 (d, J=8.4Hz, 1H), 6.44 (d, J=8.4Hz, 1H), 6.81 (s, 1H), 7.17 (s, 1H), 7.46 (dd, J=8.4, 2.1Hz, 1H), 7.68 (dd, J=8.4, 2.1Hz, 1H), 8.12 (d, J=2.1Hz, 1H), 8.29 (d, J=2.1H 1H)
174: ¹H-NMR (CDCl₃)δ 1.27 (d, J=6.3Hz, 12H), 3.79 (s, 6H), 3.87-4.02 (m, 2H), 4.48 (d, J=7.8Hz, 2H), 6.43 (d, J=8.6Hz, 2H), 6.92 (s, 2H), 7.70 (dd, J=8.6, 2.3Hz, 2H), 8.30 (d, J=2.3Hz, 2H)
175: ¹H-NMR (CDCl₃)δ 1.29 (d, J=6.3Hz, 12H), 2.01 (s, 6H), 2.10 (s, 3H), 3.35 (s, 3H), 3.87-4.00 (m, 2H), 4.40-4.50 (m, 2H), 6.47 (d, J=8.4Hz, 2H), 7.21-7.27 (m, 1H), 7.42 (dd, J=8.4, 2.4Hz, 1H), 7.87-7.91 (in, 1H), 8.01-8.05 (m, 1H)
176: ¹H-NMR (CDCl₃)δ1.49-1.85 (m, 12H), 2.02-2.16 (m, 4H), 2.10 (s, 6H), 3.36 (s, 6H), 3.97-4.09 (m, 2H), 4.65 (d, J=6.9Hz, 2H), 6.49 (d, J=8.4Hz, 2H), 7.42 (dd, J=8.4, 2.1Hz, 2H), 8.03 (d, J=2.1Hz, 2H)
177: ¹H-NMR (CDCl₃)δ 1.49-1.85 (m, 12H), 1.98-2.14 (m, 4H), 2.02 (s, 12H), 3.96-4.09 (m, 2H), 4.61 (d, J=6.9Hz, 2H), 6.50 (d, J=8.4Hz, 2H), 7.23-7.29 (m, 2H), 7.89 (s, 2H)
178: ¹H-NMR (CDCl₃)δ 1.78-1.81 (m, 8H), 2.02-2.11 (m, 8H), 2.17 (s,6H), 2.20 (s, 3H), 4.00-4.04 (m,2H), 6.52-6.60 (m,2H), 6.98 (m, 1H), 7.33-7.35 (m, 1H), 7.49-7.52 (m, 1H), 7.80-7.82 (m, 1H), 7.99-8.02 (m, 1H)
179: ¹H-NMR (CDCl₃)δ 1.49-1.83 (m, 12H), 1.84-2.15 (m, 4H), 1.98 (s, 3H), 1.99 (s, 3H), 2.02 (s, 3H), 3.87.4.10 (m, 2H), 4.62 (d, J=6.6Hz, 1H), 4.74 (d, J=7.5Hz, 1H), 4.85 (brs, 1H), 6.49 (d, J=8.4Hz, 1H), 6.54 (d, J=8.4Hz, 1H), 7.27 (d, J=8.4Hz, 1H), 7.38 (d, J=8.4Hz, 1H), 7.89 (s, 1H), 7.89 (s, 1H)
180: ¹H-NMR (CDCl₃) δ 1.49-1.83 (m, 12H), 1.98-2.14 (m, 4H), 2.01 (s, 6H), 2.09 (s, 3H), 3.35 (s, 3H), 3.96-4.08 (m, 2H), 4.59-4.66 (m, 2H), 6.49 (d, J=8.4Hz, 2H), 7.23-7.27 (m, 1H), 7.40-7.45 (m, 1H), 7.85-8.01 (m, 1H), 8.00-8.05 (m, 1H)
181: ¹H-NMR (CDCl₃)δ 0.26-0.33 (m, 4H), 0.55-0.63 (m, 4H), 1.08-1.23 (m, 2H), 2.01 (s, 12H), 3.19 (dd, J=6.9, 5.4Hz, 4H), 4.68 (t, J=5.4Hz, 2H), 6.46-6.72 (m, 2H), 7.23-7.29 (m, 2H), 7.88-7.93 (m, 2H)
182: ¹H-NMR (CDCl₃)δ 0.26-0.33 (m, 4H), 0.55-0.64 (m, 4H), 1.07-1.22 (m, 2H), 2.09 (s, 6H), 3.19 (dd, J=6.9, 5.3Hz, 4H), 3.36 (s, 6H), 4.71 (t, J=5.3Hz, 2H), 6.49 (d, J=8.6Hz, 2H), 7.42 (dd, J=8.6, 2.1Hz, 2H), 8.05 (d, J=2.1Hz, 2H)
183: ¹H-NMR (CDCl₃)δ 1.75 (s, 6H), 1.78 (s, 6H), 2.01 (s, 6H), 2.09 (s, 3H), 3.35 (s, 3H), 3.91 (t, J=5.9Hz, 2H), 4.45-4.53 (m, 2H), 5.34-5.42 (m, 2H), 6.48 (d, J=8.7Hz, 2H), 7.22-7.27 (m, 1H), 7.43 (dd, J=8.7, 2.3Hz, 1H), 7.87-7.93 (m, 1H), 8.05 (d, J=2.3Hz, 1H)
184: ¹H-NMR (CDCl₃)δ 0.26-0.37 (m, 4H), 0.55-0.65 (m, 4H), 1.09-1.21 (m, 2H), 2.01 (s, 6H), 2.09 (s, 3H), 3.19 (dd, J=6.9, 5.4Hz, 4H), 3.35 (s, 3H), 4.65-4.74 (m, 2H), 6.49 (d, J=8.6Hz, 2H), 7.23-7.26 (m, 1H), 7.42 (dd, J=8.6, 2.1Hz, 1H), 7.87-7.92 (m, 1H), 8.04 (d, J=2.1Hz 1H)
185: ¹H-NMR (CDCl₃)δ 1.32 (t, J=7.2Hz, 6H), 2.01 (s, 12H), 3.37 (dq, J=7.2, 5.4Hz, 4H), 4.45-4.54 (m, 2H), 6.46-6.53 (m, 2H), 7.24-7.31 (m, 2H), 7.87-7.93 (m, 2H)
186: ¹H-NMR (CDCl₃)δ 1.28 (s, 3H), 1.29 (s, 3H), 1.30 (s, 3H), 1.31 (s, 3H), 2.07 (s,6H), 2.20 (s, 3H), 3.87-3.96 (m,2H), 4.58 (brs, 2H), 6.43-6.50 (m,2H), 7.00 (s, 1H), 7.26-7.28 (m, 1H), 7.42-7.45 (m, 1H), 7.87-7.89 (m, 1H), 7.96-8.16 (m, 1H)
187: ¹H-NMR (CDCl₃)δ 0.28-0.32 (m, 4H), 0.55-0.62 (m, 4H), 1.08-1.20 (m, 2H), 2.06 (s,6H), 2.20 (s, 3H), 3.16-3.21 (m,4H), 4.69-4.70 (m, 2H), 6.45-6.51 (m,2H), 6.99 (s, 1H), 7.26 (dd, J=8.4, 2.4hZ, 1H), 7.43 (dd, J=8.4, 2.4hZ, 1H), 7.91 (d, J=1.5hZ, 1H), 8.09 (d, J=1.5Hz, 1H)
188: ¹H-NMR (CDCl₃)δ 1.75 (s, 6H), 1.78 (s, 6H), 2.06 (s,6H), 2.20 (s, 3H), 3.86-3.94 (m,4H), 4.52-4.65 (m, 2H), 5.33-5.42 (m, 2H), 6.45-6.51 (m,2H), 7.00 (s, 1H), 7.26-7.29 (m, 1H), 7.43-7.46 (m, 1H), 7.91 (d, J=1.5Hz, 1H), 8.10 (d, J=1.5Hz, 1H)
189: ¹H-NMR (CDCl₃)δ 1.28 (s, 3H), 1.29 (s, 3H), 1.30 (s, 3H), 1.31 (s, 3H), 2.13 (s,3H), 2.33 (s, 3H), 3.90-3.97 (m,2H), 4.50-4.57 (m, 2H), 6.42-6.49 (m,2H), 7.05 (s, 1H), 7.31 (dd, J=8.4Hz, 2.4, 1H), 7.40 (dd, J=8.4Hz, 2.4, 1H) , 7.93 (d, J=2.4Hz, 1H), 8.06 (d, J=2.4Hz, 1H)
190: ¹H-NMR (CDCl₃)δ 0.27-0.33 (m, 2H), 0.55-0.63 (m, 2H), 1.08.1.22 (m, 1H), 1.29 (d, J=6.3Hz, 6H), 2.01 (s, 12H), 3.20 (dd, J=6.6, 5.3Hz, 2H), 3.85-4.00 (m, 1H), 4.44 (d, J=5.1Hz, 1H), 4.69 (t, J=5.3Hz; 1H), 6.47 (dd, J=8.4, 1.2Hz, 1H), 6.50 (dd, J=8.7, 1.2Hz, 1H), 7.22-7.30 (m, 2H), 7.86-7.93 (m, 2H)
191: ¹H-NMR (CDCl₃)δ1.29 (d, J=6.6Hz, 6H), 1.75 (s, 3H), 1.78 (s, 3H), 2.01 (s, 12H), 3.86-3.99 (m, 3H), 4.40-4.52 (m, 2H), 5.34-5.42 (m, 1H), 6.44-6.52 (m, 214), 7.22-7.30 (m, 2H), 7.86-7.94 (m, 2H)
192: ¹H-NMR (CDCl₃)δ 1.29 (d, J=6.3Hz, 6H), 1.50-1.82 (m, 6H), 1.90-2.14 (m, 2H), 2.01 (s, 12H), 3.85-4.07 (m, 2H), 4.42 (d, J=8.1 1H), 4.63 (d, J=6.6Hz, 1H), 6.44-6.52 (m, 2H), 7.22-7.30 (m, 2H), 7.87-7.91 (m, 2H)
193: ¹H-NMR (DMSO-d₆)δ 1.28 (d, J=6.6Hz, 12H), 1.98 (s, 12H), 4.03-4.17 (m, 2H), 7.19 (d, J=8.7Hz, 2H), 7.61-7.75 (m, 2H), 8.99 (brs, 2H)
194: ¹H-NMR (DMSO-d₆)δ 1.27 (d, J=6.3Hz, 12H), 2.05 (s, 6H), 3.36 (s, 6H), 4.04-4.18 (m, 2H), 7.14 (d, J=9.0Hz, 2H), 7.77-7.88 (m, 2H), 8.90 (brs, 2H)
195: ¹H-NMR (CDCl₃)δ 1.04 (d, J=6.3Hz, 12H), 1.95 (nona, J=6.3Hz, 2H), 2.01 (s, 12H), 3.14 (dd, J=6.3, 5.4Hz, 4H), 4.66 (t, J=5.4Hz, 2H), 6.49 (dd, J=8.4, 1.5Hz, 2H), 7.23-7.30 (m, 2H), 7.86-7.92 (m, 2H)
196: ¹H-NMR (CDCl₃)δ 1.03 (d, J=6.5Hz, 12H), 1.95 (nona, J=6.5Hz, 2H), 2.10 (s, 6H), 3.14 (dd, J=6.5, 5.4Hz, 4H), 3.36 (s, 6H), 4.71 (t, J=5.4Hz, 2H), 6.48 (d, J=8.4Hz, 2H), 7.42 (dd, J=8.4, 2.1H 2H), 8.03 (d, J=2.1Hz, 2H)
197: ¹H-NMR (CDCl₃)δ 0.25-0.33 (m, 4H), 0.54-0.62 (m, 4H), 1.08-1.21 (m, 2H), 2.29 (s, 6H), 3.18 (dd, J=6.9, 5.4Hz, 4H), 4.73 (t, J=5.4Hz, 2H), 6.46 (dd, J=8.4, 0.6Hz, 2H), 7.11 (s, 2H), 7.46 (dd, J=8.4, 2.4Hz, 2H), 8.11 (dd, J=2.4, 0.6Hz, 2H)
198: ¹H-NMR (CDCl₃)δ 1.05 (t, J=7.2Hz, 6H), 1.71 (sextet, J=7.2Hz, 4H), 2.01 (s, 12H), 3.29 (dt, J=7.2, 5.7Hz, 4H), 4.59 (t, J=5.7Hz, 2H), 6.45-6.54 (m, 2H), 7.22-7.31 (in, 2H), 7.84-7.93 (m, 2H)
199: ¹H-NMR (CDCl₃)δ 1.04 (t, J=7.2Hz, 6H), 1.71 (sextet, J=7.2Hz, 4H), 2.10 (s, 6H), 3.29 (dt, J=7.2, 5.4Hz, 4H), 3.36 (s, 6H), 4.62 (t, J=5.4Hz, 2H), 6.48 (d, J=8.4Hz, 2H), 7.43 (dd, J=8.4, 2.1Hz, 2H), 8.04 (d, J=2.1Hz, 2H)
200: ¹H-NMR (D₂O)δ 0.33-0.40 (m, 4H), 0.63-0.70 (m, 4H), 1.16-1.29 (m, 2H), 2.04 (s, 12H), 3.30 (d, J=7.2Hz, 4H), 7.16 (d, J=8.9Hz, 2H), 7.57 (s, 2H), 7.68 (d, J=8.9Hz, 2H)
201: ¹H-NMR (CDCl₃)δ 0.24-0.32 (m, 4H), 0.52.0.62 (m, 4H), 1.05-1.21 (m, 2H), 2.26 (s, 3H), 3.17 (dd, J=7.2, 5.4Hz, 2H), 3.19 (dd, J=7.2, 5.4Hz, 2H), 3.79 (s, 3H), 4.70 (t, J=5.4Hz, 1H), 4.73 (t, J=5.4Hz, 1H), 6.45 (dd, J=8.7, 0.6Hz, 1H), 6.47 (dd, J=8.7, 0.6Hz, 1H), 6.81 (s, 1H), 7.17 (s, 1H), 7.47 (dd, J=8.7, 2.4Hz, 1H), 7.68 (dd, J=8.7, 2.4Hz, 1H), 8.13 (dd, J=2.4, 0.6Hz, 1H), 8.30 (dd, J=2.4, 0.6Hz, 1H)
202: ¹H-NMR (D₂O)δ 0.33-0.40 (m, 4H), 0.63-0.71 (m, 4H), 1.15-1.30 (m, 2H), 2.03 (s, 3H), 2.04 (s, 3H), 2.11 (s, 3H), 3.30 (dd, J=7.2Hz, 4H), 3.46 (s, 3H), 7.16 (d, J=9.0Hz, 1H), 7.17 (d, J=9.0Hz, 1H), 7.61 (s, 1H), 7.67-7.72 (m, 1H), 7.72 (s, 1H), 7.78-7.84 (m, 1H)
203: ¹H-NMR (CDCl₃)δ 1.26 (d, J=6.3Hz, 6H), 1.29 (d, J=6.3Hz, 6H), 3.51 (s, 3H), 3.75 (s, 3H), 3.86-4.01 (m, 2H), 4.48-4.58 (m, 2H), 6.43 (s, 1H), 6.44 (dd, J=8.7, 0.6Hz, 1H), 6.45 (dd, J=8.7, 0.6Hz, 1H), 7.58 (dd, J=8.7, 2.4Hz, 1H), 7.76 (dd, J=8.7, 2.4Hz, 1H), 8.22 (dd, J=2.4, 0.6Hz, 1H), 8.36 (dd, J=2.4, 0.6Hz, 1H)
204: ¹H-NMR (CDCl₃)δ 1.27 (d, J=6.3Hz, 6H), 1.28 (d, J=6.3Hz, 6H), 2.36 (s, 3H), 3.86-4.02 (m, 2H), 4.39-4.51 (m, 2H), 6.43 (d, J=8.7Hz, 1H), 6.44 (d, J=8.7Hz, 1H), 7.23-7.27 (m, 1H), 7.34-7.40 (m, 2H), 7.44 (dd, J=8.7, 2.4Hz, 1H), 7.68 (dd, J=8.7, 2.4Hz, 1H), 8.09 (d, J=2.4Hz, 1H), 8.36 (d, J=2.4Hz, 1H)
205: ¹H-NMR (CDCl₃)δ 0.23-0.31 (m, 4H), 0.52-0.60 (m, 4H), 1.04-1.20 (m, 2H), 2.25 (s, 3H), 3.12-3.20 (m, 4H), 4.75-4.84 (m, 2H), 6.45 (d, J=8.4Hz, 1H), 6.49 (d, J=8.9Hz, 1H), 6.87 (s, 1H), 7.12 (s, 1H), 7.49 (dd, J=8.4, 2.3Hz, 1H), 7.70 (dd, J=8.9, 2.3Hz, 1H), 8.14 (d, J=2.3Hz, 1H), 8.30 (d, J=2.3Hz, 1H)
206: ¹H-NMR (D₂O)δ 1.34 (t, J=7.2Hz, 6H), 2.01 (s, 12H), 3.45 (q, J=7.2Hz, 4H), 7.14 (d, J=9.3Hz, 2H), 7.57 (s, 2H), 7.63-7.69 (m, 2H)
207: ¹H-NMR (CDCl₃)δ 1.27 (d, J=6.6Hz, 6H), 1.28 (d, J=6.6Hz, 6H), 3.84-4.02 (m, 2H), 4.54-4.65 (m, 2H), 6.40 (d, J=8.7Hz, 1H), 6.47 (d, J=8.7Hz, 1H), 7.38 (dd, J=8.7, 2.4Hz, 1H), 7.43 (d, J=7.8Hz, 1H), 7.69 (dd, J=8.7, 2.4Hz, 1H), 7.71 (dd, J=7.8, 2.0Hz, 1H), 7.94 (d, J=2.0Hz, 1H), 8.09 (d, J=2.4Hz, 1H), 8.38 (d, J=2.4Hz, 1H)
208: ¹H-NMR (CDCl₃)δ 0.26-0.34 (m, 4H), 0.54-0.64 (m, 4H), 1.06-1.21 (m, 2H), 2.11 (s, 3H), 3.13-3.23 (m, 4H), 3.47 (s, 3H), 3.64 (s, 3H), 4.68-4.85 (m, 2H), 6.46 (d, J=8.7Hz, 1H), 6.48 (d, J=8.7Hz, 1H), 7.30 (dd, J=8.7, 2.3Hz, 1H), 7.69 (s, 1H), 7.74 (dd, J=8.7, 2.3Hz, 1H), 7.87 (d, J=2.3Hz, 1H), 8.34 (d, J=2.3Hz, 1H), 8.30 (d, J=2.3Hz, 1H)
209: ¹H-NMR (CDCl₃)δ 1.28 (d, J=6.3Hz, 6H), 1.28 (d, J=6.6Hz, 6H), 2.12 (s, 3H), 3.48 (s, 3H), 3.63 (s, 3H), 3.85-4.00 (m, 2H), 4.47 (d, J=7.8Hz, 1H), 4.52 (d, J=8.1H 1H), 6.43 (d, J=8.7Hz, 1H), 6.45 (d, J=8.7Hz, 1H), 7.29 (dd, J=8.7, 2.1Hz, 1H), 7.69 (s, 1H), 7.74 (dd, J=8.7, 2.1Hz, 1H), 7.86 (d, J=2.1Hz, 1H), 8.34 (d, J=2.3Hz, 1H), 8.33 (d, J=2.1Hz, 1H)
210: ¹H-NMR (CDCl₃)δ 1.27 (d, J=6.6Hz, 6H), 1.28 (d, J=6.6Hz, 6H), 3.80 (s, 2H), 3.86-3.98 (m, 2H), 4.40-4.50 (m, 2H), 6.43 (d, J=8.7Hz, 1H), 6.45 (d, J=8.7Hz, 1H), 6.89 (d, J=1.7Hz, 1H), 6.96 (dd, J=8.0, 1.7Hz, 1H), 7.13 (d, J=8.0Hz, 1H), 7.57 (dd, J=8.7, 2.1Hz, 1H), 7.66 (dd, J=8.7, 2.1Hz, 1H), 8.19 (d, J=2.1Hz, 1H), 8.34 (d, J=2.1 1H)
211: ¹H-NMR (CDCl₃)δ 1.27 (d, J=6.6Hz, 6H), 1.28 (d, J=6.6Hz, 6H), 1.36 (t, J=6.9Hz, 3H), 2.27 (s, 3H), 3.84-4.00 (m, 2H), 4.02 (q, J=6.9Hz, 2H), 4.41-4.50 (m, 2H), 6.42 (d, J=8.6Hz, 1H), 6.43 (d, J=8.7Hz, 1H), 6.80(s, 1H), 7.17 (s, 1H), 7.45 (dd, J=8.6, 2.3Hz, 1H), 7.72 (dd, J=8.7, 2.1Hz, 1H), 8.11 (d, J=2.3Hz, 1H), 8.32 (d, J=2.1H, 1H)
212: ¹H-NMR (CDCl₃)δ 1.27 (d, J=6.3Hz, 12H), 3.87-4.00 (m, 2H), 4.48-4.57 (m, 2H), 6.45 (d, J=8.4Hz, 2H), 7.23-7.36 (m, 2H), 7.42 (t, J=8.1Hz, 1H), 7.64-7.72 (m, 2H), 8.29-8.33 (m, 1H), 8.36 (d, J=2.1Hz, 1H)
213: ¹H-NMR (CDCl₃)δ 1.28 (d, J=6.3Hz, 6H), 1.29 (d, J=6.3Hz, 6H), 2.99 (s, 3H), 3.85-4.00 (m, 2H), 4.64-4.75 (m, 2H), 6.45 (d, J=8.7Hz, 1H), 6.46 (d, J=8.7Hz, 1H), 6.82 (s, 1H), 7.25 (d, J=8.0Hz, 1H), 7.34 (dd, J=8.0, 1.7Hz, 1H), 7.42 (dd, J=8.7, 2.4Hz, 1H), 7.70 (dd, J=8.7, 2.6Hz, 1H), 7.76 (d, J=1.7Hz, 1H), 8.01 (d, J=2.4Hz, 1H), 8.36 (d, J=2.6Hz, 1H)
214: ¹H-NMR (CDCl₃)δ 1.18 (d, J=6.2Hz, 6H), 1.27 (d, J=6.3Hz, 6H), 1.28 (d, J=6.3Hz, 6H), 3.70 (sept, J=6.2Hz, 1H), 3.86-4.00 (m, 2H), 4.44-4.54 (m, 2H), 6.44 (d, J=8.7Hz, 1H), 6.46 (d, J=8.6Hz, 1H), 6.79 (d, J=1.8Hz, 1H), 6.85 (dd, J=7.8, 1.8Hz, 1H), 7.07 (d, J=7.8Hz, 1H), 7.50 (dd, J=8.7, 2.3Hz, 1H), 7.68 (dd, J=8.6, 2.1Hz, 1H), 8.14 (d, J=2.3Hz, 1H), 8.36 (d, J=2.1Hz, 1H)
215: ¹H-NMR (D₂O)δ 1.36 (d, J=6.3Hz, 6H), 1.78 (s, 3H), 1.81 (s, 3H), 2.04 (s, 12H), 3.92-4.02 (m, 1H), 4.02 (d, J=6.0Hz, 2H), 5.36-5.45 (m, 1H), 7.18-7.16 (m, 2H), 7.54-7.72 (m,2H)
216: ¹H-NMR (CDCl₃)δ 0.31-0.39 (m, 4H), 0.60-0.68 (m, 4H), 1.12-1.26 (m, 2H), 2.27 (s, 6H), 3.27 (d, J=6.9Hz, 4H), 7.09 (d, J=9.0Hz, 2H), 7.27 (s, 2H), 7.76 (s, 2H), 7.91 (d, J=9.0Hz, 2H)
217: ¹H-NMR (D₂O)δ 1.03 (d, J=6.0Hz, 12H), 2.01-2.10 (m, 2H), 2.10 (s, 6H), 3.26 (d, J=6.3Hz, 4H), 3.47 (s, 6H), 7.13-7.20 (m, 2H), 7.73-7.85 (m, 4H)
218: ¹H-NMR (D₂O)δ 0.30-0.41 (m, 4H), 0.59-0.71 (m, 4H), 1.12-1.27 (m, 2H), 2.25 (s, 3H), 3.20-3.34 (m, 4H), 3.86 (s, 3H), 6.98-7.14 (m, 3H), 7.33 (s, 1H), 7.79 (s, 1H), 7.88-7.97 (m, 2H), 8.05-8.12 (m, 1H)
219: ¹H-NMR (CDCl₃)δ 1.27 (d, J=6.6Hz, 6H), 1.28 (d, J=6.3Hz, 6H), 3.65 (s, 3H), 3.68 (s, 3H), 3.75 (s, 3H), 3.87-4.01 (m, 2H), 4.43 (d, J=7.2Hz, 2H), 4.47 (d, J=7.8Hz, 2H), 6.44 (d, J=8.7Hz, 2H), 6.65 (s, 1H), 7.52 (dd, J=8.7, 2.3Hz, 1H), 7.73 (dd, J=8.7, 2.1H 1H), 8.16 (d, J=2.3Hz, 1H), 8.31 (dd, J=2.1Hz, 1H)
220: ¹H-NMR (CDCl₃)δ 0.23-0.33 (m, 4H), 0.53-0.62 (m, 4H), 1.06-1.23 (m, 2H), 3.17 (dd, J=6.9, 5.4Hz, 2H), 3.19 (dd, J=6.9, 5.4Hz, 2H), 3.64 (s, 3H), 3.67 (s, 3H), 3.74 (s, 3H), 4.66-4.79 (m, 2H), 6.47 (d, J=8.7Hz, 2H), 6.65 (s, 1H), 7.53 (dd, J=8.7, 2.1 1H), 7.74 (dd, J=8.7, 2.4Hz, 1H), 8.17 (d, J=2.1Hz, 1H), 8.32 (dd, J=2.4Hz, 1H)
221: ¹H-NMR (D₂O) δ 1.26-1.40 (m, 12H), 2.35 (s, 3H), 3.86-4.02 (m, 2H), 7.02.7.11 (m, 2H), 7.32-7.41 (m, 1H), 7.48-7.54 (m, 1H), 7.58 (s, 1H), 7.76 (s, 1H), 7.85-7.92 (m, 1H), 8.03 (s, 1H), 8.12-8.20 (m, 1H)
222: ¹H-NMR (CDCl₃)δ 1.28 (d, J=6.5Hz, 12H), 3.96 (dsept, J=8.0, 6.5Hz, 2H), 4.64 (d, J=8.0Hz, 2H), 6.47 (dd, J=8.7, 0.6Hz, 2H), 7.57 (dd, J=8.7, 2.1Hz, 2H), 8.25 (dd, J=2.1, 0.6Hz, 2H)
223: ¹H-NMR (CDCl₃)δ 1.05 (t, J=7.2Hz, 6H), 1.71 (sextet, J=7.2Hz, 4H), 3.30 (dt, J=7.2, 5.4Hz, 4H), 4.79 (t, J=5.4Hz, 214), 6.49 (d, J=8.6Hz, 2H), 7.58 (dd, J=8.6, 1. 8Hz, 2H), 8.26 (d, J=1.8Hz, 2H)
224: ¹H-NMR (CDCl₃)δ 1.03 (d, J=6.6Hz, 12H), 1.94 (nona, J=6.6Hz, 2H), 3.15 (dd, J=6.6Hz, 4H), 4.84 (t, J=6.6Hz, 2H), 6.49 (d, J=8.9Hz, 2H), 7.58 (d, J=8.9Hz, 2H), 8.25 (s, 2H)
225: ¹H-NMR (CDCl₃)δ 0.25-0.33 (m, 4H), 0.55-0.63 (m, 4H), 1.06-1.18 (m, 2H), 3.20 (dd, J=6.9, 5.3Hz, 4H), 4.90 (t, J=5.3Hz, 2H), 6.50 (d, J=8.7Hz, 2H), 7.58 (dd, J=8.7, 2.1Hz, 2H), 8.26 (d, J=2.1Hz, 2H)
226: ¹H-NMR (CDCl₃)δ 1.74 (s, 6H), 1.77 (s, 6H), 3.91 (t, J=6.2Hz, 4H), 4.65-4.72 (m, 2H), 5.30-5.38 (m, 2H), 6.49 (d, J=9.0Hz, 2H), 7.57 (d, J=9.0Hz, 2H), 8.27 (s, 2H)
227: ¹H-NMR(CDCl₃) δ 1.34 (d, J=6.0Hz, 12H), 3.92-4.04 (m, 2H), 7.10-7.18 (m, 2H), 7.97-8.10 (m, 4H)
228: ¹H NMR (CDCl₃) δ 0.97 (t, J = 7.2 Hz, 6H), 1.60 (m, 12H), 2.08 (s, 3H), 2.11 (s, 3H), 3.30 (m, 1H), 3.33 (s, 3H), 3.39 (s, 3H), 4.35 (m, 1H), 6.64 (d, J = 8.4 Hz, 2H), 7.06 (d, J = 8.4 Hz, 2H), 8.29 (s, 2H) ppm
229: Mp 160-161°C; ¹H NMR (CDCl₃) δ 1.75 (s, 3H), 1.78 (s, 3H), 2.09 (s, 6H), 3.30 (s, 3H), 3.36 (s, 3H), 3,57 (s, 3H), 3.62 (s, 3H), 3.75 (d, J = 6.3Hz, 2H), 3.91 (m, 1H), 6.70 (d, J = 8.7Hz, 2H), 7.11 (d, J = 8.7Hz, 2H), 8.58 (s,2H) ppm
230: Mp 169-170°C; ¹H NMR (CDCl₃) δ 0.27-0.32 (m, 2H), 0.55-0.63 (m, 2H), 1.16 (m, 1H), 2.08 (s, 3H), 2.09 (s, 3H), 3.05 (d, J = 7.5Hz, 2H), 3.32 (s, 3H), 3.36 (s, 3H), 3.57 (s, 3H), 3.63 (s, 3H), 6.71 (d, J = 6.0Hz, 2H), 7.13 (d, J = 8.4Hz, 2H), 8.58 (s, 2H) ppm
231: Mp 153-154°C; ¹H NMR (CDCl₃) δ 1.04 (d, J= 6.6Hz, 6H), 1.96 (m, 1H), 2.08 (s, 3H), 2.09 (s, 3H), 3.00 (d, J = 6.6Hz, 2H), 3.33 (s, 3H), 3.36 (s, 3H), 3.57 (s, 3H), 3.62 (s, 3H), 6.74 (d, J = 6.0Hz, 2H), 7.12 (d, J = 8.4Hz, 2H), 8.56 (s, 2H) ppm
233: Mp 114-116°C; ¹H NMR (CDCl₃) δ 1.75 (s, 3H), 1.79 (s, 3H), 2.08 (s, 3H), 2.09 (s, 3H), 3.35 (s, 3H), 3.36 (s, 3H), 3.57 (s, 3H), 3.62 (s, 3H), 3.78 (d, J = 6.3Hz, 2H), 5.40 (m, 1H), 6.80 (t, J = 8.7Hz, 1H), 6.92-6.97 (m, 2H), 7.57 (s, 2H) ppm
234: Mp 185-186°C; ¹H NMR (CDCl₃) δ 0.28-0.33 (m, 2H), 0.58-0.65 (m, 2H), 1.18 (m, 1H), 2.07 (s, 3H), 2.09 (s, 3H), 3.05 (d, J = 6.9Hz, 2H), 3.34 (s, 3H), 3.36 (s, 3H), 3.57 (s, 3H), 3.62 (s, 3H), 6.76 (t, J = 8.4Hz, 1H), 6.90-6.99 (m, 2H), 8.57 (s, 2H) ppm
235: ¹H NMR (CDCl₃)δ 1.52-1.80 (m, 8H), 2.06 (s, 3H), 2.11 (s, 3H), 3.32 (s, 3H), 3.39 (s, 3H), 3.34 (m, 1H), 5.22 (m, 2H), 6.77 (d, J = 8.1 Hz, 2H), 7.09 (d, J = 8.1 Hz, 2H), 8.29 (s, 2H) ppm
236: ¹H NMR (CDCl₃) δ 1.65 (m, 12H), 2.08 (s, 3H), 2.12 (s, 3H), 3.32 (s, 3H), 3.39 (s, 3H), 3.82 (m, 1H), 4.35 (m, 1H), 6.67 (d, J = 8.7 Hz, 2H), 7.08 (d, J = 8.7 Hz, 2H), 8.29 (s, 2H) ppm
237: ¹H NMR (CDCl₃) δ 0.27 (m, 2H), 0.58 (m, 2H), 1.14 (m, 1H), 1.67 (m, 8H), 2.08 (s, 3H), 2.12 (s, 3H), 3.01 (d, J= 6.9 Hz, 2H), 3.31 (s, 3H), 3.39 (s, 3H), 4.35 (m, 1H), 6.68 (d, J = 8.4 Hz, 2H), 7.10 (d, J = 8.4 Hz, 2H), 8.29 (s, 2H) ppm
238: ¹H NMR (CDCl₃,) δ 1.03 (d, J=6.6Hz, 12H), 1.94 (nona, J= 6.6Hz, 2H), 3.15 (dd, J = 6.6Hz, 4H), 4.84 (t, J = 6.6Hz, 2H), 6.49 (d, J = 8.9Hz, 2H), 7.58 (d, J = 8.9Hz, 2H), 8.25 (s, 2H) ppm
239: ¹H NMR (CDCl₃) δ 0.23-0.33 (m, 4H), 0.53-0.62 (m, 4H), 1.06-1.23 (m, 2H), 3.17 (dd, J = 6.9, 5.4Hz, 2H), 3.19 (dd, J = 6.9, 5.4Hz, 2H), 3.64 (s, 3H), 3.67 (s, 3H), 3.74 (s, 3H), 4.66-4.79 (m, 2H), 6.47 (d, J = 8.7Hz, 2H), 6.65 (s, 1H), 7.53 (dd, J = 8.7, 2.1Hz, 1H), 7.74 (dd, J = 8.7, 2.4Hz, 1H), 8:17 (d, J = 2.1Hz, 1H), 8.32 (dd, J = 2.4Hz, 1H) ppm
240: ¹H NMR (CDCl₃) δ 0.25-0.33 (m, 4H), 0.55-0.63 (m, 4H), 1.06-1.18 (m, 2H), 3.20 (dd, J = 6.9, 5.3Hz, 4H), 4.90 (t, J = 5.3Hz, 2H), 6.50 (d, J = 8.7Hz, 2H), 7.58 (dd, J = 8.7, 2.1Hz, 2H), 8.26 (d, J = 2.1Hz, 2H) ppm
241: ¹H NMR (CDCl₃) δ 1.74 (s, 6H), 1.77 (s, 6H), 3.91 (t, J = 6.2Hz, 4H), 4.65-4.72 (m, 2H), 5.30-5.38 (m, 2H), 6.49 (d, J = 9.0Hz, 2H), 7.57 (d, J = 9.0Hz, 2H), 8.27 (s, 2H) ppm
244: ¹H NMR (CDCl₃) δ 1.03 (t, J = 7.5Hz, 3H), 1.03 (t, J = 7.5Hz, 3H), 1.60-1.78 (m, 4H), 3.22-3.33 (m, 4H), 3.64 (s, 3H), 3.67 (s, 3H), 3.75 (s, 3H), 4.60 (t, J = 5.4Hz, 1H). 4.64 (t, J = 6.0Hz, 1H), 6.42-6.49 (m, 2H), 6.65 (s, 1H), 7.54 (dd, J = 8.6, 2.4Hz, 1H), 7.74 (dd, J = 8.9, 2.4Hz, 1H), 8.16 (d, J = 2.4Hz, 1H), 8.31 (d, J = 2.4Hz, 1H) ppm
245: ¹H NMR (CDCl₃) δ 1.74 (s, 3H), 1.74 (s, 3H), 1.77 (s, 6H), 3.64 (s, 3H), 3.68 (s, 3H), 3.74 (s, 3H), 3.86-3.94 (m, 4H), 4.47-4.56 (m, 2H), 5.36 (t, J = 6.0Hz, 2H), 6.46 (d, J = 8.7Hz, 2H), 6.65 (s, 1H), 7.53 (dd, J = 8.7, 2.4Hz, 1H), 7.73 (dd, J = 8.7, 2.4Hz, 1H), 8.17 (d, J = 2.4Hz, 1H), 8.32 (d, J = 2.4Hz, 1H) ppm
246: ¹H NMR (D2O) δ 1.34 (t, J = 6.1Hz, 12H), 3.90-4.01 (m, 2H), 7.02 (d, J = 9.4Hz, 1H), 7.10 (d, J = 9.4Hz, 1H), 7.62 (d, J = 8.1Hz, 1H), 7.81 (d, J = 9.4Hz, 1H), 7.84 (s, 1H), 7.99 (d, J = 8.1Hz, 1H), 8.15 (s, 1H), 8.19 (d, J = 9.4Hz, 1H), 8.36 (s, 1H) ppm
247: ¹H NMR (D2O) δ 1.29-1.41 (m, 15H), 2.25 (s, 3H), 3.88-3.98 (m, 2H), 4.15 (q, J = 6.7Hz, 2H), 7.00-7.08 (m, 3H), 7.34 (s, 1H), 7.77 (s, 1H), 7.88 (d, J = 8.7Hz, 1H), 7.97 (s, 1H), 8.08 (d, J = 9.9Hz, 1H) ppm
248: ¹H NMR (CDCl₃) δ 1.42-1.82 (m, 12H), 1.99 (d, J=2.4Hz, 3H), 2.01 (s, 3H), 2.03-3.12 (m, 4H), 2.12 (s, 3H),3.97-4.06 (m, 2H), 4.68 (bs, 2H), 6.50 (d, J = 8.4Hz, 2H), 7.25-7.41 (m, 2H), 7.86 (d, J=2. 1Hz, 1H),8.00 (d, J=2.1Hz, 1H)ppm
249: ¹H NMR (D2O) δ 0.31-0.33 (m, 4H), 0.60-0.63 (m, 4H), 1.11-1.24 (m, 2H), 2.05 (s, 6H), 2.16 (s, 3H),3.25 (d, J=6.9Hz, 4H), 4.51 (bs, 2H), 7.05 (m, 3H),7.57 (s, 1H), 7.64-7.68 (m, 1H), 7.68 (s, 1H), 7.81-7.84 (m, 1H)ppm
250: ¹H NMR (CDCl₃) 1.28 (d, J=6.6Hz, 6H), 1.29 (d, J=6.6Hz, 6H), 1,99 (d, J=3.0Hz, 3H), 2.01 (s, 3H), 2.12 (s, 3H), 4.87-5.00 (m, 2H), 4.49 (bs, 2H), 6.46 (d, J = 8.1Hz, 2H), 7.21-7.25 (m, 1H), 7.36-7.41 (m, 1H), 7.87 (d, J=2.1Hz 1H), 8.01 (d, J=2.1Hz 1H)ppm
251: ¹H NMR (CDCl₃) 1.75 (s, 6H), 1.78 (s, 6H), 1.99 (d, J=2.4Hz, 3H), 2.05 (s, 3H), 2.12 (s, 3H), 3.83-4.00 (m, 4H), 4.70 (bs, 2H), 5.32-5.40 (m, 2H), 6.49 (d, J = 8.1Hz, 2H), 7.33-7.42 (m, 2H), 7.88 (d, J=1.8Hz, 1H), 8.01 (d, J=1.8Hz, 1H)ppm
252: ¹H NMR (CDCl₃) 1.01-1.07 (m, 6H), 1.66-1.75 (m, 4H), 1.99(d, J=2.4Hz, 3H), 2.01 (s, 3H), 2.12 (s, 3H), 3.25-3.32 (m, 4H), 4.62 (bs, 2H), 6.49 (d, J = 8.1Hz 2H), 7.23-7.26 (m, 1H), 7.36-7.41 (m, 1H), 7.88 (d, J=1.8Hz, 1H), 8.01 (d, J=1.8Hz, 1H)ppm
253: ¹H NMR (D2O) δ 0.30-0.39 (m, 4H), 0.58-0.70 (m, 4H), 1.13-1.28 (m, 2H), 3.30 (d, J = 6.9Hz, 4H), 7.16 (d, J = 8.7Hz, 2H), 8.00- 8.08 (m, 4H) ppm
254: ¹H NMR (DMSO-d6) δ 1.17 (s, 6H), 1.19 (s, 6H), 2.01 (s, 3H), 2.23 (s, 3H), 4.02 (m, 2H), 6.40 (m, 2H), 6.51 (d, J = 5.4 Hz, 2H), 7.22 (dd, J = 2.4, 5.4 Hz, 2H), 7.84 (d, J = 2.4Hz, 2H), 10.05 (s, 2H)ppm
255: ¹H NMR (CDCl₃) δ 0.25-0.33 (m, 4H), 0.53-0.61 (m, 4H), 1.07-1.20 (m, 2H), 2.03 (s, 6H), 2.05 (s, 6H), 3.16 (dd, J = 6.9, 5.4Hz, 4H), 4.60-4.72 (m, 2H), 6.33 (s, 2H), 6.96 (s, 1H), 6.97 (s, 1H), 7.84 (s, 1H), 7.86 (s, 1H) ppm
256: ¹H NMR (CDCl₃)δ 0.23-0.32 (m, 4H), 0.52-0.62 (m, 4H), 1.06-1.20 (m, 2H), 2.04 (s, 3H), 2.06 (s, 3H), 2.13 (s, 3H), 3.12-3.19 (m, 4H), 3.71 (s, 3H), 4.61-4.75 (m, 2H), 6.31 (s, 1H), 6.34 (s, 1H), 6.66 (s, 1H), 6.99 (s, 1H), 7.88 (s, 1H), 7.91 (s, 1H) ppm
257: ¹H NMR (CDCl₃) δ 0.23-0.30 (m, 4H), 0.53-0.60 (m, 4H), 1.06-1.20 (m, 2H), 2.15 (s, 6H), 3.11-3.18 (m, 4H), 3.71 (s, 6H), 4.65-4.74 (m, 2H), 6.33 (s, 2H), 6.73 (s, 2H), 7.94 (s, 2H)ppm
258: ¹H NMR (DMSO-d6) δ 1.19 (s, 6H), 1.21 (s, 6H), 2.17 (s, 3H), 2.21 (s, 3H), 4.08 (m, 2H), 6.35 (s, 1H), 6.56 (m, 2H), 7.38 (m, 2H), 7.99 (m, 2H), 11.91 (s, 1H) ppm
259: ¹H NMR (DMSO-d6) δ 1.18 (s, 6H), 1.19 (s, 6H), 2.26 (s, 6H), 3.55 (m, 2H), 6.40 (brs, 2H), 6.69 (d, J = 8.4 Hz, 2H), 7.06 (dd, J = 2.4, 8.4 Hz, 2H), 7.83 (d, J = 2.4 Hz, 2H), 8,64 (s, 2H) ppm
260: ¹H NMR (DMSO-d6) δ 1.18 (s, 6H), 1.20 (s, 6H), 2.08 (s, 3H), 2.22 (s, 3H), 3.22 (s, 3H), 4.06 (m, 2H), 6.36 (s, 1H), 6.54 (m, 2H), 7.33 (m, 2H), 7.90 (m, 2H) ppm
261: ¹H NMR (DMSO-d6) δ 0.95 (s, 6H), 0.97 (s, 6H), 1.91 (m, 2H), 2.19 (s, 3H), 2.23 (s, 3H), 3.11 (m, 4H), 6.60 (m, 4H), 7.35 (m, 2H), 7.84 (s, 1H), 7.96 (m, 2H), 11.89 (s, 1H) ppm
262: ¹H NMR (DMSO-d6) δ 0.94 (s, 6H), 0.96 (s, 6H), 1.88 (m, 2H), 2.08 (s, 3H), 2.22 (s, 3H), 3.12 (m, 4H), 3.22 (s, 3H), 6.58 (m, 4H), 7.32 (m, 2H), 7.85 (s, 1H), 7.90 (m, 2H) ppm
263: ¹H NMR (DMSO-d6) δ 0.24 (m, 4H), 0.47 (m, 4H), 1.11 (m, 2H), 2.18 (s, 3H), 2.22 (s, 3H), 3.19 (m, 4H), 6.65 (m, 4H), 7.37 (m, 2H), 7.84 (s, 1H), 7.96 (m, 2H), 11.90 (s, 1H) ppm
264: ¹H NMR (DMSO-d6) δ 0.24 (m, 4H), 0.47 (m, 4H), 1.11 (m, 2H), 2.08 (s, 3H), 2.22 (s, 3H), 3.20 (m, 4H), 3.22 (s, 3H), 6.62 (m, 4H), 7.34 (m, 2H), 7.86 (s, 1H), 7.92 (m, 2H), 11.90 (s, 1H) ppm
265: ¹H NMR (CDCl₃), 1.27 (d, J=2.4Hz, 6H), 1.29 (d, J=2.4Hz, 6H), 2.19 (d, J=3.0Hz, 3H), 3.88-4.00 (m, 2H), 4.51 (bs, 2H), 6.43-6.47 (m, 2H), 6.97-7.03 (m, 1H), 7.37 (d, J=8.4Hz, 1H), 7.66 (d, J=8.4Hz, 1H), 8.02 (s, 1H), 8.29 (s, 1H)ppm
266: ¹H NMR (CDCl₃), 0.28-0.29 (m, 4H), 0.55-0.61 (m, 4H), 1.12-1.17 (m, 2H), 2.18 (d, J=3.0Hz, 3H), 3.17-3.21 (m, 4H), 4.67-4.81 (m, 2H), 6.46-6.51 (m, 2H), 6.97-7.03 (m, 1H), 7.38 (d, J=8.1Hz, 1H), 7.66 (d, J=8.1Hz, 1H), 8.03 (s, 1H), 8.30 (s, 1H)ppm
267: ¹H NMR (CDCl₃), 1.40-1.82 (m, 12H), 1.99-2.15 (m, 4H), 2.19 (d, J=3.0Hz, 3H), 3.99-4.06 (m, 2H), 4.74(bs, 2H), 6.46-6.51 (m, 2H), 6.97-7.03 (m, 1H), 7.38 (d, J=8.7Hz, 1H), 7.67 (d, J=8.7Hz, 1H), 8.01 (s, 1H), 8.29 (s, 1H)ppm
268: ¹H NMR (CDCl₃), 1.01 (d, J=3.0Hz, 6H), 1.03 (d, J=3.0Hz, 6H), 1.91-1.96 (m, 2H), 2.19 (d, J=3.0Hz, 3H), 3.12-3.16 (m, 4H), 4.70-4.80 (m, 2H), 6.45-6.50 (m, 2H), 6.97-7.03 (m, 1H), 7.36-7.40 (m, 1H), 7.64-7.68 (m, 1H), 8.01 (s, 1H), 8.29 (s, 1H)ppm
269: ¹H NMR (CDCl₃) 1.74 (s, 6H), 1.77 (s, 6H), 2.19 (d, J=3.0Hz, 3H), 3.90 (t, J=6.0Hz, 4H), 4.55-4.61 (m, 2H), 5.34-5.38 (m, 2H), 6.45-6.49 (m, 2H), 6.97-7.03 (m, 1H), 7.36-7.39 (m, 1H), 7.65-7.68 (m, 1H), 8.03 (s, 1H), 8.31 (s, 1H)ppm
270: ¹H NMR (DMSO-d6) δ 0.28-0.36 (m, 4H), 0.51-0.60 (m, 4H), 1.05-1.20 (in, 2H), 2.15 (s, 6H), 3.24-3.35 (m, 4H), 3.72 (s, 6H), 7.00 (s, 2H), 7.02 (s, 2H), 7.72 (s, 2H), 8.74 (brs, 2H), 13.7 (brs, 2H), ppm
271: ¹H NMR (DMSO-d6) δ 1.71 (s, 6H), 1.72 (s, 6H), 2.18 (s, 3H), 2.23 (s, 3H), 3.88 (m, 4H), 5.33 (m, 2H), 6.56 (m, 4H), 7.35 (m, 2H), 7.85 (s, 1H), 7.98 (m, 2H), 11.90 (s, 1H) ppm
272: ¹H NMR (DMSO-d6) δ 1.70 (s, 6H), 1.72 (s, 6H), 2.08 (s, 3H), 2.22 (s, 3H), 3.22 (s, 3H), 3.88 (m, 4H), 5.34 (m, 2H), 6.57 (m, 4H), 7.34 (m, 2H), 7.86 (s, 1H), 7.96 (m, 2H) ppm
273: ¹H NMR (DMSO-d6) δ 1.29 (s, 6H), 1.32 (s, 6H), 1.66 (s, 6H), 3.95 (m, 2H), 4.59 (brs, 2H), 6.53 (d, J = 8.7 Hz, 4H), 7.45 (dd, J = 2.4, 8.7 Hz, 4H), 8.05 (d, J = 2.4Hz, 4H), 8.70 (s, 2H) ppm
274: ¹H NMR (DMSO-d6) δ 1.16 (d, J = 6.3Hz, 12H), 3.97-4.11 (m, 2H), 6.47-6.55 (m, 4H), 7.59 (s, 4H), 7.70 (dd, J = 8.7, 2.4Hz, 2H), 8.33 (d, J = 2.4Hz, 2H) ppm
275: ¹H NMR (DMSO-d6) δ 0.18-0.25 (m, 4H), 0.42-0.48 (m, 4H), 1.00-1.15 (m, 2H), 3,17 (t, J = 6.3Hz, 4H), 6.58 (d, J = 8.6Hz, 2H), 6.74 (t, J = 5.6Hz, 2H), 7.60 (s, 4H), 7.72 (dd, J = 8.6, 2.6Hz, 2H), 8.33 (d, J = 2.6Hz, 2H) ppm
276: ¹H NMR (CDCl₃) 1.29 (d, J=6.3Hz, 12H), 2.12-2.13 (m, 6H), 3.89-3.97 (m, 2H), 4.58-4.61 (m, 2H), 6.47 (d, J=8.4Hz, 2H), 7.37 (dd, J=2.4,8.4Hz, 2H), 8.00 (d, J=2.4Hz, 2H) ppm
277: ¹H NMR (CDCl₃) 1.45-1.82 (m, 12H), 2.03-2.12 (m, 4H), 2.13 (s, 6H), 3.99-4.13 (m, 2H), 4.80-4.84 (m, 2H), 6.49 (d, J=8.7Hz, 2H), 7.39 (dd, J=2.4,8.7Hz, 2H), 7.99 (d, J=2.4Hz, 2H) ppm
278: ¹H NMR (DMSO-d6) δ 1.69 (s, 6H), 1.70 (s, 6H), 3.86 (t, J = 5.9Hz, 4H), 5.25-5.33 (m, 2H), 6.54 (d, J = 8.3Hz, 2H), 6.63-6.70 (m, 2H), 7.60 (s, 4H), 7.72 (dd, J = 8.3, 2.4Hz, 2H), 8.40 (d, J = 2.4Hz, 2H) ppm
279: ¹H NMR (CDCl₃) 1.74 (s, 6H), 1.77 (s, 6H), 2.12-2.13 (m, 6H), 3.91 (t, J=6.0Hz, 2H), 4.59-4.63 (m, 2H), 5.33-5.39 (m, 2H), 6.48 (d, J=8.4Hz, 2H), 7.39 (dd, J=2.1,8.4Hz, 2H), 8.03 (d,J=2.1Hz,2H) ppm
280: ¹H NMR (CDCl₃) 0.26-0.31 (m, 4H), 0.55-0.62 (m, 4H), 1.11-1.17 (m, 2H), 2.12 (m, 6H), 3.16-3.21 (m, 4H), 4.80-4.84 (m, 2H), 6.49 (d, J=8.7Hz, 2H), 7.38 (dd, J=2.1,8.7Hz, 2H), 8.02 (d, J=2.1Hz, 2H) ppm
281: Mp 125-126°C; ¹H NMR (CDCl₃) δ 1.75 (s, 3H), 1.78 (s, 3H), 2.28 (s, 3H), 2.31 (s, 3H), 3.07 (s, 3H), 3.46 (s, 3H), 3,91 (t, J = 6.0Hz, 2H), 5.35 (m, 1H), 6.48 (d, J = 8.4Hz, 1H), 7.13 (d, J = 7.8Hz, 2H), 7.45-7.51 (m, 2H), 7.37 (dd, J = 2.7, 5.7Hz, 1H), 8.10 (d, J =2.1Hz, 1H), 8.41 (dd, J = 0.9, 2.4Hz, 1H) ppm
282: ¹H NMR (CDCl₃) 1.02 (d, J=6.6Hz, 12H), 1.90-1.99 (m, 2H), 2.12 (s, 6H), 3.11-3.16 (m, 4H), 4.71-4.80 (m, 2H), 6.49 (d, J=8.7Hz, 2H), 7.38 (dd, J=1.8, 8.7Hz, 2H), 8.00 (d, J=1.8Hz, 2H) ppm
283: ¹H NMR (CDCl₃) δ 1.27 (d, J = 6.6Hz, 6H), 1.28 (d, J = 6.6Hz, 6H), 2.30 (s, 3H), 4.51 (t, J = 8.3Hz, 2H), 6.43 (d, J = 8.7Hz, 1H), 6.44 (d, J = 8.7Hz, 1H), 6.99 (d, J = 11.1Hz, 1H), 7.24-7.28 (m, 1H), 7.43 (dd, J = 8.7, 2.6Hz, 1H), 7.64-7.70 (m, 1H), 8.08 (d, J = 1.8Hz, 1H), 8.30 (s, 114) ppm
284: ¹H NMR (CDCl₃) δ 1.27 (d, J = 6.3Hz, 12H), 2.03 (s, 6H), 2.06 (s, 6H), 3.81-3.98 (m, 2H), 4.36 (d, J = 8.4Hz, 2H), 6.30 (s, 2H), 6.96 (s, 1H), 6.98 (s, 1H), 7.83 (s, 1H), 7.86 (s, 1H) ppm
285: ¹H NMR (CDCl₃) δ 1.27 (d, J = 6.6Hz, 12H), 2.15 (s, 6H), 3.71 (s, 6H), 3.84-3.96 (m, 2H), 4.51 (d, J = 7.2Hz, 2H), 6.30 (s, 2H), 6.73 (s, 2H), 7.93 (s, 2H) ppm
286: ¹H NMR (CDCl₃) δ 0.25-0.33 (m, 4H), 0.54-0.62 (m, 4H), 1.07-1.20 (m, 2H), 2.30 (s, 3H), 3,18 (dd, J = 6.9, 5.4Hz, 4H), 4.78-4.90 (m, 2H), 6.47 (d, J = 8.7Hz, 1H), 6.48 (d, J = 8.7Hz, 1H), 6.99 (d, J = 11.4Hz, 1H), 7.23-7.27 (m, 1H), 7.43 (dd, J = 8.7, 2.3Hz, 1H), 7.65-7.72 (m, 1H), 8.08 (d, J = 2.3Hz, 1H), 8.30 (s, 1H) ppm
287: Mp 154-155°C; ¹H NMR (CDCl₃) δ 1.76 (s, 3H), 1.79 (s, 3H), 1.96 (s, 6H), 2.02 (s, 6H), 3.09 (d, J = 3.3Hz, 3H), 3,48 (s, 3H), 3.92 (m, 2H), 5.38 (m, 1H), 6.52 (d, J = 8.4Hz, 1H), 7.25-7.28 (m, 1H), 7.53-7.55 (m, 2H), 7.90 (dd, J = 2.1, 4.5Hz, 1H), 8.24 (m1H) ppm
288: Mp 129-130°C; ¹H NMR (CDCl₃) δ 1.76 (s, 3H), 1.79 (s, 3H), 1.97 (s, 6H), 2.03 (s, 3H), 2.19 (s, 3H), 3.47 (s, 3H), 3,92(m, 2H), 5.38 (m, 1H),6.52 (d, J = 8.17Hz, 1H), 7.23-7.30 (m, 2H), 7.56-7.61 (m, 1H), 7.89 (t, J = 2.1Hz, 1H), 8.32 (m, 1H) ppm
289: ¹H NMR (CDCl₃) δ 1.02 (d, J = 6.6Hz, 6H), 1.02 (d, J = 6.3Hz, 6H), 1.86-2.01 (m, 2H), 2.30 (s, 3H), 3.11-3.17 (m, 4H), 4.76-4.90 (m, 2H), 6.46 (d, J = 8.6Hz, 1H), 6.47 (d, J = 8.6Hz, 1H), 6.99 (d, J = 11.4Hz, 1H), 7.23-7.28 (m, 1H), 7.44 (dd, J = 8.6, 2.3Hz, 1H), 7.69 (dt, J = 8.6, 2.1Hz, 1H), 8.07 (d, J = 2.3Hz, 1H), 8.25-8.32 (m, 1H) ppm
290: ¹H NMR (CDCl₃) δ 1.74 (s, 6H), 1.77 (s, 6H), 2.30 (s, 3H), 3.90 (t, J = 5.7Hz, 4H), 4.56-4.68 (m, 2H), 5.30-5.39 (m, 2H), 6.45 (d, J = 8.6Hz, 1H), 6.46 (d, J = 8.6Hz, 1H), 6.99 (d, J = 11.8Hz, 1H), 7.26 (d, J = 8.3Hz, 1H), 7.43 (dd, J = 8.6, 2.3Hz, 1H), 7.68 (dt, J = 8.3, 1.8Hz, 1H), 8.09 (d, J = 2.2Hz, 1H), 8.27-8.34 (s, 1H) ppm
291: ¹H NMR (CDCl₃) δ 1.27 (d, J = 6.6Hz, 12H), 2.06 (s, 6H), 2.18 (s, 6H), 3.74-3.86 (m, 2H), 4.50-4.60 (m, 2H), 6.29 (d, J = 8.4Hz, 2H), 6.96 (s, 1H), 6.98 (s, 1H), 7.26 (d, J = 8.4Hz, 2H)ppm
292: ¹H NMR (CDCl₃) δ 1.26 (d, J = 6.6Hz, 12H), 2.28 (s, 6H), 3.71 (s, 6H), 3.71-3.85 (m, 2H), 4.48-4.65 (m, 2H), 6.30 (d, J = 8.4Hz, 2H), 6.73 (s, 2H), 7.36 (d, J = 8.4Hz, 2H) ppm
293: ¹H-NMR (CDCl₃) δ 1.27 (d, J=6.3Hz, 6H), 1.28(d, J=6.3Hz, 6H), 3.78-3.94 (m, 2H), 4.95 (d, J=6.3Hz, 2H), 5.14 (bs, 1H), 6.50 (d, J=9.0Hz, 2H), 7.58 (d, J=8.1H 2H), 8.11 (B, 2H), ppm
294: ¹H-NMR (CDCl₃) δ 1.73 (s, 6H), 1.77 (s, 6H), 3.87 (bs, 4H), 5.05 (bs, 1H), 5.29-5.34 (m, 2H), 6.51 (d, J=8.7Hz, 2H), 7.59 (d, J=8.4Hz, 2H), 8.14 (s, 2H), ppm
295: ¹H-NMR (CDCl₃) δ 0.27-0.31 (m, 4H), 0.57-0.62 (m, 4H), 1.08-1.17 (m, 2H), 3.16 (dd, J=5.1Hz, 4H), 5.30 (bs, 3H), 6.53 (d, J=8.7Hz, 2H), 7.59 (d, J=8.7Hz, 2H), 8.14 (s, 2H), ppm
296: Mp 142-144°C; ¹H NMR (CDCl₃) δ 1.74 (s, 6H), 1.77 (s, 6H), 3.77 (s, 3H), 3.89-3.91 (m, 4H), 4.75-4.79 (m, 2H), 5.31-5.36 (m, 2H), 6.46 (dd, J = 6.6, 8.4Hz, 2H), 7.23 (dd, J = 6.3, 9.6Hz, 1H), 7.46-7.49 (m, 1H), 7.64-7.68 (m, 1H), 8.09 (s, 1H), 8.29 (s, 1H) ppm; IR (KBr) 3420, 3236, 1740, 1607, 1542, 1447, 1298, 1221 cm⁻¹
297: Mp 165-167°C; ¹H NMR (CDCl₃) δ 0.27-0.29 (m, 4H), 0.57-0.61 (m, 4H), 1.08-1.15 (m, 2H), 3.15-3.21 (m, 4H), 3.77 (s, 3H), 4.95-4.99 (m, 2H), 6.47 (dd, J = 6.9, 8.4Hz, 2H), 7.23 (dd, J = 6.6, 9.9Hz, 1H), 7.46-7.50 (m, 1H), 7.64-7.68 (m, 1H), 8.08 (s, 3H), 8.28 (s, 3H) ppm; IR (KBr) 3240, 1731, 1608, 1543, 1447, 1427, 1382, 1327, 1299, 1265, 1222, 1159, 1110 cm⁻¹
298: ¹H-NMR (CDCl₃) δ 1.28 (d, J=6.0Hz, 6H), 1.29 (d, J=6.6Hz, 6H), 3.70 (d, J=0.9Hz, 3H), 3.89-3.99 (m, 2H), 4.66-4.73 (m, 2H), 6.47 (d, J=8.7Hz, 2H), 7.58 (d, J=7.5Hz, 2H), 8.22 (d, J= 4.5Hz, 2H), ppm
299: ¹H-NMR (CDCl₃) δ 1.75 (s, 6H), 1.77 (s, 6H), 3.70 (d, J=0.9Hz, 3H), 3.92 (dd, J=5.6Hz, 4H), 4.71-4.79 (m, 2H), 5.33-5.37 (m, 2H), 6.49 (d, J=9.0Hz, 2H), 7.59 (d, J=7.5Hz, 2H), 8.25 (d, J=4.2Hz, 2H), ppm
300: ¹H-NMR (CDCl₃) δ 0.28-0.30 (m, 4H), 0.57-0.61 (m, 4H), 1.09-1.17 (m, 2H), 3.20 (dd, J=6.5Hz, 5.3Hz, 4H), 3.70 (s, 3H), 4.96 (bs, 1H), 5.04 (bs, 1H), 6.51 (d, J=8.4Hz, 2H), 7.59 (d, J=7.5Hz, 2H), 8.23 (d, J=3.9Hz, 2H), ppm
301: ¹H NMR (D2O) δ 0.31-0.48 (m, 4H), 0.57-0.78 (m, 4H), 1.10-1.30 (m, 2H), 2.30 (s, 3H), 3,20-3.34 (m, 4H), 7.07-7.22 (m, 3H), 7.46 (d, J = 8.1Hz, 1H), 7.79 (s, 1H), 7.90 (d, J = 9.9Hz, 1H), 8.01 (s, 1H), 8.10 (d, J = 8.1Hz, 1H) ppm
302: ¹H NMR (CDCl₃) δ 0.25-0.32 (m, 4H), 0.54-0.62 (m, 4H), 1.08-1.20 (m, 2H), 2.06 (s, 6H), 2.19 (s, 6H), 3.12 (t, J = 6.0Hz, 4H), 4.74-4.88 (m, 2H), 6.30 (d, J = 8.4Hz, 2H), 6.95 (s, 1H), 6.97 (s, 1H), 7.26 (d, J = 8.4Hz, 2H) ppm
303: ¹H NMR (CDCl₃) δ 0.24-0.31 (m, 4H), 0.54-0.60 (m, 4H), 1.06-1.20 (m, 2H), 2.29 (s, 6H), 3.12 (t, J = 5.9Hz, 4H), 3.71 (s, 6H), 4.75-4.87 (m, 2H), 6.30 (d, J = 8.6Hz, 2H), 6.72 (s, 2H), 7.36 (d, J = 8.6Hz, 2H) ppm
304: ¹H NMR (DMSO-d6) δ 1.26 (d, J = 6.3Hz, 12H), 2.07 (s, 6H), 2.27 (s, 6H), 4.08-4.22 (m, 2H), 7.02 (d, J = 9.0Hz, 2H), 7.14 (s, 2H), 7.75 (t, J = 9.6Hz, 2H), 8.33-8.65 (m, 2H) ppm
305: ¹H NMR (CDCl₃) δ 0.27-0.34 (m, 2H), 0.55-0.63 (m, 2H), 1.08-1.22 (m, 1H), 1.97 (s, 6H), 2.03 (s, 6H), 3.20 (dd, J = 6.9, 5.4Hz, 2H), 4.63-4.72 (m, 1H), 6.51 (d, J = 8.4Hz, 1H), 6.55-6.59 (m, 1H), 6.96-7.03 (m, 1H), 7.25-7.27 (m, 1H), 7.31 (dd, J = 8.4, 2.3Hz, 1H), 7.40-7.48 (m, 2H), 7.95 (d, J = 2.3Hz, 1H), 8.25 (s, 1H) ppm
306: ¹H NMR (CDCl₃) δ 1.04 (t, J = 7.2Hz, 3H), 1.29 (d, J = 6.6Hz, 6H), 1.69 (sextet, J = 7.2Hz, 2H), 1.99 (s, 6H), 2.01 (s, 6H), 3.14 (t, J = 7.2Hz, 2H), 3.64 (brs, 1H), 3.85-4.04 (m, 1H), 4.39 (d, J = 8.4Hz, 1H), 6.46 (dd, J = 8.7, 0.8Hz, 1H), 6.65-6.71 (m, 2H), 6.93-6.99 (m, 2H), 7.27 (dd, J = 8.7, 2.1Hz, 1H), 7.91 (d, J = 2.1, 0.8Hz, 1H) ppm
307: ¹H NMR (CDCl₃) δ 1.02 (d, J = 6.6Hz, 6H), 1.26 (d, J = 6.3Hz, 6H), 1.93 (sept., J = 6.6Hz, 1H), 2.10 (s, 3H), 3.09-3.15 (m, 2H), 3.48 (s, 3H), 3.51-3.72 (m, 1H), 3.62 (s, 3H), 4.72 (brs., 1H), 6.34-6.48 (m, 3H), 7.21 (t, J = 8.4Hz, 1H), 7.31 (dd, J = 8.7, 2.1Hz, 1H), 7.69 (s, 1H), 7.86 (d, J = 1.5Hz, 1H) ppm
308: ¹H NMR (CDCl₃) δ 1.03 (t, J = 7.5Hz, 3H), 1.26 (d, J = 6.0Hz, 6H), 1.61-1.76 (m, 2H), 2.10 (s, 3H), 3.22-3.32 (m, 2H), 3.48 (s, 3H), 3.50-3.78 (m, 1H), 3.62 (s, 3H), 4.63 (brs, 1H), 6.34-6.49 (m, 3H), 7.21 (t, J = 8.4Hz. 1H), 7.32 (dd, J = 8.7, 2.4Hz, 1H), 7.69 (s, 1H), 7.87 (d, J = 1.5Hz, 1H) ppm
309: ¹H NMR (CDCl₃)δ 0.24-0.32 (m, 2H), 0.53-0.62 (m, 2H), 1.06-1.21 (m, 1H), 1.26 (d, J = 6.0Hz, 6H), 2.10 (s, 3H), 3.13-3.20 (m, 2H), 3.48 (s, 3H), 3.50-3.72 (m, 1H), 3.62 (s, 3H), 4.74 (brs, 1H), 6.34-6.50 (m, 3H), 7.21 (t, J = 8.4Hz, 1H), 7.31 (dd, J = 8.7, 2.4Hz, 1H), 7.70 (s, 1H), 7.88 (d, J = 1.5Hz, 1H) ppm
310: ¹H NMR (CDCl₃)δ 1.26 (d, J = 6.0Hz, 6H), 1.46-1.84 (m, 8H), 2.10 (s, 3H), 3.48 (s, 3H), 3.54-3.75 (m, 1H), 3.62 (s, 3H), 3.95-4.08 (m, 1H), 4.62-4.73 (broad, 1H), 6.33-6.49 (m, 3H), 7.21 (t, J = 8.4Hz, 1H), 7.32 (dd, J = 8.7, 2.4Hz, 1H), 7.69 (s, 1H), 7.87 (d, J = 1.5Hz, 1H)ppm
311: ¹H NMR (CDCl₃)δ 1.28 (d, J = 6.6Hz, 6H), 2.11 (s, 3H), 3.47 (s, 3H), 3.62 (s, 3H), 3.84-3.98 (m, 1H), 4.23-4.30 (m, 1H), 4.37 (d, J = 5.1H 2H), 4.41-4.50 (m, 1H), 6.40-6.52 (m, 3H), 7.19-7.42 (m, 7H), 7.68 (s, 1H), 7.87 (d, J = 1.5Hz, 1H). ppm
312: ¹H NMR (CDCl₃) δ 1.26 (d, J = 6.0Hz, 6H), 1.28 (d, J = 6.6Hz, 6H), 2.11 (s, 3H), 3.48 (s, 3H), 3.57-3.72 (m, 1H), 3.62 (s, 3H), 3.85-3.98 (m, 1H), 4.46 (d, J = 6.9Hz, 1H), 6.33-6.46 (m, 3H), 7.21 (t, J = 2.7Hz, 1H), 7.30 (dd, J = 8.7, 2.4Hz, 1H), 7.69 (s, 1H), 7.87 (d, J = 1.8Hz,1H)ppm
313: ¹H NMR (CDCl₃) δ 1.02 (d, J = 6.6Hz, 6H), 1.28 (d, J = 6.6Hz, 6H), 1.93 (m, 1H), 2.11 (s, 3H), 3.77-4.02 (m, 2H), 6.26-6.56 (m, 3H), 6.45 (d, J = 6.3Hz, 1H), 7.15-7.36 (m, 2H), 7.68 (s, 1H), 7.87 (d, J = 1.8Hz, 1H) ppm
314: ¹H NMR (CDCl₃) δ 1.03 (t, J = 7.2Hz, 3H), 1.28 (d, J = 6.3Hz, 6H), 1.68 (q, J = 7.2Hz, 2H), 2.11 (s, 3H), 3.05-3.17 (m, 2H), 3.47 (s, 3H), 3.62 (s, 3H), 3.77-4.01 (m, 2H), 4.40-4.55 (m, 1H), 6.34-6.50 (m, 3H), 7.21 (t, J = 8.4Hz, 1H), 7.30 (dd, J = 8.7, 2.4Hz, 1H), 7.68 (s, 1H), 7.87 (d, J = 1.8Hz, 1H) ppm
315: ¹H NMR (CDCl₃) δ 0.23-0-32 (m, 2H), 0.54-0.63 (m, 2H), 1.03-1.21 (m, 1H), 1.28 (d, J = 6.3Hz, 6H), 2.11 (s, 3H), 2.99 (d, J = 6.6Hz, 2H), 3.47 (s, 3H), 3.62 (s, 3H), 3.87-3.98 (m, 1H), 4.03 (brs, 1H), 4.45 (d, J = 7.8Hz, 1H), 6.36-6.49 (m, 3H), 7.22 (t, J = 8.4Hz, 1H), 7.30 (dd, J =8.4, 2.4Hz, 1H), 7.68 (s, 1H), 7.87 (d, J = 1.5Hz, 1H). ppm
316: ¹H NMR (CDCl₃) δ 1.28 (d, J = 6.6Hz, 6H), 1.74 (s, 3H), 1.78 (s, 3H), 2.11 (s, 3H), 3.47 (s, 3H), 3.62 (s, 3H), 3.73 (d, J = 5.7Hz, 2H), 3.76-3.99 (m, 2H), 4.49 (brs, 1H), 5.24-5.40 (m, 1H), 6.30-6.54 (m, 3H), 7.14-7.36 (m, 3H), 7.69 (s, 1H), 7.87 (s, 1H) ppm
317: ¹H NMR (CDCl₃) δ 1.03 (d, J = 6.6Hz, 6H), 1.29 (d, J = 6.6Hz, 6H), 1.94 (nona, J = 6.6Hz, 1H), 1.99 (s, 6H), 2.01 (s, 6H), 2.99 (d, J = 6.9Hz, 2H), 3.70 (brs, 1H), 3.86-3.99 (m, 1H), 4.39 (d, J =6.9Hz, 1H), 6.46 (dd, J = 8.7, 0.6Hz, 1H), 6.68 (d, J = 8.6Hz, 2H), 6.96 (d, J = 8.6Hz, 2H), 7.27 (dd, J = 8.7, 2.3Hz, 1H), 7.91 (dd, 2.3, 0.6Hz, 1H) ppm
318: ¹H NMR (CDCl₃) δ 0.24-0.30 (m, 2H), 0.55-0.63 (m, 2H), 1.05-1.19 (m, 1H), 1.29 (d, J = 6.6Hz, 6H), 3.02 (d, J = 6.9Hz, 2H), 3.90-4.02 (m, 1H), 4.06 (brs, 1H), 4.66 (d, J = 7.5Hz, 1H), 6.47 (d, J = 8.7Hz, 1H), 6.70 (d, J = 8.7Hz, 2H), 7.34 (d, J = 8.7Hz, 2H), 7.55-7.60 (m, 1H), 8.23-8.27 (m, 1H) ppm
319: ¹H NMR (CDCl₃) δ 1.05 (t, J = 7.2Hz, 3H), 1.71 (sext, J = 7.2Hz, 2H), 1.97 (s, 6H), 2.03 (s, 6H), 3.26-3.33 (m, 2H), 4.57 (t, J = 5.3Hz, 1H), 6.50 (d, J = 8.4Hz, 1H), 6.53-6.60 (m, 1H), 6.97-7.03 (m, 1H), 7.25-7.27 (m, 1H), 7.32 (dd, J = 8.4, 2.1Hz, 1H), 7.40-7.48 (m, 2H), 7.95 (d, J=2.1Hz 1H), 8.25 (brs, 1H) ppm
320: ¹H NMR (CDCl₃) δ 1.28 (d, J = 6.3Hz, 6H), 1.99 (s, 6H), 2.00 (s, 6H), 3.85-3.98 (m, 1H), 4.40 (d, J = 7.8Hz, 1H), 4.69 (s, 4H), 6.46 (d, J = 8.7Hz, 1H), 6.79 (d, J = 7.5Hz, 2H), 6.95 (d, J = 7.5Hz, 2H), 7.26-7.40 (m, 11H), 7.90 (d, 2.1Hz, 1H) ppm
321: ¹H NMR (CDCl₃)δ 1.04 (d, J = 6.6Hz, 6H), 1.96 (nona, J = 6.6Hz, 1H), 1.97 (s, 6H), 2.03 (s, 6H), 4.61-4.68 (m, 1H), 6.50 (d, J = 8.6Hz, 1H), 6.53-6.59 (m, 1H), 6.97-7.02 (m,1H 7.25-7.27 (m, 1H), 7.31 (dd, J=8.6, 2.3Hz, 1H), 7.40-7.48 (m, 2H), 7.93 (d, J = 2.3Hz, 1H), 8.23 (brs, 1H) ppm
322: ¹H NMR (CDCl₃) δ 1.29 (d, J = 6.9Hz, 6H), 1.31 (d, J = 6.3Hz, 6H), 1.95 (s, 6H), 2.01 (s, 6H), 2.56 (sept, J = 6.9Hz, 1H), 3.84-3.96 (m, 1H), 4.92 (brs, 1H), 6.51 (d, J = 8.4Hz, 1H), 7.13 (d, J = 8.7Hz, 2H), 7.25 (s, 1H), 7.30 (dd, J = 8.4, 2.3Hz, 1H), 7.55-7.67 (m, 2H), 7.86 (d, 2.3Hz, 1H) ppm
323: ¹H NMR (CDCl₃) δ 1.76 (s, 3H), 1.78 (s, 3H), 1.97 (s, 6H), 2.03 (s, 6H), 3.92 (t, J = 5.9Hz, 2H), 4.42-4.50 (m, 1H), 5.39 (t, J = 5.9Hz, 1H), 6.50 (d, J = 8.7Hz, 1H), 6.55-6.60 (m, 1H), 6.98-7.02 (m, 1H), 7.24-7.27 (m,1H), 7.31 (dd, J = 8.7, 2.2Hz, 1H), 7.40-7.48 (m, 2H), 7.96 (d, J = 2.2Hz, 1H), 8.25 (brs, 1H) ppm
324: Mp 184-187°C
325: Mp 178-180°C
326: Mp 217-219°C
327: Mp 175-177°C
328: Mp 169-171°C
329: Mp 130-131°C
330: Mp 135-138°C
331: Mp 152-153°C
332: ¹H NMR (CDCl₃) δ 1.30 (d, J = 6.3Hz, 6H), 1.87 (s, 6H), 1.99 (s, 6H), 3.85-3.97 (m, 1H), 4.48-4.58 (m, 1H), 6.47 (d, J = 8.4Hz, 1H), 6.83 (brs, 1H), 7.02.7.16 (m, 4H), 7.25 (dd, J = 8.4, 1.8Hz, 1H), 7.41-7.48 (m, 2H), 7.52-7.58 (m, 1H), 7.76-7.82 (m, 2H), 7.87 (d, J = 1.8Hz, 1H) ppm
333: ¹H NMR (CDCl₃) δ 1.43 (d, J = 6.3Hz, 6H), 1.97 (s, 6H), 1.99 (s, 6H), 3.74-3.88 (m, 1H), 6.88 (d, J = 9.0Hz, 1H), 7.11-7.18 (m, 2H), 7.44-7.62 (m, 4H), 7.62-7.70 (m, 2H), 7.83 (dd, J = 9.0, 2.4Hz, 1H), 7.90-7.96 (m, 3H) ppm
334: ¹H NMR (D2O) δ 1.39(d, J = 6.3Hz, 6H), 1.60-1.80 (s, 6H), 1.99(s, 6H), 2.02-2.18 (m, 2H), 2.19 (s, 3H), 3.82-3.95 (m, 1H), 4.00-4.15 (m, 1H), 7.06 (d, J = 8.7Hz, 1H), 7.14 (s, 1H), 7.38 (d, J = 8.7Hz, 2H), 7.56 (d, J = 8.7Hz, 2H), 7.72 (s,1H), 7.83 (d, J=8.7Hz, 2H) ppm
335: ¹H NMR (D2O) δ 0.35-0.40 (m, 2H), 0.69-0.78 (m, 2H), 1.10-1.20 (m, 1H), 1.62-1.85 (m, 6H), 1.99 (s, 6H), 2.12-2.18 (m, 2H), 2.19 (s, 3H), 3.40 (d, J = 9.3Hz, 2H), 4.05-4.13 (m, 1H), 7.04-7.08 (m, 1H), 7.14 (s, 1H), 7.35-7.38 (m, 2H), 7.58-7.64 (m, 2H), 7.72 (s, 1H) 7.81-7.85 (m, 1H)ppm
336: Anal. Calcd. For C28H32F1N3O1·1.7 HCl ·0.9 AcOEt: C, 64.67%, H, 7.02%, N, 7.16%, F, 3.24%, Cl, 10.27%; Found; C, 64.46%, H, 6.99%, N, 7.71%, F, 3.46%, Cl, 9.79%
337: ¹H NMR (CDCl₃) δ 1.28 (d, J = 6.3Hz, 6H), 2.07 (s, 3H), 3.19 (s, 3H), 3.45 (s, 3H), 3,67 (s, 3H), 3.86 ∼3.99 (m, 1H), 6.68 - 6.82 (m, 2H), 7.39 (d, J = 9.0Hz, 1H), 7.45 (s, 1H), 7.48 (s, 1H), 7.59 (dd, J = 9.0 & 2.1Hz, 1H), 7.85 (s, 1H), 8.02(s, 1H). ppm
338: ¹H NMR (CDCl₃) δ 1.28 (d, J = 6.3Hz, 6H), 1.45-1.79 (m, 6H), 2.00-2.12 (m, 2H), 3.84 (quint, J = 6.6Hz, 1H), 3.85-4.02 (m, 1H), 4.64 (d, J = 8.1Hz, 1H), 6.47 (d, J = 8.7Hz, 1H), 6.68 (d, J = 8.9Hz, 2H), 7.33 (d, J = 8.9Hz, 2H), 7.58 (dd, J = 8.7, 1.8Hz, 1H), 8.25 (d, J = 1.8Hz, 1H) ppm
339: ¹H NMR (CDCl₃) δ 1.28 (d, J = 6.3Hz, 6H), 1.74 (s, 3H), 1.77 (s, 3H), 3.74 (d, J = 6.6Hz, 2H), 3.85 (brs, 1H), 3.85-4.04 (m, 1H), 4.66 (d, J = 6.9Hz, 1H), 5.35 (t, J = 6.6Hz, 1H), 6.47 (d, J = 8.6Hz, 1H), 6.69 (d, J = 8.9Hz, 2H), 7.34 (d, J = 8.9Hz, 2H), 7.58 (d, J = 8.6Hz, 1H), 8.25 (s, 1H) ppm
340: ¹H NMR (CDCl₃) δ 0.84.0.92 (m, 2H), 1.07-1.16 (m, 2H), 1.29 (d, J = 6.3Hz, 6H), 1.47-1.60 (m 1H), 1.95 (s, 6H), 2.01 (s, 6H), 3.86-3.99 (m, 1H), 4.44 (d, J = 6.0Hz, 1H), 6.47 (d, J = 8.3Hz, 1H), 7.12 (d, J = 8.4Hz, 2H), 7.26 (dd, J = 8.3, 1.9Hz, 1H), 7.45 (brs, 1H), 7.52-7.64 (m, 2H), 7.89 (d, 1.9Hz, 1H) ppm
341: ¹H NMR (DMSO-d6) δ 1.13 (d, J = 6.9Hz, 6H), 1.18 (d, J = 6.9Hz, 6H), 2.63 (sept, J = 6.9Hz, 2H), 4.00-4.13 (m, 1H), 6.59 (d, J = 8.9Hz, 1H), 6.89 (d, J = 7.5Hz, 1H), 7.48 (d, J = 8.6Hz, 2H), 7.54 (d, J = 8.9Hz, 1H), 7.79 (d, J = 8.6Hz, 2H), 8.15 (s, 1H), 10.07 (s, 1H) ppm
342: ¹H NMR (CDCl₃) δ 1.29 (d, J = 6.3Hz, 6H), 3.11 (s, 3H), 3.89-4.02 (m, 1H), 4.74 (d, J = 8.1Hz, 2H), 6.48 (d, J = 8.9Hz, 1H), 6.91 (brs, 1H), 7.35 (d, J = 8.7Hz, 2H), 7.51 (d, J = 8.7Hz, 2H), 7.58 (d, J = 8.9Hz, 1H), 8.26 (s, 1H) ppm
343: Mp 173-174°C
344: Mp 146-147°C
345: Mp 158-160°C
346: Mp 161-152.5°C
347: Mp 156-158°C
348: ¹H NMR (CDCl₃) δ 1.02 (d, J = 6.3Hz, 6H), 1.30 (d, J = 6.6Hz, 6H), 1.94 (sept. J = 6.6Hz, 1H), 2.05 (s, 3H), 2.59 (sept. J = 6.9Hz, 1H), 3.00 (d, J = 6.9Hz, 1H), 3.45 (s, 3H), 3.61 (s, 3H), 6.68 (d, J = 8.4Hz, 2H), 7.46 (d, J = 8.4Hz, 2H), 7.56 (dd, J = 8.7, 2.4Hz, 1H), 7.81 (d, J = 0.6Hz, 1H), 8.03 (brs, 1H), 8.07 (d.d, J = 2.4, 0.9Hz, 1H), 8.32 (d, J = 8.7Hz, 1H) ppm
349: ¹H NMR (CDCl₃) δ 1.29 (d, J = 6.3Hz, 6H), 1.96 (s, 6H), 2.00 (s, 6H), 3.85-3.98 (m, 1H), 4.45 (d, J = 8.1Hz, 1H), 4.54 (s, 2H), 6.47 (d, J = 8.1Hz, 1H), 6.71 (d, J = 8.0Hz, 2H), 6.98 (d, J = 8.0Hz, 2H), 7.05 (s, 2H), 7.27 (dd, J = 8.1, 2.4Hz, 1H), 7.89 (d, 2.4Hz, 1H) ppm
350: ¹H NMR (CDCl₃) δ 1.30 (d, J = 6.3Hz, 6H), 1.98 (s, 6H), 2.00 (s, 6H), 3.84-3.98 (m, 1H), 4.38 (s, 2H), 4.56-4.64 (m, 1H), 6.48 (d, J = 8.6Hz, 1H), 6.76 (d, J = 7.5Hz, 2H), 6.98 (d, J = 7.5Hz, 2H), 7.03 (s, 1H), 7.29 (dd, J = 8.6, 2.3Hz, 1H), 7.66 (s, 1H), 7.89 (d, 2.3Hz, 1H) ppm
351: ¹H NMR (CDCl₃) δ 1.26 (d, J = 6.3Hz, 6H), 2.05 (s, 3H), 3.45 (s, 3H), 3.60 (s, 3H), 3.70 (sept., J = 6.3Hz, 1H), 3.83 (s, 3H), 6.67 (d, J = 8.4Hz, 2H), .7.46 (d, J = 8.4Hz, 2H), 7.55 (dd, J = 8.7, 2.4Hz, 1H), 7.79 (s, 1H), 7.80 (s, 1H), 8.05 (d, J = 8.7Hz, 1H), 8.07 (d, J = 2.4Hz, 1H) ppm
352: ¹H NMR (CDCl₃) δ 1.26 (d, J = 6.3Hz, 6H), 1.30 (t, J = 7.2Hz, 3H), 2.10 (s, 3H), 3.28-3.40 (m, 2H), 3.44 (s, 3H), 3.62 (s, 3H), 3.69 (sept., J = 6.3Hz, 1H), 4.55 (brs., 1H), 6.45 (d, J = 8.7Hz, 1H), 6.65 (d, J = 8.7Hz, 2H), 7.31 (dd, J = 8.4-2.4Hz, 1H), 7.44 (d, J = 8.7Hz, 2H), 7.71 (s, 1H), 7.87 (d, J = 2.4Hz, 1H) ppm
353: ¹H NMR (CDCl₃) δ 1.26 (d, J = 6.3Hz, 6H), 1.54-1.80 (m, 6H), 1.97 (d,J=3.0Hz, 3H), 1.99 (s, 3H), 2.00-2.11 (m, 2H), 2.11 (s, 3H), 3.63-3.72 (m, 1H), 3.68 (bs,1H), 4.00-4.05 (m, 1H), 5.02 (bs, 1H), 6.52 (d, J = 8.4Hz, 1H), 6.66 (d, J = 8.4Hz, 2H), 6.92 (d, J = 8.4Hz, 2H), 7.42 (d, J = 8.4Hz, 2H), 7.97 (s, 1H) ppm
354: ¹H NMR (CDCl₃) δ 0.25-0.32 (m, 2H), 0.54-0.62 (m, 2H), 1.08-1.20 (m, 1H), 1.50-1.85 (m, 6H), 1.99 (s, 6H), 2.01 (s, 6H), 2.01-2.16 (m, 2H), 3.02 (d, J = 6.6Hz, 2H), 3.97-4.06 (m, 1H), 4.64 (d, J = 6.6Hz, 1H), 6.50 (d, J = 8.4Hz, 1H), 6.69 (d, J = 8.0Hz, 2H), 6.97 (d, J = 8.0Hz, 2H), 7.29 (dd, J = 8.4, 2.3Hz, 1H), 7.90 (d, 2.3Hz, 1H) ppm
355: ¹H NMR (CDCl₃) δ 1.29 (d, J = 6.9Hz, 6H), 1.50-1.90 (m, 6H), 1.95 (s, 6H), 2.01 (s, 6H), 2.01-2.16 (m, 2H), 2.56 (sept, J = 6.9Hz, 1H), 3.96-4.06 (m, 1H), 5.01 (brs, 1H), 6.54 (d, J = 8.4Hz, 1H), 7.13 (d, J = 8.4Hz, 2H), 7.25 (brs, 1H), 7.31 (dd, J = 8.4, 1.8Hz, 1H), 7.55-7.68 (m, 2H), 7.86 (d, 1.8Hz, 1H) ppm
356: ¹H NMR (CDCl₃) δ 1.04 (t, J = 7.2Hz, 3H), 1-50-1.90 (m, 7H), 1.99 (s, 6H), 2.01 (s, 6H), 2.01-2.15 (m, 2H), 3.14 (t, J = 7.2Hz, 2H), 3.96-4.06 (m, 1H), 4.67 (d, J = 5.7Hz, 1H), 6.50 (d, J = 8.3Hz, 1H), 6.68 (d, J = 8.6Hz, 2H), 6.96 (d, J = 8.6Hz, 2H), 7.29 (dd, J = 8.3, 2.0Hz, 1H), 7.89 (d, 2.0Hz, 1H) ppm
357: ¹H NMR (CDCl₃) δ 1.03 (d, J = 6.3Hz, 6H), 1.48-1.85 (m, 6H), 1.94 (nona, J = 6.3Hz, 1H), 1.99 (s, 6H), 2.01 (s, 6H), 2.01-2.14 (m, 2H), 2.99 (d, J = 6.3Hz, 2H), 3.72 (brs, 1H), 3.97-4.06 (m, 1H), 4.63 (d, J = 6.9Hz, 1H), 6.50 (d, J = 8.3Hz, 1H), 6.68 (d, J = 8.3Hz, 2H), 6.96 (d, J = 8.3Hz, 2H), 7.29 (dd, J = 8.3, 2.1Hz, 1H), 7.90 (d, 2.1Hz, 1H) ppm
358: ¹H NMR (CDCl₃) δ 0.26-0.31 (m, 2H), 0.56-0.62 (m, 2H), 1.10-1.19 (m, 1H), 1.30-1.87 (m, 6H), 1.96 (d,J=2.4Hz, 3H), 1.99 (s, 3H), 2.00-2.12 (m, 2H), 2.12 (s, 3H),.3.01 (d, J=8.4Hz, 2H), 3.99-4.05 (m, 1H), 3.99 (bs,1H), 4.70-4.81 (m, 1H), 6.50 (d, J = 8.4Hz, 1H), 6.69 (d, J = 8.4Hz, 2H), 6.94 (d, J = 8.4Hz, 2H), 7.40 (d, J = 8.4Hz, 2H), 7.99 (s, 1H) ppm
359: Mp 166-168°C
360: Mp 130-131°C
361: Mp 153-155°C
362: ¹H NMR (DMSO) δ 1.42-1.82 (m, 6H), 1.94 (s, 6H), 1.99-2.14 (m, 2H), 2.14 (s, 3H), 2.50 (s, 6H), 4.09-4.22 (m, 2H), 7.03(s, 1H), 7.17 (m, 3H),7.36 (d, J = 8.4Hz, 2H), 7.85 (s, 1H), 7.90 (d, J=8.4Hz, 1H), 9.14 (bs, 1H) ppm
363: ¹H NMR (CDCl₃) δ 1.30 (d,J=6.6Hz, 6H), 1.51-1.85 (m, 6H), 1.85-2.02 (m, 1H),1.96-2.15 (m, 2H), 2.05 (s, 6H), 2.20 (s, 3H), 2.98 (d, J=6.6Hz, 2H), 3.96-4.10 (m, 1H), 4.60-4.72 (m, 1H), 6.46 (d, J=8.4Hz, 1H), 6.68 (d, J=8.4Hz, 2H), 6.96(d, J = 8.4Hz, 2H),6.97 (s, 1H), 7.43-7.47 (m, 1H), 8.08 (s, 1H) ppm
364: ¹H NMR (CDCl₃) δ 1.48-1.82 (m, 6H), 1.98 (s, 6H), 2.01 (s, 6H), 2.01-2.13 (m, 2H), 3.98-4.08 (m, 1H), 4.37 (d, J = 4.5Hz, 2H), 4.60 (d, J = 6.6Hz, 2H), 6.29 (dd, J = 3.3, 0.8Hz, 1H), 6.36 (dd, J = 3.3, 2.0Hz, 1H), 6.50 (d, J = 8.1Hz, 1H), 6.75 (d, J= 8.3Hz, 2H); 6.99 (d, J = 8.3Hz, 2H), 7.28 (dd, J = 8.3, 2.3Hz, 1H), 7.40 (dd, J = 2.0, 0.8Hz, 114), 7.90 (d, 2.3Hz, 1H) ppm
365: ¹H NMR (CDCl₃) δ 1.02 (d, J = 6.6Hz, 6H), 1.93 (sept, J = 6.6Hz, 1H), 2.10 (s, 3H), 3.12 (t, J = 6.3Hz, 2H), 3.47 (s, 3H), 3.62 (s, 3H), 4.23-4.31 (m, 1H), 4.37 (d, J = 5.4Hz, 2H), 4.61.4.72 (m, 1H), 6.32-6.55 (m, 3H), 7.13-7.44 (m, 8H), 7.68 (s, 1H), 7.86 (d, J = 2.1Hz, 1H)ppm
366: ¹H NMR (CDCl₃) δ 1.25 (t, J = 7.2Hz, 3H), 1.28 (d, J = 6.6Hz, 6H), 2.11 (s, 3H), 2.94 -3.06 (m, 2H), 3.47 (s, 3H), 3.62 (s, 3H), 3.76-3.99 (m, 2H), 4.49 (brs, 1H), 6.30-6.54 (m, 3H), 7.14-7.36 (m, 3H), 7.69 (s, 1H), 7.87 (s, 1H) ppm
367: ¹H NMR (CDCl₃) δ 1.27 (d, J = 6.3Hz, 6H), 1.28 (d, J = 6.9Hz, 6H), 2.02 (s, 3H), 3.47 (s, 3H), 3.55 (s, 3H), 3.70 (sept., J = 6.9Hz, 1H), 4.89 (sept., J = 6.3Hz, 1H), 6.69 (d, J = 8.4Hz, 2H), 7.25 (d, J = 7.8Hz, 1H) , 7.47 (d, J = 8.4Hz, 2H), 7.64 (dd, J = 7.8, 2.1Hz, 1H), 7.88 (s, 1H), 8.39 (d, J 2.1Hz, 1H) ppm
368: Mp 167.5-168-C
369: Mp 161-163°C
370: Mp 145-147°C
371: Mp 192-193°C
372: Mp 175-176°C
373: Mp 130-131°C; ¹H NMR (CDCl₃) δ 1.02 (d, J = 6.6Hz, 6H), 1.25 (d, J = 6.6Hz, 6H), 1.92(m,1H),2.12 (s, 3H), 2.21 (s,3H), 3.11 (dd, J = 6.6, 6.0Hz, 2H),3.60 (s, 3H), 3.67 (m, 1H), 6.42 (dd, J = 8.4, 0.9Hz, 1H), 6.63 (d, J = 8.7Hz, 2H), 7.13 (d, J = 8.7Hz, 2H), 7.18 (s, 1H), 7.35 (dd, J = 8.4, 2.4Hz, 1H), 7.97 (dd, J = 2.4, 0.9Hz, 1H) ppm
374: Mp 141-143°C; ¹H NMR (CDCl₃) δ 1.03 (t, J = 7.2Hz, 3H), 1.25 (d, J = 6.3Hz, 6H), 1.60-1.76 (m, 2H), 2.12(s, 3H), 2.21 (s, 3H), 3.20-3.30 (m, 2H), 3.60 (s, 3H), 3.67 (m, 1H), 6.42 (dd, J = 8.4, 0.6Hz, 1H), 6.63 (d, J = 8.7Hz, 2H), 7.13 (d, J = 8.7Hz, 2H), 7.18(s, 1H), 7.35 (dd, J = 8.7, 2.4Hz, 1H), 7.97 (dd, J = 2.4, 0.6Hz, 1H) ppm
375: ¹H NMR (CDCl₃) δ 1.29 (d, J = 6.6Hz, 6H), 1.43 (t, J = 7.5Hz, 3H), 3.23 (q, J = 7.5Hz, 2H), 3.90-4.02 (m, 1H), 4.80 (d, J = 5.7Hz, 1H), 6.49 (d, J = 8.7Hz, 1H), 6.77 (brs, 1H), 7.34 (d, J = 8.4Hz, 2H), 7.50 (d, J = 8.4Hz, 2H), 7.59 (d, J = 8.7Hz, 1H), 8.25 (s, 1H) ppm
376: ¹H NMR (CDCl₃) δ 1.26 (d, J = 6.6Hz, 6H), 1.39-1.81 (m, 6H), 1.99 (s, 3H), 2.00-2.12 (m, 2H), 2.05 (s, 3H), 2.12 (s, 3H), 3.62-3.75 (m, 1H), 3.95-4.08 (m, 1H), 4.11 (bs, 1H), 4.78 (bs, 1H), 6.41(d, J = 9.0Hz, 1H), 6.66 (d, J = 9.0Hz, 2H), 6.90 (d, J = 9.0Hz, 2H), 7.31-7.36 (m, 1H), 7.92 (s, 1H) ppm
377: ¹H NMR (CDCl₃) δ 0.25-0.32 (m, 2H), 0.55-0.63 (m, 2H), 1.07-1.20 (m, 1H), 1.47-1.82 (m, 6H), 1.98 (s, 3H), 2.00-2.11 (m, 2H), 2.05 (s, 3H), 2.11 (s, 3H), 3.02 (d, J=7.2H_{z}, 2H), 3.85(bs, 1H), 3.98-4.06 (m, 1H), 4.60-4.80 (m, 1H), 6.50(d, J= 8.4Hz, 1H), 6.69 (d, J = 8.4Hz, 2H), 6.93 (d, J = 8.4Hz, 2H), 7.30-7.34 (m, 1H), 7.92 (s, 1H) ppm
378: ¹H NMR (CDCl₃) δ 1.51-1.87(m, 6H), 1.97 (s, 3H), 2.00-2.17 (m, 2H), 2.05 (s, 3H), 2.10 (s, 3H), 3.99-4.14 (m, 2H), 4.37 (s, 2H), 4.60.4.73 (m, 1H), 6.29 (s, 1H), 6.36 (s, 1H), 6.49(d, J = 8.4Hz, 1H), 6.75 (d, J = 8.4Hz, 2H), 6.95 (d, J = 8.4Hz, 2H), 7.29-7.33 (m, 1H), 7.40 (s, 1H), 7.92 (s, 1H) ppm
379: ¹H NMR (CDCl₃) δ 1.40.1.94(m, 12H), 1.99 (s, 3H), 2.01-2.17 (m, 4H), 2.06 (s, 3H), 2.12 (s, 3H), 3.71 (bs,1H), 3.78-3.90 (m, 1H), 3.95-4.10 (m,1H), 4.61-4.74 (m,1H), 6.50(d, J = 8.1Hz, 1H), 6.67 (d, J = 8.1Hz, 2H), 6.91 (d, J = 8.1Hz, 2H), 7.30-7.34 (m, 1H), 7.93 (s, 1H)ppm
380: ¹H NMR (CDCl₃) δ 1.03 (d, J=6.6Hz, 6H), 1.41-1.86(m, 6H), 1.86-2.04 (m, 1H), 1.99 (s, 3H), 2.02-2.17 (m, 2H), 2.05 (s, 3H), 2.12 (s, 3H), 2.99- (d, J=6.6Hz, 2H), 3.80 (bs, 1H), 3.98-4.06 (m, 1H), 4.98 (bs, 1H), 6.52 (d, J = 8.4Hz, 1H), 6.68 (d, J = 8.4Hz, 2H), 6.92 (d, J = 8.4Hz, 2H), 7.32-7.38 (m, 1H), 7.90 (s, 1H) ppm
381: ¹H NMR (CDCl₃ δ 1.52-1.83 (m, 6H), 1.94 (s, 6H), 2.01 (s, 6H), 2.01-2.14 (m, 2H), 3.10 (s, 3H), 3.96-4.06 (m, 1H), 4.74-4.84 (m, 1H), 6.52 (d, J = 8.7Hz, 1H), 6.80 (brs, 1H), 7.17 (d, J = 8.7Hz, 2H), 7.26-7.34 (m, 3H), 7.88 (d, J = 1.5Hz, 1H) ppm
382: Mp 138-140°C; ¹H NMR (CDCl₃) δ 1.25 (d, J = 6.3Hz, 6H), 1.43-1.83 (m, 6H), 1.99-2.15 (m, 2H), 2.12 (s, 3H), 2.21 (s, 3H), 3.60 (s, 3H), 3.67 (m, 1H), 4.00 (m, 3H), 6.43 (d, J=8.7Hz, 1H), 6.63 (d, J=8.7Hz, 2H), 7.13 (d, J=8.7Hz, 2H), 7.18 (s, 1H), 7.35 (dd, J = 8.7, 2.4Hz, 1H), 7.96 (d, J=2.4Hz, 1H) ppm
383: Mp 132-133°C; ¹H NMR (CDCl₃) δ 0.25-0.32 (m, 2H), 0.53 (m, 2H), 1.13 (m, 1H), 1.25 (d, J = 6.3Hz, 6H), 2.12 (s, 3H), 2.21 (s, 3H), 3.16 (dd, J= 6.9, 5.4Hz, 2H), 3.60 (s, 3H), 3.67 (m, 1H), 6.42 (d, J=8.7Hz, 1H), 6.63 (t, J=8.7Hz, 2H), 7.13 (t, J=8.7Hz, 2H), 7.18 (s, 1H), 7.35 (dd, J = 8.7, 2.4Hz, 1H), 7.98 (bd, J=2.4Hz, 1H) ppm
384: ¹H NMR (CDCl₃) δ 1.23 (d, J = 6.9Hz, 6H), 1.29 (d, J = 8.7Hz, 6H), 2.15 (s, 3H), 3.45 (s, 3H), 3.64 (s, 3H), 3.83 ∼ 4.03 (m, 1H), 4.25 (s, 2H), 4.53 (brs, 1H), 4.90 (sept., J = 6.9Hz, 1H), 6.45 (d, J = 8.1Hz), 7.15 ∼ 7.38 (m, 3H), 7.60 ∼ 7.78 (m, 3H), 7.87 (s, 1H) ppm;
385: ¹H NMR (CDCl₃) δ 1.44-1.86(m, 6H), 1.75 (s, 3H), 1.78 (s, 3H), 1.99 (s, 3H), 2.02-2.17 (m, 2H), 2.06 (s, 3H), 2.12 (s, 3H), 3.66 (bs, 1H), 3.74 (d, J=7.2Hz, 2H), 3.99-4.06 (m, 1H), 4.61-4.70(m, 1H), 5.35-5.41 (m, 1H), 6.49 (d, J = 8.1Hz, 1H), 6.69 (d, J = 8.1Hz, 2H), 6.93 (d, J = 8.1Hz, 2H), 7.30-7.34 (m, 1H), 7.92 (s, 1H) ppm
386: Mp 184-185°C
387: Mp 153-155°C
388: Mp 128-129°C
389: Mp 118-119°C
390: Mp 154-156°C
391: Mp 134-136°C
392: ¹H NMR (CDCl₃) δ 1.29 (d, J = 6.3Hz, 6H), 1.75 (s, 3H), 1.78 (s, 3H), 1.99 (s, 3H), 2.06 (s, 3H); 2.12 (s, 3H), 3.66 (bs, 1H), 3.74 (d, J = 6.6Hz, 2H), 3.88-4.00 (m, 1H), 4.59 (bs, 1H), 5.37-5.43 (m, 1H), 6.47 (d, J = 8.4Hz, 1H), 6.69 (d, J = 8.4Hz, 2H), 6.93 (d, J = 8.4Hz, 2H), 7.32 (dd, J = 2.1, 8.4Hz, 1H), 7.92 (d, J = 2.1Hz, 1H) ppm
393: Mp 197-199°C; ¹H NMR (CDCl₃) δ 1.01 (d, J = 6.6Hz, 6H), 1.25 (d, J= 6.3Hz, 6H), 1.92 (m, 1H), 2.10 (s, 3H), 3.12 (dd, J = 6.6, 6.0Hz, 2H), 3.66 (m, 1H),3.67 (s, 3H),3.73 (s, 3H), 6.41 (d, J = 8.7.Hz, 1H), 6.64 (d, J = 8.7.Hz, 2H), 6.72 (s, 1H),7.00 (d, J = 8.7Hz, 2H), 7.50 (dd, J = 8.7, 2.4Hz, 1H) 8.16 (d, J = 2.4Hz, 1H) ppm
394: Mp 189-191°C; ¹H NMR (CDCl₃) δ 1.02 (t, J = 7.5Hz, 3H), 1.25 (d, J = 6.3Hz, 6H), 1.60- 1.76 (m, 2H), 2.05 (s, 3H), 2.10 (s, 3H), 3.21-3.52 (m, 2H), 3.66 (m, 1H),3.67 (s, 3H), 3.73 (s, 3H), 6.31-6.38 (m, 2H), 6.42 (d, J = 8.7.Hz, 1H), 6.64 (d, J = 8.7.Hz, 2H), 6.72 (s, 1H), 7.00 (d, J = 8.7.Hz, 2H), 7.51 (dd, J = 8.7, 2.4Hz, 1H), 8.16 (d, J = 2.4Hz, 1H) ppm
395: Mp 219-221°C; ¹H NMR (CDCl₃) δ 0.24-0.32 (m, 2H), 0.53-0.62 (m, 2H), 1.12 (m, 1H), 1.25 (d, J= 6.3Hz, 6H), 2.10 (s, 3H), 3.17 (dd, J= 6.9, 5.4Hz, 2H), 3.66 (m, 1H), 3.67 (s, 3H), 3.73 (s, 3H), 6.42 (d, J = 8.7Hz, 1H), 6.64 (d, J = 8.7Hz, 2H), 6.72 (s, 1H), 7.00 (d, J = 8.7Hz, 2H), 7.51 (dd, J = 8.7, 2.4Hz, 1H), 8.17 (dd, J = 2.4Hz, 1H) ppm
396: Mp 167-169°C; ¹H NMR (CDCl₃) δ 1.25 (d, J = 6.3Hz, 6H), 1.43-1.84 (m, 6H), 2.00-2.14 (m, 2H), 2.10 (s, 3H), 3.66 (m, 1H), 3.67 (s, 3H), 3.73 (s, 3H), 4.01 (m, 1H), 6.42 (d, J= 8.7Hz, 1H), 6.64 (d, J = 8.7Hz, 2H), 6.72 (s, 1H), 7.00 (d, J = 8.7Hz, 2H), 7.51 (dd, J = 8.4, 2.4Hz, 1H), 8.15 (d, J = 2.4Hz, 1H) ppm
397: ¹H NMR (CDCl₃) δ 1.28 (d, J = 6.3Hz, 6H), 3.88-4.02 (m, 1H), 4.38 (d, J = 5.4Hz, 2H), 4.66 (d, J = 8.1Hz, 2H), 6.28 (dd, J = 3.3, 0.8Hz, 1H), 6.35 (dd, J = 3.3, 2.0Hz, 1H), 6.47 (d, J = 9.0Hz, 1H), 6.77 (d, J = 8.9Hz, 2H), 7.35 (d, J = 8.9Hz, 2H), 7.39 (dd, J = 2.0, 0.8Hz, 1H), 7.58 (d, J = 9.0Hz, 1H), 8.25 (s, 1H) ppm
398: Mp 126-128°C; ¹H NMR (CDCl₃) δ 1.02 (d, J = 6.9Hz, 6H),1.24 (d, J = 6.0Hz, 6H), 1.93 (m, 1H), 3.13 (m, 2H), 3.71 (s, 3H), 3.86 (s, 3H), 6.42 (d, J = 8.4Hz, 1H), 6.63 (d, J = 8.7Hz, 2H), 6.81 (s, 1H), 7.40 (d, J= 8.7Hz, 2H), 7.45 (dd, J= 8.4, 2.7Hz, 1H), 7.90 (s, 1H), 8.09 (d, J = 2.7Hz, 1H) ppm
399: Mp 141-143°C; ¹H NMR (CDCl₃) δ 0.24-0.32 (m, 2H), 0.53-0.61 (m, 2H), 1.13 (m, 1H), 1.24 (d, J = 6.3Hz, 6H), 3.18 (dd, J = 6.9. 5.1Hz, 2H), 3.67 (m, 1H), 3.71 (s, 3H), 3.86 (s, 3H), 6.43 (d, J = 8.7Hz, 1H), 6.63 (d, J = 8.7Hz, 2H), 6.81 (s, 1H), 7.40 (d, J = 8.7Hz, 2H), 7.45 (dd, J = 8.7, 2.4Hz, 1H), 7.90 (s, 1H), 8.09 (d, J = 2.4Hz, 1H) ppm
400: Mp 181-183°C; ¹H NMR (CDCl₃) δ 1.24 (d, J = 6.3Hz, 6H), 1.44.1.84 (m, 6H), 1.99-2.14 (m, 2H), 3.67 (m, 1H), 3.71 (s, 3H), 3.86 (s, 3H), 4.03 (m, 1H), 6.43 (d, J = 8.4Hz, 1H), 6.63 (d, J = 8.7Hz, 2H), 6.82 (s, 1H), 7.40 (d, J = 8.7Hz, 2H), 7.45 (dd, J = 8.4, 2.4Hz, 1H), 7.90 (s, 1H), 8.08 (d, J = 2.4Hz, 1H) ppm
401: ¹H NMR (CDCl₃) δ 0.23-0.33 (m, 4H), 0.52-0.65 (m, 4H), 1.07-1.21 (m, 2H), 1.98 (s, 3H), 2.05 (s, 3H), 2.11 (s, 3H), 3.18 (d, J=6.3Hz, 2H), 3.20 (d, J=6.3Hz, 2H), 3.85 (bs, 1H), 4.68-4.92 (m, 1H), 6.50 (d, J = 8.4Hz, 1H), 6.69 (d, J = 8.4Hz, 2H), 6.88 (d, J = 8.4Hz, 2H), 7.32 (dd, J=2.1,8.4Hz, 1H), 7.95 (d, J= 2.1Hz, 1H) ppm
402: ¹H NMR (CDCl₃) δ 1.28 (d, J = 6.3Hz, 6H), 1.35 (s, 9H), 2.12 (s, 3H), 3.42 (s, 3H), 3.63 (s, 3H), 3.86-3.99 (m, 1H), 4.48 (d, J = 6.9Hz, 1H), 6.44 (d, J =8.7Hz, 1H), 7.30 (dd, J = 8.4, 2.4Hz, 1H), 7.40 (s, 1H), 7.57-7.66 (m, 4H), 7.71 (d, J = 0.6Hz, 1H), 7.87 (d, J = 1.8Hz, 111) ppm
403: ¹H NMR (CDCl₃) δ 1.28 (d, J = 6.3Hz, 6H), 2.12 (s, 3H), 3.07 (s, 6H), 3.42 (s, 3H), 3.63 (s, 3H), 3.85-3.99 (m, 1H), 4.41-4.50 (brosd, 1H), 6.42 (s, 1H), 6.43 (d, J = 8.7Hz, 1H), 7.29 (dd, J = 8.4, 2.4Hz, 1H), 7.47 (d, J = 8.7Hz, 2H), 7.56 (d, J = 8.7Hz, 2H), 7.71 (s, 1H), 7.87 (dd, J = 2.1, 0.6Hz, 1H)ppm
404: ¹H NMR (CDCl₃) δ 0.83-0.92 (m, 1H), 1.28 (d, J = 6.6Hz, 6H), 2.12 (s, 3H), 2.89 (d, J = 4.8Hz, 3H), 3.42 (s, 3H), 3.63 (s, 3H), 3.85-3.98 (m, 1H), 4.52 (brs, 1H), 4.82 (d, J= 5.1Hz, 1H), 6.44 (d, J = 8.7Hz, 1H), 6.53 (s, 1H), 7.30 (dd, J = 8.1, 2.1Hz, 1H), 7.39 (d, J = 8.7Hz, 2H), 7.57 (d, J = 8.7Hz, 2H), 7.70 (s, 1H), 7.86 (d, J = 2.1Hz, 1H) ppm
405: ¹H NMR (CDCl₃) δ 0.23-0.32 (m, 2H), 0.56-0.63 (m, 2H), 0.93-1.00 (m, 1H), 1.03 (d,J = 6.6Hz, 6H), 1.86-2.05 (m, 1H), 1.99 (s, 3H), 2.05 (s, 3H), 2.12 (s, 3H), 2.99 (d, J = 6.6Hz, 2H), 3.18 (d, J = 6.6Hz, 2H),3.77 (bs, 1H), 4.65-4.76 (m, 1H), 6.50 (d, J = 8.4Hz, 1H), 6.68 (d, J = 8.4Hz, 2H), 6.93 (d, J = 8.4Hz, 2H), 7.28-7.36 (m, 1H), 7.95 (s, 1H) ppm
406: ¹H NMR (CDCl₃) δ 0.26-0.33 (m, 2H), 0.50-0.63 (m, 2H), 1.03 (d, J = 6.9Hz, 6H), 1.08-1.22 (m, 1H), 1.94 (nona, J = 6.9Hz, 1H), 1.99 (s, 6H), 2.00 (s, 6H), 2.98 (d, J = 6.9Hz, 2H), 3.19 (dd, J = 6.9, 5.0Hz, 2H), 3.71 (brs, 1H), 4.68-4.76 (m, 1H), 6.50 (d, J = 8.6Hz, 1H), 6.68 (d, J = 8.4Hz, 2H), 6.96 (d, J = 8.4Hz, 2H), 7.28 (dd, J = 8.6, 1.9Hz, 1H), 7.92 (d, 1.9Hz, 1H) ppm
407: ¹H NMR (CDCl₃) δ 1.28 (d, J = 6.6Hz, 6H), 2.11 (s, 3H), 3.43 (s, 3H), 3.63 (s, 3H), 3.82 (s, 3H), 3.85-3.98 (m, 1H), 3.99 (d, J = 4.5Hz, 2H), 4.36-4.43 (m, 1H), 4.44-4.60 (broad, 1H), 6.44 (d, J = 8.7Hz, 1H), 6.69 (d, J = 8.7Hz, 2H), 7.30 (dd, J = 8.4, 2.1Hz, 1H), 7.48 (d, J = 8.7Hz, 2H), 7.70 (s, 1H), 7.86 (d, J = 1.8Hz, 1H) ppm
408: ¹H NMR (CDCl₃) δ 0.29-0.34 (m, 2H), 0.58-0.63 (m, 2H), 1.01-1.21 (m, 1H), 1.97 (s, 3H), 2.05 (s, 3H), 2.10 (s, 3H), 3.19 (d, J= 6.9Hz, 2H), 3.22 (bs, 1H), 4.09 (bs, 1H), 4.37 (s, 2H), 6.28-2.30 (m, 1H), 6.36-6.40 (m, 1H), 6.55-6.60 (m, 1H), 6.76 (d, J = 8.1Hz 2H), 6.90-6.98 (m, 2H), 7.34-7.44(m, 1H), 7.41 (s, 1H), 7.88 (s, 1H) ppm
409: ¹H NMR (CDCl₃) δ 0.27-0.33 (m, 2H), 0.57-0.62 (m, 2H), 1.11-1.20 (m, 1H), 1.75 (s,3H), 1.78(s, 3H), 1.99 (s, 3H), 2.05 (s, 3H), 2.12 (s, 3H), 3.18 (d, J = 6.0Hz, 2H), 3.21 (bs, 1H), 3.74 (d, J = 7.2Hz, 2H), 5.00(bs, 1H), 5.39 (t, J = 7.2Hz, 1H), 6.52 (d, J = 8.4Hz, 1H), 6.69 (d, J = 8.4Hz, 2H), 6.93 (d, J = 8.4Hz, 2H), 7.30-7.38 (m, 1H), 7.92 (s, 1H) ppm
410: ¹H NMR (CDCl₃) 1.28 (d, J = 6.3Hz, 12H), 2.04 (s, 6H), 2.20 (s, 3H), 3.72 (bs, 1H), 3.86-3.98 (m, 2H), 4.18-4.27 (m, 1H), 4.41-4.44 (m, 1H), 6.43 (d, J = 8.4Hz, 1H), 6.73-6.81 (m, 3H), 6.97 (s, 1H), 7.43 (dd, J = 2.1, 8.4Hz, 1H), 8.08 (d, J = 2.1Hz, 1H) ppm
411: Mp 151-153°C; ¹H NMR (CDCl₃) δ 1.75 (s, 3H), 1.78 (s, 3H), 2.07 (s, 3H), 2.08 (s, 3H), 2.17 (s, 3H), 3.34 (s, 6H), 3.45 (s, 3H), 3.75 (d, J = 6.6Hz, 2H), 5.39 (m, 1H), 6.71 (d, J = 8.7Hz, 2H), 7.12 (d, J= 8.4Hz, 1H), 7.46 (m, 1H), 7.74 (dd, J= 2.1, 8.1Hz, 1H), 8.46 (d, J= 2.1Hz, 1H) ppm
412: ¹H NMR (CDCl₃) δ 1.29 (d, J = 6.3Hz, 6H), 2.19 (s, 3H), 2.29 (s, 3H), 3.09 (s, 3H), 3.81-3.96 (m, 1H), 6.48 (d, J = 9.0Hz, 1H), 7.01-7.14 (m, 4H), 7.24 (d, J = 8.1Hz 1H), 7.50 (dd, J =9.0, 2.7Hz, 1H), 8.06 (d, J =2.7Hz, 1H) ppm
413: ¹H NMR (CDCl₃) δ 1.28 (d, J = 6.3Hz, 6H), 1.29 (d, J = 6.6Hz, 6H), 2.19 (s, 3H), 2.29 (s, 3H), 2.54 (sept, J = 6.6Hz, 1H), 3.83-4.00 (m, 1H), 4.49 (d, J = 8-1Hz 1H), 6.44 (d, J = 9.0Hz, 1H), 7.10 (s, 1H), 7.12 (s, 1H), 7.17-7.28 (m, 3H), 7.46 (dd, J = 8.4, 2.1Hz 1H), 7.60 (d, J = 12.6Hz, 1H), 8.10 (d, J = 1.8Hz, 1H) ppm
414: ¹H NMR (CDCl₃) δ 1.28 (d,, J = 6.9Hz, 6H), 2.23 (s, 3H), 2.64 (s, 3H), 3.82 (s, 3H), 4.88 (sept, d = 6.6Hz, 1H), 6.73 (s, 1H), 7.09-7.29 (m, 4H), 7.43 (d, J = 10.2Hz, 1H), 7.81 (dd, J = 7.8, 2.4Hz, 1H), 8.60 (d, J = 2.4Hz, 1H) ppm
415: ¹H NMR (CDCl₃) 1.28 (d, J = 6.3Hz, 6H), 1.40-1.90 (m, 6H), 1.93-2.15 (m, 2H), 2.04 (s, 6H), 2.19 (s, 3H), 3.78-4.00 (m, 3H), 4.52 (bs, 1H), 6.43 (d, J = 9.0Hz, 1H), 6.72-6.81 (m, 3H), 6.97 (s, 1H), 7.40-7.48 (m, 1H), 8.07 (s, 1H) ppm
416: ¹H NMR (CDCl₃) δ 0.26-0.34 (m, 2H), 0.54-0.63 (m, 2H), 1.04 (t, J = 7.2Hz, 3H), 1.09-1.20 (m, 1H), 1.69 (sext, J = 7.2Hz, 2H), 1.99 (s, 6H, 2.00 (s, 6H), 3.14 (t, J = 7.2Hz, 2H), 3.19 (dd, J = 7.1, 6.2Hz, 2H), 4.64-4.71 (m, 1H), 6.49 (d, J = 8.6Hz, 1H), 6.68 (d, J = 8.7Hz, 2H), 6.96 (d, J = 8.7Hz, 2H), 7.28 (dd, J = 8.6, 1.9Hz, 1H), 7.92 (d, 1.9Hz, 1H) ppm
417: ¹H NMR (CDCl₃) δ 0.26-0.35 (m, 2H), 0.55-0.64 (m, 2H), 1.09-1.21 (m, 1H), 1.29 (d, J = 6.9Hz, 6H), 1.95 (s, 6H), 2.00 (s, 6H), 2.55 (sept, J = 6.9Hz, 1H), 3.19 (dd, J = 6.9, 5.1Hz, 2H), 4.68-4.76 (m, 1H), 6.50 (d, J = 8.4Hz, 1H), 7.13 (d, J = 8.1Hz, 2H), 7.22 (brs, 1H), 7.27 (dd, J = 8.4, 1.9Hz, 1H), 7.54-7.66 (m, 2H), 7.90 (d, 1.9Hz, 1H) ppm
418: ¹H NMR (CDCl₃) 0.27-0.32 (m, 2H), 0.55-0.61 (m, 2H), 1.11-1.20 (m, 1H), 1.29 (d, J=6.0Hz, 6H), 2.04 (s, 6H), 2.19 (s, 3H), 3.61-3.20 (m, 2H), 3.65-3.76 (m, 2H), 4.88 (bs, 1H), 6.48 (d, J = 8.4Hz, 1H), 6.75-6.81 (m, 3H), 6.97 (s, 1H), 7.45 (dd,J=2.1Hz, 8.4Hz, 1H), 8.07 (d, J=2.1Hz, 1H) ppm
419: ¹H NMR (DMSO-d6) δ 1.13 (d, J = 6.8Hz, 6H), 1.54-1.84 (m, 6H), 1.89 (s, 6H), 1.96 (s, 6H), 1.97-2.11 (m, 2H), 2.64 (sept, J = 6.8Hz, 1H), 4.09-4.19 (m, 1H), 6.96-7.05 (m, 2H), 7.16 (d, J = 9.6Hz, 1H), 7.66-7.78 (m, 4H), 8.99 (brs, 1H), 10.00 (s, 1H), 13.52 (brs, 1H) ppm
420: ¹H NMR (CDCl₃)1.04 (d, J=6.6Hz, 6H), 1.29 (d, J=6.6Hz, 6H), 1.90-2.01 (m, 1H), 2.04 (s, 6H), 2.20 (s, 3H), 3.02 (t, J=6.6Hz, 2H), 3.87-4.00 (m, 2H), 4.41 (bs, 1H), 6.44 (d, J = 8.7Hz, 1H), 6.72-6.82 (m, 3H), 6.98 (s, 1H), 7.44 (dd,J=2.1Hz 8.7Hz, 1H), 8.08 (d, J=2.1Hz, 1H) ppm
421: ¹H NMR (CDCl₃) 0.32-0.36 (m, 2H), 0.55-0.61 (m, 2H), 1.13-1.17 (m, 1H), 2.03(s, 6H), 2.19 (s, 3H), 3.16-3.20 (m, 2H), 4.30 (bs, 1H), 4.40 (d, J=6.3Hz, 2H), 4.82 (bs, 1H), 6.30-6.31 (m, 1H), 6.36-6.37 (m,1H), 6.48 (d, J=9.0Hz, 1H), 6.78-6.85 (m, 3H), 6.97 (s, 1H), 7.41-7.46 (m,2H), 8.08 (m, 1H) ppm
422: ¹H NMR (CDCl₃) δ 1.28 (d, J = 6.6Hz, 6H), 2.20 (s, 3H), 2.29 (s, 3H), 3.84-4.00 (m, 1H), 4.16 (s, 2H), 4.50 (brs, 1H), 6.44 (d, J = 8.4Hz, 1H), 6.48-6.61 (m, 2H), 7.10 (s, 1H), 7.12 (s, 1H), 7.18 (t, J = 8.4Hz, 1H), 7.47 (dd, J = 8.4, 2.1Hz, 1H), 8.11 (d, J = 2.1Hz) ppm;
423: ¹H NMR (CDCl₃) δ 1.29 (d, J=6.0Hz, 6H), 1.75 (s, 3H), 1.77 (s, 3H), 2.04(s, 6H), 2.20 (s, 3H), 3.65-3.78 (m, 2H), 3.90(t, J=6.0Hz, 2H), 4.50 (bs, 1H), 5.34-5.39 (m, 1H), 6.45 (d, J=8.7Hz, 1H), 6.72.6.81 (m, 3H), 6.97 (s, 1H), 7.44 (dd, J=2.4, 8.7Hz, 1H), 8.10 (d, J=2.4Hz, 1H) ppm
424: ¹H NMR (CDCl₃) δ 1.55-1.81 (m, 6H), 1.75 (s, 3H), 1.77 (s, 3H), 2.00-2.15 (m, 2H), 2.04(s, 6H), 2.20 (s, 3H), 3.79-3.95 (m, 4H), 4.59 (bs, 1H), 5.33-5.39 (m, 1H), 6.45 (d, J = 8.4Hz, 1H), 6.77-6.80 (m, 3H), 6.97 (s, 1H), 7.45 (dd, J = 2.4, 8.4Hz, 1H), 8.09 (d, J = 2.4Hz, 1H) ppm
425: ¹H NMR (CDCl₃) δ 1.26 (d, J = 6.0Hz, 6H), 2.23 (s, 3H), 2.27 (s, 3H), 3.64 (sept. J = 6.0Hz, 1H), 3.83 (s, 3H), 6.33-6.45 (m, 2H), 7.00-7.07 (m, 1H), 7.13 (d, J = 7.8Hz, 1H), 7.73 (dd, J = 8.7, 2.1Hz, 1H), 7.96 (s, 1H), 8.04 (dd, J = 8.7, 0.6Hz, 1H), 8.28 (dd, J = 2.4, 0.9Hz, 1H) ppm
426: ¹H NMR (CDCl₃) δ 1.26 (d, J = 6.3Hz, 6H), 2.23 (s, 3H), 2.27 (s, 3H), 3.64 (sept, J = 6.3Hz, 1H), 4.33 (s, 3H), 6.32-6.46 (m, 2H), 7.04 (t, J = 2.4Hz, 1H), 7.12 (s, 1H), 7.15 (m, 1H), 7.79 (dd, J = 8.7, 2.4Hz, 1H), 8.31 (dd, J = 2.4, 0.6Hz, 1H), 8.34 (d, J = 9.0Hz, 1H), 9.20 (s, 1H) ppm
427: ¹H NMR (CDCl₃) δ 1.34 (d, J = 6.6Hz, 6H), 2.18 (s, 3H), 2.27 (s, 3H), 2.77 (s, 3H), 3.84 (sept, J = 6.6Hz, 1H), 4.97 (brs, 1H), 6.64 (d, J = 8.4Hz, 1H), 6.99 (d.d, J = 7.8 & 2.4Hz, 1H), 7.02 ∼ 7.12 (m, 3H), 7.21 (t, J = 2.4Hz, 1H), 7.64 (d, J = 10.8Hz, 1H), 7.94 (s, 1H) ppm
428: ¹H NMR (DMSO-d6) δ 1.24 (t, J = 7.5Hz, 3H), 1.25 (d, J = 6.6Hz, 6H), 3.21 (q, J = 7.5Hz, 2H), 4.01-4.17 (m, 1H), 7.09 (d, J = 6.9Hz, 1H), 7.39 (d, J = 8.7Hz, 2H), 7.52 (d, J = 8.7Hz, 2H), 7.94 (d, J = 6.9Hz, 1H), 8.8 (brs, 1H), 10.20 (s, 1H) ppm
429: ¹H NMR (CDCl₃)δ 0.29-0.32 (m, 2H), 0.58-0.64 (m, 2H), 1.10-1.23 (m, 1H), 1.75 (s, 3H), 1.77 (s, 3H), 2.00-2.15 (m, 2H), 2.04(s, 6H), 2.20 (s, 3H), 3.04(d, J=6.9Hz, 2H), 3.88-3,92 (m, 2H), 4.05 (bs, 1H), 4.50 (bs, 1H), 5.33-5.39 (m, 1H), 6.46 (d, J=8.7Hz, 1H), 6.71-6.84 (m, 3H), 6.98 (s, 1H), 7.45 (dd, J=2.4, 8.7Hz, 1H), 8.09 (d, J=2.4Hz, 1H) ppm
430: ¹H NMR (CDCl₃)δ 1.04 (d, J=6.6Hz, 6H), 1.41-1.83 (m, 6H), 1.83-2.15 (m, 3H), 2.04(s, 6H), 2.20 (s, 3H), 3.02 (t, J=6.6Hz, 2H), 3.90-4.06 (m, 2H), 4.62 (bs, 1H), 6.46 (d, J=8.7Hz, 1H), 6.72-6.86 (m, 3H), 6.98 (s, 1H), 7.45 (dd, J=2.1, 8.7Hz, 1H), 8.09 (d, J=2.1Hz, 1H) ppm
431: ¹H NMR (CDCl₃)δ 1.04 (d, J = 6.6Hz, 6H), 1.75 (s, 3H), 1.77 (s; 3H), 1.92-2.04 (m, 1H), 2.04(s, 6H), 2.20 (s, 3H), 3.01 (t, J = 6.6Hz, 2H), 3.88-3.92 (m, 2H), 3.96 (bs, 1H), 4.50 (bs, 1H), 5.34-5.39 (m, 1H), 6.45 (d, J = 8.7Hz, 1H), 6.71-6.81 (m, 3H), 6.97 (s, 1H), 7.44 (dd, J = 2.1, 8.7Hz, 1H), 8.09 (d, J = 2. 1H) ppm
432: Mp 117-118°C; ¹H NMR (CDl₃) δ 1.75 (s, 3H), 1.79 (s, 3H), 2.05 (s, 3H), 2.07 (s, 3H), 3.09 (s, 3H), 3.32 (s, 3H), 3.35 (s, 3H), 3.48 (s, 3H), 3.79 (d, J = 6.3Hz, 2H), 5.40 (m, 1H), 6.79 (t, J= 8.1Hz, 1H), 6.93-6.98 (m, 2H), 7.51 (d, J = 8.1Hz, 1H), 7.70 (dd, J = 2.1Hz, 1H), 8.37 (d, J = 1.8Hz, 1H) ppm
433: Mp 169-171°C; ¹H NMR (CDCl₃) δ 0.25-0.33 (m, 2H), 0.58-0.65 (m, 2H), 1.18 (m, 1H), 2.07 (s, 3H), 2.09 (s, 3H), 3.06 (d, J = 7.2Hz, 2H), 3.09 (s, 3H), 3.36 (s, 3H), 3.57 (s, 3H), 3.62 )s, 3H), 6.77 (t, J = 9.0Hz, 1H), 6.92-6.99 (m, 2H), 7.52 (dd, J = 0.9, 8.4Hz, 1H), 8.57 (s, 2H) ppm
434: ¹H NMR (CDCl₃) δ 1.49-1.80 (m, 6H), 2.03-2.17 (m, 2H), 2.03(s, 6H), 2.20 (s, 3H), 3.99-4.05 (m, 1H), 4.30 (bs, 1H), 4.41 (d, J = 5.7Hz, 2H), 4.72 (bs, 1H), 6.30-6.31 (m, 1H), 6.35-6.37 (m, 1H), 6.47 (d, J=8.4Hz, 1H), 6.78-6.87 (m, 3H), 6.97 (s, 1H), 7.41 (s, 1H), 7.44 (dd, J = 2.1, 8.7Hz, 1H), 8.09 (d, J = 2.1Hz, 1H) ppm
435: ¹H NMR (CDCl₃) δ 1.25 (d, J = 6.6Hz, 6H), 1.43-1.82 (m, 6H), 1.82-2.17 (m, 2H), 2.06(s, 6H), 2.21 (s, 3H), 3.55 (bs, 1H), 3.63-3.72 (m, 1H), 3.99 (m, 1H), 4.59-4.62 (m, 1H), 6.49 (d, J = 7.8Hz, 1H), 6.64 (d, J = 8.7Hz, 2H), 7.03 (s, 1H), 7.17 (d, J = 8.7Hz, 2H), 7.28 (dd, J = 2.1, 7.8Hz, 1H), 7.90 (d, J = 2.1Hz, 1H) ppm
436: ¹H NMR (CDCl₃) δ 1.29 (d, J = 6.3Hz, 6H), 2.31 (s, 6H), 3.86-3.99 (m, 1H), 4.49 (d, J = 7.5Hz, 1H), 6.44 (d, J = 8.4Hz, 1H), 6.57-6.62 (m, 1H), 7.13 (s, 1H), 7.18-7.27 (m, 3H), 7.44 (d, J = 8.1H 1H), 7.49 (dd, J = 8.4, 2.5 Hz, 1H), 7.62 (s, 1H), 8.13 (d, J = 2.5Hz, 1H), 8.25 (brs, 1H) ppm
437: ¹H NMR (CDCl₃)δ 1.42-1.82 (m, 12H), 1.90-2.18 (m, 4H), 2.06(s, 6H), 2.22 (s, 3H), 3.72 (bs, 1H), 3.83 (bs, 1H), 3.99-4.05 (m, 1H), 4.69 (bs, 1H), 6.51 (d, J = 8.1H 1H), 6.66 (d, J=8.4Hz, 2H), 7.03 (s, 1H), 7.16 (d, J= 8.4Hz, 2H), 7.29 (dd, J= 2.1, 8.1Hz, 1H), 7.90 (d, J =2.1Hz, 114) ppm
438: ¹H NMR (CDCl₃) δ 1.26 (d, J = 6.3Hz, 6H), 2.23 (s, 3H), 2.26 (s, 3H), 3.64 (sept., J = 6.3Hz, 1H), 6.36 (dd, J= 12.6, 2.1Hz, 1H), 6.42 (dd, J = 8.4, 2.1Hz, 1H), 6.91 (d, J= 8.4Hz, 1H), 7.04 (t, J = 8.4Hz, 1H), 7.10 (s, 1H), 7.14 (s, 1H), 7.66 (dd, J = 8.4, 2.4Hz, 1H), 8.22 (d, J =2.1Hz), 8.27 ∼ 8.36 (broad, 1H). ppm
439: ¹H NMR (CDCl₃) δ 1.29 (d, J = 6.3Hz, 6H), 2.21 (s, 3H), 2.83 (s, 3H), 2.87 (d, J = 4.8Hz, 3H), 3.81-3.98 (m, 2H), 4.60 (brs, 1H), 6.38 (dd, J = 12, 2.4Hz, 1H), 6.40-6.48 (m, 2H), 7.05 (d, J = 8.4Hz, 1H), 7.10 (s, 1H), 7.11 (s, 1H), 7.48 (dd, J = 8.4, 2.4Hz, 1H), 8.09 (d, J =2.1Hz) ppm
440: ¹H NMR (CDCl₃) δ 1.75 (s, 3H), 1.77 (s, 3H), 2.21 (s, 3H), 2.28 (s, 3H), 2.87 (d, J = 4.2Hz, 3H), 3.78-3.97 (m, 3H), 4.61 (brs, 1H), 5.32-5.40 (m, 1H), 6.32-6.51 (m, 3H), 7.05 (d, J= 8.4Hz, 1H), 7.10 (s, 1H), 7.11 (s, 1H), 7.48 (d.d, J = 8.7, 2.4Hz, 2H), 8.12 (d, J = 1.8Hz, 1H) ppm
441: ¹H NMR (CDCl₃) δ 1.75 (s, 3H), 1.78 (s, 3H), 2.31 (s, 6H), 3.91 (t, J = 5.9Hz, 2H), 4.54-4.61 (m, 1H), 5.33-5.40 (m, 1H), 6.47 (d, J = 8.4Hz, 1H), 6.57-6.62 (m, 1H), 7.13 (s, 1H), 7.19-7.27 (m, 3H), 7.44 (d, J = 8.7Hz, 1H), 7.50 (dd, J = 8.4, 2.1 Hz, 1H), 7.59-7.64 (m, 1H), 8.15 (d, J = 2.1Hz, 1H), 8.24 (brs, 1H) ppm
442: ¹H NMR (CDCl₃)δ 1.75 (s, 3H), 1.78 (s, 3H), 2.03(s, 6H), 2.20 (s, 3H), 3.88-3.94 (m, 2H), 4.30 (bs, 1H), 4.40 (d, J=5.4Hz, 2H), 4.62 (bs, 1H), 5.34-5.38 (m, 1H), 6.30-6.31 (m, 1H), 6.36-6.37 (m, 1H), 6.46 (d, J=8.7Hz, 1H), 6.78-6.91 (m, 3H), 6.97 (s, 1H), 7.41 (s, 1H), 7.45 (dd, J=2.1, 8.7Hz, 1H), 8.08 (d, J=2.1H 1H) ppm
443: ¹H NMR (CDCl₃)δ 1.45-1.80 (m, 6H), 2.03-2.17 (m, 2H), 2.06(s, 6H), 2.20 (s, 3H), 3.99-4.05 (m, 1H), 4.10 (bs, 1H), 4.37 (s, 2H), 4.65 (bs, 1H), 6.27-6.29 (m, 1H), 6.34-6.36 (m, 1H), 6.50 (d, J = 8.7Hz, 1H), 6.73 (d, J = 8.7Hz, 2H), 7.02 (s, 1H), 7.17 (d, J = 8.7Hz, 2H), 7.27-7.30 (m, 1H), 7.39 (s, 1H), 7.89 (m, 1H) ppm
444: ¹H NMR (CDCl₃) δ 1.29 (d, J = 6.3Hz, 6H), 2.21 (s, 3H), 2.28 (s, 3H), 2.87 (s, 3H), 3.83-3.98 (m, 2H), 4.68 (brs, 1H), 6.34-6.49 (m, 3H), 7.03-7.13 (m, 3H), 7.48 (dd, J = 8.7, 2.4Hz, 1H), 8.09 (d, J = 2.4Hz, 1H) ppm;
445: ¹H NMR (CDCl₃) δ 1.29 (d, J = 6.3Hz, 6H), 2.20 (s, 3H), 2.30 (s, 3H), 2.92 (s, 3H), 3.38 (s, 3H), 3.86-4.01 (m, 1H), 4.52 (d, J = 6.9Hz, 1H), 6.44 (d, J = 9.0Hz, 114), 7.10 (s, 1H), 7.13 (s, 1H), 7.18-7.36 (m, 3H), 7.47 (d.d, J = 8.4, 2.1Hz, 1H), 8.11 (d, J = 6.3Hz, 6H) ppm
446: ¹H NMR (CDCl₃) δ 1.28 (d, J = 6.3Hz, 6H), 2.19 (s, 3H), 2.29 (s, 3H), 2.79 (d, J = 2.1Hz, 3H), 3.34 (s, 3H), 3.84-3.97 (m, 1H), 4.62 (brs, 2H), 6.44 (d, J = 8.7Hz, 1H), 7.10 (s, 1H), 7.12 (s, 1H), 7.16-7.33 (m, 3H), 7.47 (dd, J = 8.7, 2.1Hz, 1H), 8.09 (d, J = 2.1Hz) ppm;
447: ¹H NMR (CDCl₃)δ 1.40-1.85 (m, 6H), 1.74 (s, 3H), 1.77 (s, 3H), 2.00-2.17 (m, 2H), 2.06(s, 6H), 2.21 (s, 3H), 3.40 (bs, 1H), 3.74 (d, J = 6.9Hz, 2H), 3.99-4.08 (m, 1H), 4.63 (bs, 1H), 5.33-5.41 (m, 1H), 6.50 (d, J=8.4Hz, 1H), 6.67 (d, J= 8.4Hz, 2H), 7.03 (s, 1H), 7.18 (d, J = 8.4Hz, 2H), 7.26-7.30 (m, 1H), 7.90 (s, 1H) ppm
448: Mp 128.129°C; ¹H NMR (CDCl₃) δ 1.76 (s, 3H), 1.78 (s, 3H), 2.04 (s, 3H), 2.09 (s, 3H), 3.09 (s, 3H), 3,35 (s, 3H), 3.37 (s, 3H), 3.39 (s, 3H), 3.91 (m, 2H), 5.37 (m, 1H), 6.50 (d, J = 8.7Hz, 1H), 6.98 (d, J = 8.4Hz, 2H), 7.42 (dd, J = 2.4, 8.7Hz, 1H), 8.02 (d, J = 2.1Hz, 1H) ppm
449: ¹H NMR (CDCl₃) δ 1.03 (d, J = 6.6Hz, 6H), 1.94 (nona, J = 6.6Hz, 1H), 2.31 (s, 6H), 3.14 (t, J = 6.6Hz, 2H), 4.68-4.76 (m, 1H), 6.47 (d, J = 8.7Hz,- 114), 6.57-6.62 (m, 1H), 7.13 (s, 1H), 7.17-7.28 (m, 3H), 7.44 (d, J = 8.4Hz, 1H), 7.50 (dd, J = 8.7, 2.1 Hz, 1H), 7.61 (s, 1H), 8.13 (d, J = 2.1Hz, 1H), 8.24 (brs, 1H) ppm
450: ¹H NMR (CDCl₃) δ 1.28 (d, J = 6.6Hz, 6H), 2.21 (s, 3H), 2.30 (s, 3H), 3.36 (s, 3H), 3.78 (s, 3H), 3.85-4.00 (m, 1H), 4.50 (d, J = 7.8Hz, 1H), 6.44 (d, J = 8.4Hz, 1H), 7.06-7.16 (m, 4H), 7.21-7.30 (m, 1H), 7.47 (d,d, J = 8.4, 2.4Hz, 1H), 8.11 (dd, J = 2.4, 0.6Hz, 1H) ppm
451: ¹H NMR (CDCl₃) δ 1.30 (d, J = 6.3Hz, 6H), 2.00 (s, 3H), 2.21 (s, 3H), 2.31 (s, 3H), 3,26(s, 3H), 3.86-3.99(m, 1H), 4.57-4.69 (m, 1H), 6.46 (d, J =9.0Hz, 1H), 6.96-7.11 (m, 2H), 7.12 (s, 1H), 7.14 (s, 1H), 7.48 (dd, J= 8.7, 2.7Hz, 1H), 8.10 (d, J= 2.7Hz, 1H) ppm
452: ¹H NMR (CDCl₃+CD₃OD) δ 1.74 (s, 3H), 1.77 (s, 3H), 2.18 (s, 3H), 2.27 (s, 3H), 2.77 (s, 3H), 3.80-3.95 (m, 2H), 5.31-5.40 (m, 1H), 6.64 (d, J = 8.4Hz, 1H), 6.99 (dd, J = 7.8, 2.4Hz, 1H), 7.02-7.12 (m, 3H), 7.21 (t, J = 2.4Hz, 1H), 7.64 (d, J = 10.8Hz, 1H), 7.94 (s, 1H) ppm
453: ¹H NMR (CDCl₃) δ 1.75 (s, 3H), 1.77 (s, 3H), 2.20 (s, 3H), 2.29 (s, 3H), 2.92 (s, 3H), 3.38 (s, 3H), 3.90 (brs.,2H), 4.58 (brs., 1H), 5.27-5.42 (m, 1H), 6.45 (d, J = 8.4Hz, 1H), 7.10 (s, 1H), 7.13 (s, 1H), 7.16-7.38 (m, 3H), 7.46 (dd, J = 8.4Hz, 2.1Hz, 1H), 8.12 (d, J = 1.5Hz, 1H) ppm
454: ¹H NMR (CDCl₃) δ 1.29 (d, J = 6.3Hz, 6H), 3.90-4.03 (m, 1H), 4.74-4.81 (m, 1H), 6.50 (d, J = 8.7Hz, 1H), 6.62-6.67 (m, 1H), 7.27-7.34 (m, 2H), 7.52 (d, J = 8.1Hz, 1H), 7.59-7.64 (m, 1H), 7.80 (s, 1H), 8.27 (d, J = 1.5Hz, 1H), 8.33 (brs, 1H) ppm
455: ¹H NMR (CDCl₃) δ 1.28 (d, J = 6.3Hz, 6H), 2.21 (s, 3H), 2.28 (s, 3H), 2.87 (s, 3H), 3.80-3.82 (m, 2H), 4.47 (d, J = 6.6Hz, 1H), 6.35-6.50 (m, 3H), 7.06 (d, J = 8.7Hz, 1H), 7.10 (s, 2H), 7.46 (d.d, J = 8.4, 2.4Hz, 1H), 8.10 (d, J = 1.8Hz, 1H) ppm
456: ¹H NMR (CDCl₃) δ 1.75 (s, 3H), 1.78 (s, 3H), 2.00 (s, 3H), 2.21 (s, 3H), 2.31 (s, 3H), 3.33 (s, 3H), 3.86-3.94 (m, 2H), 6.47 (d, J = 8.4Hz, 1H), 6.98-7.11 (m, 2H), 7.13 (s, 1H), 7.15 (s, 1H), 7.34 (t, J = 7.8Hz, 1H), 7.48 (dd, J = 8.4, 2.1Hz 1H), 8.13 (dd, J = 2.4, 0.6Hz, 1H) ppm
457: ¹H NMR (CDCl₃) δ 1.75(s, 3H), 1.77(s, 3H), 2.22(s, 3H), 2.29(s, 3H), 3.36(s, 3H), 3.78(s, 3H), 3.86-3.94 (m, 1H), 4.50-4.57 (m, 1H), 5.31-5.40 (m, 1H), 6.46 (d, J = 8.7Hz, 1H), 7.07-7.16 (m, 4H), 7.22-7.29 (m, 1H), 7.47 (dd, J = 8.7, 2.4Hz, 1H), 8.13 (d, J = 2.4Hz, 1H) ppm
458: ¹H NMR (CDCl₃) δ 0.26-0.32 (m, 2H), 0.55-0.62 (m, 2H), 1.05-1.23 (m, 1H), 2.20 (s, 3H), 2.29 (s, 3H), 2.92 (s, 3H), 3.16-3.22 (m, 2H), 3.38 (s, 3H), 6.49 (d, J = 9.0Hz, 1H), 7.10 (s, 1H), 7.13 (s, 1H), 7.16-7.36 (m, 3H), 7.48 (dd, J = 8.7, 2.7Hz, 1H), 8.10 (d, J = 1.8Hz, 1H) ppm
459: ¹H NMR (CDCl₃+CD₃OD) δ 1.74 (s, 3H), 1.77 (s, 3H), 2.18 (s, 3H), 2.27 (s, 3H), 2.77 (s, 3H), 2.87 (s, 3H), 3.80-3.95 (m, 2H), 5.31-5.40 (m, 1H), 6.64 (d, J = 8.4Hz, 1H), 6.99 (dd, J = 7.8, 2.4Hz, 1H), 7.02-7.12 (m, 3H), 7.21 (t, J = 2.4Hz, 1H), 7.64 (d, J = 10.8Hz, 1H), 7.94 (s, 1H) ppm
460: ¹H NMR (CDCl₃) δ 0.26-0.32 (m, 2H), 0.55-0.61 (m, 2H), 1.08-1.20 (m, 1H), 2.21 (s, 3H), 2.29 (s, 3H), 3.15-3.22 (m, 2H), 3.36 (s, 3H), 3.78 (s, 3H), 4.82 (brs, 1H), 6.48 (d, J = 9.0Hz, 1H), 7.05-7.15 (m, 1H), 7.12 (s, 1H), 7.13 (s, 1H), 7.21-7.29 (m, 1H), 7.48 (dd, J = 8.4Hz, 2.1Hz, 1H), 8.11 (d, J = 2. 1H 1H) ppm
461: ¹H NMR (CDCl₃) δ 1.02 (d, J = 6.9Hz, 6H), 1.94 (sept, J = 6.9Hz, 1H), 2.20 (s, 3H), 2.29 (s, 3H), 2.92 (s, 3H), 3.08-3.18 (m, 2H), 3.38 (s, 3H), 4.74 (brs, 1H), 6.46 (d, J= 8.4Hz, 1H), 7.10 (s, 1H), 7.13 (s, 1H), 7.17-7.36 (m, 3H), 7.47 (dd, J = 8.4, 2.4Hz, 1H), 8.10 (d, J = 2.4Hz, 1H) ppm
462: Mp 118-120°C; ¹H NMR (CDCl₃) δ 1.75 (s, 3H), 1.78 (s, 3H), 2.29 (s, 6H), 3.09 (s, 3H), 3.35 (s, 3H), 3.91 (t, J = 6.0Hz, 2H), 5.36 (m, 1H), 6.47 (d, J = 8.4Hz, 1H), 7.00 (d, J = 8.7Hz, 2H), 7.11 (d, J = 7.2Hz, 2H), 7.46 (dd, J = 2.1, 8.7Hz, 1H), 8.09 (d, J = 2.1Hz, 1H) ppm
463: ¹H-NMR (CDCl₃) δ 1.75 (s, 3H), 1.78 (s, 3H), 1.84 (t, J = 2.3Hz, 3H), 2.06 (s, 6H), 3.41 (s, 3H), 3.89-3.92 (m, 2H), 3.95-3.98 (m, 2H), 4.17 (bs, 1H), 4.53 (bs, 1H), 5.35-5.40 (m, 1H), 6.48 (d, J=8.7Hz, 1H), 6.83 (t, J = 8.4Hz, 1H), 7.07 (s, 1H), 7.24-7.37 (m, 2H), 7.91 (d, J = 1.5Hz, 1H), ppm
464: ¹H NMR (DMSO-d6) δ 0.37-0.43 (m, 2H), 0.67-0.76 (m, 2H), 1.16-1.28 (m, 1H), 1.30 (d, J = 6.5Hz, 6H), 1.93 (s, 6H), 1.97 (s, 6H), 2.59 (sept, J = 6.5Hz, 1H), 3.21-3.28 (m, 1H), 6.90 (d, J = 9.3Hz, 1H), 7.07 (d, J = 8.7Hz, 2H), 7.44 (brs, 1H), 7.52-7.60 (m, 2H), 7.64-7.78 (m, 2H), 8.70 (brs, 1H) ppm
465: ¹H-NMR (CDC1,) δ 0.28-0.31 (m, 2H), 0.57-0.62 (m, 2H), 1.09-1.19 (m, 1H), 1.75 (s, 3H). 1.78 (s, 3H), 2.06 (s, 6H), 3.04 (dd, J = 6.0Hz, 2H), 3.41 (s, 3H), 3.90 (dd, J = 5.1Hz, 2H), 4.10 (bs, 1H), 4.56 (bs, 1H), 5.35-5.40 (m, 1H), 6.49 (d, J = 8.4Hz, 1H), 6.72 (t, J = 8.3Hz, 1H), 7.07 (s, 1H), 7.27-7.36 (m, 2H), 7.91 (s, 1H), ppm
466: ¹H NMR (CDCl₃)δ 1.26 (d, J = 6.6Hz, 6H), 1.28 (d, J= 6.6Hz, 6H), 2.11 (dd, J = 3.0, 3.9Hz, 6H), 3.60-3.72 (m, 2H), 3.87-3.99 (m, 1H), 4.51-4.54 (m, 1H), 6.46 (d, J = 8.7Hz, 1H), 6.65 (d, J = 8.1Hz, 2H), 7.09 (d, J = 8.1Hz, 2H), 7.37-7.40 (m, 1H), 8.02 (d, J = 2.1Hz, 1H) ppm
467: ¹H NMR (CDCl₃)δ 1.28 (d, J = 6.3Hz, 6H), 1.49-1.82 (m, 6H), 2.00-2.10 (m,2H), 2.11 (dd, J = 3.0, 4.2Hz, 6H), 3.83 (bs, 2H), 3.87-3.99 (m, 1H), 4.51-4.54 (m, 1H), 6.46 (d, J = 7.8Hz, 1H), 6.67 (d, J = 8.7Hz, 2H), 7.09 (d, J = 8.7Hz, 2H), 7.36-7.40 (m, 1H), 8.02 (d, J = 2.4Hz, 1H) ppm
468: ¹H NMR (CDCl₃)δ 1.28 (d, J = 6.6Hz, 6H), 1.74 (s, 3H), 1.77 (s, 3H), 2.11 (dd, J = 3.0, 4.2Hz, 6H), 3.74 (d, J = 6.3Hz, 2H), 3.88-4.00 (m, 2H), 4.50-4.60 (m, 1H), 5.34-5.40 (m, 1H), 6.46 (d, J = 8.4Hz, 1H), 6.69 (d, J = 8.1Hz, 2H), 7.10 (d, J = 8.1Hz, 2H), 7.36-7.40 (m, 1H), 8.02 (s, 1H) ppm
469: ¹H NMR (CDCl₃)δ 1.02 (d, J = 6.6Hz, 6H), 1.28 (d, J = 6.6Hz, 6H), 1.86-2.00 (m, 1H),, 2.11 (dd, J = 3.0, 4.8Hz, 6H), 2.98 (d, J = 6.6Hz, 2H), 3.84 (bs, 1H), 3.88-3.99 (m, 1H), 4.51 (d, J = 7.2Hz, 1H), 6.46 (d, J = 8.1Hz, 1H), 6.68 (d, J=8.4Hz, 2H), 7.10 (d, J = 8.4Hz, 2H), 7.37-7.40 (m, 1H), 8.02 (s, 1H)ppm
470: ¹H NMR (CDCl₃)δ 1.25 (d, J = 6.6Hz, 6H), 1.29 (d, J = 6.6Hz, 6H), 3.65-3.73(m, 1H), 3.81-3.90 (m, 1H), 6.50 (d, J = 8.7Hz, 1H), 6.67 (d, J=8.7Hz, 2H), 7.47 (d, J = 8.7Hz, 2H), 7.52(d, J = 8.7Hz, 2H), 7.59 (d, J = 8.7Hz, 2H), 7.75 (dd, J = 2.1, 8.7Hz, 1H), 8.28 (d, J = 2.1Hz, 1H) ppm
471: ¹H NMR (CDCl₃) 1.28(d, J = 6.3Hz, 6H), 1.74 (s, 3H), 1.77 (s, 3H), 3.74 (d, J = 6.6Hz, 2H), 3.82-3.91 (m, 1H), 5.33-5.40 (m, 1H), 6.49 (d, J = 9.0Hz, 1H), 6.70 (d, J = 8.4Hz, 2H), 7.47 (d, J = 8.4Hz, 2H), 7.52 (d, J = 8.4Hz, 2H), 7.59 (d, J = 8.4Hz, 2H), 7.73 (dd, J = 2.1, 9.0Hz, 1H), 8.30 (d, J = 2.1Hz, 1H)ppm
472: Mp 177-178°C; ¹H NMR (CDCl₃)δ 1.75 (s, 3H), 1.78 (s, 3H), 2.01 (s, 6H), 2.18 (s, 6H), 3.47 (s, 3H), 3.75 (d, J = 6.6Hz, 2H), 5.37 (m, 1H), 6.73 (d, J = 8.4Hz, 2H), 6.95-6.89 (m, 2H), 7.42 (m 1H), 7.60 (dd, J = 2.4, 8.1Hz, 1H), 8.33 (d, J = 3.0Hz, 1H) ppm
473: ¹H NMR (CDCl₃) δ 1.30 (d, J = 6.3Hz, 6H), 1.98 (s, 6H), 2.00 (s, 6H), 3.85-3.97 (m, 1H), 3.92 (s, 3H), 4.08-4.20 (m, 1H), 4.45 (s, 2H), 4.78 (brs, 1H), 6,50 (d, J = 8.4Hz, 1H), 6.69 (d, J = 7.2Hz, 2H), 6.96 (d, J = 7.2Hz, 2H), 7.30 (dd, J = 8.4, 1.8Hz, 1H), 7.50 (d, J = 8.6Hz, 2H), 7.87 (d, 1.8Hz, 1H), 8.05 (d, J = 8.6Hz, 2H), ppm
474: ¹H NMR (CDCl₃)δ 1.74 (s, 3H), 1.77 (s, 3H), 2.01 (s, 6H), 2.18 (s, 3H), 3.82-3.95 (m, 2H), 4.30 (bs, 1H), 4.37 (s, 2H), 4.64 (bs, 1H), 5.30-5.40 (m, 1H), 6.47 (d, J = 8.7Hz, 1H), 6.68-6.82 (m, 3H), 6.90-6.98 (m, 3H), 7.21-7.26 (m, 2H), 7.44-7.48 (m, 1H), 8.05 is, 1H) ppm
475: ¹H NMR (CDCl₃) δ 1.31 (d, J = 6.3Hz, 6H), 1.97 (s, 6H), 2.01 (s, 6H), 3.82 (s, 3H), 3.86-3.96 (m, 1H), 3.99 (d, J = 4.2Hz, 2H), 4.27-4.35 (m, 1H), 4.80-5.00 (m, 1H), 6.51 (d, J = 8.1Hz, 1H), 6.69 (d, J = 8.7Hz, 2H), 6.94-7.02 (m, 2H), 7.31 (dd, J = 8.1, 2.1Hz, 1H), 7.86 (d, 2.1H 1H) ppm
476: ¹H-NMR (CDCl₃) δ 0.28-0.32 (m, 4H), 0.58-0.63 (m, 4H), 1.11-1.20 (m, 2H), 2.00 (s, 6H), 2.05 (s, 3H), 3.05 (dd, J = 6.6Hz, 2H), 3.19 (dd, J = 5.1Hz, 2H), 3.33 (s, 3H), 4.08 (bs, 1H), 5.00 (bs, 1H), 6.52 (d, J = 8.7Hz, 1H), 6.74 (t, J = 8.7Hz, 1H), 6.92-6.98 (m, 2H), 7.29 (d, J = 9.0Hz, 1H), 7.88 (s, 1H) ppm
477: ¹H NMR (DMSO-d6) δ 1.23 (d, J = 6.6Hz, 6H), 1.90 (s, 6H), 1.93 (s, 6H), 3.84 (s, 2H), 3.97-4.08 (m, 1H), 6.63 (d, J = 8.4Hz, 2H), 6.77-6.86 (m, 3H), 7.38-7.47 (m, 1H), 7.72 (s, 1H), 11.0-13.5 (brs, 1H) ppm
478: ¹H NMR (CDCl₃) δ 1.29 (d, J = 6.3Hz, 6H), 1.98 (s, 6H), 2.01 (s, 6H), 3.83-3.96 (m, 1H), 4.28 (s, 2H), 4.68 (brs, 1H), 6.51 (d, J = 8.4Hz, 1H), 6.71 (d, J = 8.7Hz, 2H), 6.85 (d, J = 8.6Hz, 2H), 6.96 (d, J = 8.7Hz, 2H), 7.28 (d, J = 8.6Hz, 2H), 7.32 (dd, J = 8.4, 2.3Hz, 1H), 7.88 (d, 2.3Hz, 1H) ppm
479: ¹H NMR (CDCl₃)δ 1.28 (d, J = 6.3Hz, 12H), 2.11 (dd, J = 2.1,2.7Hz, 6H), 3.60-3.78 (m, 1H), 3.82 (bs, 1H), 3.88-3.96 (m, 1H), 4.63 (bs, 1H), 6.47 (d, J = 8.4Hz, 1H), 6.73-6.79 (m, 1H), 6.90-6.94 (m, 2H), 7.38 (d, J = 8.4Hz, 1H), 8.00 (s, 1H)ppm
480: ¹H NMR (CDCl₃)δ 1.29 (d, J = 6.3Hz, 6H), 1.54-1.80 (m, 6H), 2.00-2.12 (m, 2H), 2.11 (dd, J =2.7,3.0Hz, 6H), 3.80.4.00 (m, 3H), 4.66 (bs, 1H), 6.47 (d, J = 8.7Hz, 1H), 6.75-6.81 (m, 1H), 6.91 (d, J = 10.2Hz, 2H), 7.39 (d, J = 8.7Hz, 1H), 8.00 (s, 1H) ppm
481: ¹H NMR (CDCl₃)δ 0.27-0.32 (m, 2H), 0.57-0.64 (m, 2H), 1.11-1.20 (m, 1H), 1.31 (d, J = 6.6Hz, 12H), 2.11 (dd, J = 2.7,7.5Hz, 6H), 3.00-3.08 (m, 2H), 3.88-4.00 (m, 1H), 4.12 (bs,1H), 4.63 (bs, 1H), 6.47 (d, J = 8.7Hz, 1H), 6.71-6.77 (m, 1H), 6.91-6.96 (m, 2H), 7.37-7.40 (m, 1H), 8.00 (s, 1H) ppm
482: ¹H NMR (CDCl₃)δ 1.29 (d, J = 6.3Hz, 6H), 2.11 (dd, J = 2.2, 8.7Hz, 6H), 3.89-3.96 (m, 1H)_{,} 4.37 (bs, 1H), 4.41 (s, 2H), 4.76 (bs, 1H), 6.29-6.31 (m, 1H), 6.35-6.50 (m, 1H), 6.48 (d, J = 8.4Hz, 1H), 6.82-6.97 (m, 3H), 7.38-7.41 (m, 2H), 8.00 (s, 1H) ppm
483: ¹H NMR (CDCl₃)δ 1.29 (d, J=6.3Hz, 6H), 1.75(s, 3H), 1.78 (s, 3H), 2.11 (dd, J = 3.0, 4.2Hz, 6H), 3.70-3.86 (m, 2H), 3.90-3.96 (m, 2H), 4.69 (bs, 1H), 5.36-5.41 (m, 1H), 6.48 (d, J = 9.0Hz, 1H), 6.74-6.79 (m, 1H), 6.91-6.95 (m, 2H), 7.19 (d, J = 9.0Hz, 1H), 8.01 (s, 1H), ppm
484: Mp foam; ¹H NMR (CDCl₃)δ1.35 (d, J = 6.9Hz, 6H), 1.73 (s, 3H), 1.77 (s, 3H), 3.76 (s, 3H), 3.76-3.80 (m, 2H), 4.55-4.64 (m, 1H), 5.36-5.38 (m, 1H), 6.80-6.84 (m, 1H), 7.06-7.10 (m, 2H), 7.32 (dd, J = 6.3, 9.9Hz, 1H), 7.40 (d, J = 8.1H 1H), 7.99-8.02 (m, 1H), 8.75 (s, 1H) ppm; IR (KBr) 3337, 2972, 1735, 1627, 1536, 1465, 1391, 1303, 1258, 1224, 1198, 1111, 1034 cm⁻¹
485: Mp oil; ¹H NMR (CDCl₃) δ 1.28 (d, J = 6.3Hz, 6H), 1.35 (d, J = 6.6Hz, 6H), 3.76 (s, 3H), 3.46-3.76 (m, 1H), 4.55-4.64 (m, 1H), 6.70-6.76 (m, 1H), 7.02-7.04 (m, 2H), 7.36 (dd, J = 6.0, 9.9Hz, 1H), 7.39 (d, J = 8.1Hz, 1H), 7.95-8.01 (m, 1H), 8.74 (s, 1H) ppm
486: ¹H NMR (CDCl₃)δ 1.03 (d, J = 6.6Hz, 6H), 1.29 (d, J = 6.6Hz, 6H), 1.86-2.05 (m, 1H), 2.11 (dd, J= 2.7, 4.5Hz, 6H), 3.14 (t, J = 6.6Hz, 2H), 3.62-3.78 (m, 1H), 3.82 (bs, 2H), 4.85 (bs, 1H), 6.49 (d, J = 8.7Hz, 1H), 6.73-6.79 (m, 1H), 6.90-6.94 (m, 2H), 7.38-7.41 (m, 1H), 8.00(s, 1H) ppm
487: ¹H NMR (CDCl₃)δ 1.02 (d, J = 6.6Hz, 6H), 1.75 (s, 3H), 1.78 (s, 3H), 1.89-1.99 (m, 1H), 2.11 (dd, J = 2.4, 5.1Hz, 6H), 3.14 (t, J = 6.6Hz, 2H), 3.70-3.80 (m, 2H), 3.94 (bs, 1H), 4.77 (bs, 1H), 5.32-5.40 (m, 1H), 6.48 (d, J = 8.4Hz, 1H), 6.74-6.79 (m, 1H), 6.91-6.95 (m, 2H), 7.39 (d, J = 8.4Hz, 1H), 8.01(s, 1H) ppm
488: ¹H NMR (CDCl₃)δ 0.27-0.32 (m, 2H), 0.57-0.63 (m, 2H), 1.02 (d, J = 6.6Hz, 6H), 2.11 (dd, J = 2.4, 7.2Hz, 6H), 3.00-3.08 (m, 2H), 3.14 (t, J = 6.6Hz, 2H), 4.13(bs, 1H), 4.82 (bs, 1H), 6.49 (d, J = 8.7Hz, 1H), 6.71-6.77 (m, 1H), 6.91-6.95 (m, 2H), 7.38-7.40 (m, 1H), 8.01(s, 1H), ppm
489: Mp 121-123°C; ¹H NMR (CDCl₃ δ 1.75 (s, 3H), 1.76 (s, 3H), 1.77 (s, 3H), 1.79 (s, 3H), 1.96 (s, 3H), 1.99 (s, 3H), 2.11 (s, 3H), 3.71 (m, 2H), 3.90 (m, 2H), 5.34-5.42 (m, 2H), 6.47 (d, J = 8.4Hz, 1H), 6.75-6.77 (m, 3H), 7.38 (dd, J = 2.1, 8.4Hz, 1H), 8.02 (d, J = 1.5Hz, 114) ppm
490: Mp 147-148°C; ¹H NMR (CDCl₃) δ 1.04 (d, 6.6Hz, 6H), 1.75 (s, 3H), 1.77 (s, 3H), 1.95-1.97 (m, 1H), 1.96 (d, J = 2.4Hz, 3H), 2.04 (s, 3H), 2.11 (s, 3H),3.02 (m, 2H), 3.91 (m, 2H), 5.39 (m, 1H), 6.50 (d, J = 8.7Hz, 1H), 6.74-6.79 (m, 3H), 7.38 (dd, J = 2.1, 8.7Hz, 1H), 8,02 (d, J = 1.5Hz, 1H) ppm
491: Mp 135-136°C; ¹H NMR (CDCl₃) δ 1.29 (d, J = 6.3Hz, 6H), 1.76 (s, 3H), 1.79 (s, 3H), 1.96 (d, J = 2.7Hz, 3H), 1.99 (s, 3H), 2.12 (s, 3H), 3.78 (m, 2H), 3.94 (m, 1H), 5.40 (m, 1H), 6.47 (d, J= 8.4Hz, 1H), 6.75-6.79 (m, 3H), 7.38 (dd, J= 2.1, 8.7Hz, 1H), 8.00 (d, J = 1.8Hz, 1H)ppm
492: Mp 136-137°C; ¹H NMR (CDCl₃) δ 1.04 (d, J = 6.9Hz, 6H), 1.29 (d, J = 6.3Hz, 6H), 1.94 (m, 1H), 1.97 (d, J = 2.7Hz, 3H), 1.99 (s, 3H), 2.11 (s, 3H), 3.02 (m, 1H), 3,91 (m, 1H), 6.48 (d, J = 8.4Hz, 1H), 6.74-6.79 (m,3H), 7.39 (dd, J = 1.2, 8.4Hz, 1H), 8.00 (d, J = 2.1Hz, 1H) ppm
493: Mp 188-189°C; ¹H NMR (CDCl₃) δ 1.28 (d, J = 1.5Hz, 6H), 1.30 (d, J = 1.2Hz, 6H), 1.96 (d, J = 2.4Hz, 3H), 1.99 (s, 3H), 2.11 (s, 3H), 3.70 (m, 1H), 3,93 (m, 1H), 6.47 (d, J = 8.4Hz, 1H), 6.75-6.80 (m, 3H), 7.39 (dd, J = 1.2, 8.4Hz, 1H), 8.00 (d, J = 2.1Hz, 1H) ppm
494: Mp 129-130°C; ¹H NMR (CDCl₃) δ 1.28 (d, J = 1.5Hz, 6H), 1.30 (s, 3H), 1.74 (s, 3H), 1.96 (d, J = 2.4Hz, 3H), 1.99 (s, 3H), 2.11 (s, 3H), 3.68 (m, 1H), 3,90 (m, 2H), 5,36 (m, 1H), 6.49 (d, J = 8.7Hz, 1H), 6.74-6.79 (m, 3H), 7.39 (dd, J = 1.2, 8.4Hz, 1H), 8.00 (d, J = 2.1Hz, 1H) ppm
495: ¹H NMR (CDCl₃)δ 1.28 (d, J = 6.0Hz, 6H), 1.75 (s, 3H), 1.78 (s, 3H), 2.11 (dd, J = 2.7, 4.5Hz, 6H), 3.69 (bs, 1H), 3.82 (bs, 1H), 3.90 (t, J=6.0Hz, 2H), 4.60-4.62 (m, 1H),5.34-5.38 (m, 1H), 6.48 (d, J = 8.4Hz, 1H), 6.73-6.79 (m, 1H), 6.90-6.94 (m, 2H), 7.37-7.40 (m, 1H), 8.03(s, 1H) ppm
496: ¹H NMR (CDCl₃)δ 1.41-1.81 (m, 6H), 1.75 (s, 3H), 1.77 (s, 3H), 2.02-2.12 (m, 2H), 2.11 (dd, J = 2.7, 4.5Hz, 6H), 3.85 (bs, 1H), 3.90 (t, J = 6.0Hz, 2H), 3.97 (bs, 1H), 4.63 (bs, 1H), 5.31-5.40 (m, 1H), 6.48 (d, J = 8.7Hz, 1H), 6.75-6.81 (m, 1H), 6.89-6.93 (m, 2H), 7.37-7.41 (m, 1H), 8.02(s, 1H) ppm
497: Mp 134-136°C; ¹H NMR (CDCl₃)δ 1.30 (d, J = 6.3Hz, 6H), 1.75 (s, 3H), 1.79 (s, 3H), 2.00 (s, 3H), 2.02 (s, 3H), 2.14 (s, 3H), 3.76-3.78 (m, 2H), 3.87-3.96 (m, 2H), 4.64-4.65 (m, 1H), 5.38-5.42 (m, 1H), 6.49 (d, J= 8.7Hz, 1H), 6.77-6.85 (m, 3H), 6.85-7.26 (m, 1H), 7.85 (d, J = 1.8Hz, 1H) ppm; IR (KBr) 3432, 3245, 2968, 1609, 1527, 1458, 1389, 1338, 1265, 1179 cm⁻¹
498: Mp 153-155°C; ¹H NMR (CDCl₃)δ 1.04 (d, J = 6.9Hz, 6H), 1.30 (d, J = 6.0Hz, 6H), 1.96-2.02 (m, 1H), 2.00 (s, 3H), 2.02 (s, 3H), 2.14 (s, 3H), 3.02 (t, J = 6.0Hz, 2H), 3.89-4.01 (m, 2H), 4.64 (brs, 1H), 6.49 (d, J = 8.7Hz, 1H), 6.71-6.87 (m, 3H), 7.22-7.26 (m, 1H), 7.85 (s, 1H) ppm; IR (KBr) 3432, 3236, 2962, 1627, 1607, 1526, 1459, 1387, 1340, 1273, 1176, 1107 cm⁻¹
499: ¹H NMR (CDCl₃) δ 1.29 (d, J = 6.3Hz, 12H), 1.99 (s, 6H), 2.01 (s, 6H), 3.13∼ 3.32 (m, 1H), 3.57∼ 3.76 (m, 1H), 3.85 ∼ 3.98 (m, 1H), 4.44 (d, J = 7.2Hz, 1H), 6.47 (d, J = 8.4Hz, 1H), 6.70∼ 6.87 (m, 3H), 7.27 (d,d, J = 8.4 & 2.4Hz, 1H), 7.90 (d, J = 1.5Hz, 1H) ppm
500: ¹H NMR (CDCl₃) δ 1.30 (d, J,= 6.3Hz, 61H), 1.76 (s, 3H), 1.79 (s, 3H), 1.99 (s, 6H), 2.00 (s, 6H), 3.77 (d, J = 6.9Hz) , 3.81 ∼ 3.99 (m, 1H), 4.60 ∼ 4.87 (broad, 1H), 5.33 ∼ 5.45 (m, 1H), 6.50 (d, J = 8.4Hz, 1H), 6.68 ∼ 6.86 (m, 3H), 7.29 (d.d, J = 6.6 & 2.1Hz, 1H), 7.87 (d, J = 1.8Hz, 1H) ppm
501: ¹H NMR (CDCl₃)δ 1.75 (s, 6H), 1.78 (s, 6H), 2.11 (dd, J = 2.4, 5.1Hz, 6H), 3.70-3.81 (m, 2H), 3.89-3.97 (m, 3H), 4.57 (bs, 1H), 5.33-5.40 (m, 1H), 6.48 (d, J = 8.4Hz, 1H), 6.73-6.79 (m, 1H), 6.91-6.94 (m, 2H), 7.36-7.40 (m, 1H), 8.03(s, 1H) ppm
502: ¹H NMR (CDCl₃)δ 0.27-0.32 (m, 2H), 0.58-0.64 (m, 2H), 1.14-1.19 (m, 1H), 1.75 (s, 3H), 1.78 (s, 3H), 2.11 (dd, J = 2.7, 6.6Hz, 6H), 3.05 (d, J = 5.1Hz, 2H), 3.91 (t, J = 6.0Hz, 2H), 4.13 (bs, 1H), 4.68 (bs, 1H), 5.33-5.39 (m, 1H), 6.49 (d, J = 8.4Hz, 1H), 6.71-6.77 (m, 1H), 6.92-6.96 (m, 2H), 7.38-7.41 (m, 1H), 8.03(s, 1H) ppm
503: ¹H NMR (CDCl₃)δ 1.75 (s, 3H), 1.78 (s, 3H), 2,10 (dd, J = 2.1, 7.8Hz, 6H), 3.91 (t, J = 6.0Hz, 2H), 4.39 (bs, 1H), 4.41 (s, 2H), 4.56-4.60 (m, 1H), 5.35-5.39 (m, 1H), 6.29-6.31 (m, 1H), 6.35-6.37 (m, 1H), 6.48 (d, J = 9.0Hz, 1H), 6.82-6.88 (m, 1H), 6.91-6.97 (m, 2H), 7.37-7.41 (m, 2H), 8.03(s, 1H) ppm
504: Mp 194-196°C; ¹H NMR (CDCl₃)δ 1.29 (d, J = 6.3Hz, 6H), 1.30 (d, J = 6.3Hz, 6H), 2.00 (s, 3H), 2.02 (s, 3H), 2.14 (s, 3H), 3.66-3.77 (m, 2H), 3.87-3.98 (m, 1H), 4.61 (d, J = 7.2Hz, 1H), 6.48 (d, J = 8.4Hz, 1H), 6.73-6.85 (m, 3H), 7.22-7.26 (m, 1H), 7.85 (d, J = 2.1Hz, 1H) ppm; IR (KBr) 3422, 3272, 2967, 1629, 1606, 1525, 1458, 1387, 1336, 1265, 1176 cm⁻¹
505: Mp 140-142°C; ¹H NMR (CDCl₃)δ 1.29 (d, J = 6.3Hz, 6H), 1.75 (s, 3H), 1.78 (s, 3H), 2.00 (s, 3H), 2.02 (s, 3H), 2.13 (s, 3H), 3.66-3.77 (m, 2H), 3.91 (t, J = 5.4Hz, 2H), 4.61 (brs, 1H), 5.35-5.40 (m, 1H), 6.49 (d, J = 8.4Hz, 1H), 6.76-6.86 (m, 3H), 7.22-7.26 (m, 1H), 7.88 (d, J = 2.1Hz, 1H) ppm; IR (KBr) 3430, 3237, 2965, 1609, 1523, 1459, 1385, 1337, 1265, 1199, 1176 cm⁻¹
506: Mp 136-138°C; ¹H NMR (CDCl₃)δ 1.75 (s, 6H), 1.78 (s, 6H), 2.00 (s, 3H), 2.02 (s, 3H), 2.14 (s, 3H), 3.77 (t, J = 5.1H 2H), 3.89-3.93 (m, 3H), 4.61 (brs, 1H), 5.35-5.42 (m, 2H), 6.50 (d, J = 8.4Hz, 1H), 6.73-6.87 (m, 3H), 7.22-7.26 (m, 1H), 7.88 (d, J = 2.1Hz, 1H) ppm; IR (KBr) 3433, 3231, 1712, 1606, 1528, 1455, 1388, 1316, 1264, 1109 cm⁻¹
507: Mp 139-141°C; ¹H NMR (CDCl₃) δ 1.04 (d, J = 6.6Hz, 6H), 1.76 (s, 3H), 1.78 (s, 3H), 1.84-2.00 (m, 1H), 2.00 (s, 3H), 2.02 (s, 3H), 2.14 (s, 3H), 3.02 (t, J = 6.0Hz, 2H), 3.91 (t, J = 5.7Hz, 2H), 4.00 (brs, 1H), 4.64-4.66 (m, 1H), 5.35-5.40 (m, 1H), 6.50 (d, J = 8.7Hz, 1H), 6.71-6.87 (m, 3H), 7.22-7.26 (m, 1H), 7.87 (s, 1H) ppm; IR (KBr) 3432, 3235; 2958, 1609, 1530, 1470, 1389, 1263, 1144, 1106 cm⁻¹
508: ¹H NMR (CDCl₃) δ 1.29 (d, J = 6.3Hz, 6H), 1.98 (s, 6H), 2.01 (s, 6H), 3.33-3.42 (m, 1H), 3.44 (s, 3H), 3.68 (t, J = 5.1Hz, 1H), 3.84-3.89 (m, 1H), 4.27 (brs, 1H), 4.44 (d, J = 7.2Hz, 1H), 6.47 (d, J = 8.4Hz, 1H), 6.71-6.85 (m, 3H), 7.23-7.29 (m, 1H), 7.90 (d, J = 2.1Hz, 111) ppm
509: ¹H NMR (CDCl₃) δ 1.04 (d, J = 6.9Hz, 6H), 1.29 (d, J = 6.0Hz, 6H), 1.89-2.20 (m, 1H), 1.97 (s, 6H), 1.99 (s, 6H), 3.11 (d, J = 7.2Hz, 2H), 3.40-4.00 (broad, 1H), 3.69 (sept, J = 6.0Hz, 1H), 5.60-5.93 (broad, 1H), 6.52 (d, J = 8.4Hz, 1H), 6.68-6.87 (m, 3H), 7.32 (dd, J = 8.7, 2.4Hz, 1H), 7.81 (d, J = 1.5Hz, 1H) ppm
510: ¹H NMR (CDCl₃) δ 1.04 (d, J = 6.9Hz, 12H), 1.86 ∼ 2.03 (m, 2H), 1.98 (s, 6H), 2.00 (s, 6H), 3.02 (d, J = 6.6Hz, 2H), 3.13 (d, J = 6.3Hz, 2H), 5.00 (brs. 1H), 6.50 (d, J = 8.7Hz, 1H), 6.70-6.83 (m, 3H), 7.29 (dd, J = 8.4, 2.4Hz, 1H), 7.87 (d, J = 2.1Hz, 1H) ppm
511: ¹H NMR (CDCl₃) δ 1.04 (d, J = 6.9Hz, 6H), 1.76 (s, 3H), 1.79 (s, 3H), 1.89-2.05 (m, 1H), 1.99 (s, 6H), 2.00 (s, 6H), 3.14 (t, J = 6.0Hz, 2H), 3.77 (d, J = 6.9Hz, 2H), 4.72 (brs, 1H), 5.41 (brs, 1H), 6.49 (d, J = 8.7Hz), 6.72-6.84 (m, 3H), 7.21-7.32 (m, 1H), 7.89 (d, J = 2.1Hz, 1H)ppm
512: ¹H NMR (CDCl₃)δ 1.28 (d, J = 6.0Hz, 12H), 1.29 (d, J = 6.0Hz, 3H), 2.06 (s, 6H), 2.20 (s, 3H), 3.20-3.27 (m, 1H), 3.68 (bs, 1H), 3.88-3.96 (m, 1H), 4.47-4.51 (m, 1H), 6.47 (d, J = 8.1Hz 1H), 6.71-6.76 (m, 1H), 6.96-7.00 (m, 2H), 7.00 (s, 1H), 7.25-7.28 (m, 1H), 7.89 (s, 1H), ppm
513: ¹H NMR (CDCl₃) 1.29 (d, J = 6.3Hz, 6H), 1.45-1.83 (m, 6H), 2.00-2.17 (m, 2H), 2.06 (s, 6H), 2.20 (s, 3H), 3.84 (bs, 1H), 3.87-3.99 (m, 2H), 4.41 (bs, 1H), 6.48 (d, J = 8.4Hz, 1H), 6.73-6.78 (m, 1H), 6.95-6.99 (m, 2H), 7.00 (s, 1H), 7.25-7.29 (m, 1H), 7.88 (s, 1H), ppm
514: Mp 153-156°C; ¹H NMR (CDCl₃) δ 1.29 (d, J = 6.0Hz, 12H), 1.75 (s, 3H), 1.78 (s, 3H), 1.98 (s, 3H), 2.05 (s, 3H), 2.11 (s, 3H), 3.69 (m, 1H), 3.91 (m, 2H), 5.38 (m, 1H), 6.48 (d, J = 8.4Hz, 1H), 6.76 (m, 3H), 7.31 (m, 1H), 7.94 (m, 1H) ppm
515: Mp 197-199°C; ¹H NMR (CDCl₃) δ 1.29 (d, J = 6.3Hz, 12H), 1.98 (s, 3H), 2.05 (s; 3H), 2.11 (s, 3H), 3.70 (m, 1H), 3.93 (m, 1H), 6.46 (d, J = 8.7Hz, 1H), 6.76 (m, 3H), 7.29 (m, 1H), 7.92 (m, 1H) ppm
516: Mp 159-162°C; ¹H NMR (CDCl₃) δ 1.03 (d, J = 6.6Hz, 6H), 1.29 (d, J = 6.0Hz, 6H), 1.95 (m, 1H), 1.98 (s, 3H), 2.05 (s, 3H), 2.11 (s, 3H), 3.14 (m, 2H), 3.71 (m, 1H), 6.84 (d, J = 8.4Hz, 1H), 6.76 (m, 3H), 7.31 (m, 1H), 7.92 (m, 1H) ppm
517: ¹H NMR (CDCl₃)δ 1.03 (d, J = 6.6Hz, 6H), 1.29 (d, J = 6.3Hz, 6H), 1.91-2.00 (m, 1H), 2.06 (s, 6H), 2.20 (s, 3H), 2.99-3.03 (m, 2H), 3.86-3.96 (m, 1H), 3.98 (bs, 1H), 4.49 (bs, 1H), 6.47 (d, J = 8.7Hz, 1H), 6.69-6.75 (m, 1H), 6.96-6.99 (m, 2H), 7.00 (s, 1H), 7.25-7.28 (m, 1H), 7.90(s, 1H), ppm
518: ¹H NMR (CDCl₃)δ 1.29 (d, J = 6.3Hz, 6H), 1.75 (s, 3H), 1.78 (s, 3H), 2.06 (s, 6H), 2.20 (s, 3H), 3.75-3.80 (m, 2H), 3.87 (bs, 1H), 3.87-3.96 (m, 1H), 4.54 (bs, 1H), 5.36-5.41 (m, 1H), 6.48 (d, J = 8.7Hz, 1H), 6.71-6.77 (m, 1H), 6.96-6.99 (m, 2H), 7.00 (s, 1H), 7.25-7.29 (m, 1H), 7.90(s, 1H), ppm
519: ¹H NMR (CDCl₃) δ 1.04 (d, J = 6.6Hz, 6H), 1.31 (d, J = 6.9Hz, 6H), 1.80-2.03 (m, 1H), 1.95 (s, 6H), 2.00 (s, 6H), 2.62 (sept. J = 6.9Hz, 1H), 3.14 (t, J = 6.6Hz, 2H), 4.61-4.72 (m, 1H), 6.49 (d, J = 8.4Hz, 1H), 6.87-6.98 (m, 2H), 7.19-7.30 (m, 1H), 7.45 (s, 1H), 7.88 (d, J= 2.1Hz, 1H), 8.41 (t, J = 5.1Hz, 1H)) ppm;
520: ¹H NMR (CDCl₃)δ 1.04 (d, J = 6.6Hz, 6H), 1.95 (s, 6H), 2.00 (s, 6H), 3.06.3.19 (m, 2H), 3.83 (s, 3H), 4.67-4.76 (m, 1H), 6.49 (d, J = 8.4Hz, 1H), 6.86-7.04 (m, 3H), 7.20∼ 7.30 (m, 1H), 7.89 (d, J = 1.5Hz, 1H), 8.12 (brs, 1H) ppm
521: ¹H NMR (CDCl₃) δ 1.30 (d, J = 6.0Hz, 6H), 1.95 (s, 6H), 2.01 (s, 6H), 3.83 (s, 3H), 3.83-4.00 (m, 1H), 4.51 (d, J = 7.2Hz, 1H), 6.83 ∼ 6.99 (m, 3H), 7.17-7.33 (m, 1H), 7.88 (d, J = 1.5Hz, 1H), 8.12 (brs,1H) ppm
522: ¹H NMR (DMSO-d6) δ 1.28 (d, J = 6.3Hz, 6H), 1.91 (s, 6H), 1.95 (s, 6H), 3.68 (s, 3H), 3.97 (s, 2H), 4.00-4.10 (m, 1H), 6.66 (d, J = 9.0Hz, 2H), 6.76-6.85 (m, 2H), 7.18 (d, J = 9.9Hz, 1H), 7.67-7.78 (m, 2H), 8.98 (brs, 1H), 13.7 (brs, 1H) ppm
523: Mp 145-147°C; ¹H NMR (CDCl₃) δ 1.04 (d, J = 6.9Hz, 6H), 1.29 (d, J = 6.0Hz, 6H), 1.83-2.00 (m, 1H), 2.00 (s, 3H), 2.02 (s, 3H), 2.14 (s, 3H), 3.12-3.16 (m, 2H), 3.66-3.72 (m, 1H), 3.77 (brs, 1H), 4.68 (t, J = 5.7Hz, 1H), 6.49 (d, J = 8.7Hz, 1H), 6.73-6.86 (m, 3H), 7.21-7.26 (m, 1H), 7.87 (d, J = 2.1Hz, 1H) ppm; IR (KBr) 3435, 3249, 2960, 1609, 1522, 1458, 1326, 1263, 1198, 1173, 1159 cm⁻¹
524: Mp 144-146°C; ¹H NMR (CDCl₃) δ 1.04 (d, J = 6.6Hz, 6H), 1.85-2.00 (m, 2H), 2.00 is, 3H), 2.02 (s, 3H), 2.14 (s, 3H), 3.02 (t, J = 6.0Hz, 2H), 3.12.3.17 (m, 2H), 4.00 (brs, 1H), 4.68 (t, J = 5.4Hz, 1H), 6.49 (d, J = 8.4Hz, 1H), 6.72-6.87 (m, 3H), 7.21-7.27 (m, 1H), 7.87 (s, 1H) ppm; IR (KBr) 3427, 3250, 2956, 1628, 1607, 1530, 1472, 1326, 1263, 1156, 1107 cm⁻¹
525: Mp 105-107°C; ¹H NMR (CDCl₃) δ 1.04 (d, J = 6.3Hz, 6H), 1.75 (s, 3H), 1.79 (s, 3H), 2.00 (s, 3H), 2.02 (s, 3H), 2.14 (s, 3H), 3.12-3.17 (m, 2H), 3.76-3.78 (m, 2H), 3.87 (brs, 1H), 4.70 (brs, 1H), 5.38-5.42 (m, 1H), 6.49 (d, J = 8.4Hz, 1H), 6.74-6.87 (m, 3H), 7.22-7.26 (m, 1H), 7.87 (d, J = 2.1Hz, 1H) ppm; IR (KBr) 3433, 3254, 2955, 1627, 1607, 1524, 1459, 1386, 1338, 1326, 1263, 1156cm⁻¹

### Experiment 1 Suppressive effect on the IgE production against ovalbumin (OVA)

### 1) Animals

BALB/c mice (female, 8-10 weeks old) and Wistar rats (female, 8-10 weeks old) which were bought from Japan SLC, Inc. (Shizuoka) were used.

### 2) Immunizing method

BALB/c mice were immunized by an intraperitoneal administration of 0.2 ml suspension of 2 µg of ovalbumin (OVA) and 2 mg of aluminium hydroxide gel in physiological saline. After 10 days, blood was collected from hearts, then sera were separated and stocked at -40 °C till the measurement of IgE antibody titer.

### 3) Compounds

After the compound of the present invention was dissolved or suspended in N, N-dimethylacetoamide, the mixture was diluted 20 times with miglyol 812 neutral oil. The obtained solution was orally administered to mice at 0.1 ml per mouse (dose 40 mg/kg). The administration was continued for 10 days from the immunizing day to the day before the blood collection.

### 4) Measurement of anti-OVA IgE antibody titer (PCA titer)

The obtained mouse serum was 2-fold diluted with physiological saline, then each 50 µl of the solution was intradermally injected at dorsal skin of Wistar rats which previously hair cut. After 24 hours, a passive cutaneous anaphylaxis reaction (PCA) was induced by an intravenous injection of 0.5 ml of physiological saline containing 1 mg of OVA and 5 mg of Evans' blue dye. The rats were sacrified 30 minutes later and the highest dilution giving bluing with a diameter of 5 mm or more was recorded as the PCA titer. For example, when a serum is positive for the PCA reaction till 2⁷ times dilution, the anti-OVA IgE antibody titer of the mouse is defined as 7. The results are shown in Table 45.

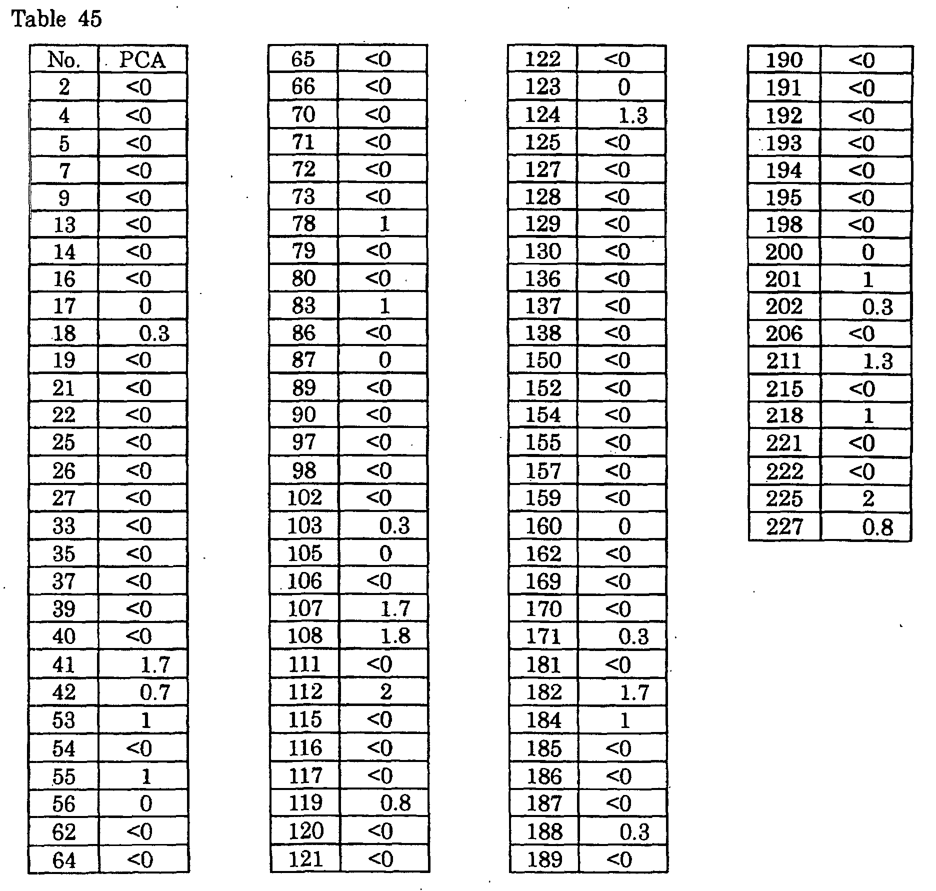

As shown in the above, the compound of the present invention has a suppressive effect on the IgE production.

| Formulation Example 1 Tablet | |
|---|---|
| The compound of the present invention (Ia-1) | 15 mg |
| Starch | 15 mg |
| Lactose | 15 mg |
| Crystalline cellulose | 19 mg |
| Polyvinyl alcohol | 3 mg |
| Distilled water | 30 ml |
| Calcium stearate | 3 mg |

After all of the above ingredients except for calcium stearate were uniformly mixed, the mixture was crushed and granulated, and dried to obtain a suitable size of granules. After calcium stearate was added to the granules, tablets were formed by compression molding.

### Industrial Applicability

As explained in the above experiments, the compounds of the present invention have potent IgE production suppressive activities. The compounds of the present invention are useful as an immunosuppressant and/or an anti-allergic agent.

## Claims

1. A compound of the formula (I): 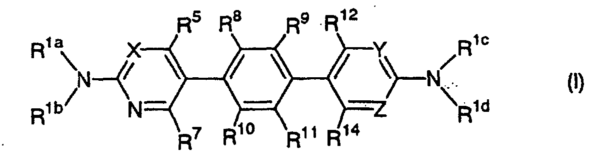 wherein X is N or CR⁴, Y is N or CR¹⁸, Z is N or CR¹⁵,
R^{1a}, R^{1b}, R^{1c} and R^{1d} are each independently hydrogen;
lower alkyl optionally substituted with at least one group selected from the group of (i)halogen, (ii)hydroxy, (iii)lower alkylthio, (iv)lower alkoxy, (v)amino, (vi)lower alkylamino, (vii)cycloalkyl, (viii)heterocyclyl, (ix)optionally substituted aryl wherein the substituents are lower alkoxycarbonyl or hydroxy, (x)lower alkoxycarbonyl, (xi)cyano, (xii)oxo and (xiii)carboxy;
lower alkenyl;
lower alkynyl;
acyl optionally substituted with halogen or lower alkoxy;
lower alkylsulfonyl optionally substituted with halogen;
lower alkoxycarbonyl;
cycloalkyl;
arylsulfonyl;
carbamoyl optionally substituted with alkyl;
sulfamoyl optionally substituted with lower alkyl,
R⁴, R⁶, R⁷, R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, optionally substituted lower alkenyl, optionally substituted lower alkenyloxy, optionally substituted cycloalkyoxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino, optionally substituted carbamoyl, optionally substituted sulfamoyl, guanidino, nitro, cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy,
R⁴ and R^{1a} taken together may form optionally substituted C2 or C3 alkenylene,
R¹³ and R^{1c} taken together may form optionally substituted C2 or C3 alkenylene,
R⁸ and R⁹ taken together may form optionally substituted C2 or C3 alkenylenediamino or optionally substituted C2 or C3 alkylenediamino,
R¹⁰ and R¹¹ taken together may form optionally substituted C2 or C3 alkenylenediamino or optionally substituted C2 or C3 alkylenediamino,
a prodrug, a pharmaceutically acceptable salt or solvate thereof.

2. The compound as claimed in claim 1 wherein R^{1a} is lower alkylsulfonyl or 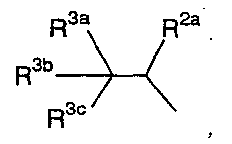 R^{1c} is lower alkylsulfonyl or 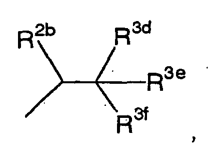 R^{1b} and R^{1d} are each independently hydrogen, lower alkyl or acyl,
R^{2a} and R^{2b} are each independently hydrogen or lower alkyl,
R^{3a} and R^{3d} are each independently hydrogen, lower alkyl or lower alkenyl,
R^{3b}, R^{3e}, R^{3c} and R^{3f} are each independently hydrogen or lower alkyl,
R^{3a} and R^{2a} or R^{3b} taken together may form optionally substituted lower alkylene,
R^{3d} and R^{2b} or R^{3e} taken together may form optionally substituted lower alkylene, a prodrug, a pharmaceutically acceptable salt or solvate thereof.

3. The compound as claimed in claim 1 wherein X is N or CR⁴, Y is CR¹³ and Z is CR¹⁵, a prodrug, a pharmaceutically acceptable salt or solvate thereof.

4. The compound as claimed in claim 1 wherein X is N or CR⁴, Y is CR¹³ and Z is N, a prodrug, a pharmaceutically acceptable salt or solvate thereof.

5. The compound as claimed in any one of claims 1 to 4 wherein at least one of R^{1b} and R^{1d} is hydrogen, a prodrug, a pharmaceutically acceptable salt or solvate thereof.

6. The compound as claimed in any one of claims 1 to 4 wherein both of R^{1b} and R^{1d} are hydrogen, a prodrug, a pharmaceutically acceptable salt or solvate thereof.

7. The compound as claimed in any one of claims 2 to 6 wherein R^{2a} and R^{2b} are each independently hydrogen or C1 to C3 alkyl, a prodrug, a pharmaceutically acceptable salt or solvate thereof.

8. The compound as claimed in any one of claims 2 to 7 wherein both of R^{3a} and R^{3d} are hydrogen or C1 to C3 alkyl, a prodrug, a pharmaceutically acceptable salt or solvate thereof.

9. The compound as claimed in any one of claims 2 to 6 wherein R^{3a} and R^{2a} or R^{3b} taken together may form unsubstituted alkylene and/or R^{3d} and R^{2b} or R^{3e} taken together may form unsubstituted alkylene, a prodrug, a pharmaceutically acceptable salt or solvate thereof.

10. The compound as claimed in any one of claims 2 to 9 wherein R^{3b} and R^{3e} are each independently hydrogen or C1 to C3 alkyl, a prodrug, a pharmaceutically acceptable salt or solvate thereof.

11. The compound as claimed in any one of claims 2 to 10 wherein R^{3c} and R^{3f} are each independently hydrogen or C1 to C3 alkyl, a prodrug, a pharmaceutically acceptable salt or solvate thereof.

12. The compound as claimed in any one of claims 2 to 4 wherein both of R^{1b} and R^{1d} are hydrogen,
R^{2a}, R^{2b}, R^{3a}, R^{3d}, R^{3b}, R^{3e}, R^{3c} and R^{3f} are each independently hydrogen or all of R^{2a}, R^{2b}, R^{3a}, R^{3d}, R^{3b}, R^{3e}, R^{3c} and R^{3f} are C1 to C3 alkyl,
R^{3a} and R^{2a} or R^{3b} taken together may form unsubstituted alkylene and/or R^{3a} and R^{2b} or R^{3e} taken together may form alkylene,
a prodrug, a pharmaceutically acceptable salt or solvate thereof.

13. The compound as claimed in any one of claims 2 to 4 wherein both of R^{1b} and R^{1d} are hydrogen, R^{2a}, R^{2b}, R^{3a}, R^{3d}, R^{3b}, R^{3e}, R^{3c} and R^{3f} are each independently hydrogen or methyl, a prodrug, a pharmaceutically acceptable salt or solvate thereof.

14. The compound as claimed in any one of claims 1 to 4 wherein both of R^{1a} and R^{1c} are isopropyl and both of R^{1b} and R^{1d} are hydrogen, a prodrug, a pharmaceutically acceptable salt or solvate thereof.

15. The compound as claimed in any one of claims 1 to 14 wherein X is N or CH and R⁶ and R7 are each independently hydrogen or lower alkyl, a prodrug, a pharmaceutically acceptable salt or solvate thereof.

16. The compound as claimed in any one of claims 1 to 15 wherein R¹² is hydrogen, Y is CH, Z is N or CR¹⁵, R¹⁴ and R¹⁵ are each independently hydrogen, halogen, lower alkyl or lower alkoxy, a prodrug, a pharmaceutically acceptable salt or solvate thereof.

17. The compound as claimed in any one of claims 1 to 16 wherein 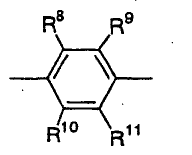 is 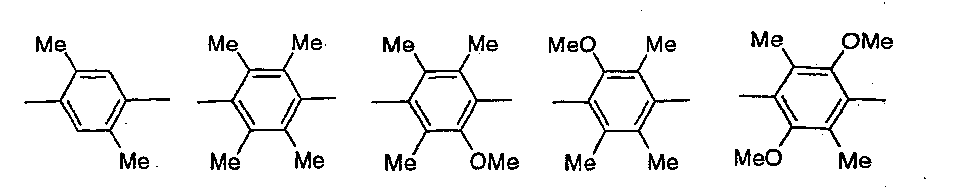 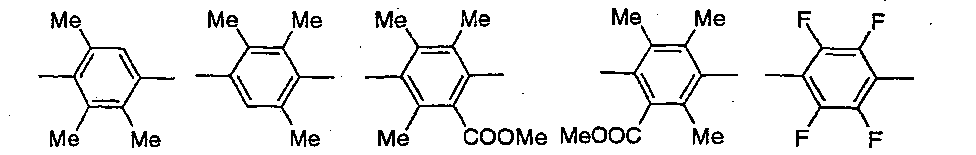 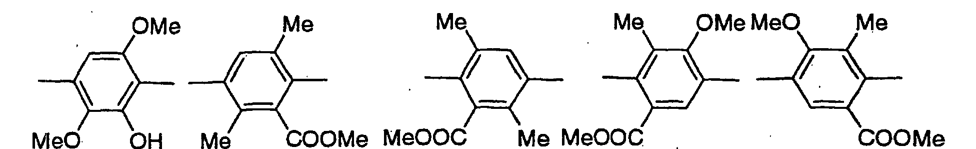 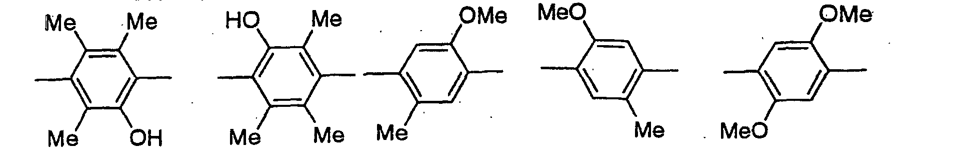 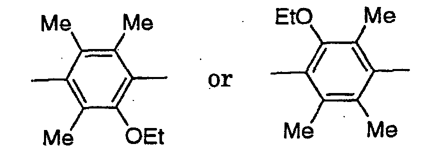 a prodrug, a pharmaceutically acceptable salt or solvate thereof.

18. The compound as claimed in any one of claims 1 to 16 wherein 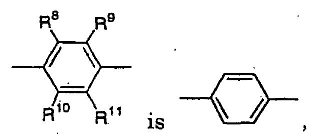 a prodrug, a pharmaceutically acceptable salt or solvate thereof.

19. The compound as claimed in any one of claims 1 to 16 wherein 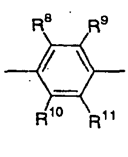 is 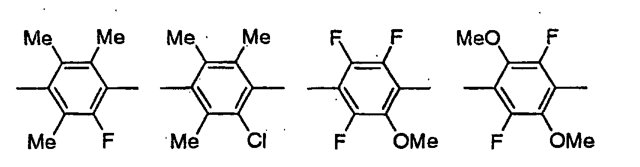 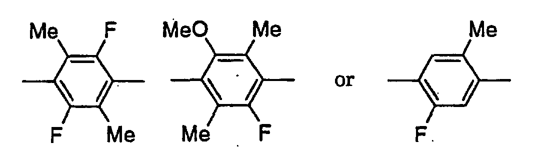 a prodrug, a pharmaceutically acceptable salt or solvate thereof.

20. The compound as claimed in claim 1 wherein X and Y are CH, Z is N, R^{1a}, R^{1b}, R^{1c} and R^{1d} are each independently hydrogen; lower alkyl optionally substituted with cycloalkyl; lower alkenyl; acyl; lower alkylsulfonyl; or cycloalkyl,
R⁵, R⁷, R¹² and R¹⁴ are each independently hydrogen or lower alkyl,
R⁸, R⁹, R¹⁰ and R¹¹ are each independently hydrogen, halogen, lower alkyl or lower alkoxy,
provided tat at least two of R⁸, R⁹, R¹⁰ and R¹¹ are each independently halogen, lower alkyl or lower alkoxy,
a prodrug, a pharmaceutically acceptable salt or solvate thereof.

21. The compound as claimed in claim 1 wherein X is N, Y and Z is CH, R^{1a}, R^{1b}, R^{1c} and R^{1d} are each independently hydrogen, lower alkyl or cycloalkyl,
R⁵, R⁷, R¹² and R¹⁴ are hydrogen,
R⁸, R⁹, R¹⁰ and R¹¹ are each independently lower alkyl or lower alkoxy,
a prodrug, a pharmaceutically acceptable salt or solvate thereof.

22. The compound as claimed in claim 1 wherein X is CH, Y is CR¹³, Z is CR¹⁵, R^{1a}, R^{1b}, R^{1c} and R^{1d} are each independently
hydrogen;
lower alkyl optionally substituted with at least one group selected from the group of cycloalkyl and heterocyclyl;
lower alkenyl;
acyl;
lower alkylsulfonyl;
or cycloalkyl,
R⁵, R⁷, R¹² and R¹⁴ are hydrogen,- R¹³ and R¹⁵ are each independently hydrogen or halogen,
R⁸, R⁹, R¹⁰ and R¹¹ are each independently hydrogen, halogen, lower alkyl, lower alkoxy or lower alkoxycarbonyl,
provided that at least three of R⁸, R⁹, R¹⁰ and R¹¹ are each independently halogen, lower alkyl, lower alkoxy or lower alkoxycarbonyl,
R¹³ and R^{1c} taken together may form optionally substituted C2 or C3 alkenylene, a prodrug, a pharmaceutically acceptable salt or solvate thereof.

23. A pharmaceutical composition comprising a compound, a prodrug, a pharmaceutically acceptable salt or solvate thereof of any one of claims 1 to 22.

24. An IgE production suppressive composition comprising a compound, a prodrug, a pharmaceutically acceptable salt or solvate thereof of any one of claims 1 to 22.

25. An anti-allergic composition comprising a compound, a prodrug, a pharmaceutically acceptable salt or solvate thereof of any one of claims 1 to 22.

26. An immunosuppressive composition comprising a compound, a prodrug, a pharmaceutically acceptable salt or solvate thereof of any one of claims 1 to 22.

27. A method for suppressing IgE production comprising administering a compound, a prodrug, a pharmaceutically acceptable salt or solvate thereof of any one of claims 1 to 22.

28. A method for treating and/or preventing an allergic disease comprising administering a compound, a prodrug, a pharmaceutically acceptable salt or solvate thereof of any one of claims 1 to 22.

29. A method for suppressing an immune reaction comprising administering a compound, a prodrug, a pharmaceutically acceptable salt or solvate thereof of any one of claims 1 to 22.

30. Use of a compound, a prodrug, a pharmaceutically acceptable salt or solvate thereof of any one of claims 1 to 22 for preparing a medicine for suppressing IgE production.

31. Use of a compound, a prodrug, a pharmaceutically acceptable salt or solvate thereof of any one of claims 1 to 22 for preparing an anti-allergic agent.

32. Use of a compound, a prodrug, a pharmaceutically acceptable salt or solvate thereof of any one of claims 1 to 22 for preparing an immunosuppressive agent.
